# EUROPEAN PATENT APPLICATION

(11) **EP 4 793 271 A1**
(43) Date of publication of application: **19.08.2026**
(21) Application number: 24877274.1
(22) Date of filing: 11.10.2024
(51) Int. Cl.: C07D 213/55

(54) **NEUROPROTECTIVE AGENT**

(30) Priority: 13.10.2023 JP 2023177619; 28.06.2024 JP 2024105766
(71) Applicant: National Center of Neurology and Psychiatry, Kodaira-shi Tokyo 187-8551 (JP); Tokyo University of Pharmacy & Life Sciences, Hachioji-shi, Tokyo 192-0392 (JP)
(72) Inventor: ARAKI, Toshiyuki, Kodaira-shi, Tokyo 187-8551 (JP); TOKUNAGA, Shinji, Kodaira-shi, Tokyo 187-8551 (JP); FUNAKOSHI, Masabumi, Kodaira-shi, Tokyo 187-8551 (JP); ITO, Hisanaka, Hachioji-shi, Tokyo 192-0392 (JP); KAWAMOTO, Yuichiro, Hachioji-shi, Tokyo 192-0392 (JP); ABE, Hideki, Hachioji-shi, Tokyo 192-0392 (JP); YAMADA, Atsushi, Odawara-shi, Kanagawa 250-0852 (JP); ISHIDA, Natsuki, Tokyo 104-8002 (JP); NOMOTO, Masahiro, Tokyo 104-8002 (JP); NANGOU, Fumi, Odawara-shi, Kanagawa 250-0852 (JP)
(74) Representative: Hoffmann Eitle
(86) International application number: PCT/JP2024/036416
(87) International publication number: WO 2025/079680

(57) **Abstract**

Provided are: a novel compound that is useful for the treatment of diseases to be prevented, ameliorated, and/or treated by suppressing axon degeneration; and a pharmaceutical composition containing the same. The present invention relates to: a compound represented by general formula (I) [in the formula, R^{a}, R^{b}, X¹, R¹, L¹, L², and R² are as disclosed in the specification and as set forth in the claims] or a pharmaceutically acceptable salt thereof; and a pharmaceutical composition containing the same.

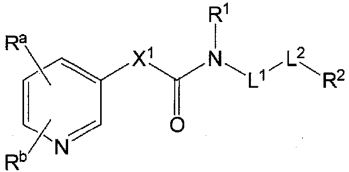

## Description

### Technical Field

The present invention relates to a nicotinamide derivative and a pharmaceutical composition containing the same. The present invention particularly relates to a nicotinamide derivative for treatment of a disease subject to prevention, alleviation, and/or therapy through the suppression of axonal degeneration, and a pharmaceutical composition containing the same.

### Background Art

Many neurodegenerative diseases are known to cause axonal degeneration. A report states that deficiency in sterile alpha and TIR motif-containing 1 (SARM1) drastically attenuates axonal degeneration in some models, suggesting that SARM1 controls axonal degeneration via its enzymatic activity. Hence, a compound that can inhibit SARM1 has received attention (see, for example, Patent Literature 1).

Recent research also suggests that an active state of SARM1 is controlled by the abundance ratios of a substrate and an intermediate of nicotinamide adenine dinucleotide (NAD) and the NAD biosynthesis pathway (see, for example, Non Patent Literature 1). Since nicotinamide phosphoribosyl transferase (NAMPT) is reported to be a rate-determining enzyme of the NAD biosynthesis pathway, a compound is expected to be found which can inhibit SARM1 involved in NAD degradation, without inhibiting NAMPT.

### Citation List

### Patent Literature

Patent Literature 1: JP 2023-501969 A

### Non Patent Literature

Non Patent Literature 1: Li et al., eLife 2021; 10: e67381

### Summary of Invention

### Technical Problem

The present invention provides a novel compound useful for treatment of a disease subject to prevention, alleviation, and/or therapy through the suppression of axonal degeneration, and a pharmaceutical composition containing the same.

### Solution to Problem

The present inventors have conducted diligent studies on compounds having SARM1 inhibitory activity and consequently completed the present invention by finding that a series of derivatives having a nicotinamide structure or salts thereof have SARM1 inhibitory activity and/or have weak NAMPT inhibitory activity and are useful for treatment of a disease subject to prevention, alleviation, and/or therapy through the suppression of axonal degeneration, particularly, a neurodegenerative disease, via these properties.

The present invention is as follows.
[1] A compound represented by the following general formula (I): 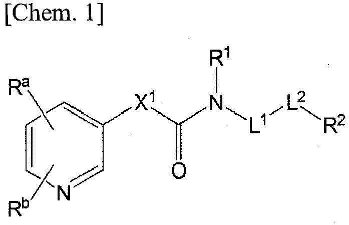
   or a pharmaceutically acceptable salt thereof, wherein
   R^{a} and R^{b} are each independently a hydrogen atom, halogen, nitro, cyano, amino, hydroxy, C₁₋₆ alkyl, halo-C₁₋₆ alkyl, amino-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, or C₂₋₆ alkynyloxy, or R^{a} and R^{b}, which present on adjacent carbon atoms, optionally form a substituted or unsubstituted carbocyclic ring or heterocyclic ring condensed with a pyridine ring, together with the carbon atoms to which they are bonded,
   X¹ is a direct bond, -CR¹¹=CR¹¹-, or -(CONR¹¹)ₘCH₂-,
   R¹ is a hydrogen atom or C₁₋₆ alkyl,
   L¹ is C₁₋₁₀ alkylene, arylene, C₁₋₁₀ alkylene-arylene, arylene-C₁₋₁₀ alkylene, C₁₋₁₀ alkylene-arylene-C₁₋₁₀ alkylene, arylene-C₁₋₁₀ alkylene-arylene, heterocyclylene, C₁₋₁₀ alkylene-heterocyclylene, heterocyclylene-C₁₋₁₀ alkylene, or C₁₋₁₀ alkylene-heterocyclylene-C₁₋₁₀ alkylene, wherein the alkylene, arylene, or heterocyclylene moiety is optionally substituted with halogen, -COOR¹², -CONHR¹², or oxo,
   L² is a direct bond, -O-, -NR¹³-, -NR¹³SO₂-, or -(OCH₂CH₂)ₙO-, and
   R² is a hydrogen atom, azide, substituted or unsubstituted C₁₋₆ alkyl, substituted or unsubstituted C₂₋₆ alkenyl, substituted or unsubstituted C₂₋₆ alkynyl, -COR¹⁴, substituted or unsubstituted aryl, or substituted or unsubstituted heterocyclyl,
   wherein
   m is 0 or 1,
   n is 1 to 6,
   each R¹¹ is independently a hydrogen atom or C₁₋₆ alkyl,
   R¹² is a hydrogen atom or C₁₋₆ alkyl,
   R¹³ is a hydrogen atom, C₁₋₆ alkyl, or -COR¹⁴, and
   R¹⁴ is substituted or unsubstituted C₁₋₆ alkyl, substituted or unsubstituted C₂₋₆ alkenyl, substituted or unsubstituted C₂₋₆ alkynyl, substituted or unsubstituted C₃₋₁₀ cycloalkyl, substituted or unsubstituted C₃₋₁₀ cycloalkenyl, substituted or unsubstituted aryl, or substituted or unsubstituted heterocyclyl,
   provided that the compound is not a compound in which L¹ is arylene, C₁₋₃ alkylene-arylene, arylene-C₁₋₃ alkylene, or C₁₋₃ alkylene-arylene-C₁₋₃ alkylene, and L² is a direct bond, a compound in which L¹ is C₁₋₃ alkylene-arylene, and L² is -O-, or a compound in which L¹ is C₁₋₃ alkylene, L² is -NH-, and R² is -CO-5-membered heterocyclyl.
[2] The compound or the pharmaceutically acceptable salt thereof according to [1], wherein the compound is a compound represented by the following general formula (Ia): 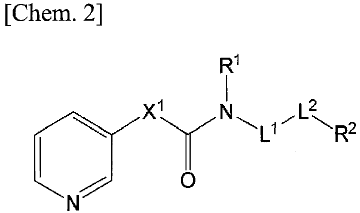 wherein
   X¹ is a direct bond, -CR¹¹=CR¹¹-, or -CONR¹¹CH₂-,
   R¹ is a hydrogen atom or C₁₋₆ alkyl,
   L¹ is C₁₋₁₀ alkylene, arylene, C₁₋₃ alkylene-arylene, arylene-C₁₋₃ alkylene, C₁₋₃ alkylene-arylene-C₁₋₃ alkylene, heterocyclylene, C₁₋₃ alkylene-heterocyclylene, heterocyclylene-C₁₋₃ alkylene, or C₁₋₃ alkylene-heterocyclylene-C₁₋₃ alkylene, wherein the alkylene moiety is optionally substituted with -COOR¹² or -CONHR¹²,
   L² is a direct bond, -O-, -NR¹³-, -NR¹³SO₂-, or -(OCH₂CH₂)ₙO-, and
   R² is a hydrogen atom, azide, substituted or unsubstituted C₁₋₆ alkyl, substituted or unsubstituted C₂₋₆ alkenyl, substituted or unsubstituted C₂₋₆ alkynyl, -COR¹⁴, substituted or unsubstituted aryl, or substituted or unsubstituted heterocyclyl,
   wherein
   n is 1 to 6,
   each R¹¹ is independently a hydrogen atom or C₁₋₆ alkyl,
   R¹² is a hydrogen atom or C₁₋₆ alkyl,
   R¹³ is a hydrogen atom, C₁₋₆ alkyl, or -COR¹⁴,
   R¹⁴ is substituted or unsubstituted C₁₋₆ alkyl, substituted or unsubstituted C₂₋₆ alkenyl, substituted or unsubstituted aryl, or substituted or unsubstituted heterocyclyl,
   provided that the compound is not a compound in which L¹ is arylene, C₁₋₃ alkylene-arylene, arylene-C₁₋₃ alkylene, or C₁₋₃ alkylene-arylene-C₁₋₃ alkylene, and L² is a direct bond, a compound in which L¹ is C₁₋₃ alkylene-arylene, and L² is -O-, or a compound in which L¹ is C₁₋₃ alkylene, L² is -NH-, and R² is -CO-5-membered heterocyclyl.
[3] The compound or the pharmaceutically acceptable salt thereof according to [1] or [2], wherein L² is -O- or -NH-.
[4] The compound or the pharmaceutically acceptable salt thereof according to any of [1] to [3], wherein the compound is represented by the following general formula (II): 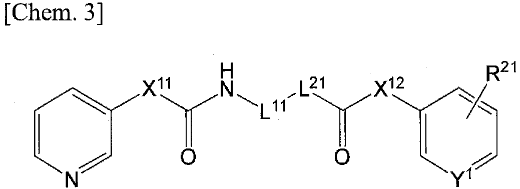 wherein
   X¹¹ is a direct bond, -CR¹¹=CR¹¹-, or -CONR¹¹CH₂-,
   X¹² is a direct bond, C₁₋₃ alkylene, -CR¹¹=CR¹¹-, or -CH₂NR¹¹CO-,
   L¹¹ is C₁₋₁₀ alkylene, arylene, C₁₋₃ alkylene-arylene, arylene-C₁₋₃ alkylene, C₁₋₃ alkylene-arylene-C₁₋₃ alkylene, heterocyclylene, C₁₋₃ alkylene-heterocyclylene, heterocyclylene-C₁₋₃ alkylene, or C₁₋₃ alkylene-heterocyclylene-C₁₋₃ alkylene,
   L²¹ is -O- or -NH-,
   Y¹ is N or CH,
   each R¹¹ is independently a hydrogen atom or C₁₋₆ alkyl,
   R¹² is a hydrogen atom or C₁₋₆ alkyl, and
   each R²¹ is independently selected from the group consisting of halogen, nitro, cyano, amino, hydroxy, C₁₋₆ alkyl, halo-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, and C₂₋₆ alkynyloxy.
[5] The compound or the pharmaceutically acceptable salt thereof according to [1] or [2], wherein L² is a direct bond or -(OCH₂CH₂)ₙO-.
[6] The compound or the pharmaceutically acceptable salt thereof according to [1], [2], or [5], wherein R² is a hydrogen atom, substituted or unsubstituted C₁₋₆ alkyl, substituted or unsubstituted C₂₋₆ alkenyl, substituted or unsubstituted C₂₋₆ alkynyl, substituted or unsubstituted aryl, or substituted or unsubstituted heterocyclyl.
[7] The compound or the pharmaceutically acceptable salt thereof according to [1], wherein the compound is the following compound: 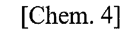 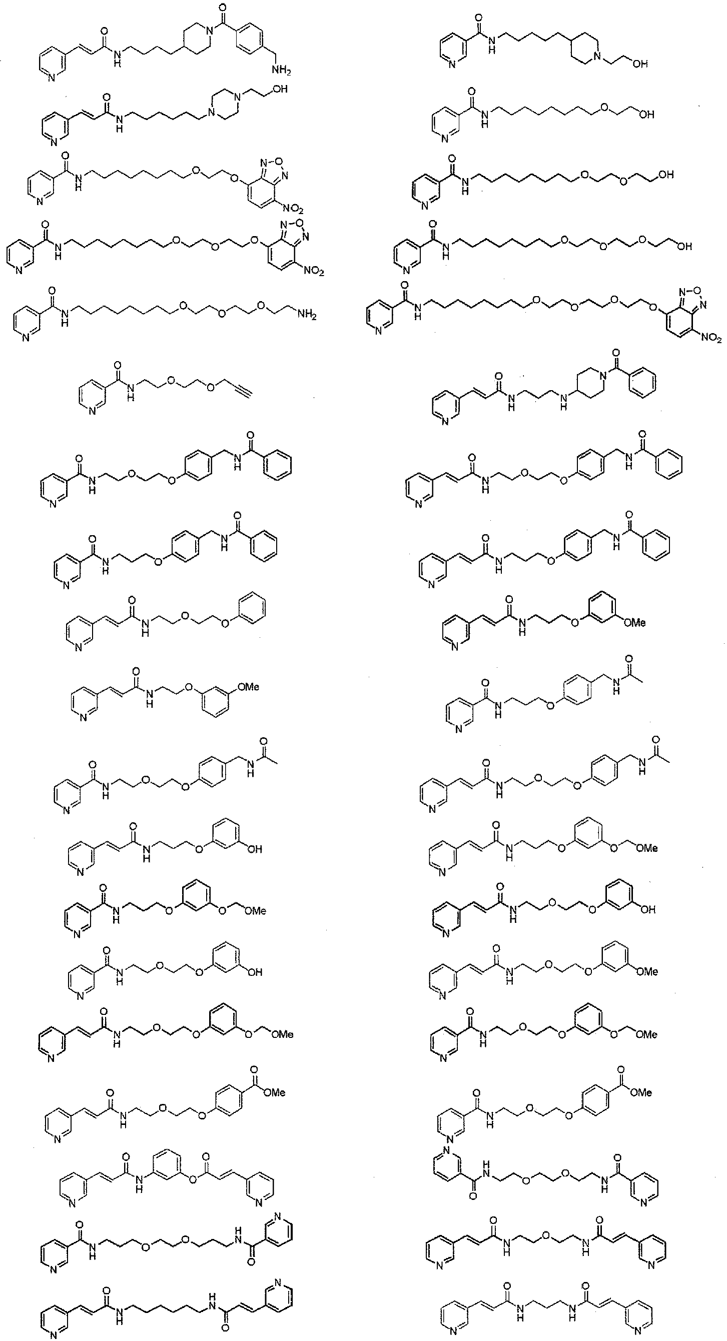 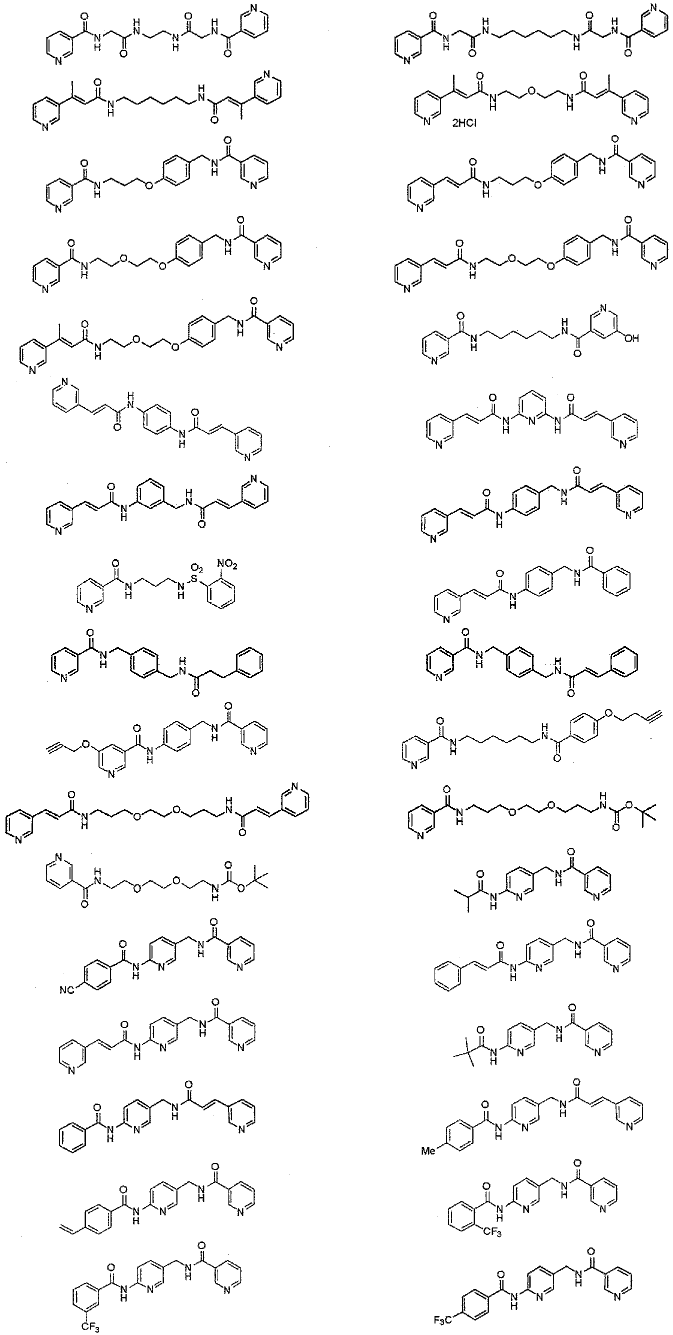 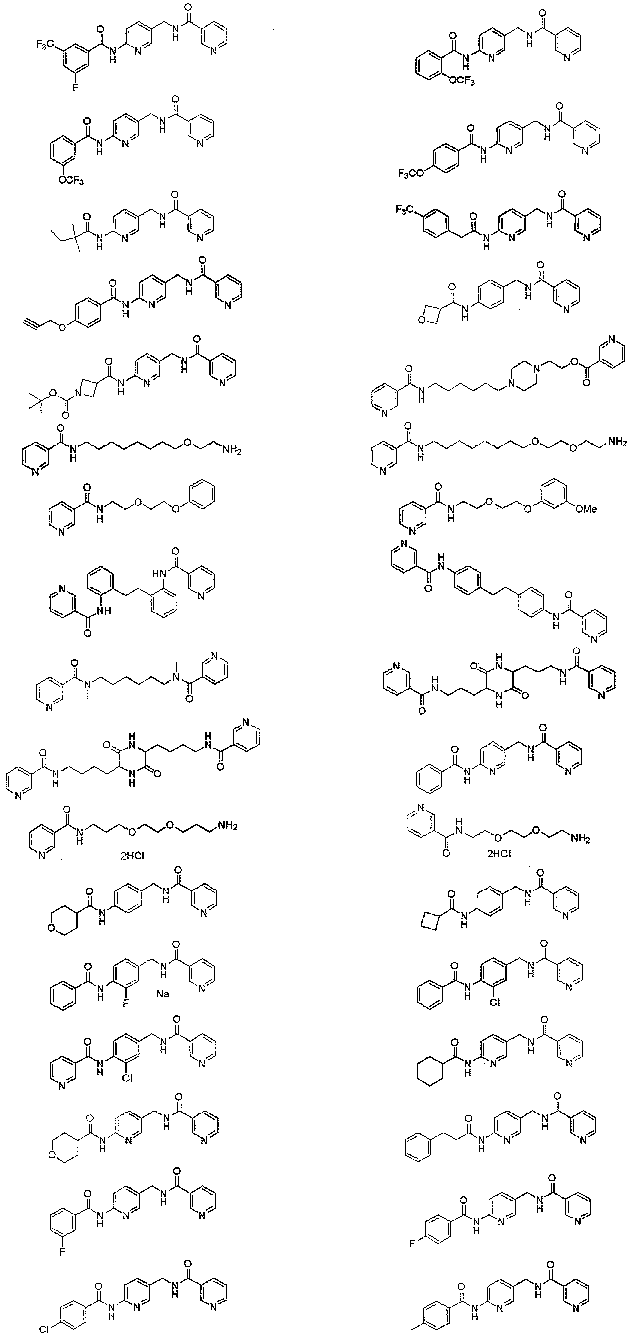 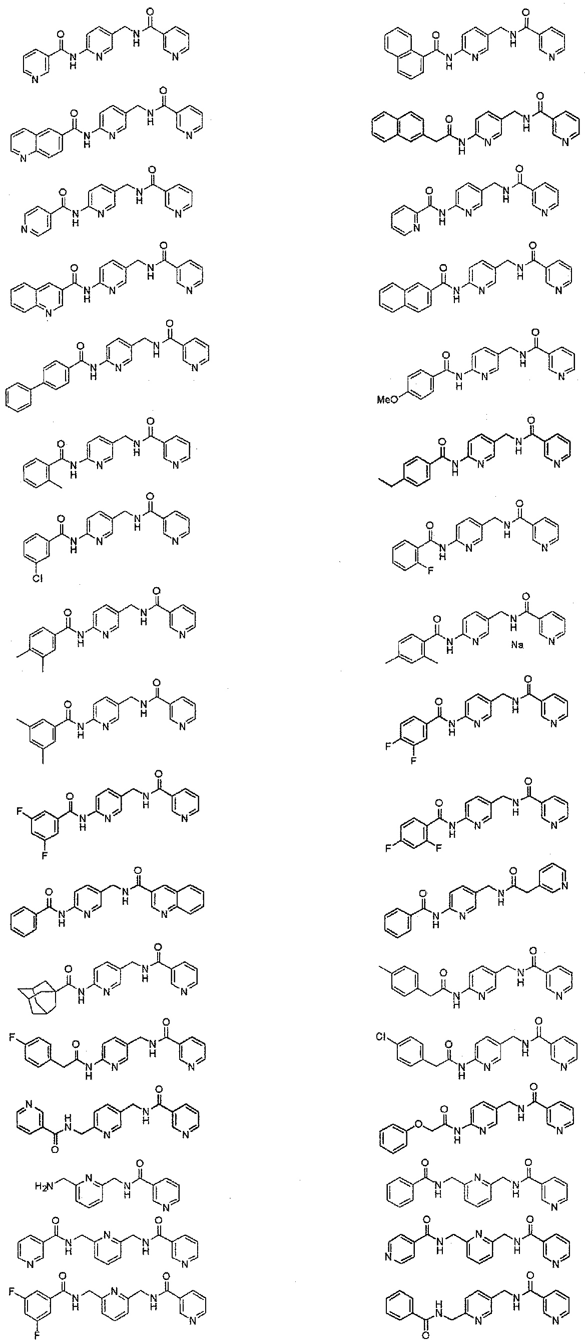 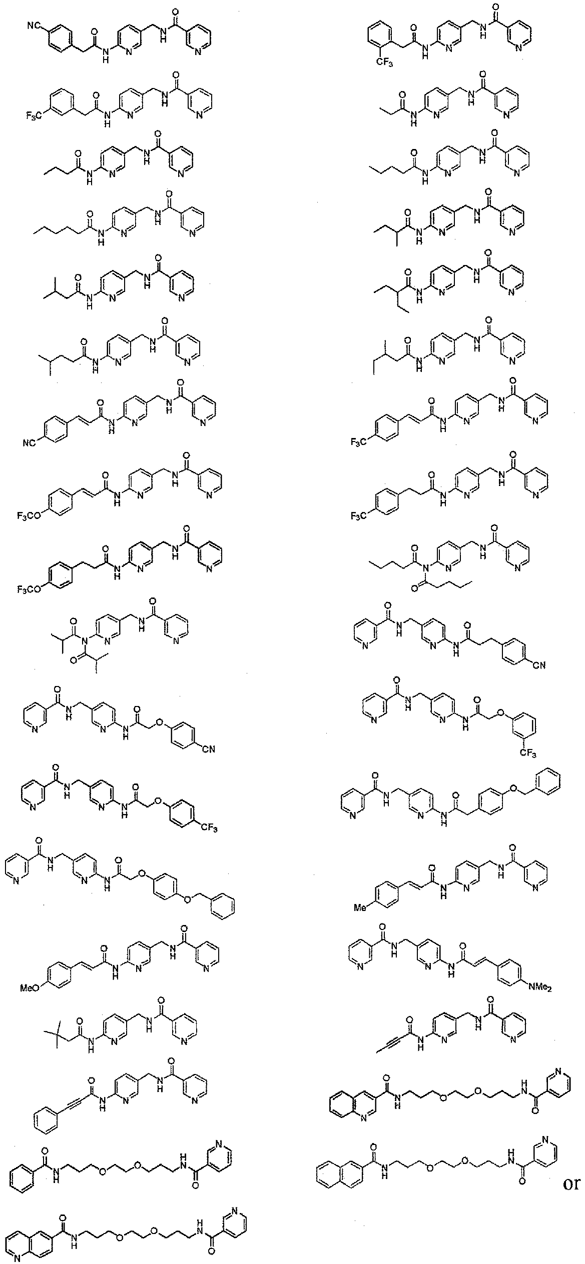
[8] A pharmaceutical composition comprising the compound or a pharmaceutically acceptable salt thereof according to any of [1] to [7] and at least one pharmaceutically acceptable additive.
[9] The pharmaceutical composition according to [8] for treatment of a disease subject to prevention, alleviation, and/or therapy through the suppression of axonal degeneration.
[10] The pharmaceutical composition according to [9], wherein the disease is a neurodegenerative disease.
[11] Use of the compound or a pharmaceutically acceptable salt thereof according to any of [1] to [7] for producing a medicament for treatment of a disease subject to prevention, alleviation, and/or therapy through the suppression of axonal degeneration.
[12] A method for treatment of a disease subject to prevention, alleviation, and/or therapy through the suppression of axonal degeneration, comprising administering a therapeutically effective amount of the compound or a pharmaceutically acceptable salt thereof according to any of [1] to [7] to a patient in need thereof.
[13] The pharmaceutical composition according to [8] for treatment of a disease subject to prevention, alleviation, and/or therapy through the inhibition of SARM1.
[14] The pharmaceutical composition according to [13], wherein the disease is a neurodegenerative disease.
[15] Use of the compound or a pharmaceutically acceptable salt thereof according to any of [1] to [7] for producing a medicament for treatment of a disease subject to prevention, alleviation, and/or therapy through the inhibition of SARM1.
[16] A method for treatment of a disease subject to prevention, alleviation, and/or therapy through the inhibition of SARM1, comprising administering a therapeutically effective amount of the compound or a pharmaceutically acceptable salt thereof according to any of [1] to [7] to a patient in need thereof.

### Advantageous Effects of Invention

The compound represented by the general formula (I) of the present invention or a pharmaceutically acceptable salt thereof has SARM1 inhibitory activity and/or has weak NAMPT inhibitory activity, and has excellent pharmacokinetic characteristics, and is therefore useful for treatment of a disease subject to prevention, alleviation, and/or therapy through the suppression of axonal degeneration, typically, a neurodegenerative disease, via these properties.

### Description of Embodiments

The meanings of terms used in the present specification and claims will be described below. The terms used in the present specification and claims are as defined below unless otherwise specified.

In the present specification, a numeric range represented by using the term "to" refers to a range including numeric values described before and after the term "to" as the smallest value and the largest value, respectively.

In the present invention, a compound of the general formula (I) encompasses its isotopic isomers. Specifically, in the compound of the general formula (I), all or some of individual atoms may be replaced with their corresponding isotopes. The isotope refers to an atom having a mass number different from a naturally found mass number. Examples of such an isotope include a hydrogen atom (2H and 3H), a carbon atom (13C and 14C), a nitrogen atom (15N), and an oxygen atom (17O and 18O). Particularly, a deuterium atom (2H) is also referred to as "D". In such a case, in the compound of the general formula (I), all hydrogen atoms at specific locations represented by D are replaced with deuterium atoms, and the resulting molecular weight is different from a molecular weight calculated from a naturally found mass number.

The term "halogen atom" or "halo" means a fluorine atom, a chlorine atom, a bromine atom, or an iodine atom singly or in combination with an additional group.

The term "C₁₋₆ alkyl" means a monovalent group of linear or branched saturated aliphatic hydrocarbon having 1 to 6 carbon atoms, singly or in combination with an additional group. Examples of the C₁₋₆ alkyl include methyl, ethyl, propyl, butyl, pentyl, and hexyl (including various isomers). A preferred form of the C₁₋₆ alkyl is C_{1 to 4} alkyl, for example, methyl, ethyl, propyl, isopropyl, butyl, isobutyl, sec-butyl, or tert-butyl, and a more preferred form thereof is C₁₋₃ alkyl.

The term "C₃₋₁₀ cycloalkyl" means a monovalent group of monocyclic or polycyclic saturated alicyclic hydrocarbon having 3 to 10 carbon atoms, singly or in combination with an additional group. Examples of the C₃₋₁₀ cycloalkyl include monocyclic groups such as cyclopropyl, cyclobutyl, cyclopentyl, cyclohexyl, cycloheptyl, cyclooctyl, cyclononyl, and cyclodecyl, and polycyclic groups such as bicyclo[1.1.0]butyl, bicyclo[3.2.1]octyl, and adamantyl. A preferred form of the C₃₋₁₀ cycloalkyl is C_{3 to 6} cycloalkyl, for example, cyclopropyl, cyclobutyl, or cyclohexyl, and a more preferred form thereof is C_{4 to 6} cycloalkyl.

The term "C₂₋₆ alkenyl" means a monovalent group of linear or branched unsaturated aliphatic hydrocarbon having 2 to 6 carbon atoms and containing at least one double bond, singly or in combination with an additional group. Examples of the C₂₋₆ alkenyl include vinyl, allyl, isopropenyl, 1-butenyl, 2-butenyl, 1,3-butadienyl, and 2-pentenyl (including various isomers). A preferred form of the C₂₋₆ alkenyl is C_{2 to 4} alkenyl, for example, vinyl, allyl, isopropenyl, 1-butenyl, 2-butenyl, or 1,3-butadienyl, and a more preferred form thereof is C_{2 to 3} alkenyl.

The term "C₃₋₁₀ cycloalkenyl" means a monovalent group of monocyclic or polycyclic unsaturated alicyclic hydrocarbon having 3 to 10 carbon atoms, singly or in combination with an additional group. Examples of the C₃₋₁₀ cycloalkenyl include monocyclic groups such as cyclopropenyl, cyclobutenyl, cyclopentenyl, cyclohexenyl, cycloheptenyl, cyclooctenyl, cyclononenyl, and cyclodecenyl, and polycyclic groups such as bicyclo[2.2.2]octenyl. A preferred form of the C₃₋₁₀ cycloalkenyl is C_{3 to 6} cycloalkenyl, for example, cyclopropenyl, cyclobutenyl, or cyclohexenyl, and a more preferred form thereof is C_{4 to 6} cycloalkenyl.

The term "C₂₋₆ alkynyl" means a monovalent group of linear or branched unsaturated aliphatic hydrocarbon having 2 to 6 carbon atoms and containing at least one triple bond, singly or in combination with an additional group. Examples of the C₂₋₆ alkynyl include ethynyl, 1-propynyl, 2-propynyl, 1-butynyl, 2-butynyl, 3-butynyl, 4-pentynyl, and 5-hexynyl (including various isomers). A preferred form of the C₂₋₆ alkynyl is C_{2 to 4} alkynyl, for example, ethynyl, 1-propynyl, 2-propynyl, 1-butynyl, 2-butynyl, or 3-butynyl, and a more preferred form thereof is C_{2 to 3} alkynyl.

The term "mono- or di-C₁₋₆ alkylamino" means a group -NR'R" (wherein R' is the C₁₋₆ alkyl, R" is a hydrogen atom or the C₁₋₆ alkyl) singly or in combination with an additional group. Examples of the mono- or di-C₁₋₆ alkylamino include mono-C₁₋₆ alkylamino such as methylamino, ethylamino, and propylamino, and di-C₁₋₆ alkylamino such as dimethylamino, diethylamino, and dipropylamino.

The term "C₁₋₆ alkoxy" means a group -O-R' (wherein R' is the C₁₋₆ alkyl) singly or in combination with an additional group. Examples of the C₁₋₆ alkoxy include methoxy, ethoxy, propoxy, butyloxy, pentyloxy, and hexyloxy (including various isomers). A preferred form of the C₁₋₆ alkoxy is C_{1 to 4} alkoxy, for example, methoxy, ethoxy, propoxy, isopropoxy, butyloxy, isobutyloxy, sec-butyloxy, or tert-butyloxy, and a more preferred form thereof is C₁₋₃ alkoxy.

The term "C₂₋₆ alkenyloxy" means a group -O-R' (wherein R' is the C₂₋₆ alkenyl) singly or in combination with an additional group. Examples of the C₂₋₆ alkenyloxy include vinyloxy, allyloxy, isopropenyloxy, 1-butenyloxy, 2-butenyloxy, 1,3-butadienyloxy, and 2-pentenyloxy (including various isomers). A preferred form of the C₂₋₆ alkenyloxy is C_{2 to 4} alkenyloxy, for example, vinyloxy, allyloxy, isopropenyloxy, 1-butenyloxy, 2-butenyloxy, or 1,3-butadienyloxy, and a more preferred form thereof is C_{2 to 3} alkenyloxy.

The term "C₂₋₆ alkynyloxy" means a group -O-R' (wherein R' is the C₂₋₆ alkynyl) singly or in combination with an additional group. Examples of the C₂₋₆ alkynyloxy include ethynyloxy, 1-propynyloxy, 2-propynyloxy, 1-butynyloxy, 2-butynyloxy, 3-butynyloxy, 4-pentynyloxy, and 5-hexynyloxy (including various isomers). A preferred form of the C₂₋₆ alkynyloxy is C_{2 to 4} alkynyloxy, for example, ethynyloxy, 1-propynyloxy, 2-propynyloxy, 1-butynyloxy, 2-butynyloxy, or 3-butynyloxy, and a more preferred form thereof is C_{2 to 3} alkynyloxy.

The term "halo-C₁₋₆ alkyl" means a group in which at least one hydrogen atom at an arbitrary position of the "C₁₋₆ alkyl" is replaced with the "halogen atom". Examples of the halo-C₁₋₆ alkyl include fluoromethyl, difluoromethyl, trifluoromethyl, 2,2,2-trifluoroethyl, dichloromethyl, and trichloromethyl.

The term "amino-C₁₋₆ alkyl" means a group in which at least one hydrogen atom at an arbitrary position of the "C₁₋₆ alkyl" is replaced with amino (NH₂). Examples of the amino-C₁₋₆ alkyl include aminomethyl, 2-aminoethyl, 3-aminopropyl, 2,3-diaminopropyl, and 4-aminobutyl.

The term "hydroxy-C₁₋₆ alkyl" means a group in which at least one hydrogen atom at an arbitrary position of the "C₁₋₆ alkyl" is replaced with hydroxy (OH). Examples of the hydroxy-C₁₋₆ alkyl include hydroxymethyl, 2-hydroxyethyl, 3-hydroxypropyl, 2,3-dihydroxypropyl, and 4-hydroxybutyl.

The term "halo-C₁₋₆ alkoxy" means a group in which at least one hydrogen atom at an arbitrary position of the "C₁₋₆ alkoxy" is replaced with the "halogen atom". Examples of the halo-C₁₋₆ alkoxy include fluoromethoxy, difluoromethoxy, trifluoromethoxy, 2,2,2-trifluoroethoxy, dichloromethoxy, and trichloromethoxy.

The term "C₁₋₆ alkoxy-C₁₋₆ alkoxy" means a group in which one hydrogen atom at an arbitrary position of the "C₁₋₆ alkoxy" is replaced with another "C₁₋₆ alkoxy". Examples of the C₁₋₆ alkoxy-C₁₋₆ alkoxy include methoxymethoxy, ethoxymethoxy, 2-methoxyethoxy, and 2-ethoxyethoxy.

The term "C₁₋₆ alkoxycarbonyl" means a group of the "C₁₋₆ alkoxy" bonded to carbonyl (-C(=O)-). Examples of the C₁₋₆ alkoxycarbonyl include methoxycarbonyl, ethoxycarbonyl, and tert-butoxycarbonyl (Boc).

The term "aryl" means a monovalent group of aromatic hydrocarbon singly or in combination with an additional group and preferably means a monovalent group of aromatic hydrocarbon having 6 to 10 carbon atoms. Examples of the aryl include phenyl, 1-naphthyl, and 2-naphthyl.

The term "heterocyclyl" means a saturated or unsaturated monocyclic, dicyclic, or tricyclic heterocyclic monovalent group containing 1 to 5 heteroatoms selected from the group consisting of O, N, and S, singly or in combination with an additional group and preferably means a saturated or unsaturated monocyclic or dicyclic heterocyclic monovalent group having 2 to 9 carbon atoms and 1, 2, or 3 heteroatoms selected from the group consisting of O, N, and S. Examples of the heterocyclyl include: 3- to 10-membered saturated heterocyclic groups such as oxetanyl, azetidinyl, pyrrolidinyl, pyrazolidinyl, piperidyl, piperazinyl, tetrahydropyranyl, morpholino, thiomorpholino, homomorpholino, and homopiperazinyl; monocyclic 5- to 10-membered unsaturated heterocyclic groups such as 2-thienyl, 3-thienyl, 2-furyl, 3-furyl, 2-pyranyl, 3-pyranyl, 4-pyranyl, 1-pyrrolyl, 2-pyrrolyl, 3-pyrrolyl, 1-imidazolyl, 2-imidazolyl, 4-imidazolyl, 1-pyrazolyl, 3-pyrazolyl, 4-pyrazolyl, 5-pyrazolyl, 2-thiazolyl, 4-thiazolyl, 5-thiazolyl, 3-isothiazolyl, 4-isothiazolyl, 5-isothiazolyl, 1,2,4-triazol-1-yl, 1,2,4-triazol-3-yl, 1,2,4-triazol-5-yl, 1,2,3-triazol-1-yl, 1,2,3-triazol-4-yl, 1,2,3-triazol-5-yl, 2-oxazolyl, 4-oxazolyl, 5-oxazolyl, 3-isoxazolyl, 4-isoxazolyl, 5-isoxazolyl, 2-pyridyl, 3-pyridyl, 4-pyridyl, 2-pyrazinyl, 2-pyrimidinyl, 4-pyrimidinyl, 5-pyrimidinyl, 3-pyridazinyl, 4-pyridazinyl, 1,3,4-oxadiazol-2-yl, 1,3,4-thiadiazol-2-yl, 1,2,4-oxadiazol-3-yl, 1,2,4-oxadiazol-5-yl, 1,2,4-thiadiazol-3-yl, 1,2,4-thiadiazol-5-yl, 1,2,5-oxadiazol-3-yl, and 1,2,5-thiadiazol-3-yl; and condensed polycyclic 5- to 10-membered unsaturated heterocyclic groups such as 2-benzofuranyl, 3-benzofuranyl, 4-benzofuranyl, 5-benzofuranyl, 6-benzofuranyl, 7-benzofuranyl, 1-isobenzofuranyl, 4-isobenzofuranyl, 5-isobenzofuranyl, 2-benzoxazolyl, 4-benzoxazolyl, 5-benzoxazolyl, 6-benzoxazolyl, 7-benzoxazolyl, 4-benzoxadiazolyl, 5-benzoxadiazolyl, 2-benzothienyl, 3-benzothienyl, 4-benzothienyl, 5-benzothienyl, 6-benzothienyl, 7-benzothienyl, 1-isobenzothienyl, 4-isobenzothienyl, 5-isobenzothienyl, 2-benzothiazolyl, 4-benzothiazolyl, 5-benzothiazolyl, 6-benzothiazolyl, 7-benzothiazolyl, 2-chromenyl, 3-chromenyl, 4-chromenyl, 5-chromenyl, 6-chromenyl, 7-chromenyl, 8-chromenyl, 1-indolizinyl, 2-indolizinyl, 3-indolizinyl, 5-indolizinyl, 6-indolizinyl, 7-indolizinyl, 8-indolizinyl, 1-isoindolyl, 2-isoindolyl, 4-isoindolyl, 5-isoindolyl, 1-indolyl, 2-indolyl, 3-indolyl, 4-indolyl, 5-indolyl, 6-indolyl, 7-indolyl, 1-indazolyl, 2-indazolyl, 4-indazolyl, 5-indazolyl, 6-indazolyl, 7-indazolyl, 2-purinyl, 6-purinyl, 7-purinyl, 8-purinyl, 2-quinolyl, 3-quinolyl, 4-quinolyl, 5-quinolyl, 6-quinolyl, 7-quinolyl, 8-quinolyl, 1-isoquinolyl, 3-isoquinolyl, 4-isoquinolyl, 5-isoquinolyl, 6-isoquinolyl, 7-isoquinolyl, 8-isoquinolyl, 1-phthalazinyl, 5-phthalazinyl, 6-phthalazinyl, 2,7-naphthyridin-1-yl, 2,7-naphthyridin-3-yl, 2,7-naphthyridin-4-yl, 2,6-naphthyridin-1-yl, 2,6-naphthyridin-3-yl, 2,6-naphthyridin-4-yl, 1,8-naphthyridin-2-yl, 1,8-naphthyridin-3-yl, 1,8-naphthyridin-4-yl, 1,7-naphthyridin-2-yl, 1,7-naphthyridin-3-yl, 1,7-naphthyridin-4-yl, 1,7-naphthyridin-5-yl, 1,7-naphthyridin-6-yl, 1,7-naphthyridin-8-yl, 1,6-naphthyridin-2-yl, 1,6-naphthyridin-3-yl, 1,6-naphthyridin-4-yl, 1,6-naphthyridin-5-yl, 1,6-naphthyridin-7-yl, 1,6-naphthyridin-8-yl, 1,5-naphthyridin-2-yl, 1,5-naphthyridin-3-yl, 1,5-naphthyridin-4-yl, 2-quinoxalinyl, 5-quinoxalinyl, 6-quinoxalinyl, 2-quinazolinyl, 4-quinazolinyl, 5-quinazolinyl, 6-quinazolinyl, 7-quinazolinyl, 8-quinazolinyl, 3-cinnolinyl, 4-cinnolinyl, 5-cinnolinyl, 6-cinnolinyl, 7-cinnolinyl, 8-cinnolinyl, 2-pteridinyl, 4-pteridinyl, 6-pteridinyl, and 7-pteridinyl.

The term "aryloxy" means a group -O-R' (wherein R' is the aryl) singly or in combination with an additional group. Examples of the aryloxy include phenoxy and naphthoxy.

The term "aryl-C₁₋₆ alkyl" means a group in which at least one hydrogen atom at an arbitrary position of the "C₁₋₆ alkyl" is replaced with the "aryl". Examples of the aryl-C₁₋₆ alkyl include benzyl, phenethyl, phenylpropyl, phenylbutyl, phenylpentyl, and phenylhexyl. The term "aryl-C₁₋₆ alkyl" can be used interchangeably with "aralkyl".

The term "aryl-C₁₋₆ alkoxy" means a group in which at least one hydrogen atom at an arbitrary position of the "C₁₋₆ alkoxy" is replaced with the "aryl". Examples of the aryl-C₁₋₆ alkoxy include benzyloxy and phenethyloxy. The term "aryl-C₁₋₆ alkoxy" can be used interchangeably with "aralkyloxy".

The term "aryl-C₂₋₆ alkenyl" means a group in which at least one hydrogen atom at an arbitrary position of the "C₂₋₆ alkenyl" is replaced with the "aryl". Examples of the aryl-C₂₋₆ alkenyl include 2-phenylvinyl and 3-phenylallyl.

The term "heterocyclyl-C₁₋₆ alkyl" means a group in which at least one hydrogen atom at an arbitrary position of the "C₁₋₆ alkyl" is replaced with the "heterocyclyl". Examples of the heterocyclyl-C₁₋₆ alkyl include 3-pyridylmethyl, 2-(3-pyridyl)ethyl, and 3-(3-pyridyl)propyl.

The term "heterocyclyl-C₂₋₆ alkenyl" means a group in which at least one hydrogen atom at an arbitrary position of the "C₂₋₆ alkenyl" is replaced with the "heterocyclyl". Examples of the heterocyclyl-C₂₋₆ alkenyl include 2-(3-pyridyl)vinyl and 2-(3-pyridyl)-1-propen-1-yl.

The term "substituted or unsubstituted" means that the group is not substituted or is substituted with at least one substituent selected from a predetermined substituent group, for example, the group consisting of halogen, nitro, cyano, amino, hydroxy, C₁₋₆ alkyl, halo-C₁₋₆ alkyl, amino-C₁₋₆ alkyl, hydroxy-C₁₋₆ alkyl, mono- or di-C₁₋₆ alkylamino, C₁₋₆ alkoxy, halo-C₁₋₆ alkoxy, C₁₋₆ alkoxy-C₁₋₆ alkoxy, C₂₋₆ alkenyl, C₂₋₆ alkenyloxy, C₂₋₆ alkynyl, C₂₋₆ alkynyloxy, -NHCOR¹⁵, -COR¹⁵, -C₁₋₆ alkyl-NHCOR¹⁵, aryl, heterocyclyl, aryloxy, aryl-C₁₋₆ alkyl, aryl-C₁₋₆ alkoxy, aralkyloxy-aryl, and aralkyloxy-aryloxy and oxo.

In this context, R¹⁵ is C₁₋₆ alkyl, C₁₋₆ alkoxy, aryl, heterocyclyl, aryl-C₁₋₆ alkyl, aryl-C₂₋₆ alkenyl, heterocyclyl-C₁₋₆ alkyl, or heterocyclyl-C₂₋₆ alkenyl, and the aryl or heterocyclyl moiety in R¹⁵ is optionally substituted with at least one substituent selected from the group consisting of halogen, nitro, cyano, amino, hydroxy, C₁₋₆ alkyl, halo-C₁₋₆ alkyl, amino-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, and oxo. Preferably, R¹⁵ is C₁₋₆ alkyl, C₁₋₆ alkoxy, aryl, heterocyclyl, aryl-C₂₋₆ alkenyl, or heterocyclyl-C₂₋₆ alkenyl, and the aryl or heterocyclyl moiety is optionally substituted with at least one substituent selected from the group consisting of halogen, amino, C₁₋₆ alkyl, halo-C₁₋₆ alkyl, and amino-C₁₋₆ alkyl.

A preferred form of the "substituted or unsubstituted C₁₋₆ alkyl" is (unsubstituted) C₁₋₆ alkyl or is C₁₋₆ alkyl substituted with at least one substituent selected from the group consisting of amino, -NHCOR¹⁵ (wherein R¹⁵ is C₁₋₆ alkoxy, heterocyclyl, or heterocyclyl-C₂₋₆ alkenyl), aryl, heterocyclyl, and aryloxy. In this context, the aryl or heterocyclyl moiety contained in the substituent is optionally substituted with at least one substituent selected from the group consisting of halogen, nitro, cyano, amino, hydroxy, C₁₋₆ alkyl, halo-C₁₋₆ alkyl, hydroxy-C₁₋₆ alkyl, mono- or di-C₁₋₆ alkylamino, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, aryl, heterocyclyl, aryloxy, aryl-C₁₋₆ alkyl, and aryl-C₁₋₆ alkoxy, and preferably optionally substituted with at least one substituent selected from the group consisting of halogen, cyano, C₁₋₆ alkyl, halo-C₁₋₆ alkyl, C₁₋₆ alkoxy, halo-C₁₋₆ alkoxy, aryl-C₁₋₆ alkyl, and aryl-C₁₋₆ alkoxy.

A preferred form of the "substituted or unsubstituted C₂₋₆ alkenyl" is (unsubstituted) C₂₋₆ alkenyl or is C₂₋₆ alkenyl substituted with at least one substituent selected from the group consisting of amino, -NHCOR¹⁵ (wherein R¹⁵ is C₁₋₆ alkoxy, heterocyclyl, or heterocyclyl-C₂₋₆ alkenyl), aryl, heterocyclyl, and aryloxy, and a more preferred form thereof is (unsubstituted) C₂₋₆ alkenyl or is C₂₋₆ alkenyl substituted with at least one substituent selected from the group consisting of aryl and heterocyclyl. In this context, the aryl or heterocyclyl moiety contained in the substituent is optionally substituted with at least one substituent selected from the group consisting of halogen, nitro, cyano, amino, hydroxy, C₁₋₆ alkyl, halo-C₁₋₆ alkyl, hydroxy-C₁₋₆ alkyl, mono- or di-C₁₋₆ alkylamino, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, aryl, heterocyclyl, aryloxy, aryl-C₁₋₆ alkyl, and aryl-C₁₋₆ alkoxy, and preferably optionally substituted with at least one substituent selected from the group consisting of halogen, cyano, C₁₋₆ alkyl, halo-C₁₋₆ alkyl, mono- or di-C₁₋₆ alkylamino, C₁₋₆ alkoxy, and halo-C₁₋₆ alkoxy.

A preferred form of the "substituted or unsubstituted C₂₋₆ alkynyl" is (unsubstituted) C₂₋₆ alkynyl or is C₂₋₆ alkynyl substituted with at least one substituent selected from the group consisting of amino, -NHCOR¹⁵ (wherein R¹⁵ is C₁₋₆ alkoxy, heterocyclyl, or heterocyclyl-C₂₋₆ alkenyl), aryl, heterocyclyl, and aryloxy, and a more preferred form thereof is (unsubstituted) C₂₋₆ alkynyl. In this context, the aryl or heterocyclyl moiety contained in the substituent is optionally substituted with at least one substituent selected from the group consisting of halogen, nitro, cyano, amino, hydroxy, C₁₋₆ alkyl, halo-C₁₋₆ alkyl, hydroxy-C₁₋₆ alkyl, mono- or di-C₁₋₆ alkylamino, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, aryl, heterocyclyl, aryloxy, aryl-C₁₋₆ alkyl, and aryl-C₁₋₆ alkoxy.

A preferred form of the "substituted or unsubstituted aryl" is (unsubstituted) aryl or is aryl substituted with at least one substituent selected from the group consisting of halogen, nitro, cyano, amino, hydroxy, C₁₋₆ alkyl, halo-C₁₋₆ alkyl, amino-C₁₋₆ alkyl, hydroxy-C₁₋₆ alkyl, mono- or di-C₁₋₆ alkylamino, C₁₋₆ alkoxy, halo-C₁₋₆ alkoxy, C₁₋₆ alkoxy-C₁₋₆ alkoxy, C₂₋₆ alkenyl, C₂₋₆ alkenyloxy, C₂₋₆ alkynyl, C₂₋₆ alkynyloxy, - COR¹⁵, -C₁₋₆ alkyl-NHCOR¹⁵, aryl, heterocyclyl, aryloxy, aryl-C₁₋₆ alkyl, and aryl-C₁₋₆ alkoxy. In this context, R¹⁵ is C₁₋₆ alkyl, C₁₋₆ alkoxy, aryl, or heterocyclyl.

A preferred form of the "substituted or unsubstituted heterocyclyl" is (unsubstituted) heterocyclyl or is heterocyclyl substituted with at least one substituent selected from the group consisting of halogen, nitro, cyano, amino, hydroxy, C₁₋₆ alkyl, halo-C₁₋₆ alkyl, amino-C₁₋₆ alkyl, hydroxy-C₁₋₆ alkyl, mono- or di-C₁₋₆ alkylamino, C₁₋₆ alkoxy, halo-C₁₋₆ alkoxy, C₁₋₆ alkoxy-C₁₋₆ alkoxy, C₂₋₆ alkenyl, C₂₋₆ alkenyloxy, C₂₋₆ alkynyl, C₂₋₆ alkynyloxy, -COR¹⁵, -C₁₋₆ alkyl-NHCOR¹⁵, aryl, heterocyclyl, aryloxy, aryl-C₁₋₆ alkyl, aryl-C₁₋₆ alkoxy, and oxo, and a more preferred form thereof is (unsubstituted) heterocyclyl or is heterocyclyl substituted with at least one substituent selected from the group consisting of halogen, nitro, hydroxy, C₁₋₆ alkyl, halo-C₁₋₆ alkyl, hydroxy-C₁₋₆ alkyl, C₂₋₆ alkenyl, C₂₋₆ alkynyl, -COR¹⁵, -C₁₋₆ alkyl-NHCOR¹⁵, aryl, heterocyclyl, aryloxy, aryl-C₁₋₆ alkyl, aryl-C₁₋₆ alkoxy, and oxo. In this context, R¹⁵ is C₁₋₆ alkyl, aryl, or heterocyclyl, and the aryl or heterocyclyl is optionally substituted with at least one substituent selected from the group consisting of halogen, nitro, cyano, amino, hydroxy, C₁₋₆ alkyl, halo-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, and oxo.

The term "C₁₋₁₀ alkylene" means linear or branched alkylene having 1 to 10 carbon atoms, singly or in combination with an additional group. Examples of the C₁₋₁₀ alkylene include methylene, ethylene, propylene, trimethylene, tetramethylene, 1-methylpropylene, 2-methylpropylene, dimethylethylene, ethylethylene, pentamethylene, 1-methyl-tetramethylene, 2-methyl-tetramethylene, 1,1-dimethyl-trimethylene, 1,2-dimethyl-trimethylene, 2,2-dimethyl-trimethylene, 1-ethyl-trimethylene, hexamethylene, octamethylene, and decamethylene. A preferred form of the C₁₋₁₀ alkylene is C_{2 to 8} alkylene, for example, ethylene, propylene, trimethylene, tetramethylene, hexamethylene, or octamethylene.

The term "C₁₋₃ alkylene" means linear or branched alkylene having 1 to 3 carbon atoms, singly or in combination with an additional group. Examples thereof include methylene, ethylene, propylene, and trimethylene.

The alkylene is optionally substituted with halogen, -COOR¹², -CONHR¹², or oxo (wherein R¹² is a hydrogen atom or C₁₋₆ alkyl), and preferably optionally substituted with -COOR¹² or -CONHR¹² (wherein R¹² is a hydrogen atom or methyl).

The term "arylene" means a divalent group obtained by removing one hydrogen atom at an arbitrary position of the "aryl", singly or in combination with an additional group. Examples of the arylene include 1,4-phenylene, 1,3-phenylene, and 1,2-phenylene.

The arylene is optionally substituted with halogen, -COOR¹², -CONHR¹², or oxo (wherein R¹² is a hydrogen atom or C₁₋₆ alkyl) and preferably optionally substituted with halogen.

The term "C₁₋₁₀ alkylene-arylene", "arylene-C₁₋₁₀ alkylene", "C₁₋₁₀ alkylene-arylene-C₁₋₁₀ alkylene", or "arylene-C₁₋₁₀ alkylene-arylene" means a divalent group formed from the "C₁₋₁₀ alkylene" bonded to the "arylene".

Likewise, the term "C₁₋₃ alkylene-arylene", "arylene-C₁₋₃ alkylene", or "C₁₋₃ alkylene-arylene-C₁₋₃ alkylene" means a divalent group formed from the "C₁₋₃ alkylene" bonded to the "arylene".

The term "heterocyclylene" means a divalent group obtained by removing one hydrogen atom at an arbitrary position of the "heterocyclyl", singly or in combination with an additional group. Examples of the heterocyclylene include 1,4-piperazinediyl, 2,5-piperazinediyl, 2,5-pyridinediyl, and 2,6-pyridinediyl.

The heterocyclylene is optionally substituted with halogen, -COOR¹², - CONHR¹², or oxo (wherein R¹² is a hydrogen atom or C₁₋₆ alkyl), and preferably optionally substituted with oxo.

The term "C₁₋₁₀ alkylene-heterocyclylene", "heterocyclylene-C₁₋₁₀ alkylene", or "C₁₋₁₀ alkylene-heterocyclylene-C₁₋₁₀ alkylene" means a divalent group formed from the "C₁₋₁₀ alkylene" bonded to the "heterocyclylene".

Likewise, the term "C₁₋₃ alkylene-heterocyclylene", "heterocyclylene-C₁₋₃ alkylene", or "C₁₋₃ alkylene-heterocyclylene-C₁₋₃ alkylene" means a divalent group formed from the "C₁₋₃ alkylene" bonded to the "heterocyclylene".

The compound represented by the general formula (I) of the present invention includes its stereoisomers (if present). The stereoisomers mean isomers differing in spatial arrangement of atoms. Examples thereof include optical isomers and geometric isomers, such as diastereomers and enantiomers. For example, when the compound represented by the general formula (I) of the present invention has one or more chiral centers, the compound represented by the general formula (I) of the present invention can be present in the form of an optically pure enantiomer, an enantiomer mixture such as a racemate, an optically pure diastereomer, a diastereomer mixture, a diastereomer racemate, a diastereomer racemate mixture, or the like.

Examples of the pharmaceutically acceptable salt of the compound represented by the general formula (I) of the present invention include: inorganic acid salts such as hydrochloride, hydrobromide, hydroiodide, nitrate, sulfate, and phosphate; and organic acid salts such as acetate, trifluoroacetate, benzoate, oxalate, malonate, succinate, maleate, fumarate, tartrate, citrate, methanesulfonate, ethanesulfonate, trifluoromethanesulfonate, benzenesulfonate, p-toluenesulfonate, glutamate, and aspartate. A preferred form of the organic acid salt is dicarboxylate such as oxalate, malonate, succinate, maleate, fumarate, or tartrate.

Other examples of the pharmaceutically acceptable salt of the compound represented by the general formula (I) of the present invention include: metal salts such as sodium salt, potassium salt, calcium salt, and magnesium salt; inorganic salts such as ammonium salt; and organic amine salts such as triethylamine salt and guanidine salt.

The compound represented by the general formula (I) of the present invention or the pharmaceutically acceptable salt thereof includes pharmaceutically acceptable solvates. A preferred form of the solvate is a hydrate. The hydrate may result from the moisture absorption of the compound represented by the general formula (I) of the present invention or the pharmaceutically acceptable salt thereof.

The compound represented by the general formula (I) of the present invention or the pharmaceutically acceptable salt thereof, when being crystals, may exhibit crystal polymorphs. The crystal polymorphs mean identical substances differing in crystal structure. Individual crystals or their mixtures at an arbitrary ratio are encompassed by the present invention.

Hereinafter, embodiments of the present invention will be described in detail.

The present invention relates to a compound represented by the general formula (I): 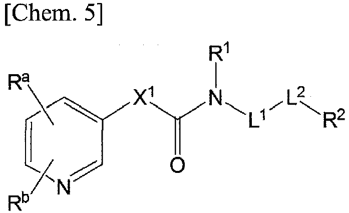 wherein
R^{a} and R^{d} are each independently a hydrogen atom, halogen, nitro, cyano, amino, hydroxy, C₁₋₆ alkyl, halo-C₁₋₆ alkyl, amino-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, or C₂₋₆ alkynyloxy, or R^{a} and R^{d}, which present on adjacent carbon atoms, optionally form a substituted or unsubstituted carbocyclic ring or heterocyclic ring condensed with a pyridine ring, together with the carbon atoms to which they are bonded,
X¹ is a direct bond, -CR¹¹=CR¹¹-, or -(CONR¹¹)ₘCH₂-,
R¹ is a hydrogen atom or C₁₋₆ alkyl,
L¹ is C₁₋₁₀ alkylene, arylene, C₁₋₁₀ alkylene-arylene, arylene-C₁₋₁₀ alkylene, C₁₋₁₀ alkylene-arylene-C₁₋₁₀ alkylene, arylene-C₁₋₁₀ alkylene-arylene, heterocyclylene, C₁₋₁₀ alkylene-heterocyclylene, heterocyclylene-C₁₋₁₀ alkylene, or C₁₋₁₀ alkylene-heterocyclylene-C₁₋₁₀ alkylene, wherein the alkylene, arylene, or heterocyclylene moiety is optionally substituted with halogen, -COOR¹², -CONHR¹², or oxo,
L² is a direct bond, -O-, -NR¹³-, -NR¹³SO₂-, or -(OCH₂CH₂)ₙO-, and
R² is a hydrogen atom, azide, substituted or unsubstituted C₁₋₆ alkyl, substituted or unsubstituted C₂₋₆ alkenyl, substituted or unsubstituted C₂₋₆ alkynyl, -COR¹⁴, substituted or unsubstituted aryl, or substituted or unsubstituted heterocyclyl,
wherein
m is 0 or 1,
n is 1 to 6,
each R¹¹ is independently a hydrogen atom or C₁₋₆ alkyl,
R¹² is a hydrogen atom or C₁₋₆ alkyl,
R¹³ is a hydrogen atom, C₁₋₆ alkyl, or -COR¹⁴, and
R¹⁴ is substituted or unsubstituted C₁₋₆ alkyl, substituted or unsubstituted C₂₋₆ alkenyl, substituted or unsubstituted C₂₋₆ alkynyl, substituted or unsubstituted C₃₋₁₀ cycloalkyl, substituted or unsubstituted C₃₋₁₀ cycloalkenyl, substituted or unsubstituted aryl, or substituted or unsubstituted heterocyclyl,
or a pharmaceutically acceptable salt thereof.

However, a compound in which L¹ is arylene, C₁₋₃ alkylene-arylene, arylene-C₁₋₃ alkylene, or C₁₋₃ alkylene-arylene-C₁₋₃ alkylene, and L² is a direct bond, a compound in which L¹ is C₁₋₃ alkylene-arylene, and L² is -O-, and a compound in which L¹ is C₁₋₃ alkylene, L² is -NH-, and R² is -CO-5-membered heterocyclyl, are excluded.

The present invention also relates to a compound represented by the following general formula (Ia): 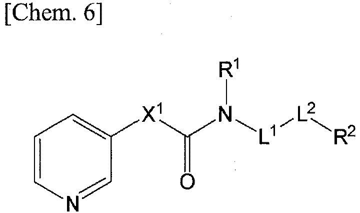 wherein
X¹ is a direct bond, -CR¹¹=CR¹¹-, or -CONR¹¹CH₂-,
R¹ is a hydrogen atom or C₁₋₆ alkyl,
L¹ is C₁₋₁₀ alkylene, arylene, C₁₋₃ alkylene-arylene, arylene-C₁₋₃ alkylene, C₁₋₃ alkylene-arylene-C₁₋₃ alkylene, heterocyclylene, C₁₋₃ alkylene-heterocyclylene, heterocyclylene-C₁₋₃ alkylene, or C₁₋₃ alkylene-heterocyclylene-C₁₋₃ alkylene, wherein the alkylene moiety is optionally substituted with -COOR¹² or -CONHR¹²,
L² is a direct bond, -O-, -NR¹³-, -NR¹³SO₂-, or -(OCH₂CH₂)ₙO-, and
R² is a hydrogen atom, azide, substituted or unsubstituted C₁₋₆ alkyl, substituted or unsubstituted C₂₋₆ alkenyl, substituted or unsubstituted C₂₋₆ alkynyl, -COR¹⁴, substituted or unsubstituted aryl, or substituted or unsubstituted heterocyclyl,
wherein
n is 1 to 6,
each R¹¹ is independently a hydrogen atom or C₁₋₆ alkyl,
R¹² is a hydrogen atom or C₁₋₆ alkyl,
R¹³ is a hydrogen atom, C₁₋₆ alkyl, or -COR¹⁴,
R¹⁴ is substituted or unsubstituted C₁₋₆ alkyl, substituted or unsubstituted C₂₋₆ alkenyl, substituted or unsubstituted aryl, or substituted or unsubstituted heterocyclyl,
or a pharmaceutically acceptable salt thereof.

However, a compound in which L¹ is arylene, C₁₋₃ alkylene-arylene, arylene-C₁₋₃ alkylene, or C₁₋₃ alkylene-arylene-C₁₋₃ alkylene, and L² is a direct bond, a compound in which L¹ is C₁₋₃ alkylene-arylene, and L² is -O-, and a compound in which L¹ is C₁₋₃ alkylene, L² is -NH-, and R² is -CO-5-membered heterocyclyl, are excluded.

The substitution means substitution with at least one substituent selected from, for example, the following substituent group:
halogen, nitro, cyano, amino, hydroxy, C₁₋₆ alkyl, halo-C₁₋₆ alkyl, amino-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, - NHCOR¹⁵, -COR¹⁵, -C₁₋₆ alkyl-NHCOR¹⁵, aryl, heterocyclyl, and oxo,
wherein R¹⁵ is C₁₋₆ alkyl, C₁₋₆ alkoxy, aryl, heterocyclyl, aryl-C₁₋₆ alkyl, aryl-C₂₋₆ alkenyl, heterocyclyl-C₁₋₆ alkyl, or heterocyclyl-C₂₋₆ alkenyl, and the aryl or heterocyclyl moiety in R¹⁵ is optionally substituted with at least one substituent selected from the group consisting of halogen, nitro, cyano, amino, hydroxy, C₁₋₆ alkyl, halo-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, C₂₋₆ alkynyloxy, and oxo.

In a specific embodiment, the present invention relates to the compound represented by the general formula (I) of the present invention or the pharmaceutically acceptable salt thereof, wherein L² is -O- or -NH-.

The present invention relates to, for example, a compound represented by the general formula (II) or a pharmaceutically acceptable salt thereof.

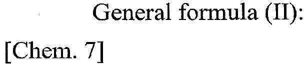

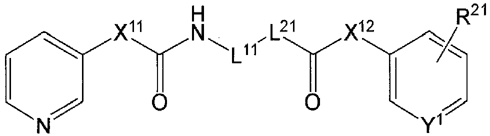

In the formula,
X¹¹ is a direct bond, -CR¹¹=CR¹¹-, or -CONR¹¹CH₂-,
X¹² is a direct bond, C₁₋₃ alkylene, -CR¹¹=CR¹¹-, or -CH₂NR¹¹CO-,
L¹¹ is C₁₋₁₀ alkylene, arylene, C₁₋₃ alkylene-arylene, arylene-C₁₋₃ alkylene, C₁₋₃ alkylene-arylene-C₁₋₃ alkylene, heterocyclylene, C₁₋₃ alkylene-heterocyclylene, heterocyclylene-C₁₋₃ alkylene, or C₁₋₃ alkylene-heterocyclylene-C₁₋₃ alkylene,
L²¹ is -O- or -NH-,
Y¹ is N or CH,
each R¹¹ is independently a hydrogen atom or C₁₋₆ alkyl,
R¹² is a hydrogen atom or C₁₋₆ alkyl, and
each R²¹ is independently selected from the group consisting of halogen, nitro, cyano, amino, hydroxy, C₁₋₆ alkyl, halo-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, and C₂₋₆ alkynyloxy.

In an alternative specific embodiment, the present invention also relates to the compound represented by the general formula (I) of the present invention or the pharmaceutically acceptable salt thereof, wherein L² is a direct bond or - (OCH₂CH₂)ₙO-.

The present invention further relates to the compound represented by the general formula (I) of the present invention or the pharmaceutically acceptable salt thereof, wherein R² is a hydrogen atom, substituted or unsubstituted C₁₋₆ alkyl, substituted or unsubstituted C₂₋₆ alkenyl, substituted or unsubstituted C₂₋₆ alkynyl, substituted or unsubstituted aryl, or substituted or unsubstituted heterocyclyl.

In an alternative specific embodiment, the present invention relates to the compound represented by the general formula (I) of the present invention or the pharmaceutically acceptable salt thereof, wherein each of R^{a} and R^{b} is a hydrogen atom, or R^{a} and R^{d}, which present on adjacent carbon atoms, optionally form a benzene ring condensed with a pyridine ring, together with the carbon atoms to which they are bonded.

In an alternative specific embodiment, the present invention relates to the compound represented by the general formula (I) of the present invention or the pharmaceutically acceptable salt thereof, wherein X¹ is a direct bond, -CH₂-, -CH=CH-, -C(CH₃)=CH-, -CH=C(CH₃)-, or -CONHCH₂-.

In an alternative specific embodiment, the present invention relates to the compound represented by the general formula (I) of the present invention or the pharmaceutically acceptable salt thereof, wherein R¹ is a hydrogen atom or methyl.

In an alternative specific embodiment, the present invention relates to the compound represented by the general formula (I) of the present invention or the pharmaceutically acceptable salt thereof, wherein L¹ is ethylene, propylene, trimethylene, tetramethylene, pentamethylene, hexamethylene, octamethylene, 1,4-phenylene(p-phenylene), 1,3-phenylene(m-phenylene), 1,2-phenylene(o-phenylene), methylene-p-phenylene, methylene-o-phenylene, methylene-m-phenylene, o-phenylene-ethylene-o-phenylene, p-phenylene-ethylene-p-phenylene, p-phenylene-methylene, methylene-p-phenylene-methylene, 2,5-pyridinediyl, 2,6-pyridinediyl, methylene-2,5-pyridinediyl, methylene-2,6-pyridinediyl, methylene-2,5-pyridinediyl-methylene, methylene-2,6-pyridinediyl-methylene, hexamethylene-piperazine-1,4-diyl-ethylene, trimethylene-3,6-dioxopiperazine-2,5-diyl-trimethylene, or tetramethylene-3,6-dioxopiperazine-2,5-diyl-tetramethylene.

In an alternative specific embodiment, the present invention relates to the compound represented by the general formula (I) of the present invention or the pharmaceutically acceptable salt thereof, wherein L² is a direct bond, -O-, -NH-, - NCH₃-, -OCH₂CH₂O-, -(OCH₂CH₂)₂O-, or -(OCH₂CH₂)₃O-.

In an alternative specific embodiment, the present invention relates to the compound represented by the general formula (I) of the present invention or the pharmaceutically acceptable salt thereof, wherein R² is a hydrogen atom, azide, 2-aminoethyl, 2-[[(3-pyridyl)carbonyl]amino]ethyl, 3-aminopropyl, 3-benzoylaminopropyl, 3-(2-naphthoylamino)propyl, 3-[[(3-pyridyl)carbonyl]amino]propyl, 2-[[3-(3-pyridyl)-2-propenoyl]amino]ethyl, 2-[[3-(3-pyridyl)-2-butenoyl]amino]ethyl, 3-[[3-(3-pyridyl)-2-propenoyl]amino]propyl, 3-[(tert-butoxycarbonyl)amino]propyl, 2-[(tert-butoxycarbonyl)amino]ethyl, 3-[(3-quinolylcarbonyl)amino]propyl, 3-[(6-quinolylcarbonyl)amino]propyl, 2-propynyl, propanoyl, butyryl, isobutyryl, pivaloyl, pentanoyl, 2-methylbutanoyl, 3-methylbutanoyl, 2-ethylbutanoyl, 3-methylpentanoyl, 4-methylpentanoyl, hexanoyl, 2,2-dimethylbutanoyl, 3,3-dimethylbutanoyl, 4-fluorophenylacetyl, 4-chlorophenylacetyl, 4-methylphenylacetyl, 2-trifluoromethylphenylacetyl, 3-trifluoromethylphenylacetyl, 4-trifluoromethylphenylacetyl, 4-cyanophenylacetyl, 4-benzyloxyphenylacetyl, phenoxyacetyl, 3-trifluoromethylphenoxyacetyl, 4-trifluoromethylphenoxyacetyl, 4-cyanophenoxyacetyl, 4-benzyloxyphenoxyacetyl, 2-naphthylacetyl, 3-(4-methylphenyl)-2-propenoyl, 3-(4-methoxyphenyl)-2-propenoyl, 3-(4-dimethylaminophenyl)-2-propenoyl, 3-(4-cyanophenyl)-2-propenoyl, 3-(4-trifluoromethylphenyl)-2-propenoyl, 3-(4-trifluoromethoxyphenyl)-2-propenoyl, 3-phenyl-2-propenoyl, 3-(3-pyridyl)-2-propenoyl, 2-[[(3-pyridyl)carbonyl]amino]ethanoyl, 3-(3-pyridyl)-2-butenoyl, 3-phenylpropanoyl, 3-(4-trifluoromethylphenyl)propanoyl, 3-(4-trifluoromethoxyphenyl)propanoyl, 3-(4-cyanophenyl)propanoyl, 2-butynoyl, 3-phenylpropynoyl, cyclobutanecarbonyl, cyclohexanecarbonyl, adamantane-1-carbonyl, benzoyl, 4-cyanobenzoyl, 2-fluorobenzoyl, 3-fluorobenzoyl, 4-fluorobenzoyl, 3,4-difluorobenzoyl, 3,5-difluorobenzoyl, 2,4-difluorobenzoyl, 3-chlorobenzoyl, 4-chlorobenzoyl, 2-toluoyl, 4-toluoyl, 2,4-dimethylbenzoyl, 3,4-dimethylbenzoyl, 3,5-dimethylbenzoyl, 4-ethylbenzoyl, 4-methoxybenzoyl, 4-vinylbenzoyl, 2-(trifluoromethyl)benzoyl, 3-(trifluoromethyl)benzoyl, 4-(trifluoromethyl)benzoyl, 3-fluoro-5-(trifluoromethyl)benzoyl, 2-(trifluoromethoxy)benzoyl, 3-(trifluoromethoxy)benzoyl, 4-(trifluoromethoxy)benzoyl, 4-(2-propynyloxy)benzoyl, 4-(3-butynyloxy)benzoyl, biphenyl-4-carbonyl, 1-naphthoyl, 2-naphthoyl, pyridine-2-carbonyl, pyridine-3-carbonyl, pyridine-4-carbonyl, (5-hydroxy-3-pyridyl)carbonyl, [5-(2-propynyloxy)-3-pyridyl]carbonyl, quinoline-3-carbonyl, quinoline-6-carbonyl, oxetane-3-carbonyl, tetrahydropyran-4-carbonyl, N-tert-butoxycarbonyl-azetidine-3-carbonyl, phenyl, 2-nitrophenyl, 3-hydroxyphenyl, 3-methoxyphenyl, 3-methoxymethoxyphenyl, 4-methoxycarbonylphenyl, 4-(N-acetylaminomethyl)phenyl, 4-[[(3-pyridyl)carbonyl]aminomethyl]phenyl, 4-[N-(benzoyl)aminomethyl]phenyl, N-(4-aminomethylbenzoyl)piperidin-4-yl, N-(2-hydroxyethyl)piperidin-4-yl, N-benzoylpiperidin-4-yl, 4-(2-hydroxyethyl)piperazin-1-yl, 4-nitro-2,1,3-benzoxazol-7-yl, or 1-oxoisoindolin-1-yl.

Examples of the compound represented by the general formula (I) of the present invention include compounds of [Table 1].

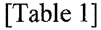

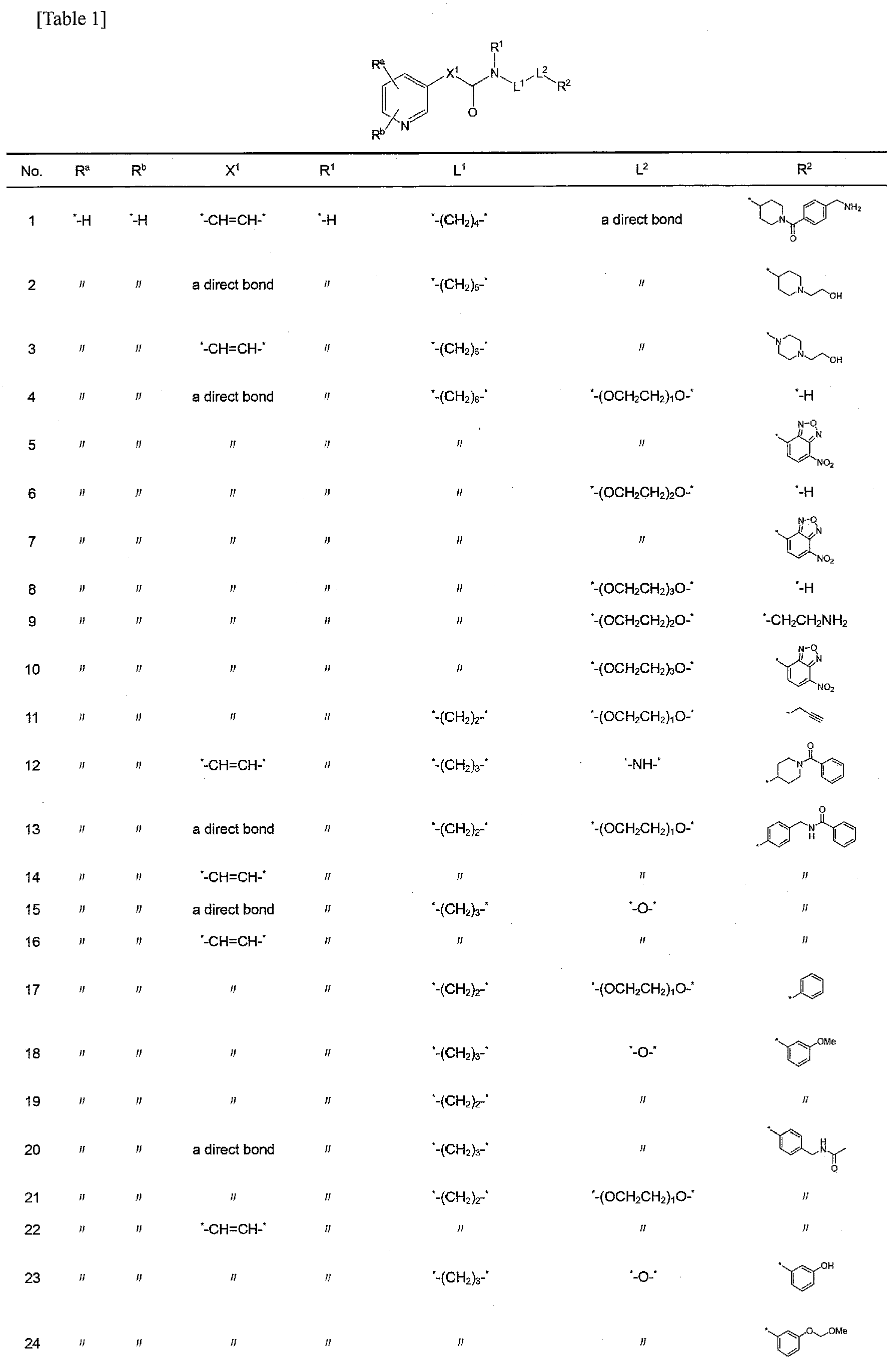

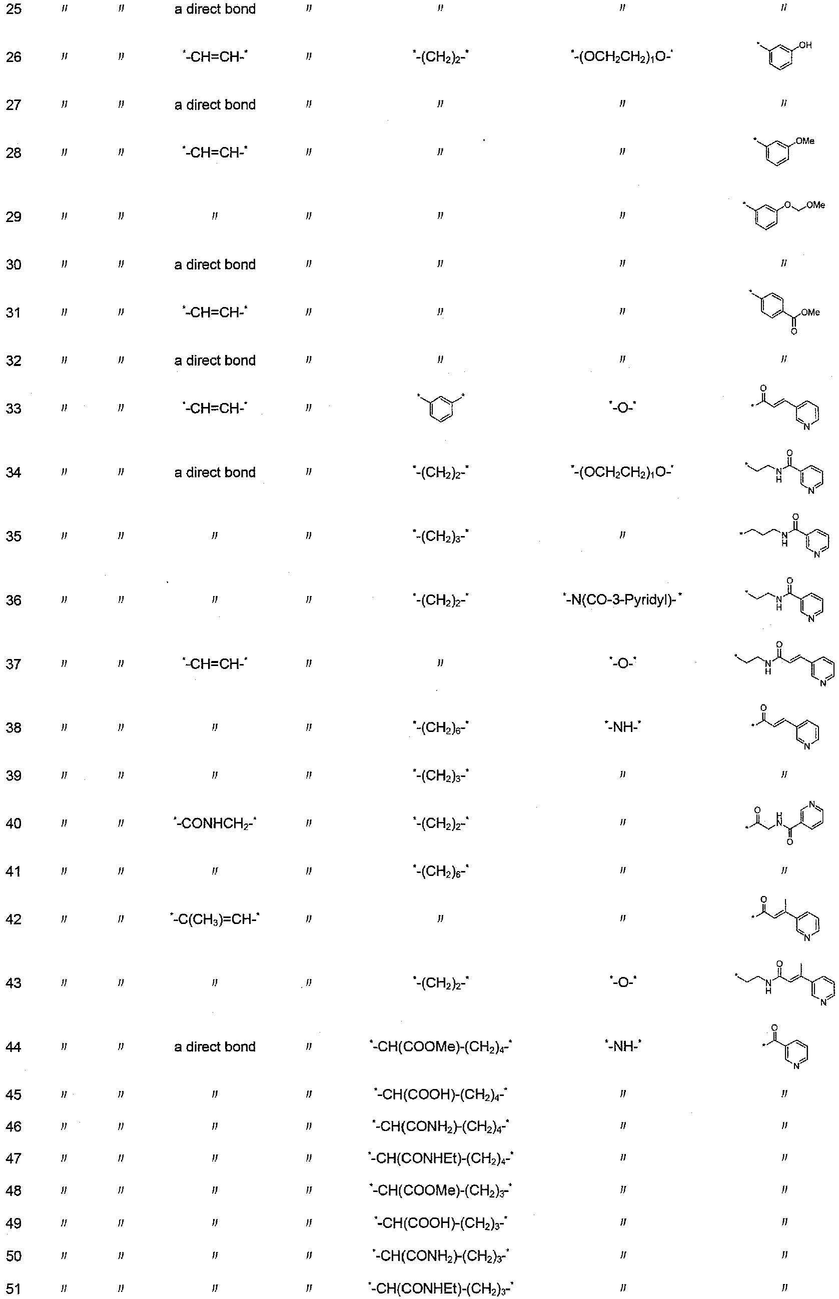

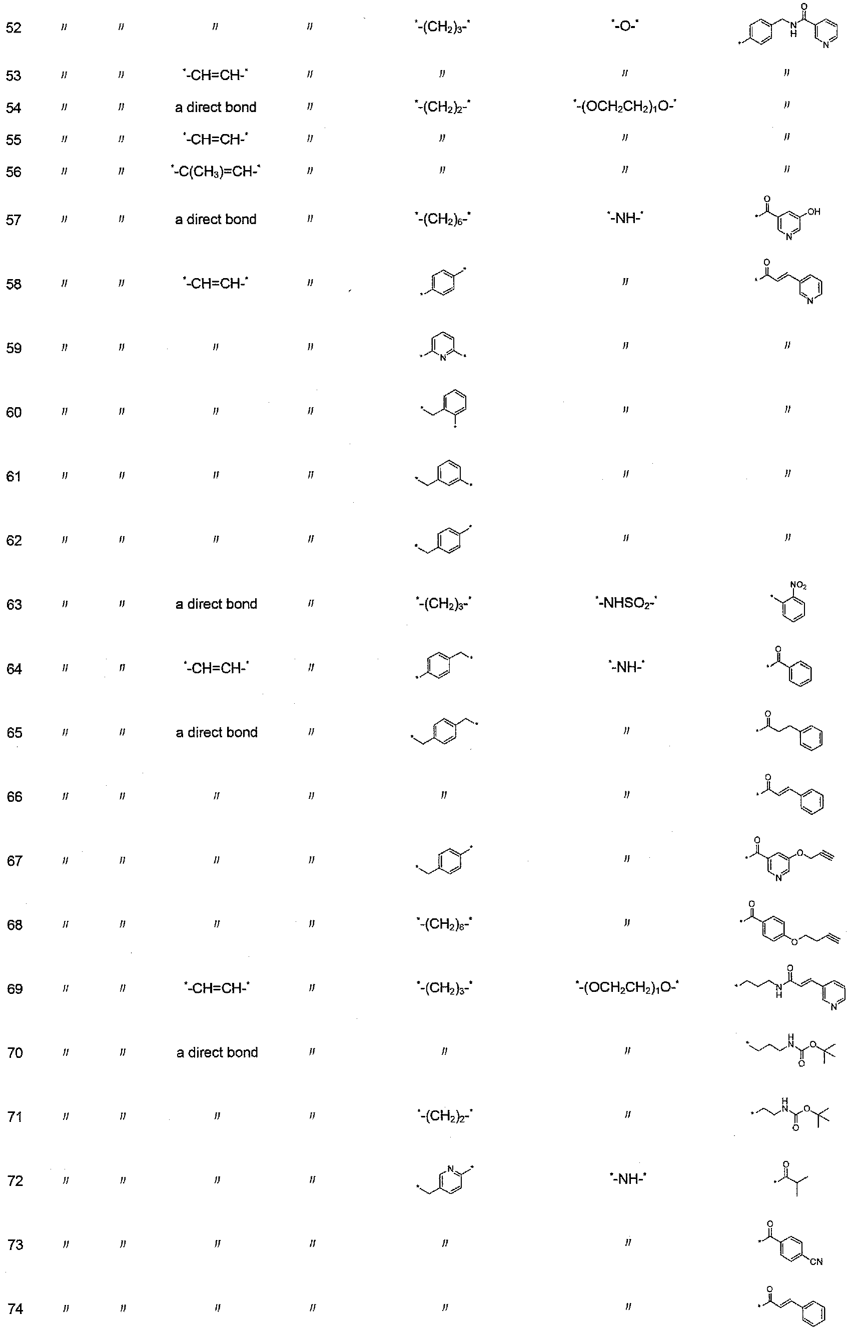

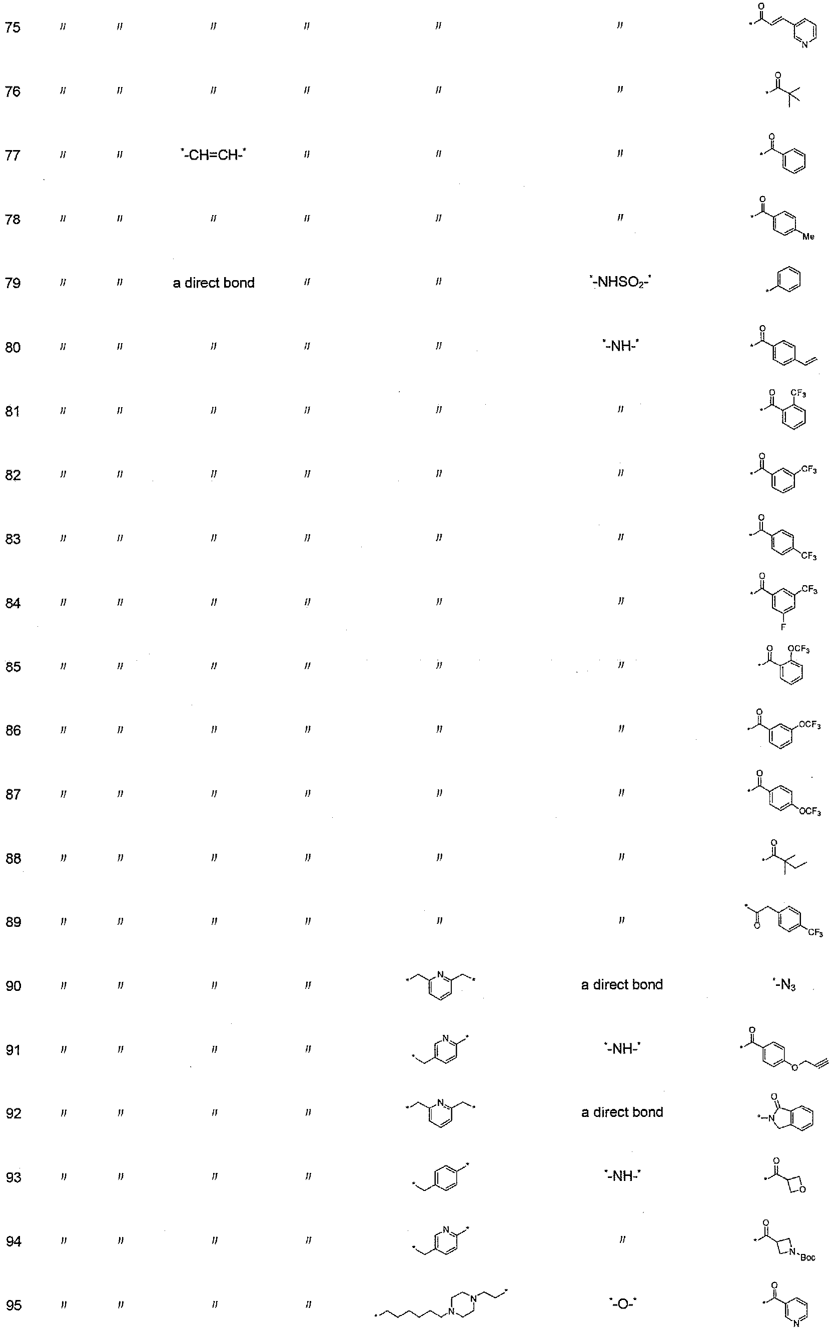

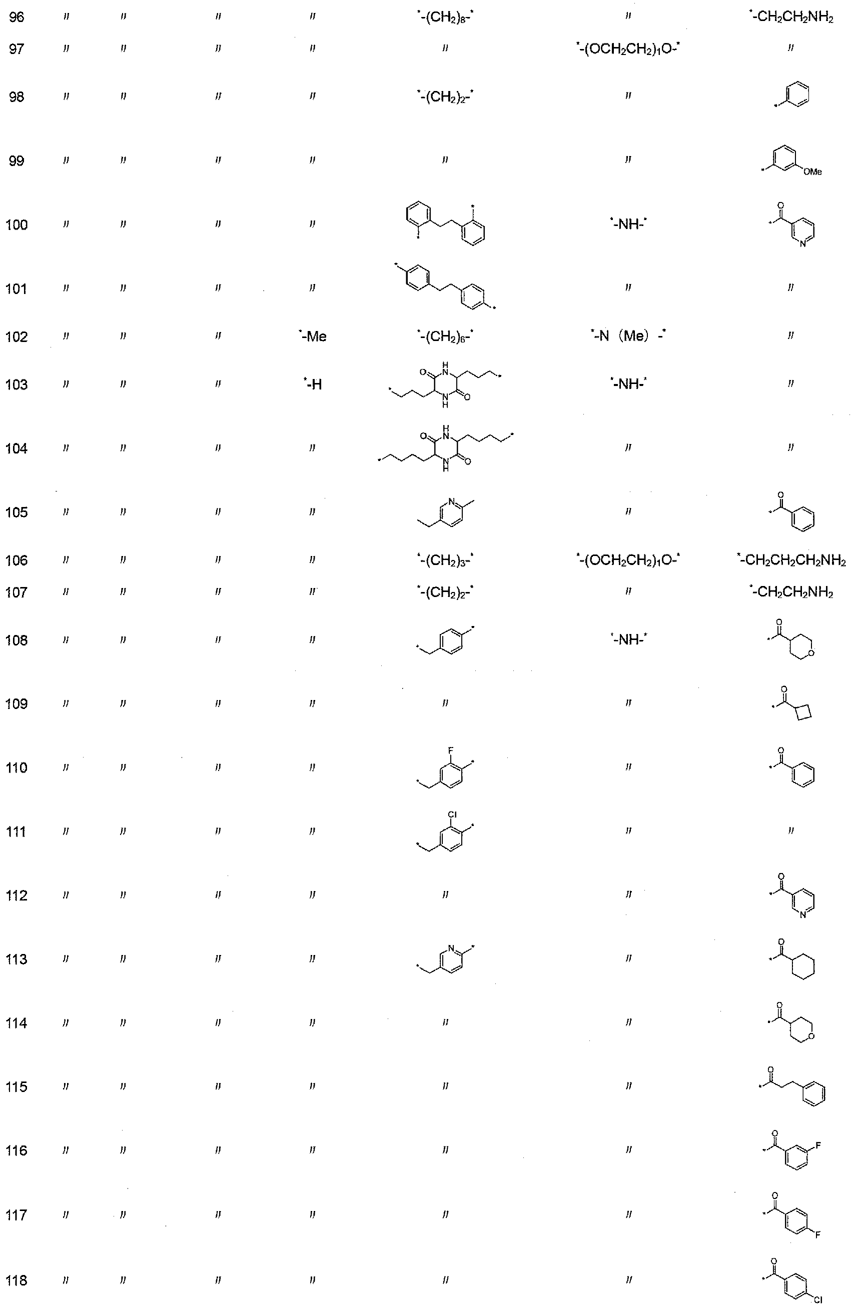

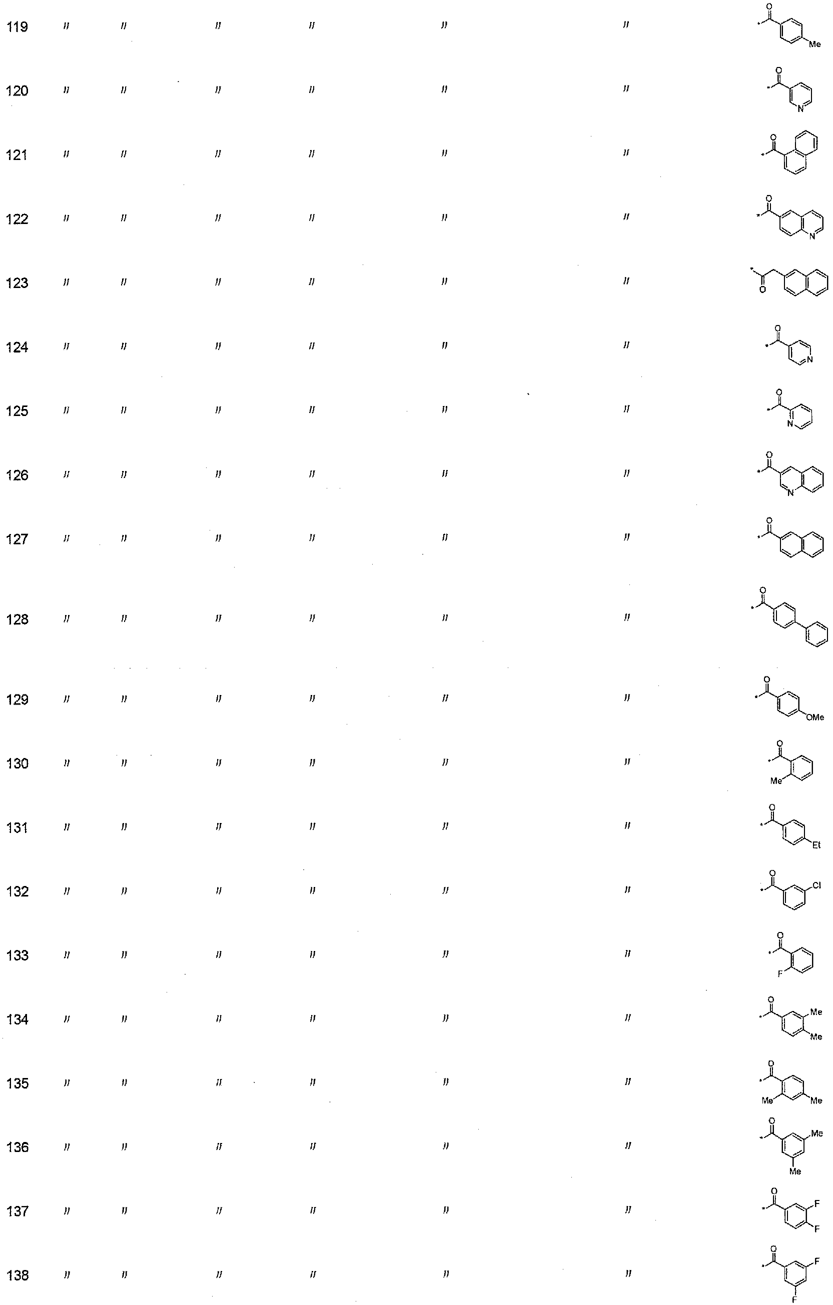

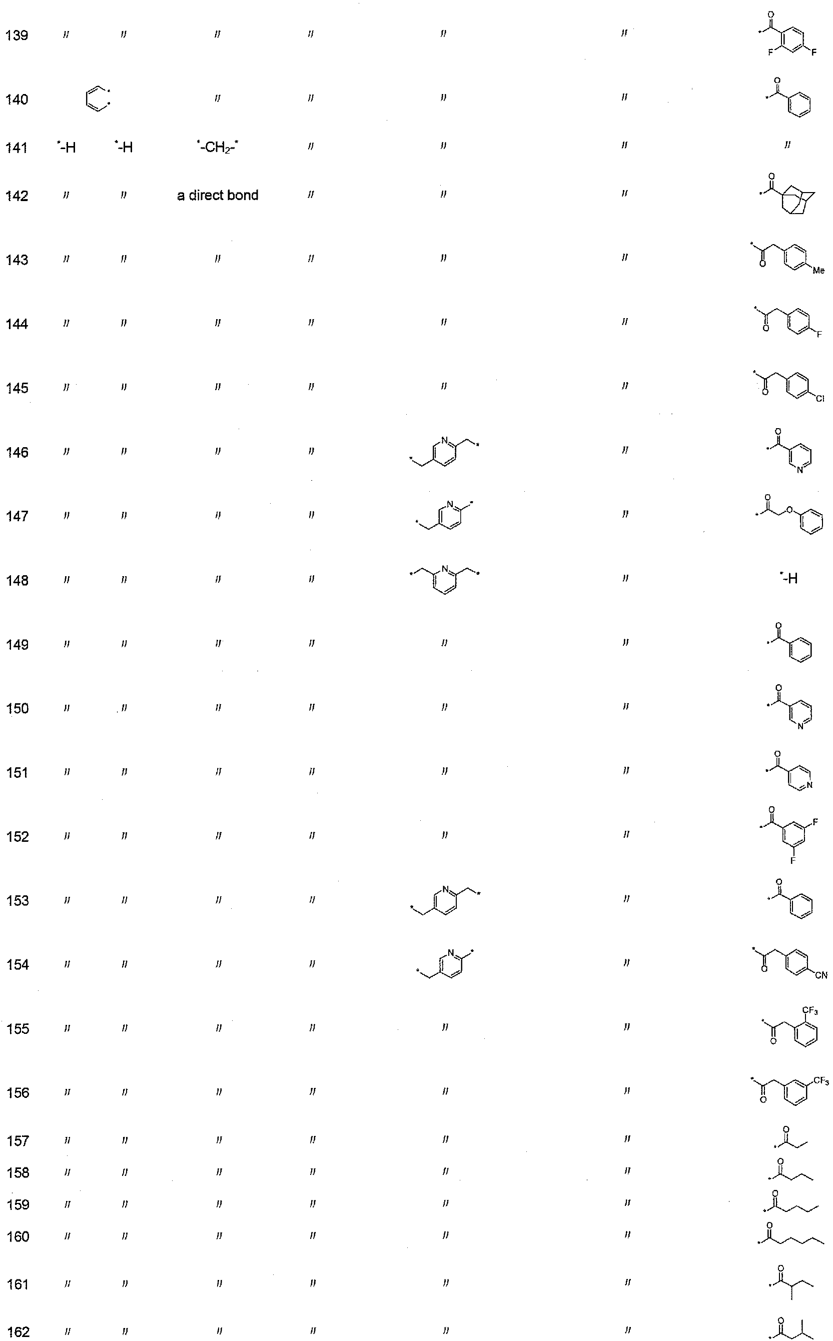

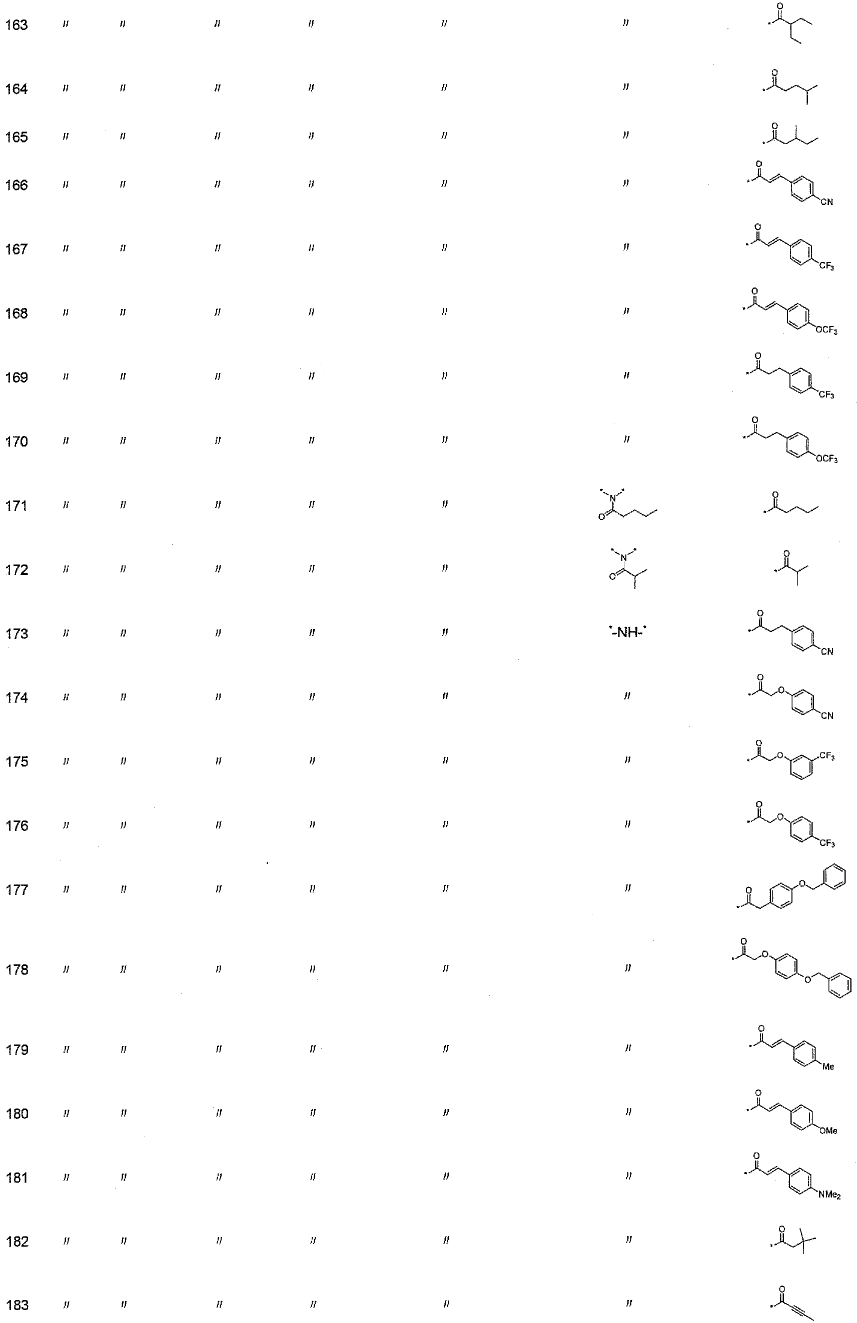

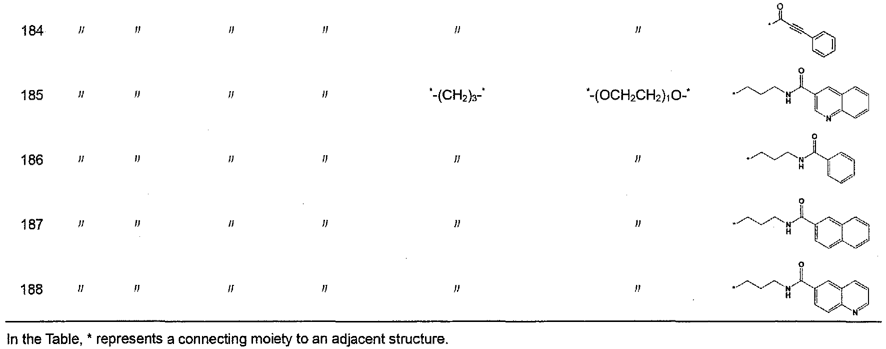

In a further specific embodiment, the present invention relates to the compounds of compound Nos. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 37, 38, 39, 40, 41, 42, 43, 48, 52, 53, 54, 55, 56, 57, 58, 59, 61, 62, 63, 64, 65, 66, 67, 68, 69, 70, 71, 72, 73,74, 75, 76, 77, 78, 80, 81, 82, 83, 84, 85, 86, 87, 88, 89, 91, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, and 188.

In a further specific embodiment, the present invention relates to the compounds of compound Nos. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 37, 38, 39, 41, 42, 43, 52, 53, 54, 55, 56, 57, 58, 59, 61, 62, 64, 65, 66, 67, 68, 69, 70, 71, 72, 73, 74, 75, 76, 77, 78, 80, 81, 82, 83, 84, 85, 86, 87, 88, 89, 91, 93, 94, 95, 96, 97, 98, 99, 100, 101, 102, 103, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 142, 143, 144, 145, 146, 147, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 186, and 188.

In a further specific embodiment, the present invention relates to the compounds of compound Nos. 1, 2, 3, 4, 5, 6, 7, 8, 9, 14, 15, 16, 17, 18, 19, 21, 22, 23, 24, 26, 28, 29, 31, 32, 34, 35, 38, 39, 41, 42, 43, 52, 53, 54, 55, 56, 58, 59, 61, 62, 64, 65, 66, 67, 68, 69, 70, 71, 73, 75, 76, 77, 78, 80, 82, 83, 87, 89, 91, 94, 95, 96, 97, 101, 102, 105, 106, 107, 108, 109, 110, 111, 113, 114, 115, 116, 117, 118, 119, 120, 122, 123, 124, 125, 126, 127, 128, 129, 130, 131, 132, 133, 134, 135, 136, 137, 138, 139, 143, 144, 145, 147, 154, 155, 156, 157, 158, 159, 160, 161, 162, 163, 164, 165, 166, 167, 168, 169, 170, 171, 172, 179, 180, 181, 182, and 188.

In a further specific embodiment, the present invention relates to the compounds of compound Nos. 1, 3, 4, 6, 9, 14, 15, 16, 17, 18, 19, 22, 23, 24, 26, 28, 29, 31, 34, 35, 38, 39, 41, 42, 43, 53, 54, 55, 58, 61, 62, 64, 65, 66, 67, 68, 69, 75, 77, 78, 80, 82, 89, 94, 96, 97, 101, 108, 109, 113, 114, 115, 116, 117, 118, 119, 122, 123, 124, 125, 126, 127, 128, 129, 130, 131, 132, 133, 134, 135, 136, 137, 138, 143, 144, 145, 147, 154, 155, 156, 160, 164, 165, 166, 167, 169, 170, 179, 180, and 182.

The compound represented by the general formula (I) of the present invention or the pharmaceutically acceptable salt thereof can be produced by a known method. Hereinafter, typical methods for producing the compound of the present invention or the pharmaceutically acceptable salt thereof will be shown, though the production methods are not limited thereto. The compound represented by the general formula (I) of the present invention or the pharmaceutically acceptable salt thereof is not limited to compounds or pharmaceutically acceptable salts thereof produced by the production methods given below.

In the production methods given below, if a compound has a partial structure (e.g., hydroxy and amino) that inhibits desired reaction or receives side reaction, the compound of interest can be obtained by introducing a protective group to the partial structure, performing the desired reaction, and then removing the protective group. Methods for introducing and removing such a protective group can be carried out in accordance with methods that are commonly used in organic synthetic chemistry (e.g., methods described in Protective Groups in Organic Synthesis 4th ed., T. W. Greene, P. G. M. Wuts, John Wiley & Sons Inc. (2006)). Specific methods for producing individual compounds of the present invention will be described in detail in Examples mentioned later.

### (Production process 1)

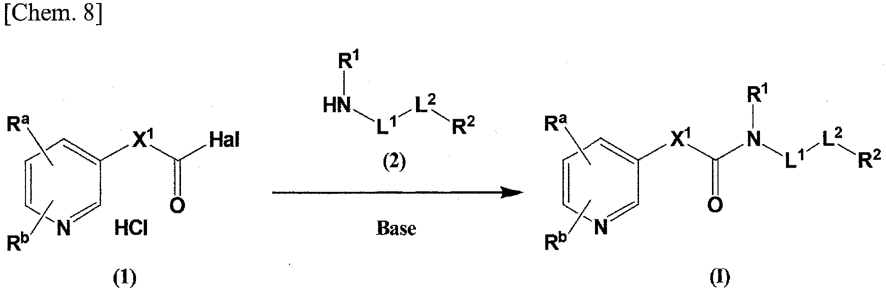

In the formulas, R^{a}, R^{b}, X¹, R¹, L¹, L², and R² are as defined above, and Hal is a halogen atom.

Production process 1 is a method of reacting a compound (1) and a compound (2) in the presence of a base in a solvent to obtain the compound represented by the general formula (I) of the present invention or the pharmaceutically acceptable salt thereof.

The compound (1) is known and is available from a reagent supplier. Examples of such a compound include nicotinic acid chloride hydrochloride and trans-3-(3-pyridyl)acrylic acid chloride hydrochloride. Alternatively, the compound (1) can be produced in accordance with a known method using a compound available from a reagent supplier.

The compound (2) is known and is available from a reagent supplier. Alternatively, the compound (2) can be produced in accordance with a known method, for example, a method described as " Production of intermediate" in Examples mentioned later, using a compound available from a reagent supplier.

The amount of the compound (2) used is usually a molar quantity of 0.9 to 5 times, preferably a molar quantity of 1.1 to 3 times, per mol of the compound (1).

Examples of the base used include: alkali metal acetate such as sodium acetate and potassium acetate; alkali metal carbonate such as sodium carbonate, potassium carbonate, and cesium carbonate; and organic bases such as triethylamine and diisopropylethylamine. The base is preferably potassium carbonate, cesium carbonate, triethylamine, or diisopropylethylamine. The amount of the base used is usually a molar quantity of 0.9 to 10 times, preferably, a molar quantity of 1 to 5 times, per mol of the compound (1).

The solvent used is not particularly limited as long as the solvent dissolves a starting material to some extent without inhibiting the reaction. Examples thereof include: aromatic hydrocarbons such as benzene, toluene, and xylene; halogenated aliphatic hydrocarbons such as methylene chloride, chloroform, and 1,2-dichloroethane; ethers such as tetrahydrofuran, 1,2-dimethoxyethane, and 1,4-dioxane; nitriles such as acetonitrile and propionitrile; amides such as N,N-dimethylformamide, N,N-dimethylacetamide, and N-methylpyrrolidone; sulfoxides such as dimethyl sulfoxide; and theirs arbitrary mixed solvents. For example, a halogenated aliphatic hydrocarbon such as methylene chloride, chloroform, or 1,2-dichloroethane, or an ether such as tetrahydrofuran, 1,2-dimethoxyethane, or 1,4-dioxane is preferably used. The amount of the solvent used is not particularly limited and is usually an amount of 1 to 50 times, preferably an amount of 5 to 20 times, based on the mass of the compound (1).

The reaction temperature differs depending on the types of the starting material, the solvent, and the like, the amounts of usage, and the like and is usually 0°C to 150°C, preferably 20°C to 120°C.

The reaction time differs depending on the reaction temperature and the like and is usually 1 minute to 48 hours, preferably 0.5 hours to 24 hours.

The reaction pressure is appropriately set according to the needs. The reaction pressure may be any of increased pressure, reduced pressure, and atmospheric pressure and is preferably atmospheric pressure. The reaction atmosphere can be an atmosphere appropriately selected according to the needs and is preferably an air atmosphere or an inert gas (such as nitrogen or argon) atmosphere.

When the obtained compound represented by the general formula (I) has a functional group (e.g., a halogen atom, a hydroxy group, and a carbonyl group), a further desired compound can be produced by reacting the functional group with a proper reaction reagent in accordance with a known method.

### (Production process 2)

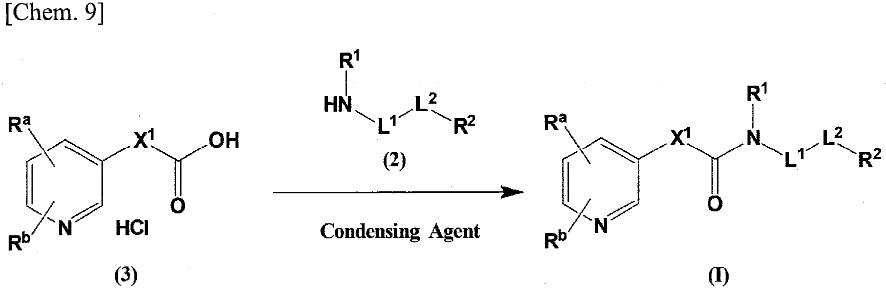

In the formulas, R^{a}, R^{b}, X¹, R¹, L¹, L², and R² are as defined above.

Production process 2 is a method of reacting a compound (3) and a compound (2) in the presence of a condensing agent in a solvent to obtain the compound represented by the general formula (I).

The compound (3) is known and is available from a reagent supplier. Examples of such a compound include nicotinic acid, trans-3-(3-pyridyl)acrylic acid, 3-(3-pyridyl)-2-butenoic acid, and N-nicotinoylglycine. Alternatively, the compound (3) can be produced in accordance with a known method using a compound available from a reagent supplier.

The compound (2) is known and is available from a reagent supplier. Alternatively, the compound (2) can be produced in accordance with a known method, for example, a method described as " Production of intermediate" in Examples mentioned later, using a compound available from a reagent supplier.

The amount of the compound (2) used is usually a molar quantity of 0.9 to 5 times, preferably a molar quantity of 1.1 to 3 times, per mol of the compound (3).

Examples of the condensing agent used include, but are not particularly limited to, carbodiimide condensing agents (e.g., N,N'-dicyclohexylcarbodiimide (DCC), N,N'-diisopropylcarbodiimide, and 1-ethyl-3-dimethylaminopropylcarbodiimide hydrochloride (EDCI)), chloroformate condensing agents (e.g., ethyl chloroformate and isobutyl chloroformate), imidazole condensing agents (e.g., 1,1'-carbonyldiimidazole (CDI)), phosphonium condensing agents (e.g., (benzotriazol-1-yloxy)tripyrrolidinophosphonium hexafluorophosphate (PyBOP(R)) and bromotripyrrolidinophosphonium hexafluorophosphate (PyBrop(R))), and uronium condensing agents (e.g., O-(benzotriazol-1-yl)-N,N,N',N'-tetramethyluronium tetrafluoroborate (TBTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU), and (1-cyano-2-ethoxy-2-oxoethylideneaminooxy)dimethylamino-morpholino-carbenium hexafluorophosphate (COMU)).

The amount of the condensing agent used is usually a molar quantity of 0.1 to 20 times, preferably a molar quantity of 1 to 10 times, more preferably a molar quantity of 1 to 5 times, per mol of the compound (3).

An additive and a base can be appropriately used without interfering with the reaction.

Examples of the additive used include, but are not particularly limited to, N,N-dimethyl-4-aminopyridine (DMAP), 1-hydroxybenzotriazole (HOBt), 1-hydroxy-1H-1,2,3-triazole-5-carboxylic acid ethyl ester (HOCt), 1-hydroxy-7-azabenzotriazole (HOAt), and (hydroxyimino)cyanoethyl acetate (OxymaPure).

The amount of the additive used is usually a molar quantity of 0.1 to 20 times, preferably a molar quantity of 0.2 to 10 times, more preferably a molar quantity of 1 to 5 times, per mol of the compound (3).

Examples of the base used include, but are not particularly limited to, aliphatic amine (e.g., triethylamine, N,N-diisopropylethylamine, and N-methylmorpholine) and aromatic amine (e.g., dimethylaminopyridine).

The amount of the base used is usually a molar quantity of 1 to 50 times, preferably a molar quantity of 1 to 10 times, more preferably a molar quantity of 1 to 5 times, per mol of the compound (3).

The solvent used is not particularly limited as long as the solvent does not interfere with the reaction. Examples thereof include halogen-containing hydrocarbon solvents (e.g., dichloromethane and chloroform), aromatic hydrocarbon solvents (e.g., toluene and xylene), ether solvents (e.g., tetrahydrofuran, 1,4-dioxane, cyclopentyl methyl ether, and methyl-t-butyl ether), amide solvents (e.g., N,N-dimethylformamide), and nitrile solvents (e.g., acetonitrile). The solvent is preferably a halogen-containing hydrocarbon solvent or an ether solvent, more preferably dichloromethane, tetrahydrofuran, or cyclopentyl methyl ether.

The solvent used is not particularly limited as long as the solvent dissolves a starting material to some extent without inhibiting the reaction. Examples thereof include: aromatic hydrocarbons such as benzene, toluene, and xylene; halogenated aliphatic hydrocarbons such as methylene chloride, chloroform, and 1,2-dichloroethane; ethers such as tetrahydrofuran, 1,2-dimethoxyethane, and 1,4-dioxane; nitriles such as acetonitrile and propionitrile; and their arbitrary mixed solvents. For example, a halogenated aliphatic hydrocarbon such as methylene chloride, chloroform, or 1,2-dichloroethane, or an ether such as tetrahydrofuran, 1,2-dimethoxyethane, or 1,4-dioxane is preferably used. The amount of the solvent used is not particularly limited and is usually an amount of 1 to 50 times, preferably an amount of 5 to 20 times, based on the mass of the compound (3).

The reaction temperature differs depending on the types of the starting material, the solvent, and the like, the amounts of usage, and the like and is usually 0°C to 150°C, preferably 20°C to 120°C.

The reaction time differs depending on the reaction temperature and the like and is usually 1 minute to 48 hours, preferably 0.5 hours to 24 hours.

The reaction pressure is appropriately set according to the needs. The reaction pressure may be any of increased pressure, reduced pressure, and atmospheric pressure and is preferably atmospheric pressure. The reaction atmosphere can be an atmosphere appropriately selected according to the needs and is preferably an air atmosphere or an inert gas (such as nitrogen or argon) atmosphere.

When the obtained compound represented by the general formula (I) has a functional group (e.g., a halogen atom, hydroxy, and carbonyl), a further desired compound can be produced by reacting the functional group with a proper reaction reagent in accordance with a known method.

The production processes 1 and 2 may be carried out by replacing the compound of the formula (2) with an intermediate capable of inducing a precursor of the compound represented by the general formula (I), such as a compound represented by the following formula (4) or (5):

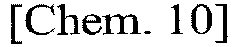

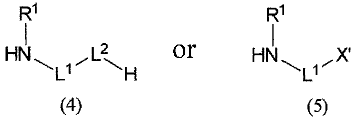

In the formulas, X¹, R¹, L¹, L², and R² are as defined above, and X' is a proper reactive group such as a halogen atom, hydroxy, or carbonyl.

In the case of using such a compound, the compound represented by the general formula (I) can be produced by further reacting the reactive group of the obtained precursor (e.g., a compound of the following formula (I') or (I")) of the compound represented by the general formula (I) with a proper reaction reagent in accordance with a known method.

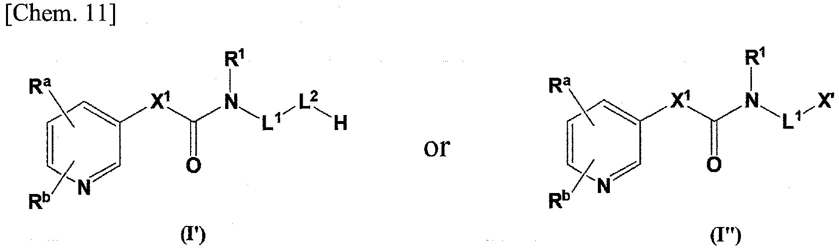

In the formulas, R^{a}, R^{b}, X¹, R¹, L¹, L², and X' are as defined above.

The obtained compound represented by the general formula (I), when having a protective group, can be subjected to a deprotection step according to the needs. When the compound represented by the general formula (I) has at least two different protective groups, only one of the protective groups may be selectively removed by selecting deprotection conditions. The deprotection conditions can be appropriately carried out in accordance with a method that is commonly used in organic synthetic chemistry (e.g., a method described in Protective Groups in Organic Synthesis 4th ed., T. W. Greene, P. G. M. Wuts, John Wiley & Sons Inc. (2006)) or Examples of the present specification.

In each production process, the obtained compound may be isolated or purified by a known approach or may be subjected directly to a next step. The isolation or the purification may be carried out by use of a usual operation, for example, filtration, extraction, crystallization, various column chromatography techniques, or the like.

In a specific embodiment, the present invention relates to a pharmaceutical composition comprising the compound represented by the general formula (I) or the pharmaceutically acceptable salt thereof as disclosed in each of the specific embodiments, and preferably relates to a pharmaceutical composition comprising the compound represented by the general formula (I) or the pharmaceutically acceptable salt thereof as disclosed in each of the specific embodiments, and at least one pharmaceutically acceptable additive.

In a specific embodiment, the present invention relates to a pharmaceutical composition for treatment of a disease subject to prevention, alleviation, and/or therapy through the suppression of axonal degeneration, the pharmaceutical composition comprising the compound represented by the general formula (I) or the pharmaceutically acceptable salt thereof as disclosed in each of the specific embodiments, and preferably relates to a pharmaceutical composition therefor comprising the compound represented by the general formula (I) or the pharmaceutically acceptable salt thereof as disclosed in each of the specific embodiments, and at least one pharmaceutically acceptable additive.

In a specific embodiment, the present invention relates to a pharmaceutical composition for treatment of a disease subject to prevention, alleviation, and/or therapy through the inhibition of SARM1, the pharmaceutical composition comprising the compound represented by the general formula (I) or the pharmaceutically acceptable salt thereof as disclosed in each of the specific embodiments, and preferably relates to a pharmaceutical composition therefor comprising the compound represented by the general formula (I) or the pharmaceutically acceptable salt thereof as disclosed in each of the specific embodiments, and at least one pharmaceutically acceptable additive.

In a specific embodiment, the present invention relates to a pharmaceutical composition for treatment of a neurodegenerative disease, particularly, for treatment of a neurodegenerative disease involving axonal degeneration as a part of its pathological condition, the pharmaceutical composition comprising the compound represented by the general formula (I) or the pharmaceutically acceptable salt thereof as disclosed in each of the specific embodiments, and preferably relates to a pharmaceutical composition therefor comprising the compound represented by the general formula (I) or the pharmaceutically acceptable salt thereof as disclosed in each of the specific embodiments, and at least one pharmaceutically acceptable additive.

In the present invention, the compound represented by the general formula (I) or the pharmaceutically acceptable salt thereof as disclosed in each of the specific embodiments can have *in vivo* axonal degeneration suppressive or SARM1 inhibitory activity directly, or indirectly via its metabolites. Thus, the pharmaceutical composition of the present invention may be not only a pharmaceutical composition containing the compound represented by the general formula (I) or the pharmaceutically acceptable salt thereof but may be a pharmaceutical composition containing its metabolite. Typical examples of the metabolite of the compound represented by the general formula (I) or the pharmaceutically acceptable salt thereof include, but are not limited to, a riboside adduct (hereinafter, also referred to as a "riboside form") having a sugar (ribose) introduced to the pyridine backbone moiety of the compound of the present invention, a nicotinamide mononucleotide (NMN)-like metabolite (hereinafter, also referred to as an "NMN-like metabolite") which is a mononucleotide adduct having a phosphoric acid moiety introduced to the riboside form, and a nicotinamide adenine dinucleotide (NAD)-like metabolite (hereinafter, also referred to as an "NAD-like metabolite") which is an adenine dinucleotide adduct further having adenosine introduced via phosphoric acid to the NMN-like metabolite, as described in Examples mentioned later.

The disease of the present invention may be a disease subject to prevention, alleviation, and/or therapy through the suppression of axonal degeneration, a disease subject to prevention, alleviation, and/or therapy through the inhibition of SARM1, a neurodegenerative disease, or a disease involving axonal degeneration, for example, axonal degeneration resulting from axonopathy, demyelinating disease, nerve damage disease or disorder, metabolic disease, mitochondrial disease, and/or metabolic axonal degeneration, as a part of its pathological condition. Examples of such a disease include central nervous system neurodegenerative disease, peripheral nervous system neurodegenerative disease, trauma-related neuropathy (including head, spine, and/or PNS trauma), hereditary neurological disease, metabolism- and/or endocrine-related neuropathy (including diabetic peripheral neuropathy), toxin-related neuropathy (including peripheral neuropathy induced by toxins (including chemotherapeutics)), inflammatory neurological disease, and cardiovascular disorder (including stroke).

Specific examples of such a disease include glaucoma, multiple sclerosis, Huntington's disease, Alzheimer's disease, Parkinson's disease, amyotrophic lateral sclerosis (ALS; Lou Gehrig's disease), Tay-Sachs disease, Gaucher's disease, Hurler syndrome, traumatic brain injury, traumatic spinal cord injury, stroke, radiation-induced injury, neurological complications of chemotherapy (chemotherapy-induced peripheral neuropathy: CIPN), neuropathy, acute ischemic optic neuropathy, age-related neurodegeneration (including dementia), chronic traumatic encephalopathy (so-called "punch drunk syndrome"), abusive head trauma, spinal cord injury (including injury caused by compression, transection, laceration, stretching, or contusion), higher functional disorder, peripheral neuropathic disease or trauma, axonal abnormalities (e.g., Wallerian degeneration), Friedreich's ataxia, Charcot-Marie-Tooth disease, diabetic neuropathy, diabetes mellitus, chronic renal failure, porphyria, amyloidosis, liver damage, hypothyroidism, Guillain-Barre syndrome, chronic inflammatory demyelinating polyradiculoneuropathy, multifocal motor neuropathy, systemic lupus erythematosus, Hansen's disease, Sjogren's syndrome, Lyme disease, sarcoidosis, polyglutamine disease (so-called "polyQ disease"), non-polyglutamine disease, Kennedy's disease, spinocerebellar ataxia 1, 2, 3, 6, 7, and 17, herpes zoster (postherpetic neuralgia), Leber's hereditary optic neuropathy, Leber congenital amaurosis, neuromyelitis optica, metachromatic leukodystrophy, acute hemorrhagic leukoencephalitis, trigeminal neuralgia, Bell's palsy, cerebral ischemia, multiple system atrophy, traumatic glaucoma, tropical spastic paraparesis/human T-lymphotropic virus (HTLV-1)-associated myelopathy, West Nile virus encephalitis, La Crosse virus encephalitis, bunyavirus Encephalitis, pediatric viral encephalitis, essential tremor, motor neuron disease, spinal muscular atrophy (SMA), hereditary sensory and autonomic neuropathy (HSAN), adrenomyeloneuropathy, progressive supranuclear palsy (PSP), hereditary ataxia, noise-induced hearing loss, congenital hearing loss, dementia with Lewy bodies, frontotemporal lobar degeneration, amyloidosis, enteric neuropathy, axonopathy, acute motor axonal neuropathy (AMAN), mitochondrial disease (e.g., chronic progressive external ophthalmoplegia, mitochondrial myopathy, encephalopathy, lactic acidosis, and stroke-like episodes, myoclonic epilepsy with ragged red fibers, and Leigh syndrome), age-related macular degeneration, retinitis pigmentosa, sickle cell disease, fibromyalgia, myalgic encephalomyelitis/chronic fatigue syndrome (ME/CSF), HIV, exposure to specific drugs/toxins (e.g., vincristine, phenytoin, nitrofurantoin, isoniazid, ethyl alcohol, chemotherapeutics, organometals, heavy metals, and fluoroquinolone drugs), vitamin B6 overdose, insufficient vitamins (e.g., vitamin B12, vitamin A, vitamin E, and vitamin B1), physical trauma, and neuropathy caused by exposure to radiation.

The pharmaceutical composition comprising the compound represented by the general formula (I) or the pharmaceutically acceptable salt thereof may be the compound itself (bulk powder alone) or may be in the form of a preparation such as a tablet, a capsule, a powder, a syrup, granules, fine granules, a pill, a suspension, an emulsion, a transdermal absorption formulation, a suppository, an ointment, a lotion, an inhalant, an instillation, or an injection, which is produced by mixing the compound or the pharmaceutically acceptable salt thereof with an appropriate pharmaceutically acceptable additive or the like, and can be administered orally or parenterally (e.g., intravenous administration, intramuscular administration, intraperitoneal administration, transdermal administration, transnasal administration, airway administration, transpulmonary administration, eye drop administration, intradermal administration, or subcutaneous administration).

These preparations are produced by well-known methods using additives such as a filler, a lubricant, a binder, a disintegrator, an emulsifier, a stabilizer, a flavoring/odor improving agent, a diluent, a tonicity agent, a buffer, a pH regulator, a solubilizer, a thickener, a dispersant, and a preservative (antiseptic).

Examples of the filler include organic fillers and inorganic fillers. Examples of the organic filler include: sugar derivatives such as lactose, sucrose, glucose, mannitol, and sorbitol; starch derivatives such as corn starch, potato starch, α- starch, and dextrin; cellulose derivatives such as crystalline cellulose; gum arabic; dextran; and pullulan. Examples of the inorganic filler include: light silicic anhydride; and sulfate such as calcium sulfate.

Examples of the lubricant include: stearic acid; stearic acid metal salts such as calcium stearate and magnesium stearate; talc: colloidal silica; waxes such as bee wax and spermaceti; boric acid; adipic acid; sulfate such as sodium sulfate; glycol; fumaric acid; sodium benzoate; D,L-leucine; sodium lauryl sulfate; silicic acids such as silicic anhydride and silicic acid hydrate; and the starch derivatives listed as the filler described above.

Examples of the binder include hydroxypropylcellulose, hydroxypropylmethylcellulose, polyvinylpyrrolidone, macrogol, and the compounds listed as the filler described above.

Examples of the disintegrator include: cellulose derivatives such as low substituted hydroxypropylcellulose, carboxymethylcellulose, calcium carboxymethylcellulose, and internally cross-linked calcium carboxymethylcellulose; cross-linked polyvinylpyrrolidone; and chemically modified starch or cellulose derivatives such as carboxymethyl starch and sodium carboxymethyl starch.

Examples of the emulsifier include: colloidal clay such as bentonite and begum; anionic surfactants such as sodium lauryl sulfate; cationic surfactants such as benzalkonium chloride; and nonionic surfactants such as polyoxyethylene alkyl ether, polyoxyethylene sorbitan fatty acid ester, and sucrose fatty acid ester.

Examples of the stabilizer include: p-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; acetic anhydride; and sorbic acid.

Examples of the flavoring/odor improving agent include: sweeteners such as saccharin sodium and aspartame; acidulants such as citric acid, malic acid, and tartaric acid; and flavors such as menthol, lemon extracts, and orange extracts.

Examples of the diluent include compounds that are usually used as diluents, for example, water, lactose, mannitol, glucose, sucrose, calcium sulfate, hydroxypropylcellulose, microcrystalline cellulose, water, ethanol, polyethylene glycol, propylene glycol, glycerol, starch, polyvinylpyrrolidone, and mixtures thereof.

Examples of the tonicity agent can include glycerin, propylene glycol, sodium chloride, potassium chloride, sorbitol, and mannitol.

Examples of the buffer can include phosphoric acid, phosphate, citric acid, acetic acid, and ε-aminocaproic acid.

Examples of the pH regulator can include hydrochloric acid, citric acid, phosphoric acid, acetic acid, sodium hydroxide, potassium hydroxide, boric acid, borax, sodium carbonate, and sodium bicarbonate.

Examples of the solubilizer can include polysorbate 80, polyoxyethylene hydrogenated castor oil 60, and macrogol 4000.

Examples of the thickener and the dispersant can include cellulosic polymers such as hydroxypropylmethylcellulose and hydroxypropylcellulose, polyvinyl alcohol, polyvinylpyrrolidone. Examples of the stabilizer can include edetic acid and sodium edetate.

Examples of the preservative (antiseptic) include general-purpose sorbic acid, potassium sorbate, benzalkonium chloride, benzethonium chloride, methyl p-hydroxybenzoate, propyl p-hydroxybenzoate, and chlorobutanol. These preservatives may be used in combination.

In addition, a proper additive can be used depending on a dosage form. For example, in the case of preparing the compound represented by the general formula (I) of the present invention or the pharmaceutically acceptable salt thereof into an aerosol for transnasal administration or airway administration, for example, a chlorofluorocarboxylic acid (CFC) (such as dichlorodifluoromethane, trichlorofluoromethane, or dichlorotetrafluoroethane) or carbon dioxide can be used as a propellant.

In a specific embodiment, the present invention relates to the compound represented by the general formula (I) or the pharmaceutically acceptable salt thereof as disclosed in each of the specific embodiments for use in treatment of a disease subject to prevention, alleviation, and/or therapy through the suppression of axonal degeneration.

In a specific embodiment, the present invention relates to the compound represented by the general formula (I) or the pharmaceutically acceptable salt thereof as disclosed in each of the specific embodiments for use in treatment of a disease subject to prevention, alleviation, and/or therapy through the suppression of axonal degeneration.

In a specific embodiment, the present invention relates to use of the compound represented by the general formula (I) or the pharmaceutically acceptable salt thereof as disclosed in each of the specific embodiments for producing a medicament for treatment of a disease subject to prevention, alleviation, and/or therapy through the suppression of axonal degeneration.

In a specific embodiment, the present invention relates to use of the compound represented by the general formula (I) or the pharmaceutically acceptable salt thereof as disclosed in each of the specific embodiments for producing a medicament for treatment of a disease subject to prevention, alleviation, and/or therapy through the inhibition of SARM1.

In a specific embodiment, the present invention relates to a method for treatment of a disease subject to prevention, alleviation, and/or therapy through the suppression of axonal degeneration, comprising administering a therapeutically effective amount of the compound represented by the general formula (I) or the pharmaceutically acceptable salt thereof as disclosed in each of the specific embodiments to a patient in need thereof.

In a specific embodiment, the present invention relates to a method for treatment of a disease subject to prevention, alleviation, and/or therapy through the inhibition of SARM1, comprising administering a therapeutically effective amount of the compound represented by the general formula (I) or the pharmaceutically acceptable salt thereof as disclosed in each of the specific embodiments to a patient in need thereof.

As used herein, the "treatment" of a disease includes (1) interference with the disease, i.e., the disease is blocked from manifesting clinical symptoms in a subject exposed to the disease or likely to develop the disease but having not yet experienced or shown any symptom of the disease, (2) inhibition of the disease, i.e., the development of the disease or its clinical symptoms is suppressed, and (3) mitigation of the disease, i.e., temporal or permanent regression of the disease or its clinical symptoms is caused.

As used herein, the term "therapeutically effective amount" means an amount that (i) effects therapy for or prevention of the disease, (ii) reduces, improves, or removes one or more symptoms of the disease, or (iii) interferes with or delays the onset of one or more symptoms of the disease when the compound represented by the general formula (I) of the present invention is administered to a subject. The therapeutically effective amount varies depending on the compound represented by the general formula (I) of the present invention to be used, the severity of the pathological condition to be subjected to the treatment, the severity of the disease to be subjected to the treatment, the age and relative health condition of the subject, an administration route, a dosage form, the judgment of a physician or a veterinarian in charge of diagnosis, and other factors.

### Examples

### [Example 1: Production of compound 1]

### (1) Production of intermediate 1

A known compound represented by the following structural formula (intermediate 1) was synthesized in accordance with the method described in WO2012/154194.

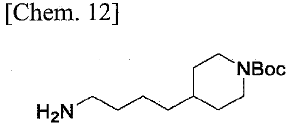

### (2) Production of intermediate 2

To a solution of intermediate 1 (1.38 g, 5.38 mmol) in dichloromethane (25 ml), trans-3-(3-pyridyl)acrylic acid chloride hydrochloride (1.21 g, 5.92 mmol) and triethylamine (3.00 ml, 21.5 mmol) were added under an ice bath, and the mixture was stirred at room temperature for 1 hour. Water was added to the reaction mixture, followed by extraction with chloroform. The organic layer was washed with saturated saline, dried over anhydrous magnesium sulfate, then filtered, and concentrated under reduced pressure. Intermediate 2 represented by the following structural formula was obtained as a yellow solid (1.62, 4.18 mmol, 78%).

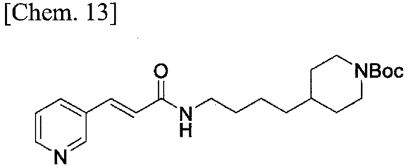

### (3) Production of intermediate 3

To a solution of intermediate 2 (219 mg, 0.565 mmol) in dichloromethane (6 ml), trifluoroacetic acid (3 ml) was added under an ice bath, and the mixture was stirred at room temperature for 1 hour. The reaction mixture was concentrated under reduced pressure, and the residue was then purified by silica gel column chromatography (chloroform:methanol = 1:1). Intermediate 3 represented by the following structural formula was obtained as a yellow solid.

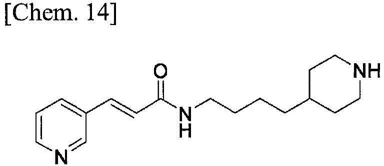

### (4) Production of intermediate 4

A known compound represented by the following structural formula (intermediate 4) was synthesized in accordance with the method described in European Journal of Medicinal Chemistry, 2020, 201, 112411.

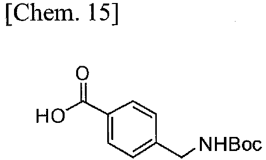

### (5) Production of intermediate 5

To a solution of intermediate 3 in acetonitrile (10 ml), intermediate 4 (486 mg, 1.94 mmol), (benzotriazol-1-yloxy)tripyrrolidinophosphonium hexafluorophosphate (Pybop) (1.34 g, 2.58 mmol), and diisopropylethylamine (0.787 ml, 4.52 mmol) were added at room temperature, and the mixture was stirred at the same temperature as above for 14 hours. The reaction mixture was concentrated under reduced pressure, and the residue was then purified by silica gel column chromatography (chloroform:methanol = 25:1). Intermediate 5 represented by the following structural formula was obtained as a yellow solid (292 mg, 0.565 mmol, 99%).

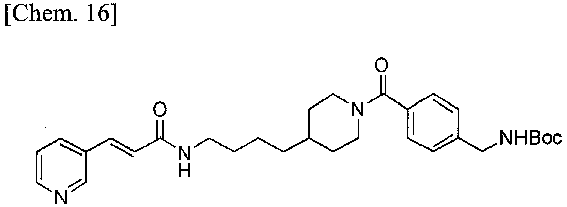

### (6) Production of compound 1

To a solution of intermediate 5 (292 mg, 0.565 mmol) in dichloromethane (4 ml), trifluoroacetic acid (2 ml) |was added under an ice bath, and the mixture was stirred at room temperature for 1 hour. The reaction mixture was concentrated under reduced pressure, and the residue was then purified by silica gel column chromatography (chloroform:methanol = 10:1). Compound 1 was obtained as a yellow solid (239 mg, 0.565 mmol, 99%). NMR measurement spectra and results of mass spectrometry by HR-ESI-MS are as follows.
¹H NMR (DMSO, 400 MHz): δ 0.97-1.53 (11H, m), 3.13-3.47 (6H, m), 3.73 (2H, s), 6.71 (1H, d, J = 16.0 Hz), 7.28 (2H, d, J = 8.0 Hz), 7.37 (2H, d, J = 8.0 Hz), 7.40-7.48 (2H, m), 7.97 (1H, d, J = 8.4 Hz), 8.16 (1H, br), 8.54 (1H, d, J = 4.8 Hz), 8.75 (1H, s); HRMS (ESI-TOF) m/z: [M + Na]⁺ calcd for C₂₅H₃₂N₄O₂Na 421.2604; Found 421.2601.

The confirmed structure is as follows.

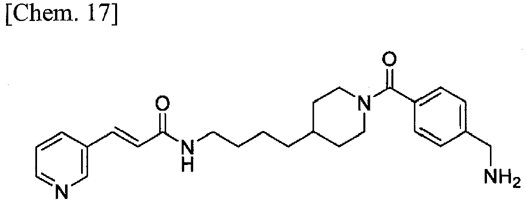

### [Example 2: Production of compound 2]

### (1) Production of intermediate 6

To a solution of t-butyl 4-(5-aminopentyl)piperidine-1-carboxylate (1.16 g, 4.29 mmol) in dichloromethane (20 ml), nicotinoyl chloride hydrochloride (1.15 g, 6.43 mmol) and triethylamine (2.99 ml, 21.4 mmol) were added at room temperature, and the mixture was stirred at the same temperature as above for 3 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 25:1). Intermediate 6 represented by the following structural formula was obtained as a yellow solid (1.61 g, 4.29 mmol, 69%).

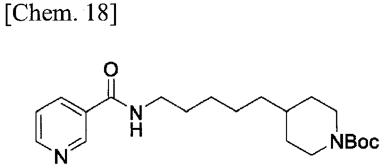

### (2) Production of intermediate 7

To a solution of intermediate 6 (516 mg, 1.37 mmol) in dichloromethane (14 ml), trifluoroacetic acid (5 ml) |was added under an ice bath, and the mixture was stirred at room temperature for 1 hour. The reaction mixture was concentrated under reduced pressure, and the residue was then purified by silica gel column chromatography (chloroform:methanol = 4:1). Intermediate 7 represented by the following structural formula was obtained as a yellow solid (266 mg, 0.966 mmol, 71%).

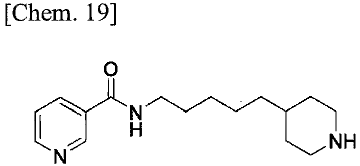

### (3) Production of compound 2

To a solution of intermediate 7 (266 mg, 0.966 mmol) in dichloromethane (14 ml), triethylamine(0.404 ml, 2.90 mmol), bromoethanol (145 mg, 1.16 mmol) |was added under an ice bath, and the mixture was stirred at room temperature for 10 hours. The reaction mixture was concentrated under reduced pressure, and the residue was then purified by silica gel column chromatography (chloroform:methanol = 8:1). Compound 2 was obtained as a yellow solid (284 mg, 0.889 mmol, 92%). NMR measurement spectra and results of mass spectrometry by HR-ESI-MS are as follows. ¹H NMR (DMSO, 400 MHz): δ 1.04-1.63 (12H, m), 1.89-2.00 (1H, m), 2.35-3.52 (10H, m), 7.50 (1H, t, J = 6.0 Hz), 8.16 (1H, d, J = 8.4 Hz), 8.63 (1H, br), 8.69 (1H, d, J = 4.4 Hz), 8.98 (1H, s); HRMS (ESI-TOF) m/z: [M + Na]⁺ calcd for C₁₈H₂₉N₃O₂Na 342.2157; Found 342.2153.

The confirmed structure is as follows.

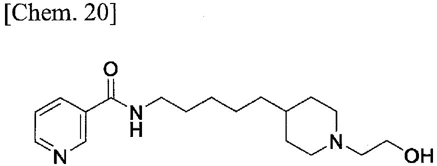

### [Example 3: Production of compound 3]

### (1) Production of intermediate 8

A known compound represented by the following structural formula (intermediate 8) was synthesized in accordance with the method described in Journal of Materials Chemistry B: Materials for Biology and Medicine, 2016, 4 (16), 2819-2827.

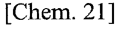

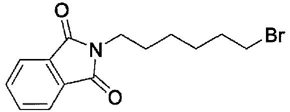

### (2) Production of intermediate 9

To a solution of intermediate 8 (3.82 g, 12.4 mmol) in dimethylformamide (15 ml), piperidineethanol (1.26 ml, 10.3 mmol) and potassium carbonate (14.2 g, 103 mmol) were added at room temperature, and the mixture was stirred at 100°C for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 25:1). Intermediate 9 represented by the following structural formula was obtained as a yellow solid (911 mg, 2.53 mmol, 25%).

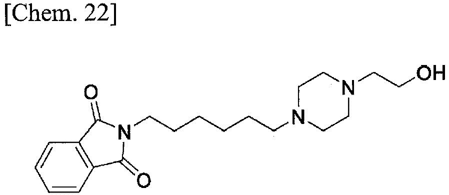

### (3) Production of intermediate 10

To a solution of intermediate 9 (911 mg, 2.53 mmol) in ethanol (50 ml), hydrazine monohydrate (0.246 ml, 5.07 mmol) was added at room temperature, and the mixture was stirred under heating to reflux for 3 hours. The reaction mixture was concentrated under reduced pressure, and the residue was then purified by silica gel column chromatography (chloroform:methanol = 4:1). Intermediate 10 represented by the following structural formula was obtained as a yellow oil (245 mg, 1.07 mmol, 42%).

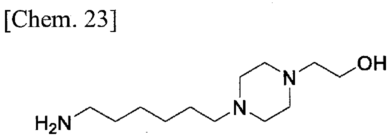

### (4) Production of compound 3

To a solution of intermediate 10 (245 mg, 1.07 mmol) in dichloromethane (10 ml), trans-3-(3-pyridyl)acrylic acid chloride hydrochloride (262 mg, 1.28 mmol) and triethylamine (0.596 ml, 4.27 mmol) were added at room temperature, and the mixture was stirred at the same temperature as above for 2 hours. The reaction mixture was concentrated under reduced pressure, and the residue was then purified by silica gel column chromatography (chloroform:methanol = 9:1). Compound 3 was obtained as a yellow solid (291 mg, 0.807 mmol, 75%). NMR measurement spectra and results of mass spectrometry by HR-ESI-MS are as follows.
¹H NMR (DMSO, 400 MHz): δ 1.22-1.50 (8H, m), 2.18-2.54 (12H, m), 3.16 (2H, q, J = 6.4 Hz), 3.46 (2H, q, J = 5.6 Hz), 4.35 (1H, t, J = 5.6 Hz), 6.72 (1H, d, J = 8.4 Hz), 7.40-7.47 (2H, m), 7.97 (1H, d, J = 8.0 Hz), 8.15 (1H, t, J = 5.2 Hz), 8.54 (1H, d, J = 5.2 Hz), 8.75 (1H, s); HRMS (ESI-TOF) m/z: [M + Na]⁺ calcd for C₂₀H₃₂N₄O₂Na 383.2423;

### Found 383.2418.

The confirmed structure is as follows.

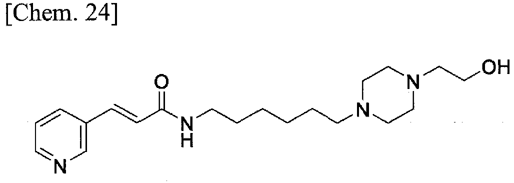

### [Example 4: Production of compound 4]

### (1) Production of intermediate 11

A known compound represented by the following structural formula (intermediate 11) was synthesized in accordance with the method described in WO2020/148153.

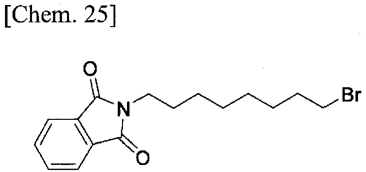

### (2) Production of intermediate 12

To a solution of intermediate 11 (5.81 g, 17.2 mmol) in dimethylformamide (50 ml), ethylene glycol (2.87 ml, 51.5 mmol) and sodium hydride (1.50 g, 34.4 mmol) were added at room temperature, and the mixture was stirred at the same temperature as above for 14 hours. A saturated aqueous solution of ammonium chloride was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (ethyl acetate:hexane = 1:1). Intermediate 12 represented by the following structural formula was obtained as a yellow oil (2.83 g, 8.86 mmol, 52%).

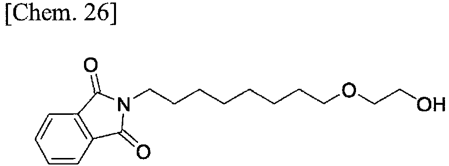

### (3) Production of intermediate 13

To a solution of intermediate 12 (2.83 g, 8.86 mmol) in ethanol (177 ml), hydrazine monohydrate (1.29 ml, 26.6 mmol) was added at room temperature, and the mixture was stirred under heating to reflux for 3 hours. The reaction mixture was concentrated under reduced pressure, and the residue was then purified by silica gel column chromatography (chloroform:methanol = 3:1). Intermediate 13 represented by the following structural formula was obtained as a yellow oil (1.43 g, 7.55 mmol, 85%).

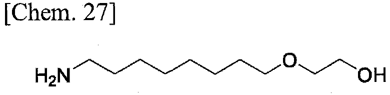

### (4) Production of compound 4

To a solution of intermediate 13 (1.43 g, 7.55 mmol) in dichloromethane (80 ml), nicotinoyl chloride hydrochloride (1.61 g, 9.06 mmol) and triethylamine (4.21 ml, 30.2 mmol) were added at room temperature, and the mixture was stirred at the same temperature as above for 6 hours. The reaction mixture was concentrated under reduced pressure, and the residue was then purified by silica gel column chromatography (chloroform:methanol = 20:1). Compound 4 was obtained as a yellow oil (2.02 g, 6.86 mmol, 92%). NMR measurement spectra and results of mass spectrometry by HR-ESI-MS are as follows.
¹H NMR (DMSO, 400 MHz): δ 1.23-1.56 (12H, m), 3.22-3.50 (8H, m), 4.56 (1H, br), 7.49 (1H, t, J = 6.4 Hz), 8.16 (1H, d, J = 7.6 Hz), 8.63 (1H, br), 8.69 (1H, d, J = 4.4 Hz), 8.98 (1H, s); HRMS (ESI-TOF) m/z: [M + Na]⁺ calcd for C₁₆H₂₆N₂O₃Na 317.1841; Found 317.1840.

The confirmed structure is as follows.

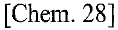

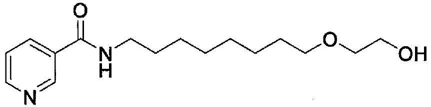

### [Example 5: Production of compound 5]

### (1) Production of intermediate 14

A known compound represented by the following structural formula (intermediate 14) was synthesized in accordance with the method described in Tetrahedron Letters, 2011, 52 (20), 2533-2535.

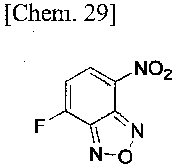

### (2) Production of compound 5

To a solution of compound 4 (161 mg, 0.546 mmol) in dichloromethane (5 ml), intermediate 14 (200 mg, 1.09 mmol) and diisopropylethylamine (0.381 ml, 2.18 mmol) were added at room temperature, and the mixture was stirred at the same temperature as above for 48 hours. The reaction mixture was concentrated under reduced pressure, and the residue was then purified by silica gel column chromatography (chloroform:methanol = 10:1). Compound 5 was obtained as a yellow solid (179 mg, 0.391 mmol, 72%). NMR measurement spectra and results of mass spectrometry by HR-ESI-MS are as follows.
¹H NMR (DMSO, 400 MHz): δ 1.22-1.55 (12H, m), 3.21-3.27 (2H, m), 3.48 (2H, t, J = 6.0 Hz), 3.82-3.86 (2H, m), 4.50-4.59 (2H, m), 7.09 (1H, d, J = 8.0 Hz), 7.46-7.52 (1H, m), 8.15 (1H, d, J = 9.2 Hz), 8.60-8.76 (3H, m), 8.97 (1H, s); HRMS (ESI-TOF) m/z: [M + Na]⁺ calcd for C₂₂H₂₇N₅O₆Na 480.1859; Found 480.1855.

The confirmed structure is as follows.

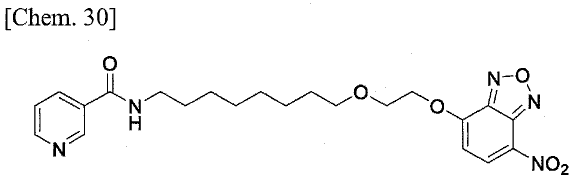

### [Example 6: Production of compound 6]

### (1) Production of intermediate 15

To a solution of intermediate 11 (2.87 g, 8.48 mmol) in dimethylformamide (25 ml), diethylene glycol (2.42 ml, 25.5 mmol) and sodium hydride (740 mg, 17.0 mmol) were added under an ice bath, and the mixture was stirred at the same temperature as above for 4 hours. A saturated aqueous solution of ammonium chloride was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (ethyl acetate:hexane = 1:9). Intermediate 15 represented by the following structural formula was obtained as a yellow oil (1.34 g, 3.69 mmol, 43%).

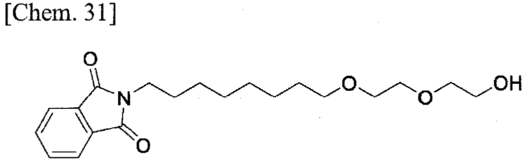

### (2) Production of intermediate 16

To a solution of intermediate 15 (1.30 g, 3.58 mmol) in ethanol (75 ml), hydrazine monohydrate (0.520 ml, 10.7 mmol) was added at room temperature, and the mixture was stirred under heating to reflux for 10 hours. The reaction mixture was concentrated under reduced pressure, and the residue was then purified by silica gel column chromatography (chloroform:methanol = 3:1). Intermediate 16 represented by the following structural formula was obtained as a yellow oil (738 mg, 3.16 mmol, 88%).

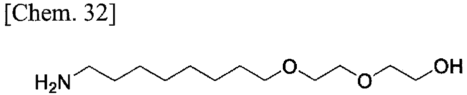

### (3) Production of compound 6

To a solution of intermediate 16 (721 mg, 3.09 mmol) in dichloromethane (40 ml), nicotinoyl chloride hydrochloride (660 mg, 3.71 mmol) and triethylamine (1.72 ml, 12.4 mmol) were added at room temperature, and the mixture was stirred at the same temperature as above for 6 hours. The reaction mixture was concentrated under reduced pressure, and the residue was then purified by silica gel column chromatography (chloroform:methanol = 15:1). Compound 6 was obtained as a yellow oil (2.02 g, 6.86 mmol, 92%). NMR measurement spectra and results of mass spectrometry by HR-ESI-MS are as follows.
¹H NMR (DMSO, 400 MHz): δ 1.24-1.57 (12H, m), 3.23-3.52 (12H, m), 4.58 (1H, t, J = 5.2 Hz), 7.50 (1H, t, J = 6.4 Hz), 8.16 (1H, d, J = 7.6 Hz), 8.63 (1H, br), 8.69 (1H, d, J = 4.8 Hz), 8.98 (1H, s); HRMS (ESI-TOF) m/z: [M + Na]⁺ calcd for C₁₈H₃₀N₂O₄Na 361.2103; Found 361.2098.

The confirmed structure is as follows.

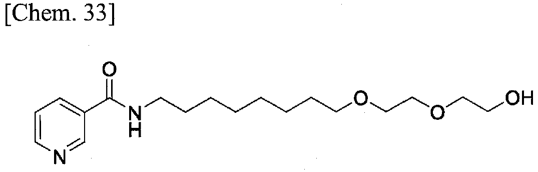

### [Example 7: Production of compound 7]

To a solution of compound 6 (185 mg, 0.546 mmol) in dichloromethane (5 ml), intermediate 14 (200 mg, 1.09 mmol) and diisopropylethylamine (0.381 ml, 2.18 mmol) were added at room temperature, and the mixture was stirred at the same temperature as above for 48 hours. The reaction mixture was concentrated under reduced pressure, and the residue was then purified by silica gel column chromatography (chloroform:methanol = 10:1). Compound 7 was obtained as a yellow solid (202 mg, 0.403 mmol, 74%). NMR measurement spectra and results of mass spectrometry by HR-ESI-MS are as follows.
¹H NMR (DMSO, 400 MHz): δ 1.18-1.33 (12H, m), 3.20-3.52 (6H, m), 3.60-3.65 (2H, m), 3.87-3.93 (2H, m), 4.55-4.59 (2H, m), 7.09 (1H, d, J = 8.4 Hz), 7.50-7.57 (1H, m), 8.17-8.24 (1H, m), 8.61-8.76 (3H, m), 8.99 (1H, s); HRMS (ESI-TOF) m/z: [M + Na]⁺ calcd for C₂₄H₃₁N₅O₇Na 524.2121; Found 524.2120.

The confirmed structure is as follows.

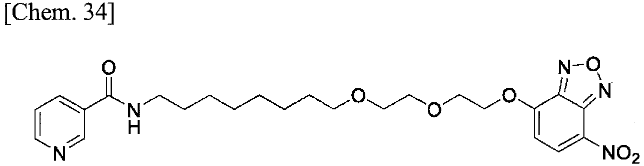

### [Example 8: Production of compound 8]

### (1) Production of intermediate 17

To a solution of intermediate 11 (5.61 g, 16.6 mmol) in dimethylformamide (60 ml), triethylene glycol (6.65 ml, 49.8 mmol) and sodium hydride (1.45 g, 33.2 mmol) were added under an ice bath, and the mixture was stirred at room temperature for 14 hours. A saturated aqueous solution of ammonium chloride was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (ethyl acetate:hexane = 4:1). Intermediate 17 represented by the following structural formula was obtained as a yellow oil (2.83 g, 6.94 mmol, 42%).

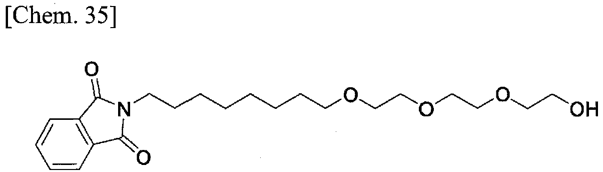

### (2) Production of intermediate 18

To a solution of intermediate 17 (1.06 g, 2.60 mmol) in ethanol (50 ml), hydrazine monohydrate (0.379 ml, 7.80 mmol) was added at room temperature, and the mixture was stirred under heating to reflux for 10 hours. The reaction mixture was concentrated under reduced pressure, and the residue was then purified by silica gel column chromatography (chloroform:methanol = 3:1). Intermediate 18 represented by the following structural formula was obtained as a yellow oil (670 mg, 2.42 mmol, 93%).

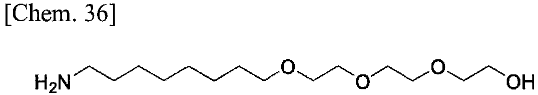

### (3) Production of compound 8

To a solution of intermediate 18 (670 mg, 2.42 mmol) in dichloromethane (25 ml), nicotinoyl chloride hydrochloride (516 mg, 2.90 mmol) and triethylamine (1.35 ml, 9.66 mmol) were added at room temperature, and the mixture was stirred at the same temperature as above for 14 hours. The reaction mixture was concentrated under reduced pressure, and the residue was then purified by silica gel column chromatography (chloroform:methanol = 20:1). Compound 8 was obtained as a yellow oil (771 mg, 2.02 mmol, 83%). NMR measurement spectra and results of mass spectrometry by HR-ESI-MS are as follows.
¹H NMR (DMSO, 400 MHz): δ 1.23-1.57 (12H, m), 3.23-3.51 (16H, m), 4.58 (1H, t, J = 5.2 Hz), 7.49 (1H, dd, J = 8.0, 4.4 Hz), 8.16 (1H, d, J = 8.0 Hz), 8.63 (1H, t, J = 5.2 Hz), 8.69 (1H, d, J = 4.8 Hz), 8.98 (1H, s); HRMS (ESI-TOF) m/z: [M + Na]⁺ calcd for C₂₀H₃₄N₂O₅Na 405.2365; Found 405.2361.

The confirmed structure is as follows.

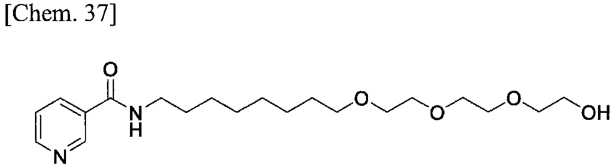

### [Example 9: Production of compound 9]

### (1) Production of intermediate 19

To a solution of compound 8 (560 mg, 1.46 mmol) in dichloromethane (5 ml), triethylamine (0.612 ml, 4.39 mmol) and mesyl chloride (0.134 ml, 1.76 mmol) were added under an ice bath, and the mixture was stirred at room temperature for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 20:1). Intermediate 19 represented by the following structural formula was obtained as a yellow oil (643 mg, 1.39 mmol, 95%).

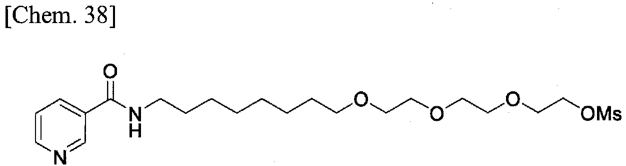

### (2) Production of intermediate 20

To a solution of intermediate 19 (643 mg, 1.39 mmol) in dimethylformamide (5 ml), sodium azide (109 mg, 1.67 mmol) was added at room temperature, and the mixture was stirred at 80°C for 16 hours. The reaction mixture was concentrated under reduced pressure, and the residue was then purified by silica gel column chromatography (chloroform:methanol = 25:1). Intermediate 20 represented by the following structural formula was obtained as a yellow oil (566 mg, 1.39 mmol, 99%).

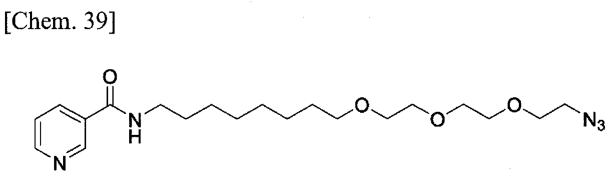

### (3) Production of compound 9

To a solution of intermediate 20 (595 mg, 1.46 mmol) in tetrahydrofuran (7.3 ml), triphenylphosphine (421 mg, 1.61 mmol) was added at room temperature, and the mixture was stirred at room temperature for 24 hours. Water (0.0788 ml, 4.38 mmol) was added at the same temperature as above, and the mixture was stirred at room temperature for 24 hours. The reaction mixture was concentrated under reduced pressure, and the residue was then purified by silica gel column chromatography (chloroform:methanol = 7:1). Compound 9 was obtained as a yellow oil (474 mg, 1.24 mmol, 85%). NMR measurement spectra and results of mass spectrometry by HR-ESI-MS are as follows.
¹H NMR (DMSO, 400 MHz): δ 1.22-1.56 (12H, m), 3.21-3.60 (16H, m), 7.47-7.52 (1H, m), 8.16 (1H, d, J = 7.6 Hz), 8.60-8.73 (2H, m), 8.98 (1H, s); HRMS (ESI-TOF) m/z: [M + Na]⁺ calcd for C₂₀H₃₅N₃O₄Na 404.2525; Found 404.2520.

The confirmed structure is as follows.

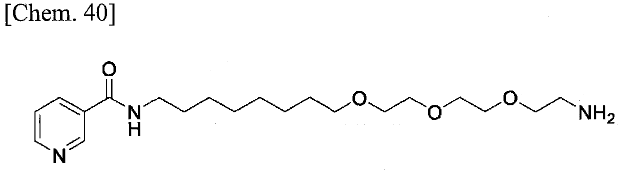

### [Example 10: Production of compound 10]

To a solution of compound 8 (209 mg, 0.546 mmol) in dichloromethane (5 ml), intermediate 14 (200 mg, 1.09 mmol) and diisopropylethylamine (0.381 ml, 2.18 mmol) were added at room temperature, and the mixture was stirred at the same temperature as above for 48 hours. The reaction mixture was concentrated under reduced pressure, and the residue was then purified by silica gel column chromatography (chloroform:methanol = 20:1). Compound 10 was obtained as a yellow solid (166 mg, 0.297 mmol, 54%). NMR measurement spectra and results of mass spectrometry by HR-ESI-MS are as follows.
¹H NMR (DMSO, 400 MHz): δ 1.20-1.56 (12H, m), 3.20-3.63 (12H, m), 3.88-3.93 (2H, m), 4.56-4.60 (2H, m), 7.09 (1H, d, J = 8.8 Hz), 7.48-7.53 (1H, m), 8.14-8.20 (1H, m), 8.60-8.76 (3H, m), 8.98 (1H, s); HRMS (ESI-TOF) m/z: [M + Na]⁺ calcd for C₂₆H₃₅N₅O₈Na 568.2383; Found 568.2386.

The confirmed structure is as follows.

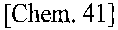

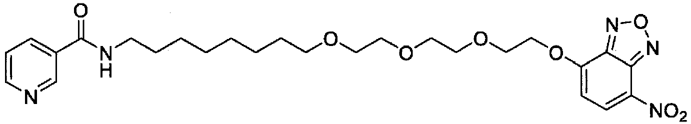

### [Example 11: Production of compound 11]

To a solution of 2-[2-(2-propyn-1-yloxy)ethoxy]ethanamine (50.0 mg, 0.349 mmol) in dichloromethane (3 ml), nicotinoyl chloride hydrochloride (74.7 mg, 0.420 mmol) and triethylamine (0.120 ml, 0.861 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 10:1). SYK-79 was obtained as a yellow oil (81.4 mg, 0.328 mmol, 94%). NMR measurement spectra and results of mass spectrometry by HR-ESI-MS are as follows.
¹H NMR (CDCl₃, 400 MHz): δ 2.44 (1H, t, J = 2.4 Hz), 3.68-3.73 (8H, m), 4.20 (2H, d, J = 2.4 Hz), 6.86 (1H, br), 7.39 (1H, dd, J = 7.6, 4.0 Hz), 8.14 (1H, dt, J = 8.0, 2.0 Hz), 8.72 (1H, dd, J = 4.8, 1.6 Hz), 9.01 (1H, d, J = 1.6 Hz); HRMS (ESI-TOF) m/z: [M + Na]⁺ calcd for C₁₃H₁₆N₂O₃Na 271.1059; Found 271.1060.

The confirmed structure is as follows.

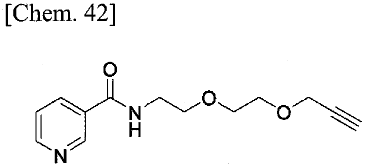

### [Example 12: Production of compound 12]

### (1) Production of intermediate 21

To a solution of trans-3-(3-pyridyl)acrylic acid (100 mg, 0.670 mmol) in dichloromethane (6 ml), t-butyl (3-aminopropyl)-carbamate (140 mg, 0.803 mmol), N,N-diisopropylethylamine (0.240 ml, 1.38 mmol), 1-[bis(dimethylamino)methylene]-1H-1,2,3-triazolo[4,5-b]pyridinium3-oxide hexafluorophosphate (HATU) (510 mg, 1.34 mmol), and dimethylaminopyridine (16.0 mg, 0.132 mmol) were added at room temperature, and the mixture was stirred at the same temperature as above for 2 hours. A saturated aqueous solution of sodium bicarbonate was added to the reaction mixture, followed by extraction with dichloromethane. The organic layer was washed with saturated saline, dried over anhydrous magnesium sulfate, then filtered, and concentrated under reduced pressure. The residue was purified by silica gel column chromatography (chloroform:methanol = 5:1). Intermediate 21 represented by the following structural formula was obtained as a yellow oil (187 mg, 0.612 mmol, 91%). HRMS (ESI-TOF) m/z: [M + Na]⁺ calcd for C₁₆H₂₃N₃O₃Na 328.1637; Found 328.1637.

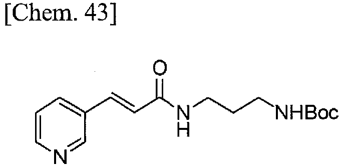

### (2) Production of intermediate 22

To a solution of intermediate 21 (230 mg, 0.753 mmol) in dichloromethane (5 ml), hydrochloric acid (4 M solution in dioxane, 3.8 ml) was added at room temperature, and the mixture was stirred at room temperature for 1 hour. The reaction mixture was concentrated under reduced pressure. Intermediate 22 represented by the following structural formula was obtained as a pale yellow solid (148 mg, 0.721 mmol, 96%).
HRMS (ESI-TOF) m/z: [M + H]⁺ calcd for C₁₁H₁₆N₃O 206.1293; Found 206.1289.

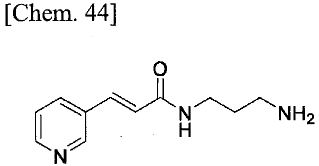

### (3) Production of compound 12

To a solution of intermediate 22 (45.0 mg, 0.219 mmol) in methanol (1.5 ml), 1-benzoyl-4-piperidone (445 mg, 2.19 mmol), sodium triacetoxyborohydride (93.0 mg, 0.439 mmol), and acetic acid (1 ml) were added at 60°C, and the mixture was stirred at the same temperature as above for 14 hours. A saturated aqueous solution of sodium bicarbonate was added to the reaction mixture, followed by extraction with dichloromethane. The organic layer was washed with saturated saline, dried over anhydrous magnesium sulfate, then filtered, and concentrated under reduced pressure. The residue was purified by silica gel column chromatography (chloroform:methanol = 5:1). Compound 12 was obtained as a yellow oil (5.00 mg, 0.0127 mmol, 5.8%). NMR measurement spectra and results of mass spectrometry by HR-ESI-MS are as follows.
¹H NMR (CD₃OD, 400 MHz): δ 1.35-2.20 (6H, m), 2.85-3.46 (5H, m), 3.73-3.86 (2H, m), 4.62-4.74 (2H, m), 6.75 (1H, d, J = 16.4 Hz), 7.38-7.52 (6H, m), 7.59 (1H, d, J = 16.4 Hz), 8.06 (1H, d, J = 7.6 Hz), 8.53 (1H, s), 8.72 (1H, s); HRMS (ESI-TOF) m/z: [M + H]⁺ calcd for C₂₃H₂₉N₄O₂ 393.2291; Found 393.2283.

The confirmed structure is as follows.

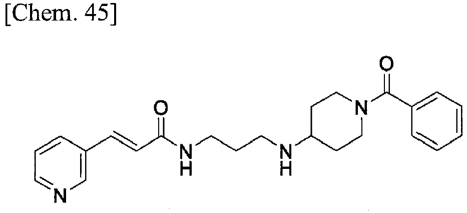

### [Example 13: Production of compound 13]

### (1) Production of intermediate 23

A known compound represented by the following structural formula (intermediate 23) was synthesized in accordance with the method described in European Journal of Organic Chemistry, 2020, 32, 5153-5160.

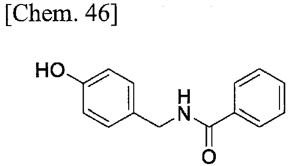

### (2) Production of intermediate 24

A known compound represented by the following structural formula (intermediate 24) was synthesized in accordance with the method described in Dalton Transactions, 2017, 46 (47), 16387-16389.

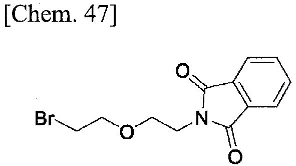

### (3) Production of intermediate 25

To a solution of intermediate 23 (830 mg, 3.65 mmol) in dimethylformamide (20 ml), intermediate 24 (1.50 g, 5.03 mmol) and potassium carbonate (690 mg, 4.99 mmol) were added at room temperature, and the mixture was stirred at 80°C for 20 hours. Water was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (100% ethyl acetate). Intermediate 25 represented by the following structural formula was obtained as a yellow solid (1.20 g, 2.70 mmol, 74%).
¹H NMR (DMSO, 400 MHz): δ 3.65-3.78 (6H, m), 3.96-3.99 (2H, m), 4.38 (2H, d, J = 6.0 Hz), 6.78 (2H, d, J = 8.8 Hz), 7.17 (2H, d, J = 8.8 Hz), 7.43-7.55 (3H, m), 7.79-7.90 (6H, m), 8.97 (1H, t, J = 6.0 Hz).

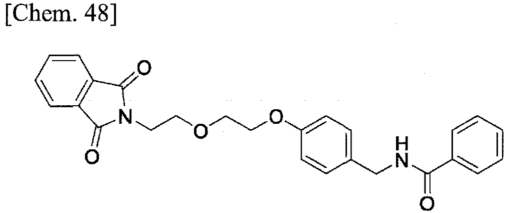

### (4) Production of intermediate 26

To a solution of intermediate 25 (1.20 g, 2.70 mmol) in ethanol (5.4 ml), hydrazine monohydrate (0.650 ml, 13.5 mmol) was added at room temperature, and the mixture was stirred at 80°C for 14 hours. The reaction mixture was concentrated under reduced pressure, and the residue was then purified by silica gel column chromatography (chloroform:methanol = 1:1, ammonia water:methanol = 5:95). Intermediate 26 represented by the following structural formula was obtained as a yellow oil (400 mg, 1.27 mmol, 47%).
¹H NMR (DMSO, 400 MHz): δ 2.63-2.68 (2H, m), 3.48 (2H, q, J = 5.2 Hz), 3.68-3.72 (2H, m), 4.04-4.08 (2H, m), 4.40 (2H, d, J = 6.0 Hz), 6.89 (2H, d, J = 8.8 Hz), 7.23 (2H, d, J = 8.8 Hz), 7.43-7.55 (3H, m), 7.87 (2H, d, J = 6.8 Hz).

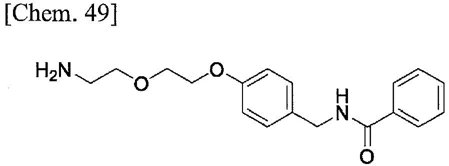

### (5) Production of compound 13

To a solution of intermediate 26 (130 mg, 0.413 mmol) in dichloromethane (15 ml), nicotinic acid (102 mg, 0.829 mmol), 1-(3-dimethylaminopropyl)-3-ethylcarbodiimide hydrochloride(158 mg, 0.824 mmol), and dimethylaminopyridine (10.1 mg, 0.0827 mmol) were added at room temperature, and the mixture was stirred at the same temperature as above for 24 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 13 was obtained as a yellow solid (167 mg, 0.398 mmol, 96%). NMR measurement spectra and results of mass spectrometry by HR-ESI-MS are as follows.
¹H NMR (DMSO, 400 MHz): δ 3.46 (2H, q, J = 6.0 Hz), 3.61 (2H, t, J = 6.0 Hz), 3.75 (2H, t, J = 4.8 Hz), 4.06 (2H, t, J = 4.8 Hz), 4.40 (2H, d, J = 6.0 Hz), 6.88 (2H, d, J = 8.8 Hz), 7.22 (2H, d, J = 8.8 Hz), 7.44-7.55 (4H, m), 7.86-7.89 (2H, m), 8.16 (1H, dt, J = 8.0, 2.0 Hz), 8.68 (1H, dd, J = 4.8, 2.4 Hz), 8.77 (1H, t, J = 5.6 Hz), 8.96-9.01 (2H, m); HRMS (ESI-TOF) m/z: [M + Na]⁺ calcd for C₂₄H₂₅N₃O₄Na 442.1743; Found 442.1735.

The confirmed structure is as follows.

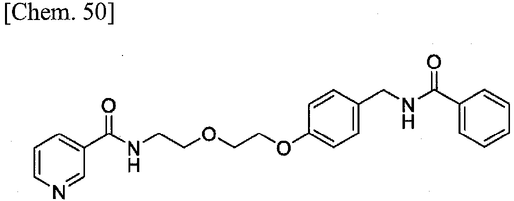

### [Example 14: Production of compound 14]

To a solution of intermediate 26 (130 mg, 0.413 mmol) in dichloromethane (15 ml), trans-3-(3-pyridyl)acrylic acid (123 mg, 0.825 mmol), 1-(3-dimethylaminopropyl)-3-ethylcarbodiimide hydrochloride (158 mg, 0.824 mmol), and dimethylaminopyridine (10.1 mg, 0.0827 mmol) were added at room temperature, and the mixture was stirred at the same temperature as above for 24 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 14 was obtained as a yellow solid (180 mg, 0.404 mmol, 98%). NMR measurement spectra and results of mass spectrometry by HR-ESI-MS are as follows.
¹H NMR (DMSO, 400 MHz): δ 3.37 (2H, q, J = 5.6 Hz), 3.54 (2H, t, J = 5.6 Hz), 3.74 (2H, t, J = 4.8 Hz), 4.07 (2H, t, J = 4.8 Hz), 4.39 (2H, d, J = 6.0 Hz), 6.77 (1H, d, J = 16.0 Hz), 6.90 (2H, d, J = 8.4 Hz), 7.22 (2H, d, J = 8.4 Hz), 7.41-7.55 (5H, m), 7.87 (2H, d, J = 7.2 Hz), 7.96 (1H, dt, J = 7.6, 2.0 Hz), 8.28 (1H, t, J = 5.6 Hz), 8.54 (1H, dd, J = 4.8, 1.6 Hz), 8.74 (1H, d, J = 2.0 Hz), 8.98 (1H, t, J = 2.0 Hz); HRMS (ESI-TOF) m/z: [M + Na]⁺ calcd for C₂₆H₂₇N₃O₄Na 468.1899; Found 468.1896.

The confirmed structure is as follows.

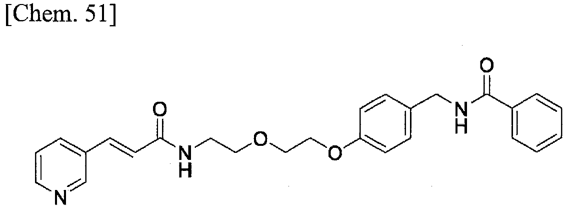

### [Example 15: Production of compound 15]

### (1) Production of intermediate 27

A known compound represented by the following structural formula (intermediate 27) was synthesized in accordance with the method described in European Journal of Medicinal Chemistry, 2018, 158, 51-67.

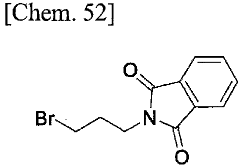

### (2) Production of intermediate 28

To a solution of intermediate 23 (932 mg, 4.10 mmol) in dimethylformamide (10 ml), intermediate 27 (1.00 g, 3.73 mmol), potassium carbonate (773 mg, 5.59 mmol), and sodium iodide (112 mg, 0.747 mmol) were added at room temperature, and the mixture was stirred at 60°C for 24 hours. Water was added to the reaction mixture, followed by extraction with ethyl acetate. The organic layer was washed with saturated saline, dried over anhydrous magnesium sulfate, then filtered, and concentrated under reduced pressure. Intermediate 28 represented by the following structural formula was obtained as a yellow solid (1.54 g, 3.72 mmol, 99%).
¹H NMR (DMSO, 400 MHz): δ 1.98-2.09 (2H, m), 3.71-3.78 (2H, m), 3.94-4.01 (2H, m), 4.36 (2H, d, J = 6.0 Hz), 6.74 (2H, d, J = 6.4 Hz), 7.18 (2H, d, J = 6.4 Hz), 7.43-7.55 (3H, m), 7.80-7.89 (6H, m), 8.97 (1H, br).

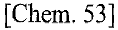

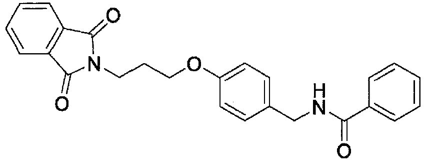

### (3) Production of intermediate 29

To a solution of intermediate 28 (2.00 g, 4.82 mmol) in ethanol (60 ml), hydrazine monohydrate (1.17 ml, 24.1 mmol) was added at room temperature, and the mixture was stirred at 80°C for 20 hours. The reaction mixture was concentrated under reduced pressure, and the residue was then purified by silica gel column chromatography (chloroform:methanol = 9:1, ammonia water:methanol = 10:90). Intermediate 29 represented by the following structural formula was obtained as a yellow oil (600 mg, 2.11 mmol, 44%).
¹H NMR (DMSO, 400 MHz): δ1.73-1.80 (2H, m), 2.68 (2H, t, J = 6.4 Hz), 3.99 (2H, t, J = 6.4 Hz), 4.40 (2H, d, J = 6.0 Hz), 6.87 (2H, d, J = 8.4 Hz), 7.23 (2H, d, J = 8.4 Hz), 7.44-7.55 (3H, m), 7.87 (2H, d, J = 6.8 Hz).

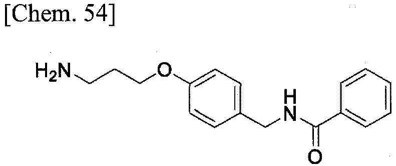

### (4) Production of compound 15

To a solution of intermediate 29 (200 mg, 0.704 mmol) in dichloromethane (15 ml), nicotinic acid (102 mg, 0.829 mmol), 1-(3-dimethylaminopropyl)-3-ethylcarbodiimide hydrochloride (158 mg, 0.824 mmol), and dimethylaminopyridine (10.1 mg, 0.0827 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 98:2). Compound 15 was obtained as a yellow solid (210 mg, 0.540 mmol, 73%). NMR measurement spectra and results of mass spectrometry by HR-ESI-MS are as follows.
¹H NMR (DMSO, 400 MHz): δ 1.97 (2H, q, J = 6.4 Hz), 3.43 (2H, q, J = 6.0 Hz), 4.02 (2H, t, J = 6.0 Hz), 4.40 (2H, d, J = 6.0 Hz), 6.89 (2H, d, J = 8.4 Hz), 7.23 (2H, d, J = 8.4 Hz), 7.43-7.55 (4H, m), 7.87 (2H, d, J = 7.2 Hz), 8.17 (1H, dt, J =8.0, 2.0 Hz), 8.69 (1H, dd, J = 4.8, 1.6 Hz), 8.74 (1H, t, J = 5.6 Hz), 8.96-9.01 (2H, m); HRMS (ESI-TOF) m/z: [M + Na]⁺ calcd for C₂₃H₂₃N₃O₃Na 412.1637; Found 412.1631.

The confirmed structure is as follows.

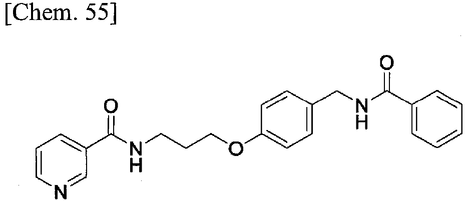

### [Example 16: Production of compound 16]

To a solution of intermediate 29 (200 mg, 0.704 mmol) in dichloromethane (15 ml), trans-3-(3-pyridyl)acrylic acid(209 mg, 1.40 mmol), 1-(3-dimethylaminopropyl)-3-ethylcarbodiimide hydrochloride (268 mg, 1.40 mmol) and dimethylaminopyridine (17.1 mg, 0.140 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 96:4). Compound 16 was obtained as a yellow solid (50.0 mg, 0.120 mmol, 17%). NMR measurement spectra and results of mass spectrometry by HR-ESI-MS are as follows.
¹H NMR (CDCl₃, 400 MHz): δ 1.90 (2H, q, J = 6.4 Hz), 3.34 (2H, q, J = 6.0 Hz), 3.99 (2H, t, J = 6.0 Hz), 4.40 (2H, d, J = 6.4 Hz), 6.72 (1H, d, J = 8.4 Hz), 6.89 (2H, d, J = 8.8 Hz), 7.24 (2H, d, J = 8.8 Hz), 7.41-7.55 (5H, m), 7.87 (2H, d, J = 8.0 Hz), 7.97 (1H, dt, J = 7.6, 2.0 Hz), 8.28 (1H, t, J = 5.6 Hz), 8.54 (1H, dd, J = 4.8, 2.0 Hz), 8.75 (1H, d, J = 2.4 Hz), 8.98 (1H, t, J = 6.0 Hz); HRMS (ESI-TOF) m/z: [M + Na]⁺ calcd for C₂₅H₂₅N₃O₃Na 438.1794; Found 438.1790.

The confirmed structure is as follows.

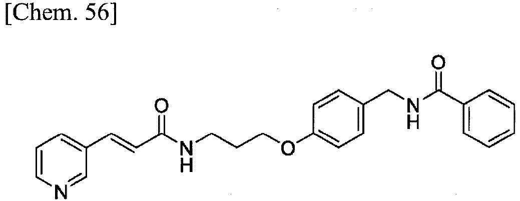

### [Example 17: Production of compound 17]

### (1) Production of intermediate 30

A known compound represented by the following structural formula (intermediate 30) was synthesized in accordance with the method described in WO97/02242.

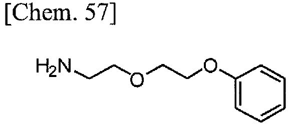

### (2) Production of compound 17

To a solution of intermediate 30 (75.0 mg, 0.414 mmol) in dichloromethane (4 ml), trans-3-(3-pyridyl)acrylic acid (123 mg, 0.825 mmol), 1-(3-dimethylaminopropyl)-3-ethylcarbodiimide hydrochloride (159 mg, 0.829 mmol), and dimethylaminopyridine (10.1 mg, 0.0827 mmol) were added at room temperature, and the mixture was stirred at the same temperature as above for 9 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 17 was obtained as a yellow solid (86.5 mg, 0.277 mmol, 67%). NMR measurement spectra and results of mass spectrometry by HR-ESI-MS are as follows.
¹H NMR (DMSO, 400 MHz): δ 3.37 (2H, q, J = 6.0 Hz), 3.55 (2H, t, J = 5.6 Hz), 3.76 (2H, t, J = 4.4 Hz), 4.09 (2H, t, J = 4.4 Hz), 6.77 (1H, d, J = 8.0 Hz), 6.89-6.96 (3H, m), 7.25-7.29 (2H, m), 7.42-7.48 (2H, m), 7.97 (1H, dt, J = 8.4, 2.0 Hz), 8.28 (1H, t, J = 5.2 Hz), 8.55 (1H, dd, J = 4.8, 1.6 Hz), 8.74 (1H, d, J = 2.0 Hz); HRMS (ESI-TOF) m/z: [M + Na]⁺ calcd for C₁₈H₂₀N₂O₃Na 335.1372; Found 335.1370.

The confirmed structure is as follows.

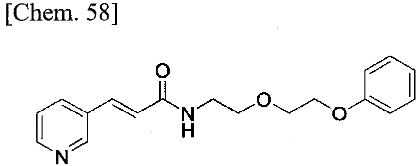

### [Example 18: Production of compound 18]

### (1) Production of intermediate 31

A known compound represented by the following structural formula (intermediate 31) was synthesized in accordance with the method described in WO2015/071657.

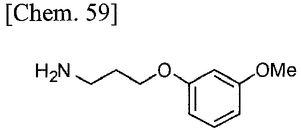

### (2) Production of compound 18

To a solution of intermediate 31 (100 mg, 0.552 mmol) in dichloromethane (5 ml), trans-3-(3-pyridyl)acrylic acid (164 mg, 1.10 mmol), 1-(3-dimethylaminopropyl)-3-ethylcarbodiimide hydrochloride (211 mg, 1.10 mmol), and dimethylaminopyridine (13.4 mg, 0.110 mmol) were added at room temperature, and the mixture was stirred at the same temperature as above for 20 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 18 was obtained as a yellow solid (72.0 mg, 0.230 mmol, 41%). NMR measurement spectra and results of mass spectrometry by HR-ESI-MS are as follows.
¹H NMR (DMSO, 400 MHz): δ1.90 (2H, q, J = 6.4 Hz), 3.33 (2H, t, J = 6.8 Hz), 3.72 (3H, s), 4.00 (2H, t, J = 6.4 Hz), 6.47-6.54 (3H, m), 6.72 (1H, d, J = 16.0 Hz), 7.17 (1H, t, J = 8.0 Hz), 7.42-7.48 (2H, m), 7.98 (1H, dt, J = 8.0, 2.0 Hz), 8.29 (1H, t, J = 5.6 Hz), 8.55 (1H, dd, J = 4.8, 1.6 Hz), 8.75 (1H, d, J = 2.4 Hz); HRMS (ESI-TOF) m/z: [M + Na]⁺ calcd for C₁₈H₂₀N₂O₃Na 335.1372; Found 335.1368.

The confirmed structure is as follows.

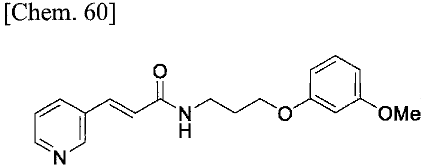

### [Example 19: Production of compound 19]

### (1) Production of intermediate 32

A known compound represented by the following structural formula (intermediate 32) was synthesized in accordance with the method described in New Journal of Chemistry, 2021, 45 (12), 5621-5630.

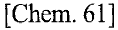

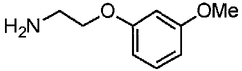

### (2) Production of compound 19

To a solution of intermediate 32 (55.0 mg, 0.329 mmol) in dichloromethane (3 ml), trans-3-(3-pyridyl)acrylic acid (98 mg, 0.657 mmol), 1-(3-dimethylaminopropyl)-3-ethylcarbodiimide hydrochloride (126 mg, 0.657 mmol), and dimethylaminopyridine (8.00 mg, 0.0655 mmol) were added at room temperature, and the mixture was stirred at the same temperature as above for 20 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 19 was obtained as a yellow solid (63.6 mg, 0.213 mmol, 65%). NMR measurement spectra and results of mass spectrometry by HR-ESI-MS are as follows.
¹H NMR (DMSO, 400 MHz): δ 3.56 (2H, q, J = 5.6 Hz), 3.73 (3H, s), 4.04 (2H, t, J = 5.6 Hz), 6.50-6.56 (3H, m), 6.79 (1H, d, J = 16.0 Hz), 7.18 (1H, t, J = 8.4 Hz), 7.42-7.51 (2H, m), 7.98 (1H, d, J = 8.4 Hz), 8.45 (1H, t, J = 5.2 Hz), 8.55 (1H, d, J = 5.2 Hz), 8.76 (1H, d, J = 1.6 Hz); HRMS (ESI-TOF) m/z: [M + Na]⁺ calcd for C₁₇H₁₈N₂O₃Na 321.1215; Found 321.1209.

The confirmed structure is as follows.

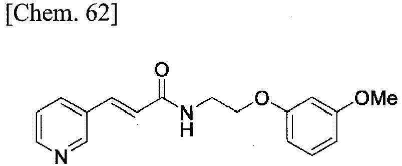

### [Example 20: Production of compound 20]

### (1) Production of intermediate 33

A known compound represented by the following structural formula (intermediate 33) was synthesized in accordance with the method described in Journal of Medicinal Chemistry, 2008, 51 (24), 7800-7805.

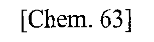

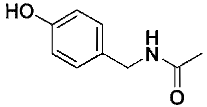

### (2) Production of intermediate 34

To a solution of intermediate 33 (677 mg, 4.10 mmol) in dimethylformamide (10 ml), intermediate 27 (1.00 g, 3.73 mmol), potassium carbonate (773 mg, 5.59 mmol), and sodium iodide (112 mg, 0.747 mmol) were| added at room temperature, and the mixture was stirred at 60°C for 9 hours. Water was added to the reaction mixture, followed by extraction with ethyl acetate. The organic layer was washed with saturated saline, dried over anhydrous magnesium sulfate, then filtered, and concentrated under reduced pressure. Intermediate 34 represented by the following structural formula was obtained as a yellow solid (1.30 g, 3.69 mmol, 99%).
¹H NMR (DMSO, 400 MHz): δ 1.84 (3H, s), 2.01-2.07 (2H, m), 3.75 (2H, t, J = 6.8 Hz), 3.98 (2H, t, J = 6.8 Hz), 4.14 (2H, d, J = 6.0 Hz), 6.73 (2H, d, J = 8.8 Hz), 7.10 (2H, d, J = 8.8 Hz), 7.80-7.89 (4H, m).

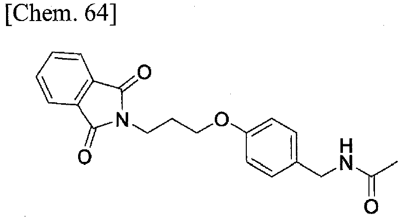

### (3) Production of intermediate 35

To a solution of intermediate 34 (1.30 g, 3.69 mmol) in ethanol (7.4 ml), hydrazine monohydrate (0.900 ml, 18.5 mmol) was added at room temperature, and the mixture was stirred at 80°C for 17 hours. The reaction mixture was concentrated under reduced pressure, and the residue was then purified by silica gel column chromatography (chloroform:methanol = 9:1, ammonia water:methanol = 10:90). Intermediate 35 represented by the following structural formula was obtained as a yellow oil (600 mg, 2.70 mmol, 73%).
¹H NMR (DMSO, 400 MHz): δ 1.80-1.88 (2H, m), 1.84 (3H, s), 2.78 (2H, t, J = 7.2 Hz), 4.00 (2H, t, J = 7.2 Hz), 4.15 (2H, d, J = 6.0 Hz), 6.86 (2H, d, J = 8.8 Hz), 7.15 (2H, d, J = 8.8 Hz).

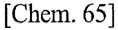

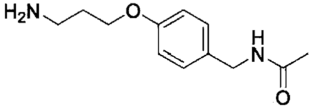

### (4) Production of compound 20

To a solution of intermediate 35 (270 mg, 1.21 mmol) in dichloromethane (12 ml), nicotinic acid (299 mg, 2.43 mmol), 1-(3-dimethylaminopropyl)-3-ethylcarbodiimide hydrochloride (464 mg, 2.42 mmol), and dimethylaminopyridine (29.6 mg, 0.242 mmol) were added at room temperature, and the mixture was stirred at the same temperature as above for 16 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 20 was obtained as a yellow solid (5.00 mg, 0.0153 mmol, 1.3%). NMR measurement spectra and results of mass spectrometry by HR-ESI-MS are as follows.
¹H NMR (DMSO, 400 MHz): δ 1.84 (3H, s), 1.97 (2H, q, J = 6.4 Hz), 3.43 (2H, q, J =6.0 Hz), 4.02 (2H, t, J = 6.0 Hz), 4.16 (2H, d, J = 5.6 Hz), 6.87 (2H, d, J = 8.4 Hz), 7.15 (2H, d, J = 8.4 Hz), 7.50 (1H, dd, J = 8.0, 5.2 Hz), 8.17 (1H, dt, J = 8.4, 2.0 Hz), 8.25 (1H, t, J = 5.2 Hz), 8.69 (1H, dd, J = 4.8, 2.0 Hz), 8.74 (1H, t, J = 2.4 Hz), 8.99 (1H, d, J = 2.4 Hz); HRMS (ESI-TOF) m/z: [M + Na]⁺ calcd for C₁₈H₂₁N₃O₃Na 350.1481; Found 350.1474.

The confirmed structure is as follows.

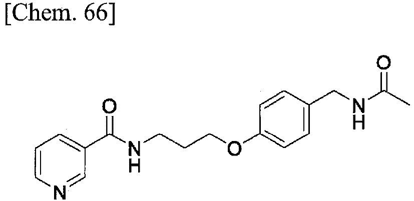

### [Example 21: Production of compound 21]

### (1) Production of intermediate 36

To a solution of intermediate 33 (609 mg, 3.69 mmol) in dimethylformamide (10 ml), intermediate 24 (1.00 g, 3.35 mmol), potassium carbonate (694 mg, 5.02 mmol), and sodium iodide (100 mg, 0.667 mmol) were added at room temperature, and the mixture was stirred at 60°C for 4 hours. Water was added to the reaction mixture, followed by extraction with ethyl acetate. The organic layer was washed with saturated saline, dried over anhydrous magnesium sulfate, then filtered, and concentrated under reduced pressure. Intermediate 36 represented by the following structural formula was obtained as a yellow solid (1.28 g, 3.35 mmol, 99%).
¹H NMR (CDCl₃, 400 MHz): δ 2.00 (3H, s), 3.72-3.85 (6H, m), 3.89-3.04 (2H, m), 4.33 (2H, d, J = 5.6 Hz), 6.81 (2H, d, J = 8.4 Hz), 7.14 (2H, d, J = 8.4 Hz), 7.69-7.73 (2H, m), 7.80-7.87 (2H, m).

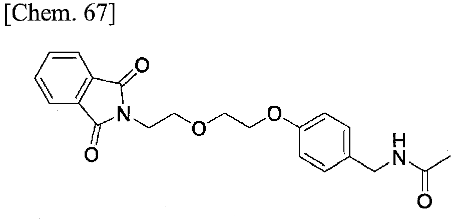

### (2) Production of intermediate 37

To a solution of intermediate 36 (1.30 g, 3.40 mmol) in ethanol (34 ml), hydrazine monohydrate (0.830 ml, 17.0 mmol) was added at room temperature, and the mixture was stirred at 80°C for 14 hours. The reaction mixture was concentrated under reduced pressure, and the residue was then purified by silica gel column chromatography (chloroform:methanol = 9:1, methanol 100%). Intermediate 37 represented by the following structural formula was obtained as a yellow oil (510 mg, 2.02 mmol, 59%).
¹H NMR (DMSO, 400 MHz): δ 1.94 (3H, s), 2.78-2.84 (2H, m), 3.50-3.55 (2H, m), 3.73-3.80 (2H, m), 4.04-4.09 (2H, m), 4.27 (2H, d, J = 5.6 Hz), 6.83 (2H, d, J = 8.4 Hz), 7.14 (2H, d, J = 8.4 Hz).

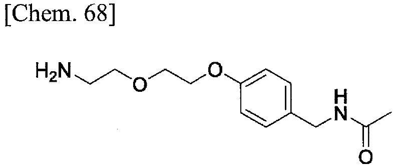

### (3) Production of compound 21

To a solution of intermediate 37 (200 mg, 0.793 mmol) in dichloromethane (15 ml), nicotinic acid (195 mg, 1.58 mmol), 1-(3-dimethylaminopropyl)-3-ethylcarbodiimide hydrochloride (303 mg, 1.58 mmol), and dimethylaminopyridine (19.3 mg, 0.158 mmol) were added at room temperature, and the mixture was stirred at the same temperature as above for 22 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 21 was obtained as a yellow solid (18.0 mg, 0.0504 mmol, 6.4%). NMR measurement spectra and results of mass spectrometry by HR-ESI-MS are as follows.
¹H NMR (CDCl₃, 400 MHz): δ 2.03 (3H, s), 3.67-3.76 (4H, m), 3.86 (2H, t, J = 3.2 Hz), 4.17 (2H, t, J =2.4 Hz), 4.36 (2H, d, J = 5.6 Hz), 6.52 (1H, br), 6.58 (1H, br), 6.88 (2H, d, J = 7.2 Hz), 7.19 (2H, d, J = 7.2 Hz), 7.37 (1H, dd, J = 7.2, 5.6 Hz), 8.13 (1H, d, J = 8.0 Hz), 8.59 (1H, s), 8.68 (1H, d, J = 4.4 Hz); HRMS (ESI-TOF) m/z: [M + Na]⁺ calcd for C₁₉H₂₃N₃O₄Na 380.1586; Found 380.1580.

The confirmed structure is as follows.

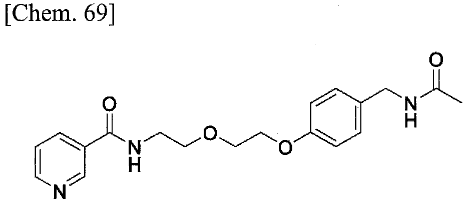

### [Example 22: Production of compound 22]

To a solution of intermediate 37 (200 mg, 0.793 mmol) in dichloromethane (3 ml), trans-3-(3-pyridyl)acrylic acid (236 mg, 1.58 mmol), 1-(3-dimethylaminopropyl)-3-ethylcarbodiimide hydrochloride (303 mg, 1.58 mmol), and dimethylaminopyridine (19.3 mg, 0.158 mmol) were added at room temperature, and the mixture was stirred at the same temperature as above for 22 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 22 was obtained as a yellow solid (20.0 mg, 0.0522 mmol, 6.6%). NMR measurement spectra and results of mass spectrometry by HR-ESI-MS are as follows.
¹H NMR (CDCl₃, 400 MHz): δ 2.06 (3H, s), 3.62 (2H, q, J = 4.8 Hz), 3.76 (2H, t, J = 4.8 Hz), 3.90 (2H, t, J = 4.4 Hz), 4.15 (2H, t, J = 4.4 Hz), 4.40 (2H, d, J = 4.8 Hz), 6.26 (1H, d, J = 15.6 Hz), 6.54 (1H, br), 6.94 (2H, d, J = 8.0 Hz), 7.22-7.57 (5H, m), 7.93 (1H, br), 8.24 (1H, s), 8.56 (1H, d, J = 4.4 Hz); HRMS (ESI-TOF) m/z: [M + Na]⁺ calcd for C₂₁H₂₅N₃O₄Na 406.1743; Found 406.1739.

The confirmed structure is as follows.

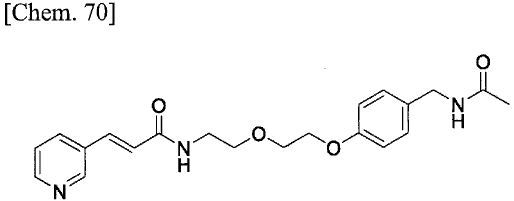

### [Example 23: Production of compound 25]

### (1) Production of intermediate 38

A known compound represented by the following structural formula (intermediate 38) was synthesized in accordance with the method described in WO2011/146845.

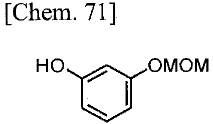

### (2) Production of intermediate 39

To a solution of intermediate 38 (948 mg, 6.15 mmol) in dimethylformamide (15 ml), intermediate 27 (1.50 g, 5.59 mmol) and potassium carbonate (1.16 g, 8.39 mmol) were added at room temperature, and the mixture was stirred at 80°C for 17 hours. Water was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (hexane:ethyl acetate = 2:1). Intermediate 39 represented by the following structural formula was obtained as a yellow solid (1.31 g, 3.83 mmol, 69%).
¹H NMR (CDCl₃, 400 MHz): δ 2.17 (2H, q, J = 6.8 Hz), 3.46 (3H, s), 3.90 (2H, t, J = 6.8 Hz), 4.01 (2H, t, J = 6.8 Hz), 5.13 (2H, s), 6.46 (1H, d, J = 8.4 Hz), 6.51 (1H, s), 6.61 (1H, d, J = 8.4 Hz), 7.13 (1H, t, J = 8.4 Hz), 7.70-7.74 (2H, m), 7.83-7.86 (2H, m).

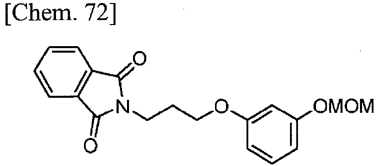

### (3) Production of intermediate 40

To a solution of intermediate 39 (1.31 g, 3.83 mmol) in ethanol (10 ml), hydrazine monohydrate (0.372 ml, 7.66 mmol) was added at room temperature, and the mixture was stirred at 80°C for 14 hours. After filtration through Celite, the reaction mixture was concentrated under reduced pressure. Intermediate 40 represented by the following structural formula was obtained as a yellow oil (809 mg, 3.83 mmol, 99%). ¹H NMR (DMSO, 400 MHz): δ 1.80 (2H, q, J = 6.4 Hz), 2.72 (2H, t, J = 6.4 Hz), 3.36 (3H, s), 3.99 (2H, t, J = 6.4 Hz), 5.16 (2H, s), 7.16 (2H, t, J = 8.0 Hz), 7.78-7.82 (1H, m), 8.04-8.07 (1H, m).

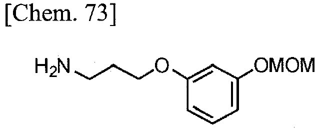

### (4) Production of compound 25

To a solution of intermediate 40 (150 mg, 0.710 mmol) in dichloromethane (5 ml), nicotinic acid (175 mg, 1.42 mmol), 1-(3-dimethylaminopropyl)-3-ethylcarbodiimide hydrochloride (272 mg, 1.42 mmol), and dimethylaminopyridine (17.3 mg, 0.142 mmol) were added at room temperature, and the mixture was stirred at the same temperature as above for 19 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 25 was obtained as a yellow solid (143 mg, 0.452 mmol, 64%). NMR measurement spectra and results of mass spectrometry by HR-ESI-MS are as follows.
¹H NMR (DMSO, 400 MHz): δ 1.97 (2H, q, J = 6.4 Hz), 3.36 (3H, s), 3.44 (2H, q, J = 6.4 Hz), 4.02 (2H, t, J = 6.4 Hz), 5.16 (2H, s), 6.55-6.61 (3H, m), 7.18 (1H, t, J = 8.0 Hz), 7.50 (1H, dd, J = 8.0, 4.8 Hz), 8.18 (1H, dt, J = 6.4, 2.0 Hz), 8.69 (1H, dd, J = 4.8, 1.6 Hz), 8.76 (1H, t, J = 5.6 Hz), 8.99 (1H, d, J = 2.0 Hz); HRMS (ESI-TOF) m/z: [M + Na]⁺ calcd for C₁₇H₂₀N₂O₄Na 339.1321; Found 339.1314.

The confirmed structure is as follows.

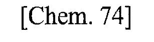

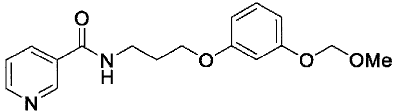

### [Example 24: Production of compound 24]

To a solution of intermediate 40 (150 mg, 1.41 mmol) in dichloromethane (5 ml), trans-3-(3-pyridyl)acrylic acid (212 mg, 1.42 mmol), 1-(3-dimethylaminopropyl)-3-ethylcarbodiimide hydrochloride (272 mg, 1.42 mmol), and dimethylaminopyridine (17.3 mg, 0.142 mmol) were added at room temperature, and the mixture was stirred at the same temperature as above for 19 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 24 was obtained as a yellow solid (152 mg, 0.444 mmol, 63%). NMR measurement spectra and results of mass spectrometry by HR-ESI-MS are as follows.
¹H NMR (DMSO, 400 MHz): δ 1.90 (2H, q, J = 2.4 Hz), 3.34 (2H, q, J = 6.4 Hz), 3.36 (3H, s), 3.99 (2H, t, J = 6.4 Hz), 5.16 (2H, s), 6.56-6.61 (3H, m), 6.72 (1H, d, J = 16.0 Hz), 7.18 (1H, t, J = 8.0 Hz), 7.42-7.48 (2H, m), 7.98 (1H, dt, J = 8.0, 2.0 Hz), 8.29 (1H, t, J = 5.6 Hz), 8.55 (1H, dd, J = 4.8, 1.6 Hz), 8.75 (1H, d, J = 2.4 Hz); HRMS (ESI-TOF) m/z: [M + Na]⁺ calcd for C₁₉H₂₂N₂O₄Na 365.1477; Found 365.1473.

The confirmed structure is as follows.

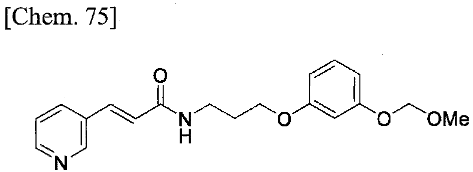

### [Example 25: Production of compound 23]

To a solution of compound 24 (50.0 mg, 0.146 mmol) in acetonitrile (0.7 ml), trimethylsilyl iodide (0.0400 ml, 0.292 mmol) was added at room temperature, and the mixture was stirred at the same temperature as above for 3 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. Compound 23 was obtained as a yellow solid (16.2 mg, 0.0543 mmol, 37%). NMR measurement spectra and results of mass spectrometry by HR-ESI-MS are as follows.
¹H NMR (DMSO, 400 MHz): δ 1.89 (2H, q, J = 6.4 Hz), 3.34 (2H, q, J = 6.4 Hz), 3.94 (2H, t, J = 6.4 Hz), 6.30-6.38 (2H, m), 6.72 (1H, d, J = 16.0 Hz), 7.04 (1H, t, J = 8.0 Hz), 7.41-7.49 (2H, m), 7.98 (1H, d, J = 7.6 Hz), 8.29 (1H, t, J = 5.6 Hz), 8.54 (1H, d, J = 4.4 Hz), 8.75 (1H, s), 9.38 (1H, s); HRMS (ESI-TOF) m/z: [M + H]⁺ calcd for C₁₇H₁₉N₂O₃ 299.1396; Found 299.1394.

The confirmed structure is as follows.

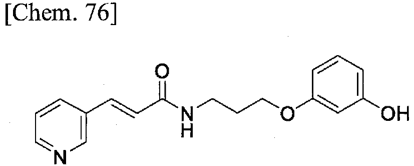

### [Example 26: Production of compound 30]

### (1) Production of intermediate 41

To a solution of intermediate 38 (853 mg, 5.53 mmol) in dimethylformamide (20 ml), intermediate 24 (1.50 g, 5.03 mmol), potassium carbonate (1.04 g, 7.55 mmol), and sodium iodide (150 mg, 1.00 mmol) were added at room temperature, and the mixture was stirred at 80°C for 16 hours. Water was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (hexane:ethyl acetate = 2:1). Intermediate 41 represented by the following structural formula was obtained as a yellow solid (402 mg, 1.08 mmol, 22%). ¹H NMR (CDCl₃, 400 MHz): δ 3.46 (3H, s), 3.80-3.86 (4H, m), 3.91-3.96 (2H, m), 4.02-4.06 (2H, m), 5.14 (2H, s), 6.49 (1H, d, J = 8.4 Hz), 6.55 (1H, s), 6.60 (1H, d, J = 8.4 Hz), 7.12 (1H, t, J = 8.4 Hz), 7.68-7.72 (2H, m), 7.81-7.85 (2H, m).

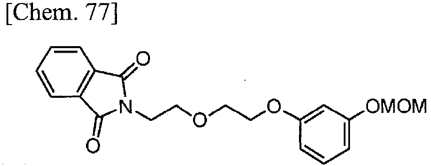

### (2) Production of intermediate 42

To a solution of intermediate 41 (400 mg, 1.08 mmol) in ethanol (10 ml), hydrazine monohydrate (0.100 ml, 2.15 mmol) was added at room temperature, and the mixture was stirred at 80°C for 14 hours. After filtration through Celite, the reaction mixture was concentrated under reduced pressure. Intermediate 42 represented by the following structural formula was obtained as a yellow oil (260 mg, 1.08 mmol, 99%). ¹H NMR (DMSO, 400 MHz): δ 2.68 (2H, t, J = 6.0 Hz), 3.36 (3H, s), 3.43 (2H, t, J = 6.0 Hz), 3.70 (2H, t, J = 4.4 Hz), 4.06 (2H, t, J = 4.4 Hz), 5.16 (2H, s), 6.55-6.62 (1H, m), 7.15-7.20 (1H, m), 7.83-7.88 (1H, m), 8.04-8.08 (1H, m).

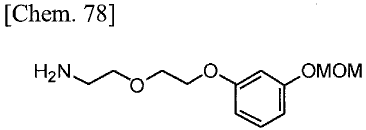

### (3) Production of compound 30

To a solution of intermediate 42 (150 mg, 0.622 mmol) in dichloromethane (6 ml), nicotinic acid (153 mg, 1.24 mmol), 1-(3-dimethylaminopropyl)-3-ethylcarbodiimide hydrochloride (238 mg, 1.24 mmol), and dimethylaminopyridine (15.1 mg, 0.124 mmol) were added at room temperature, and the mixture was stirred at the same temperature as above for 7 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 30 was obtained as a yellow solid (90.0 mg, 0.260 mmol, 42%). NMR measurement spectra and results of mass spectrometry by HR-ESI-MS are as follows.
¹H NMR (DMSO, 400 MHz): δ 3.35 (3H, s), 3.46 (2H, q, J = 6.0 Hz), 3.61 (2H, t, J = 6.0 Hz), 3.75 (2H, t, J = 4.4 Hz), 4.06 (2H, t, J = 4.4 Hz), 5.15 (2H, s), 6.54-6.61 (3H, m), 7.16 (1H, t, J = 8.8 Hz), 7.49 (1H, dd, J = 8.0, 4.8 Hz), 8.17 (1H, dt, J = 7.6, 2.0 Hz), 8.69 (1H, d, J = 5.2 Hz), 8.77 (1H, t, J = 5.2 Hz), 8.99 (1H, d, J = 2.0 Hz); HRMS (ESI-TOF) m/z: [M + Na]⁺ calcd for C₁₈H₂₂N₂O₅Na 369.1426; Found 369.1424.

The confirmed structure is as follows.

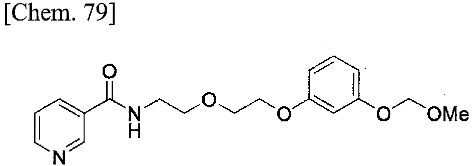

### [Example 27: Production of compound 29]

To a solution of intermediate 42 (150 mg, 0.622 mmol) in dichloromethane (6 ml), trans-3-(3-pyridyl)acrylic acid (185 mg, 1.24 mmol), 1-(3-dimethylaminopropyl)-3-ethylcarbodiimide hydrochloride (238 mg, 1.24 mmol), and dimethylaminopyridine (15.1 mg, 0.124 mmol) were added at room temperature, and the mixture was stirred at the same temperature as above for 15 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 29 was obtained as a yellow solid (118 mg, 0.318 mmol, 51%). NMR measurement spectra and results of mass spectrometry by HR-ESI-MS are as follows.
¹H NMR (DMSO, 400 MHz): δ 3.46 (3H, s), 3.48 (2H, q, J = 6.0 Hz), 3.66 (2H, t, J = 5.6 Hz), 3.85 (2H, t, J = 4.4 Hz), 4.18 (2H, t, J = 4.4 Hz), 5.26 (2H, s), 6.66-6.71 (3H, m), 6.88 (1H, d, J = 7.6 Hz), 7.28 (1H, t, J = 8.8 Hz), 7.52-7.59 (2H, m), 8.07 (1H, d, J = 8.0 Hz), 8.38 (1H, t, J = 5.6 Hz), 8.65 (1H, d, J = 4.8 Hz), 8.85 (1H, s); HRMS (ESI-TOF) m/z: [M + Na]⁺ calcd for C₂₀H₂₄N₂O₅Na 395.1583; Found 395.1580.

The confirmed structure is as follows.

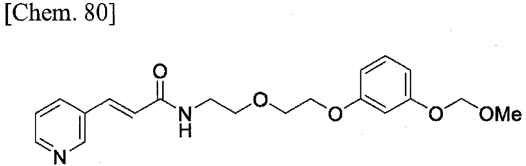

### [Example 28: Production of compound 27]

To a solution of compound 30 (27 mg, 0.146 mmol) in acetonitrile (0.5 ml), trimethylsilyl iodide (0.0210 ml, 0.156 mmol) was added at room temperature, and the mixture was stirred at the same temperature as above for 1 hour. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. Compound 27 was obtained as a yellow solid (20.0 mg, 0.662 mmol, 85%). NMR measurement spectra and results of mass spectrometry by HR-ESI-MS are as follows.
¹H NMR (DMSO, 400 MHz): δ 3.46 (2H, q, J = 6.0 Hz), 3.60 (2H, t, J = 6.0 Hz), 3.73 (2H, t, J = 4.4 Hz), 4.01 (2H, t, J = 4.4 Hz), 6.28-6.36 (3H, m), 7.02 (1H, t, J = 8.0 Hz), 7.49 (1H, dd, J = 8.0, 4.8 Hz), 8.17 (1H, d, J = 7.6 Hz), 8.69 (1H, d, J = 4.8 Hz), 8.77 (1H, t, J = 5.6 Hz), 8.99 (1H, s), 9.39 (1H, s); HRMS (ESI-TOF) m/z: [M + Na]⁺ calcd for C₁₆H₁₈N₂O₄Na 325.1164; Found 325.1162.

The confirmed structure is as follows.

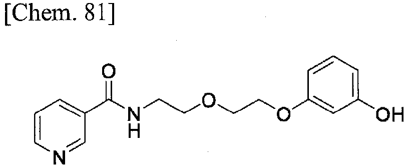

### [Example 29: Production of compound 26]

To a solution of compound 29 (46 mg, 0.124 mmol) in acetonitrile (0.7 ml), trimethylsilyl iodide (0.0340 ml, 0.248 mmol) was added at room temperature, and the mixture was stirred at the same temperature as above for 1 hour. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. Compound 26 was obtained as a yellow solid (27.2 mg, 0.0828 mmol, 67%). NMR measurement spectra and results of mass spectrometry by HR-ESI-MS are as follows.
¹H NMR (DMSO, 400 MHz): δ 3.37 (2H, q, J = 5.6 Hz), 3.54 (2H, t, J = 5.6 Hz), 3.73 (2H, t, J = 4.8 Hz), 4.02 (2H, t, J = 4.8 Hz), 6.30-6.37 (3H, m), 6.77 (1H, d, J = 16.0 Hz), 7.03 (1H, t, J = 8.0 Hz), 7.42-7.48 (2H, m), 7.97 (1H, dt, J = 6.0, 2.0 Hz), 8.27 (1H, t, J = 6.0 Hz), 8.55 (1H, dd, J = 4.8, 1.6 Hz), 8.75 (1H, d, J = 2.0 Hz), 9.38 (1H, s); HRMS (ESI-TOF) m/z: [M + Na]⁺ calcd for C₁₈H₂₀N₂O₄Na 351.1321; Found 351.1317.

The confirmed structure is as follows.

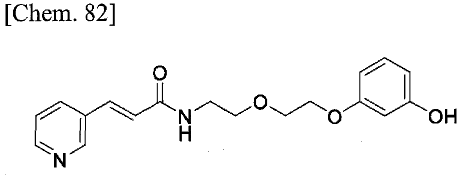

### [Example 30: Production of compound 28]

### (1) Production of intermediate 43

To a solution of 3-methoxyphenol (0.400 ml, 3.69 mmol) in dimethylformamide (11 ml), intermediate 24 (1.00 g, 3.35 mmol), potassium carbonate (695 mg, 5.03 mmol), and sodium iodide (100 mg, 0.667 mmol) were added at room temperature, and the mixture was stirred at 80°C for 14 hours. Water was added to the reaction mixture, followed by extraction with ethyl acetate. The organic layer was washed with saturated saline, dried over anhydrous magnesium sulfate, then filtered, and concentrated under reduced pressure. Intermediate 43 represented by the following structural formula was obtained as a yellow solid (569 mg, 1.67 mmol, 50%). ¹H NMR (CDCl₃, 400 MHz): δ3.76 (3H, s), 3.80-3.86 (4H, m), 3.93 (2H, t, J = 5.2 Hz), 4.05 (2H, t, J = 4.8 Hz), 6.40-6.49 (3H, m), 7.12 (1H, t, J = 8.0 Hz), 7.67-7.72 (2H, m), 7.80-7.85 (2H, m).

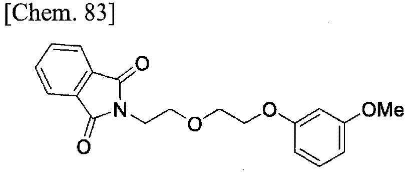

### (2) Production of intermediate 44

To a solution of intermediate 43 (569 mg, 1.67 mmol) in ethanol (17 ml), hydrazine monohydrate (0.240 ml, 4.94 mmol) was added at room temperature, and the mixture was stirred at 80°C for 4 hours. After filtration through Celite, the reaction mixture was concentrated under reduced pressure. Intermediate 44 represented by the following structural formula was obtained as a yellow oil (352 mg, 1.67 mmol, 99%). ¹H NMR (DMSO, 400 MHz): δ2.66 (2H, t, J = 5.6 Hz), 3.41 (2H, t, J = 6.0 Hz), 3.70 (2H, t, J = 4.4 Hz), 4.06 (2H, t, J = 4.4 Hz), 6.47-6.54 (2H, m), 7.12-7.28 (2H, m).

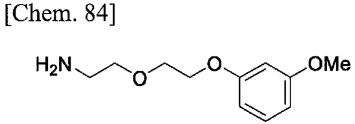

### (3) Production of compound 28

To a solution of intermediate 44 (181 mg, 0.857 mmol) in dichloromethane (5 ml), trans-3-(3-pyridyl)acrylic acid (255 mg, 1.71 mmol), 1-(3-dimethylaminopropyl)-3-ethylcarbodiimide hydrochloride (328 mg, 1.71 mmol), and dimethylaminopyridine (20.9 mg, 0.171 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 28 was obtained as a yellow solid (182 mg, 0.532 mmol, 62%). NMR measurement spectra and results of mass spectrometry by HR-ESI-MS are as follows.
¹H NMR (DMSO, 400 MHz): δ 3.37 (2H, q, J = 6.0 Hz), 3.55 (2H, t, J = 6.0 Hz), 3.71 (3H, s), 3.74 (2H, t, J = 4.8 Hz), 4.08 (2H, t, J = 4.8 Hz), 6.48-6.54 (3H, m), 6.77 (1H, d, J = 16.0 Hz), 7.16 (1H, t, J = 8.4 Hz), 7.42-7.48 (2H, m), 7.96 (1H, dt, J = 7.6, 2.0 Hz), 8.27 (1H, t, J = 5.6 Hz), 8.55 (1H, dd, J = 4.8, 1.6 Hz), 8.74 (1H, d, J = 2.0 Hz); HRMS (ESI-TOF) m/z: [M + Na]⁺ calcd for C₁₉H₂₂N₂O₄Na 365.1477; Found 365.1475.

The confirmed structure is as follows.

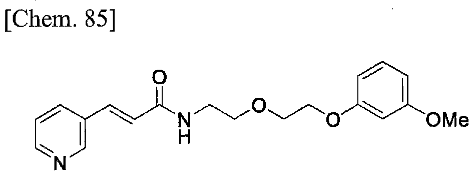

### [Example 31: Production of compound 32]

### (1) Production of intermediate 45

To a solution of methyl 4-hydroxybenzoate (1.64 g, 10.8 mmol) in dimethylformamide (25 ml), intermediate 24 (2.00 g, 6.71 mmol), potassium carbonate (1.39 g, 10.1 mmol), and sodium iodide (201 mg, 1.34 mmol) were added at room temperature, and the mixture was stirred at 60°C for 24 hours. Water was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (100% ethyl acetate). Intermediate 45 represented by the following structural formula was obtained as a yellow solid (3.50 g, 9.48 mmol, 88%). ¹H NMR (CDCl₃, 400 MHz): δ 3.80-3.89 (4H, m), 3.88 (3H, s), 3.93 (2H, t, J = 6.0 Hz), 4.09-4.15 (2H, m), 6.83 (2H, d, J = 8.8 Hz), 7.67-7.71 (2H, m), 7.80-7.83 (2H, m), 7.91 (2H, d, J = 8.8 Hz).

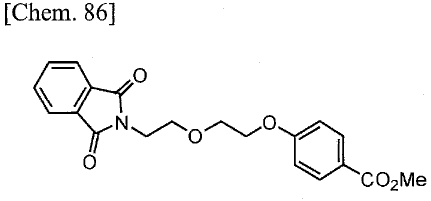

### (2) Production of intermediate 46

To a solution of intermediate 45 (2.50 g, 6.77 mmol) in ethanol (68 ml), hydrazine monohydrate (1.65 ml, 33.9 mmol) was added at room temperature, and the mixture was stirred at 80°C for 1 hour. After filtration through Celite, the reaction mixture was concentrated under reduced pressure. The residue was purified by silica gel column chromatography (chloroform:methanol = 1:1, ammonia water:methanol = 10:90). Intermediate 46 represented by the following structural formula was obtained as a yellow oil (1.60 g, 6.60 mmol, 99%).
¹H NMR (CDCl₃, 400 MHz): δ 2.88 (2H, t, J = 5.2 Hz), 3.57 (2H, t, J = 5.2 Hz), 3.83 (2H, t, J = 4.8 Hz), 3.87 (3H, s), 4.17 (2H, t, J = 4.8 Hz), 6.92 (2H, d, J = 8.8 Hz), 7.97 (2H, d, J = 8.8 Hz).

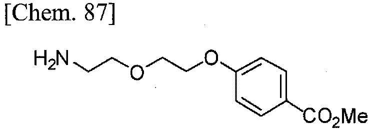

### (3) Production of compound 32

To a solution of intermediate 46 (800 mg, 3.34 mmol) in dimethylformamide (10 ml), nicotinic acid (823 mg, 6.69 mmol), 1-(3-dimethylaminopropyl)-3-ethylcarbodiimide hydrochloride (1.28 g, 6.68 mmol), and dimethylaminopyridine (81.6 mg, 0.668 mmol) were added at room temperature, and the mixture was stirred at the same temperature as above for 18 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 32 was obtained as a yellow solid (40.0 mg, 0.116 mmol, 3.5%). NMR measurement spectra and results of mass spectrometry by HR-ESI-MS are as follows.
¹H NMR (DMSO, 400 MHz): δ 3.56 (2H, q, J = 6.0 Hz), 3.72 (2H, t, J = 6.0 Hz), 3.89 (2H, t, J = 4.4 Hz), 3.91 (3H, s), 4.29 (2H, t, J = 4.4 Hz), 7.13 (2H, d, J = 8.8 Hz), 7.59 (1H, dd, J = 8.0, 4.8 Hz), 7.98 (2H, d, J = 8.8 Hz), 8.26 (1H, dt, J = 8.0, 2.0 Hz), 8.79 (1H, dd, J = 4.8, 2.0 Hz), 8.87 (1H, t, J = 4.8 Hz), 9.09 (1H, dd, J = 2.4, 0.8 Hz); HRMS (ESI-TOF) m/z: [M + Na]⁺ calcd for C₁₈H₂₀N₂O₅Na 367.1270; Found 367.1263.

The confirmed structure is as follows.

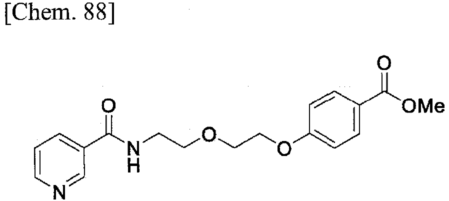

### [Example 32: Production of compound 31]

To a solution of intermediate 46 (800 mg, 3.34 mmol) in dimethylformamide (10 ml), trans-3-(3-pyridyl)acrylic acid (996 mg, 6.68 mmol), 1-(3-dimethylaminopropyl)-3-ethylcarbodiimide hydrochloride (1.28 g, 6.68 mmol), and dimethylaminopyridine (81.6 mg, 0.668 mmol) were added at room temperature, and the mixture was stirred at the same temperature as above for 19 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 93:7). Compound 31 was obtained as a yellow solid (400 mg, 1.08 mmol, 32%). NMR measurement spectra and results of mass spectrometry by HR-ESI-MS are as follows.
¹H NMR (DMSO, 400 MHz): δ 3.37 (2H, q, J = 6.0 Hz), 3.56 (2H, t, J = 6.0 Hz), 3.78 (2H, t, J = 4.4 Hz), 3.79 (3H, s), 4.19 (2H, t, J = 4.4 Hz), 6.75 (1H, d, J = 16.0 Hz), 7.05 (2H, d, J = 8.8 Hz), 7.41-7.47 (2H, m), 7.88 (2H, d, J = 8.8 Hz), 7.95 (1H, dt, J = 8.0, 2.0 Hz), 8.27 (1H, t, J = 5.6 Hz), 8.54 (1H, dd, J = 4.8, 1.6 Hz), 8.74 (1H, d, J = 2.4 Hz); HRMS (ESI-TOF) m/z: [M + Na]⁺ calcd for C₂₀H₂₂N₂O₅Na 393.1426; Found 393.1419.

The confirmed structure is as follows.

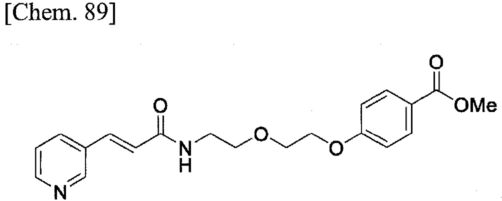

### [Example 33: Production of compound 33]

To a solution of 3-aminophenol (500 mg, 4.58 mmol) in dichloromethane (10 ml), trans-3-(3-pyridyl)acrylic acid (683 mg, 4.58 mmol), 1-(3-dimethylaminopropyl)-3-ethylcarbodiimide hydrochloride (2.63 g, 13.7 mmol), and dimethylaminopyridine (112 mg, 0.916 mmol) were added at room temperature, and the mixture was stirred at the same temperature as above for 25 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 92:8). Compound 33 was obtained as a yellow solid (23.1 mg, 0.0961 mmol, 2.1%). NMR measurement spectra and results of mass spectrometry by HR-ESI-MS are as follows.
¹H NMR (CDCl₃, 400 MHz): δ 6.64 (1H, d, J = 16.0 Hz), 6.71 (1H, d, J =16.8 Hz), 6.96 (1H, d, J = 6.8 Hz), 7.32-7.43 (4H, m), 7.64-7.95 (6H, m), 8.63 (2H, dd, J = 24.0, 4.0 Hz), 8.81 (2H, d, J = 8.4 Hz); HRMS (ESI-TOF) m/z: [M + Na]⁺ calcd for C₂₂H₁₇N₃O₃Na 394.1168; Found 394.1166.

The confirmed structure is as follows.

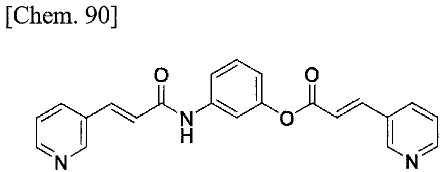

### [Example 34: Production of compound 34]

To a solution of 2,2'-(ethane-1,2-diylbis(oxy))bis(ethan-1-amine) (100 mg, 0.675 mmol) in dichloromethane (3 ml), nicotinoyl chloride hydrochloride (300 mg, 1.69 mmol) and triethylamine (0.376 ml, 2.70 mmol) were added under an ice bath, and the mixture was stirred at the same temperature as above for 1 hour. Water was added to the reaction mixture, followed by extraction with chloroform. The organic layer was washed with saturated saline, dried over anhydrous magnesium sulfate, then filtered, and concentrated under reduced pressure. Compound 34 was obtained as a yellow solid (129 mg, 0.360 mmol, 53%). NMR measurement spectra and results of mass spectrometry by HR-ESI-MS are as follows.
¹H NMR (CDCl₃, 400 MHz): δ3.64-3.76 (12H, m), 7.46 (2H, dd, J =7.6, 4.8 Hz), 7.71 (2H, br), 8.30 (2H, d, J = 7.6 Hz), 8.71 (2H, d, J = 5.2 Hz), 9.22 (2H, s); HRMS (ESI-TOF) m/z: [M + Na]⁺ calcd for C₁₈H₂₂N₄O₄Na 381.1539; Found 381.1531.

The confirmed structure is as follows.

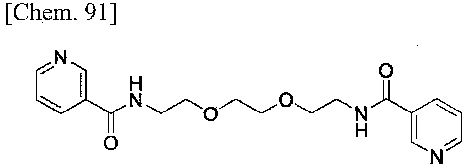

### [Example 35: Production of compound 35]

To a solution of 3,3'-(ethane-1,2-diylbis(oxy))bis(propan-1-amine) (100 mg, 0.567 mmol) in dichloromethane (2 ml), triethylamine (0.32 ml, 2.27 mmol) and nicotinoyl chloride hydrochloride (252 mg, 1.42 mmol) were added under an ice bath, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. Compound 35 was obtained as a yellow solid (18.2 mg, 0.0471 mmol, 8.3%). NMR measurement spectra and results of mass spectrometry by HR-ESI-MS are as follows.
¹H NMR (CDCl₃, 400 MHz): δ 1.91 (4H, q, J = 6.0 Hz), 3.61 (4H, q, J = 5.2 Hz), 3.66 (4H, s), 3.70 (4H, t, J = 5.6 Hz), 7.41 (2H, dd, J = 8.0, 4.8 Hz), 8.01 (2H, br), 8.28 (2H, dt, J = 7.6, 2.0 Hz), 8.60 (2H, dd, J = 4.8, 1.6 Hz), 9.06 (2H, d, J = 1.6 Hz); HRMS (ESI-TOF) m/z: [M + H]⁺ calcd for C₂₀H₂₇N₄O₄ 387.2032; Found 387.2026.

The confirmed structure is as follows.

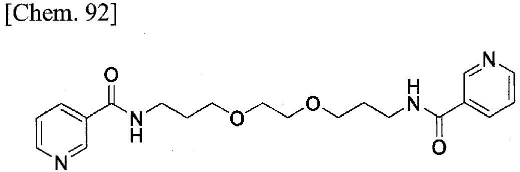

### [Example 36: Production of compound 36]

To a solution of N-(2-aminoethyl)ethane-1,2-diamine (1.00 g, 9.69 mmol) in dichloromethane (20 ml), triethylamine (5.40 ml, 38.8 mmol) and nicotinoyl chloride hydrochloride (3.62 g, 20.4 mmol) were added under an ice bath, and the mixture was stirred at the same temperature as above for 6 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 36 was obtained as a yellow solid (74.8 mg, 0.179 mmol, 1.8%). NMR measurement spectra and results of mass spectrometry by HR-ESI-MS are as follows.
¹H NMR (CDCl₃, 400 MHz): δ 3.59-3.95 (8H, m), 7.17 (1H, t, J = 6.0 Hz), 7.37-7.48 (3H, m), 7.69 (1H, br), 8.10-8.26 (3H, m), 8.44 (1H, s), 8.57 (1H, d, J = 4.8 Hz), 8.72 (2H, s), 9.15 (2H, s); HRMS (ESI-TOF) m/z: [M + Na]⁺ calcd for C₂₂H₂₂N₆O₃Na 441.1651; Found 441.1645.

The confirmed structure is as follows.

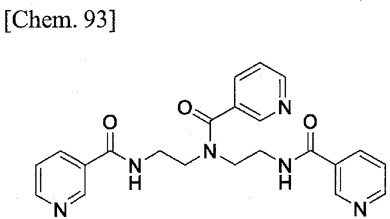

### [Example 37: Production of compound 37]

To a solution of 2,2'-oxybis(ethan-1-amine)(200 mg, 1.92 mmol) in dichloroethane (19 ml), trans-3-(3-pyridyl)acrylic acid (604 mg, 4.05 mmol), 1-(3-dimethylaminopropyl)-3-ethylcarbodiimide hydrochloride (1.10 g, 5.74 mmol), and dimethylaminopyridine (47.0 mg, 0.385 mmol) were added at room temperature, and the mixture was stirred at 60°C for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 37 was obtained as a yellow solid (16.0 mg, 0.0437 mmol, 2.3%). NMR measurement spectra and results of mass spectrometry by HR-ESI-MS are as follows.
¹H NMR (CDCl₃, 400 MHz): δ 3.56-3.64 (8H, m), 6.61 (2H, d, J = 16.0 Hz), 7.01 (2H, t, J = 4.8 Hz), 7.23 (2H, dd, J = 8.0, 4.8 Hz), 7.59 (2H, d, J = 16.0 Hz), 7.70 (2H, dt, J = 7.6, 2.0 Hz), 8.51 (2H, dd, J = 4.8, 1.6 Hz), 8.68 (2H, d, J = 2.4 Hz); HRMS (ESI-TOF) m/z: [M + Na]⁺ calcd for C₂₀H₂₂N₄O₃Na 389.1590; Found 389.1587.

The confirmed structure is as follows.

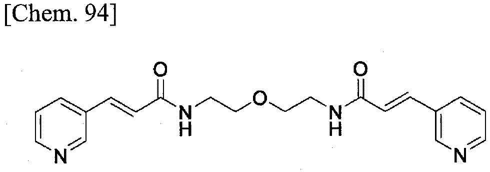

### [Example 38: Production of compound 38]

To a solution of hexane-1,6-diamine (100 mg, 0.960 mmol) in dichloroethane (10 ml), trans-3-(3-pyridyl)acrylic acid (286 mg, 1.92 mmol), 1-(3-dimethylaminopropyl)-3-ethylcarbodiimide hydrochloride (552 mg, 2.88 mmol), and dimethylaminopyridine (23.0 mg, 0.188 mmol) were added at room temperature, and the mixture was stirred at 60°C for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 38 was obtained as a yellow solid (8.50 mg, 0.0225 mmol, 2.3%). NMR measurement spectra and results of mass spectrometry by HR-ESI-MS are as follows.
¹H NMR (DMSO, 400 MHz): δ 1.29-1.35 (4H, m), 1.42-1.51 (4H, m), 3.18 (4H, q, J = 6.4 Hz), 6.72 (2H, d, J = 16.4 Hz), 7.41-7.47 (4H, m), 7.97 (2H, dt, J = 8.4, 2.0 Hz), 8.18 (2H, t, J = 5.2 Hz), 8.54 (2H, dd, J = 4.8, 1.6 Hz), 8.74 (2H, d, J = 2.4 Hz); HRMS (ESI-TOF) m/z: [M + Na]⁺ calcd for C₂₂H₂₆N₄O₂Na 401.1953; Found 401.1950.

The confirmed structure is as follows.

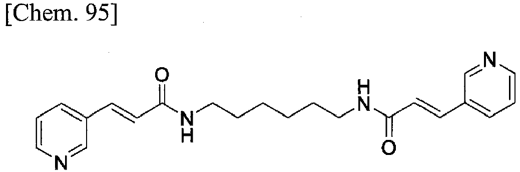

### [Example 39: Production of compound 39]

To a solution of propane-1,3-diamine (50.0 mg, 0.674 mmol) in dichloromethane (3 ml), trans-3-(3-pyridyl)acrylic acid (251 mg, 1.69 mmol), 1-(3-dimethylaminopropyl)-3-ethylcarbodiimide hydrochloride (388 mg, 2.02 mmol), and dimethylaminopyridine (16.5 mg, 0.135 mmol) were added at room temperature, and the mixture was stirred at the same temperature as above for 14 hours Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (NH₂-silica gel, chloroform:methanol = 19:1). Compound 39 was obtained as a yellow solid (43.5 mg, 0.129 mmol, 19%). NMR measurement spectra and results of mass spectrometry by HR-ESI-MS are as follows.
¹H NMR (CDCl₃, 400 MHz): δ 1.78 (2H, q, J = 6.0 Hz), 3.49 (4H, q, J = 6.0 Hz), 6.54 (2H, d, J = 16.0 Hz), 6.59 (2H, t, J = 6.0 Hz), 7.32 (2H, dd, J = 7.6, 5.2 Hz), 7.65 (2H, d, J = 16.0 Hz), 7.82 (2H, dt, J = 7.6, 2.0 Hz), 8.58 (2H, dd, J = 4.8, 1.6 Hz), 8.76 (2H, d, J = 2.4 Hz); HRMS (ESI-TOF) m/z: [M + Na]⁺ calcd for C₁₉H₂₀N₄O₂Na 359.1484; Found 359.1482.

The confirmed structure is as follows.

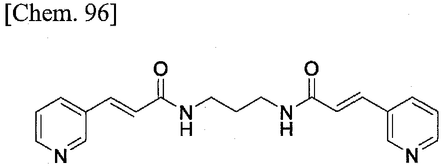

### [Example 40: Production of compound 40]

To a solution of ethane-1,2-diamine (100 mg, 1.66 mmol) in dichloromethane (5 ml), N-nicotinoylglycine (658 mg, 3.65 mmol), 1-(3-dimethylaminopropyl)-3-ethylcarbodiimide hydrochloride (636 mg, 3.32 mmol), and dimethylaminopyridine (40.6 mg, 0.332 mmol) were added at room temperature, and the mixture was stirred at the same temperature as above for 1 hour. Water was added to the reaction mixture, and concentrated under reduced pressure. Crystals were collected by filtration using a Kiriyama funnel and washed with methanol. Compound 40 was obtained as a yellow solid (257 mg, 0.669 mmol, 40%). NMR measurement spectra and results of mass spectrometry by HR-ESI-MS are as follows.
¹H NMR (DMSO, 400 MHz): δ 3.15 (4H, t, J = 2.4 Hz), 3.86 (4H, d, J = 6.0 Hz), 7.51 (2H, dd, J = 7.6, 4.4 Hz), 8.05 (2H, br), 8.21 (2H, dt, J = 8.0, 2.0 Hz), 8.71 (2H, dd, J = 4.4, 2.0 Hz), 8.98 (2H, t, J = 6.0 Hz), 9.04 (2H, dd, J = 2.4, 0.8 Hz); HRMS (ESI-TOF) m/z: [M + Na]⁺ calcd for C₁₈H₂₀N₆O₄Na 407.1444; Found 407.1444.

The confirmed structure is as follows.

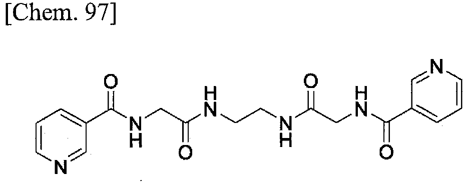

### [Example 41: Production of compound 41]

To a solution of hexane-1,6-diamine (100 mg, 0.861 mmol) in dichloromethane (4 ml), N-nicotinoylglycine (341 mg, 1.89 mmol), 1-(3-dimethylaminopropyl)-3-ethylcarbodiimide hydrochloride (330 mg, 1.72 mmol), and dimethylaminopyridine (21.0 mg, 0.172 mmol) were added at room temperature, and the mixture was stirred at the same temperature as above for 15 hours. Water was added to the reaction mixture, and concentrated under reduced pressure. Crystals were collected by filtration using a Kiriyama funnel and washed with methanol. Compound 41 was obtained as a yellow solid (267 mg, 0.607 mmol, 71%). NMR measurement spectra and results of mass spectrometry by HR-ESI-MS are as follows.
¹H NMR (CDCl₃, 400 MHz): δ 1.22-1.29 (4H, m), 1.35-1.43 (4H, m), 3.06 (4H, q, J = 6.4 Hz), 3.85 (4H, d, J = 5.6 Hz), 7.52 (2H, dd, J = 8.4, 4.8 Hz), 7.94 (2H, t, J = 5.6 Hz), 8.21 (2H, dt, J = 8.4, 2.0 Hz), 8.71 (2H, dd, J = 4.8, 1.6 Hz), 8.95 (2H, t, J = 6.0 Hz), 9.03 (2H, d, J = 1.2 Hz); HRMS (ESI-TOF) m/z: [M + Na]⁺ calcd for C₂₂H₂₈N₆O₄Na 463.2070; Found 463.2067.

The confirmed structure is as follows.

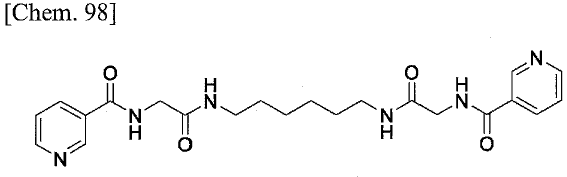

### [Example 42: Production of compound 42]

To a solution of hexane-1,6-diamine (20 mg, 0.172 mmol) in dichloromethane (2 ml), trans-3-pyridyl-2-butenoic acid (70.0 mg, 0.429 mmol), 1-(3-dimethylaminopropyl)-3-ethylcarbodiimide hydrochloride (165 mg, 0.860 mmol), and dimethylaminopyridine (8.40 mg, 0.0688 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. Ethyl acetate was added to the residue, and the resulting solid was collected by filtration using a Kiriyama funnel and then washed with ethyl acetate. Compound 42 was obtained as a yellow solid (30.5 mg, 0.0750 mmol, 43%). NMR measurement spectra and results of mass spectrometry by HR-ESI-MS are as follows.
¹H NMR (CD₃OD, 400 MHz): δ 1.40-1.46 (4H, m), 1.55-1.63 (4H, m), 2.51 (6H, d, J = 1.6 Hz), 3.29 (4H, q, J = 6.8 Hz), 6.25 (2H, d, J = 1.6 Hz), 7.46 (2H, dd, J = 8.0, 4.8 Hz), 7.97 (2H, dt, J = 8.4, 2.0 Hz), 8.50 (2H, d, J = 3.6 Hz), 8.67 (2H, s); HRMS (ESI-TOF) m/z: [M + Na]⁺ calcd for C₂₄H₃₀N₄O₂Na 429.2266; Found 429.2262.

The confirmed structure is as follows.

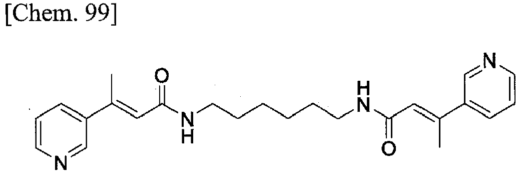

### [Example 43: Production of compound 43]

To a solution of 2,2'-oxybis(ethan-1-amine) (30 mg, 0.288 mmol) in dichloromethane (3 ml), trans-3-pyridyl-2-butenoic acid (103 mg, 0.634 mmol), 1-(3-dimethylaminopropyl)-3-ethylcarbodiimide hydrochloride (221 mg, 1.15 mmol), and dimethylaminopyridine (14.1 mg, 0.115 mmol) were added at room temperature, and the mixture was stirred at the same temperature as above for 4 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). 1,4-Dioxane (1.5 ml) and hydrochloric acid (4 M solution in dioxane) were added to the residue, and crystals were collected by filtration using a Kiriyama funnel and then washed with chloroform. Compound 43 was obtained as a yellow solid (31.5 mg, 0.0719 mmol, 28%). NMR measurement spectra and results of mass spectrometry by HR-ESI-MS are as follows. ¹H NMR (CD₃OD, 400 MHz): δ 2.57 (6H, s), 3.50 (4H, t, J = 5.6 Hz), 3.63 (4H, t, J = 5.6 Hz), 6.56 (2H, s), 8.12 (2H, dd, J = 8.0, 5.6 Hz), 8.78 (2H, dd, J = 4.4, 0.8 Hz), 8.84 (2H, d, J = 5.6 Hz), 9.03 (2H, s); HRMS (ESI-TOF) m/z: [M + H]⁺ calcd for C₂₂H₂₇N₄O₃ 395.2083; Found 395.2082.

The confirmed structure is as follows.

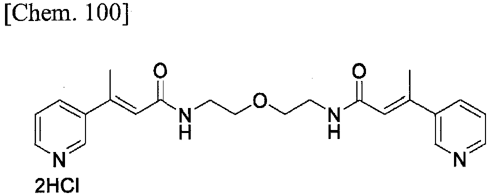

### [Example 44: Production of compound 44]

To a solution of lysine methyl ester (1.00 g, 4.29 mmol) in dichloromethane (30 ml), nicotinoyl chloride hydrochloride (1.91 g, 10.7 mmol) and triethylamine (3.59 ml, 25.8 mmol) were added under an ice bath, and the mixture was stirred at the same temperature as above for 3 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 44 was obtained as a yellow solid (1.10 g, 2.96 mmol, 69%). NMR measurement spectra and results of mass spectrometry by HR-ESI-MS are as follows.
¹H NMR (CDCl₃, 400 MHz): δ1.43-2.08 (6H, m), 3.46-3.58 (2H, m), 3.80 (3H, s), 4.76-4.84 (1H, m), 6.76 (1H, br), 7.23 (1H, br), 7.36-7.45 (2H, m), 8.16 (2H, t, J = 8.0 Hz), 8.67-8.78 (2H, m), 9.06-9.18 (2H, m); HRMS (ESI-TOF) m/z: [M + Na]⁺ calcd for C₁₉H₂₂N₄O₄Na 393.1539; Found 393.1536.

The confirmed structure is as follows.

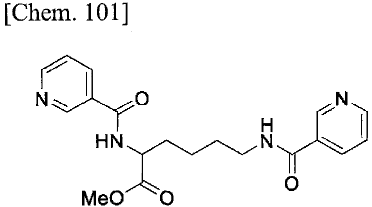

### [Example 45: Production of compound 45]

To a solution of compound 44 (152 mg, 0.410 mmol) in methanol (1 ml), potassium carbonate (142 mg, 1.03 mmol) was added at room temperature, and the mixture was stirred at 50°C for 4 hours. A saturated aqueous solution of ammonium chloride was added to the reaction mixture, followed by extraction with chloroform. The organic layer was washed with saturated saline, dried over anhydrous magnesium sulfate, then filtered, and concentrated under reduced pressure. Methanol was added to the residue, and insoluble matter was filtered off, followed by the concentration of the filtrate. Compound 45 was obtained as a yellow solid (144 mg, 0.405 mmol, 99%). NMR measurement spectra and results of mass spectrometry by HR-ESI-MS are as follows.
¹H NMR (CD₃OD, 400 MHz): δ 1.50-2.09 (6H, m), 3.42 (2H, t, J = 3.2 Hz), 4.54 (1H, dd, J = 8.8, 4.8 Hz), 7.50-7.56 (2H, m), 8.21 (1H, dt, J = 6.4, 1.6 Hz), 8.28 (1H, dt, J = 8.0, 1.6 Hz), 8.67 (2H, t, J = 4.8 Hz), 8.97 (2H, d, J = 24.0 Hz); HRMS (ESI-TOF) m/z: [M + H]⁺ calcd for C₁₃H₂₁N₄O₄ 357.1563; Found 357.1559.

The confirmed structure is as follows.

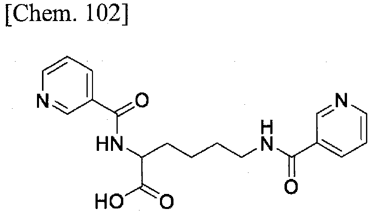

### [Example 46: Production of compound 46]

To a solution of compound 44 (157 mg, 0.424 mmol) in methanol (0.8 ml), ammonia water (1 M, 0.424 ml, 0.424 mmol) was added at room temperature, and the mixture was stirred at the same temperature as above for 13 hours and concentrated under reduced pressure. Ethyl acetate was added to the residue, and crystals were collected by filtration using a Kiriyama funnel. Then, compound 46 was obtained as a yellow solid (43.1 mg, 0.121 mmol, 29%). NMR measurement spectra and results of mass spectrometry by HR-ESI-MS are as follows.
¹H NMR (CD₃OD, 400 MHz): δ 1.50-2.03 (6H, m), 3.43 (2H, t, J = 6.4 Hz), 4.57 (1H, dd, J = 8.8, 4.8 Hz), 7.48-7.57 (2H, m), 8.20 (1H, d, J = 8.4 Hz), 8.27 (1H, d, J = 7.6 Hz), 8.68 (2H, br), 8.94 (1H, s), 9.02 (1H, s); HRMS (ESI-TOF) m/z: [M + Na]⁺ calcd for C₁₈H₂₁N₅O₃Na 378.1542; Found 378.1540.

The confirmed structure is as follows.

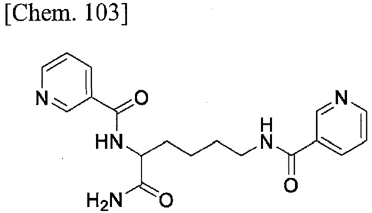

### [Example 47: Production of compound 47]

To a solution of compound 44 (91.9 mg, 0.248 mmol) in methanol (0.5 ml), an aqueous ethylamine solution (70%, 0.0210 ml, 0.372 mmol) was added at room temperature, and the mixture was stirred at 50°C for 3 hours. Water was added to the reaction mixture, followed by extraction with chloroform. The organic layer was washed with saturated saline, dried over anhydrous magnesium sulfate, then filtered, and concentrated under reduced pressure. Ethyl acetate was added to the residue, and crystals were collected by filtration using a Kiriyama funnel. Then, compound 47 was obtained as a yellow solid (2.20 mg, 0.00597 mmol, 2.4%). NMR measurement spectra and results of mass spectrometry by HR-ESI-MS are as follows.
¹H NMR (CD₃OD, 400 MHz): δ 1.13 (3H, t, J = 7.2 Hz), 1.46-1.98 (6H, m), 3.24 (2H, q, J = 7.2 Hz), 3.43 (2H, t, J = 6.8 Hz), 4.51 (1H, dd, J = 8.8, 5.2 Hz), 7.50-7.55 (2H, m), 8.20 (1H, d, J = 7.6 Hz), 8.27 (1H, d, J = 7.6 Hz), 8.67 (2H, t, J = 5.6 Hz), 8.94 (1H, s), 9.01 (1H, s); HRMS (ESI-TOF) m/z: [M + Na]⁺ calcd for C₂₀H₂₅N₅O₃Na 406.1855; Found 406.1851.

The confirmed structure is as follows.

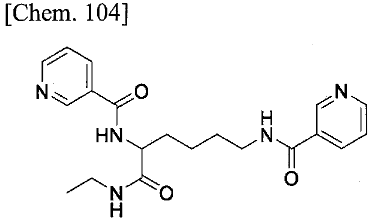

### [Example 48: Production of compound 48]

To a solution of methyl 2,5-diaminopentanoate (1.00 g, 4.56 mmol) in dichloromethane (25 ml), nicotinoyl chloride hydrochloride (2.03 g, 11.4 mmol) and triethylamine (3.81 ml, 27.4 mmol) were added under an ice bath, and the mixture was stirred at the same temperature as above for 1.5 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 85:15). Compound 48 was obtained as a yellow solid (1.22 g, 3.43 mmol, 75%). NMR measurement spectra and results of mass spectrometry by HR-ESI-MS are as follows.
¹H NMR (CD₃OD, 400 MHz): δ1.72-2.12 (4H, m), 3.47 (2H, t, J = 6.8 Hz), 3.76 (3H, s), 4.68 (1H, dd, J =9.2, 5.2 Hz), 7.52-7.58 (2H, m), 8.24 (1H, dt, J = 8.4, 2.0 Hz), 8.28 (1H, dt, J = 8.0, 2.0 Hz), 8.66-8.72 (2H, m), 8.97 (1H, s), 9.01 (1H, s); HRMS (ESI-TOF) m/z: [M + Na]⁺ calcd for C₁₈H₂₀N₄O₄Na 379.1382; Found 379.1377.

The confirmed structure is as follows.

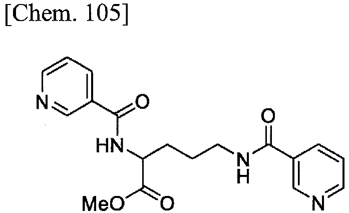

### [Example 49: Production of compound 49]

To a solution of compound 48 (146 mg, 0.409 mmol) in methanol (1 ml), potassium carbonate (113 mg, 0.817 mmol) was added at room temperature, and the mixture was stirred at 50°C for 19 hours. A saturated aqueous solution of ammonium chloride was added to the reaction mixture, and the mixture was concentrated under reduced pressure. Methanol was added to the residue, and insoluble matter was filtered off, followed by the concentration of the filtrate. Compound 49 was obtained as a yellow solid (105 mg, 0.305 mmol, 75%). NMR measurement spectra and results of mass spectrometry by HR-ESI-MS are as follows.
¹H NMR (CD₃OD, 400 MHz): δ 1.73-2.12 (4H, m), 3.44-3.49 (2H, m), 4.56 (1H, dd, J = 8.4, 4.8 Hz), 7.51-7.57 (2H, m), 8.25 (1H, dt, J = 7.6, 1.6 Hz), 8.31 (1H, dt, J = 7.6, 1.6 Hz), 8.65-8.69 (2H, m), 8.37 (1H, s), 8.42 (1H, s);
HRMS (ESI-TOF) m/z: [M + H]⁺ calcd for C₁₇H₁₉N₄O₄ 343.1406; Found 343.1406.

The confirmed structure is as follows.

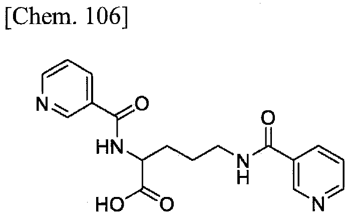

### [Example 50: Production of compound 50]

To a solution of compound 48 (145 mg, 0.408 mmol) in methanol (1 ml), ammonia water (1 M, 0.408 ml, 0.408 mmol) was added at room temperature, and the mixture was stirred at 50°C for 6 hours and concentrated under reduced pressure. Diisopropyl ether and methanol were added to the residue, and crystals were collected by filtration using a Kiriyama funnel. Then, compound 50 was obtained as a yellow solid (41.1 mg, 0.120 mmol, 30%). NMR measurement spectra and results of mass spectrometry by HR-ESI-MS are as follows.
¹H NMR (CD₃OD, 400 MHz): δ 1.71-2.08 (4H, m), 3.42-3.55 (2H, m), 4.60-4.66 (1H, m), 7.52-7.58 (2H, m), 8.24 (1H, dt, J = 8.0, 1.6 Hz), 8.30 (1H, dt, J = 7.6, 2.0 Hz), 8.69 (2H, t, J = 4.8 Hz), 8.97 (1H, s), 9.04 (1H, s); HRMS (ESI-TOF) m/z: [M + Na]⁺ calcd for C₁₇H₁₉N₅O₃Na 364.1386; Found 364.1386.

The confirmed structure is as follows.

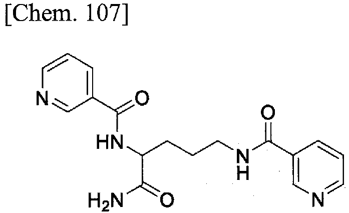

### [Example 51: Production of compound 51]

To a solution of compound 48 (188 mg, 0.528 mmol) in tetrahydrofuran and methanol (2 ml/1 ml), an aqueous ethylamine solution (70%, 0.170 ml, 2.12 mmol) was added at room temperature, and the mixture was stirred at the same temperature as above for 3 days. The reaction mixture was concentrated under reduced pressure, and the residue was then purified by silica gel column chromatography (chloroform:methanol = 9:1). Compound 51 was obtained as a yellow solid (45.9 mg, 0.124 mmol, 23%). NMR measurement spectra and results of mass spectrometry by HR-ESI-MS are as follows.
¹H NMR (CD₃OD, 400 MHz): δ1.14 (3H, t, J = 7.2 Hz), 1.68-2.03 (4H, m), 3.25 (2H, q, J = 7.2 Hz), 3.43-3.52 (2H, m), 4.53-4.60 (1H, m), 7.52-7.58 (2H, m), 8.24 (1H, d, J = 6.4 Hz), 8.30 (1H, d, J = 8.0 Hz), 8.65-8.71 (2H, m), 8.97 (1H, s), 9.03 (1H, s); HRMS (ESI-TOF) m/z: [M + Na]⁺ calcd for C₁₉H₂₃N₅O₃Na 392.1699; Found 392.1694.

The confirmed structure is as follows.

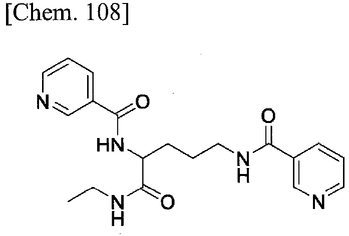

### [Example 52: Production of compound 52]

### (1) Production of intermediate 47

A known compound represented by the following structural formula (intermediate 47) was synthesized in accordance with the method described in European Journal of Medicinal Chemistry, 2019, 182, 111588.

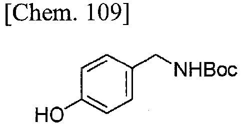

### (2) Production of intermediate 48

To a solution of intermediate 47 (2.50 g, 11.2 mmol) in dimethylformamide (20 ml), intermediate 27 (3.70 g, 12.4 mmol), potassium carbonate (2.30 g, 16.6 mmol), and sodium iodide (336 mg, 62.3 mmol) were added at room temperature, and the mixture was stirred at 60°C for 18 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (ethyl acetate:hexane = 2:1). Intermediate 48 represented by the following structural formula was obtained as a yellow solid (3.60 g, 8.17 mmol, 73%).
¹H NMR (DMSO, 400 MHz): δ1.38 (9H, s), 3.64-3.80 (6H, m), 3.95-3.99 (2H, m), 6.75 (2H, d, J = 8.4 Hz), 7.07 (2H, d, J = 8.4 Hz), 7.79-7.88 (4H, m).

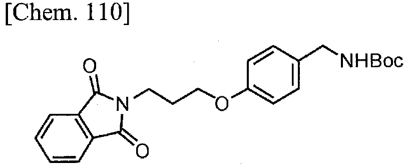

### (3) Production of intermediate 49

To a solution of intermediate 48 (3.60 g, 8.77 mmol) in dichloromethane (20 ml), trifluoroacetic acid (10 ml) was added at room temperature, and the mixture was stirred at room temperature for 1 hour. The reaction mixture was concentrated under reduced pressure. Then, chloroform and a saturated aqueous solution of sodium bicarbonate were added to the residue, and the mixture was stirred at room temperature for 1 hour. After separation between organic and aqueous layers, the separated organic layer was concentrated under reduced pressure. Intermediate 49 represented by the following structural formula was obtained as a yellow oil (2.72 g, 8.77 mmol, 99%). ¹H NMR (DMSO, 400 MHz): δ2.05 (2H, q, J = 6.0 Hz), 3.76 (2H, t, J = 6.8 Hz), 3.90-4.04 (4H, m), 6.82 (2H, d, J = 8.4 Hz), 7.31 (2H, d, J = 8.4 Hz), 7.82-7.87 (4H, m).

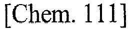

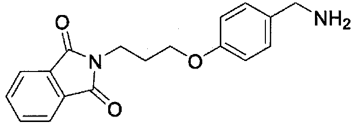

### (4) Production of intermediate 50

To a solution of intermediate 49 (300 mg, 0.967 mmol) in dichloromethane (10 ml), triethylamine (0.297 ml, 2.13 mmol) and nicotinoyl chloride hydrochloride (206 mg, 1.16 mmol) were added under an ice bath, and the mixture was stirred at room temperature for 23 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 8:2). Intermediate 50 represented by the following structural formula was obtained as a yellow solid (401 mg, 0.965 mmol, 99%).
¹H NMR (DMSO, 400 MHz): δ 2.04 (2H, q, J = 6.4 Hz), 3.75 (2H, t, J = 6.4 Hz), 3.98 (2H, t, J = 6.4 Hz), 4.40 (2H, d, J = 6.0 Hz), 6.75 (2H, d, J = 8.8 Hz), 7.20 (2H, d, J = 8.8 Hz), 7.48-7.52 (1H, m), 7.81-7.87 (4H, m), 8.21 (1H, dt, J = 7.6, 2.0 Hz), 8.70 (1H, dd, J = 5.2, 1.6 Hz), 9.03 (1H, d, J = 2.0 Hz), 9.16 (1H, t, J = 5.6 Hz).

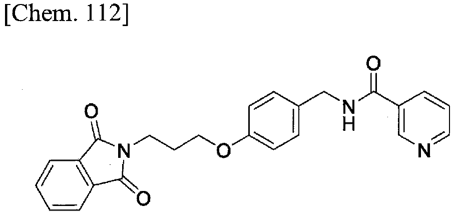

### (5) Production of intermediate 51

To a solution of intermediate 50 (401 mg, 0.965 mmol) in ethanol (10 ml), hydrazine monohydrate (0.235 ml, 4.83 mmol) was added at room temperature, and the mixture was stirred at 80°C for 22 hours. The reaction mixture was concentrated under reduced pressure. Intermediate 51 represented by the following structural formula was obtained as a yellow oil (275 mg, 0.964 mmol, 99%).
¹H NMR (DMSO, 400 MHz): δ 1.93-2.02 (2H, m), 3.40-3.44 (2H, m), 4,00-4.40 (2H, m), 4.42 (2H, d, J = 6.0 Hz), 6.90 (2H, d, J = 8.8 Hz), 7.25 (2H, d, J = 8.8 Hz), 7.48-7.52 (1H, m), 8.19-8.23 (1H, m), 8.68-8.71 (1H, m), 9.03 (1H, d, J = 2.0 Hz), 9.20 (1H, t, J = 6.0 Hz).

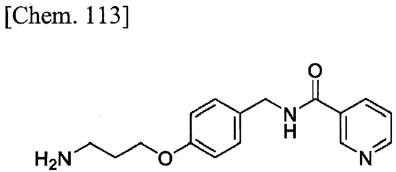

### (6) Production of compound 52

To a solution of intermediate 51 (100 mg, 0.350 mmol) in dichloromethane (3 ml), nicotinoyl chloride hydrochloride (68.6 mg, 0.385 mmol) and triethylamine (0.970 ml, 6.96 mmol) were added under an ice bath, and the mixture was stirred at the same temperature as above for 4 hours. Water was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 9:1). Compound 52 was obtained as a yellow solid (13.9 mg, 0.0356 mmol, 10%). NMR measurement spectra and results of mass spectrometry by HR-ESI-MS are as follows.
¹H NMR (CDCl₃, 400 MHz): δ 2.13-2.18 (2H, m), 3.69-3.76 (2H, m), 4.12-4.16 (2H, m), 4.80 (2H, s), 6.87 (2H, d, J = 8.8 Hz), 7.31 (2H, d, J = 8.8 Hz), 7.40-7.48 (2H, m), 7.91 (1H, d, J = 7.6 Hz), 8.15-8.23 (1H, m), 8.39 (1H, d, J = 8.0 Hz), 8.64 (1H, d, J = 3.2 Hz), 8.72-8.84 (3H, m), 9.30 (1H, s); HRMS (ESI-TOF) m/z: [M + Na]⁺ calcd for C₂₂H₂₂N₄O₃Na 413.1590; Found 413.1584.

The confirmed structure is as follows.

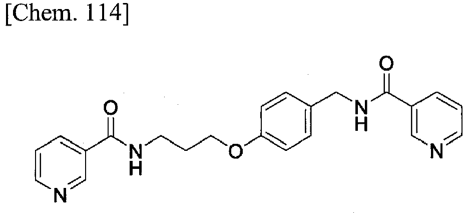

### [Example 53: Production of compound 53]

To a solution of intermediate 51 (100 mg, 0.350 mmol) in dichloromethane (3 ml), trans-3-(3-pyridyl)acrylic acid (104 mg, 0.697 mmol), 1-(3-dimethylaminopropyl)-3-ethylcarbodiimide hydrochloride (134 mg, 0.699 mmol), and dimethylaminopyridine (8.60 mg, 0.0704 mmol) were added at room temperature, and the mixture was stirred at the same temperature as above for 13 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 53 was obtained as a yellow solid (7.80 mg, 0.0187 mmol, 5.3%). NMR measurement spectra and results of mass spectrometry by HR-ESI-MS are as follows.
¹H NMR (CDCl₃, 400 MHz): δ 2.02 (2H, q, J = 6.0 Hz), 3.54 (2H, q, J = 6.0 Hz), 3.98 (2H, t, J = 6.0 Hz), 4.52 (2H, d, J = 5.6 Hz), 6.47 (1H, d, J = 15.2 Hz), 6.78 (2H, d, J = 8.0 Hz), 6.89 (1H, t, J = 7.2 Hz), 7.17-7.36 (4H, m), 7.48 (1H, d, J = 15.2 Hz), 7.48-7.53 (1H, m), 7.70 (1H, d, J = 8.4 Hz), 8.16 (1H, d, J = 8.0 Hz), 8.48 (1H, s), 8.54 (1H, s), 8.65 (1H, s), 9.00 (1H, br); HRMS (ESI-TOF) m/z: [M + Na]⁺ calcd for C₂₄H₂₄N₄O₃Na 439.1746; Found 439.1744.

The confirmed structure is as follows.

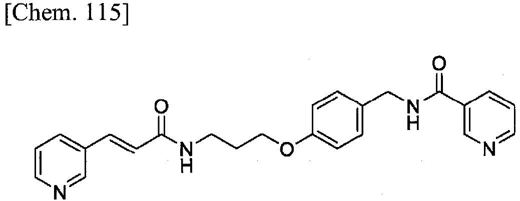

### [Example 54: Production of compound 54]

### (1) Production of intermediate 52

A known compound represented by the following structural formula (intermediate 52) was synthesized in accordance with the method described in DE2320387 A1.

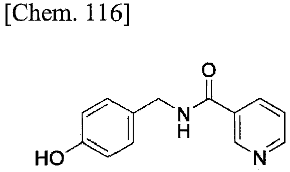

### (2) Production of intermediate 53

To a solution of intermediate 52 (500 mg, 2.19 mmol) in dimethylformamide (20 ml), intermediate 24 (718 mg, 2.41 mmol), potassium carbonate (454 mg, 3.29 mmol), and sodium iodide (65.7 mg, 0.438 mmol) were added at room temperature, and the mixture was stirred at the same temperature as above for 16 hours. Water was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 95:5). Intermediate 53 represented by the following structural formula was obtained as a yellow solid (200 mg, 0.449 mmol, 21%).
¹H NMR (CDCl₃, 400 MHz): δ 3.78-3.86 (4H, m), 3.91 (2H, t, J = 5.6 Hz), 4.05 (2H, t, J = 4.8 Hz), 4.57 (2H, d, J = 5.6 Hz), 6.81 (2H, d, J = 8.8 Hz), 7.25 (2H, d, J = 8.8 Hz), 7.51 (1H, dd, J = 7.6, 4.8 Hz), 7.68-7.71 (2H, m), 7.80-7.83 (2H, m), 8.32 (1H, d, J = 8.0 Hz), 8.70 (1H, d, J = 3.6 Hz), 9.19 (1H, s).

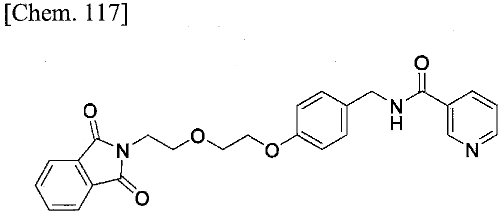

### (3) Production of intermediate 54

To a solution of intermediate 53 (200 mg, 0.449 mmol) in ethanol (4 ml), hydrazine monohydrate (0.110 ml, 2.26 mmol) was added at room temperature, and the mixture was stirred at 80°C for 5 hours. After filtration through Celite, the reaction mixture was concentrated under reduced pressure. Intermediate 54 represented by the following structural formula was obtained as a yellow oil (141 mg, 0.447 mmol, 99%). ¹H NMR (CDCl₃, 400 MHz): δ 2.87-2.91 (2H, m), 3.57 (2H, t, J = 4.8 Hz), 3.81 (2H, t, J = 4.8 Hz), 4.12 (2H, t, J = 4.8 Hz), 4.57 (2H, d, J = 5.2 Hz), 6.76 (1H, br), 6.89 (2H, d, J = 8.4 Hz), 7.26 (2H, d, J = 8.4 Hz), 7.37 (1H, dd, J = 8.0, 4.8 Hz), 8.14 (1H, dt, J = 7.6, 2.0 Hz), 8.69 (1H, dd, J = 4.8, 2.4 Hz), 8.98 (1H, d, J = 1.6 Hz).

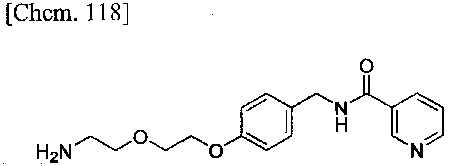

### (4) Production of compound 54

To a solution of intermediate 54 (200 mg, 0.634 mmol) in dichloromethane (6 ml), nicotinic acid (117 mg, 0.950 mmol), 1-(3-dimethylaminopropyl)-3-ethylcarbodiimide hydrochloride (242 mg, 1.26 mmol), and dimethylaminopyridine (15.4 mg, 0.126 mmol) were added at room temperature, and the mixture was stirred at the same temperature as above for 21 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 85:15). Compound 54 was obtained as a yellow solid (10.3 mg, 0.0245 mmol, 3.9%). NMR measurement spectra and results of mass spectrometry by HR-ESI-MS are as follows.
¹H NMR (CDCl₃, 400 MHz): δ 3.65-3.75 (4H, m), 3.83-3.87 (2H, m), 4.17-4.20 (2H, m), 4.59 (2H, d, J = 5.6 Hz), 6.65 (1H, br), 6.89 (2H, d, J = 8.4 Hz), 7.25 (2H, d, J = 8.4 Hz), 7.33-7.41 (2H, m), 7.79 (1H, br), 8.15 (1H, d, J = 6.4 Hz), 8.22 (1H, d, J = 6.0 Hz), 8.46 (1H, s), 8.54 (1H, d, J = 3.6 Hz), 8.70 (1H, d, J = 3.6 Hz), 9.10 (1H, s); HRMS (ESI-TOF) m/z: [M + Na]⁺ calcd for C₂₃H₂₄N₄O₄Na 443.1695; Found 443.1692.

The confirmed structure is as follows.

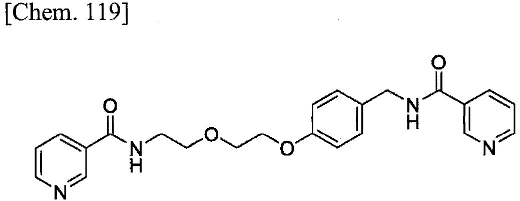

### [Example 55: Production of compound 55]

To a solution of intermediate 54 (50 mg, 0.159 mmol) in dichloromethane (2 ml), trans-3-(3-pyridyl)acrylic acid (28.0 mg, 0.188 mmol), 1-(3-dimethylaminopropyl)-3-ethylcarbodiimide hydrochloride (60.9 mg, 0.318 mmol), and dimethylaminopyridine (3.90 mg, 0.0319 mmol) were added at room temperature, and the mixture was stirred at the same temperature as above for 19 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 89:11). Compound 55 was obtained as a yellow solid (9.40 mg, 0.0187 mmol, 2.1%). NMR measurement spectra and results of mass spectrometry by HR-ESI-MS are as follows.
¹H NMR (CDCl₃, 400 MHz): δ 3.58 (2H, q, J = 5.2 Hz), 3.76 (2H, t, J = 4.8 Hz), 3.91 (2H, t, J = 4.0 Hz), 4.13 (2H, t, J = 4.4 Hz), 4.64 (2H, d, J = 6.0 Hz), 6.06 (1H, d, J = 16.0 Hz), 6.55 (1H, br), 6.97 (2H, d, J = 8.4 Hz), 7.28-7.43 (5H, m), 7.67 (1H, d, J = 8.0 Hz), 7.97 (1H, s), 8.25 (1H, dt, J = 7.6, 2.0 Hz), 8.37 (1H, d, J = 5.2 Hz), 8.65 (1H, br), 8.69 (1H, d, J = 4.8 Hz), 9.13 (1H, s); HRMS (ESI-TOF) m/z: [M + Na]⁺ calcd for C₂₅H₂₆N₄O₄Na 469.1852; Found 469.1847.

The confirmed structure is as follows.

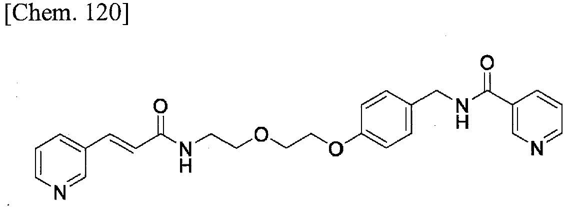

### [Example 56: Production of compound 56]

To a solution of intermediate 54 (50 mg, 0.159 mmol) in dichloromethane (2 ml), trans-3-pyridyl-2-butenoic acid (31.1 mg, 0.191 mmol), 1-(3-dimethylaminopropyl)-3-ethylcarbodiimide hydrochloride (60.9 mg, 0.318 mmol), and dimethylaminopyridine (3.90 mg, 0.0319 mmol) were added at room temperature, and the mixture was stirred at the same temperature as above for 23 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 88:12). Compound 56 was obtained as a yellow solid (24.5 mg, 0.0532 mmol, 33%). NMR measurement spectra and results of mass spectrometry by HR-ESI-MS are as follows.
¹H NMR (CDCl₃, 400 MHz): δ 2.48 (3H, s), 3.55 (2H, q, J = 5.2 Hz), 3.75 (2H, t, J = 5.2 Hz), 3.88 (2H, t, J = 4.0 Hz), 4.13 (2H, t, J = 4.0 Hz), 4.60 (2H, d, J = 6.0 Hz), 5.75 (1H, s), 6.35 (1H, br), 6.92 (2H, d, J = 8.8 Hz), 7.26-7.39 (5H, m), 7.72 (1H, dt, J = 8.0, 2.0 Hz), 8.03 (1H, d, J = 1.6 Hz), 8.24 (1H, dt, J = 8.4, 2.0 Hz), 8.49 (1H, br), 8.69 (1H, dd, J = 4.4, 1.6 Hz), 9.10 (1H, d, J = 1.6 Hz); HRMS (ESI-TOF) m/z: [M + Na]⁺ calcd for C₂₆H₂₈N₄O₄Na 483.2008; Found 483.2003.

The confirmed structure is as follows.

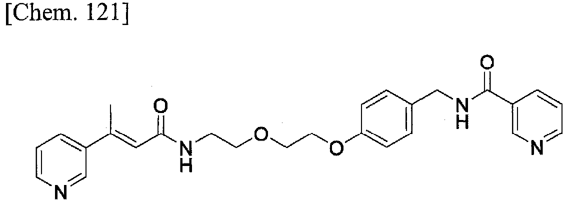

### [Example 57: Production process for compound 57]

### (1) Production of intermediate 55

A known compound represented by the following structural formula (intermediate 55) was synthesized in accordance with the method described in Journal of Inorganic Biochemistry, 2015, 148, 2-10.

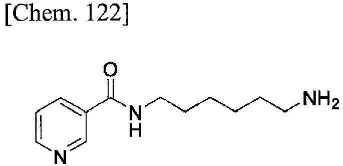

### (2) Production process for compound 57

To a solution of intermediate 55 (120 mg, 0.542 mmol) in dimethylformamide (5 ml), 5-hydroxynicotinic acid (90.5 mg, 0.651 mmol), 1-(3-dimethylaminopropyl)-3-ethylcarbodiimide hydrochloride (208 mg, 1.08 mmol), and dimethylaminopyridine (13.2 mg, 0.108 mmol) were added at room temperature, and the mixture was stirred at the same temperature as above for 16 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 57 was obtained as a yellow solid (5.6 mg, 0.0164 mmol, 1.6%). NMR measurement spectra and results of mass spectrometry by HR-ESI-MS are as follows.
¹H NMR (CDCl₃, 400 MHz): δ 1.43-1.49 (4H, m), 1.61-1.71 (4H, m), 3.34-3.43 (4H, m), 7.42 (1H, t, J = 2.0 Hz), 7.54 (1H, dd, J = 8.0, 4.8 Hz), 8.08 (1H, d, J = 2.8 Hz), 8.19 (1H, s), 8.23 (1H, dt, J = 7.6, 2.0 Hz), 8.67 (1H, d, J = 4.8 Hz), 8.96 (1H, d, J = 2.0 Hz); HRMS (ESI-TOF) m/z: [M + Na]⁺ calcd for C₁₈H₂₂N₄O₃Na 365.1590; Found 365.1584.

The confirmed structure is as follows.

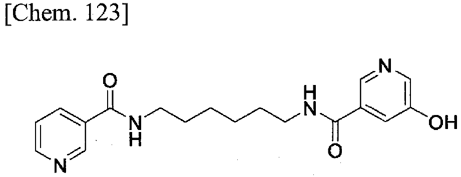

### [Example 58: Production of compound 58]

To a solution of benzene-1,4-diamine (100 mg, 0.925 mmol) in dichloromethane (3 ml), trans-3-(3-pyridyl)acrylic acid (290 mg, 1.94 mmol), 1-(3-dimethylaminopropyl)-3-ethylcarbodiimide hydrochloride (709 mg, 3.70 mmol), and dimethylaminopyridine (45.2 mg, 0.370 mmol) were added at room temperature, and the mixture was stirred at the same temperature as above for 3 hours. The reaction mixture was concentrated under reduced pressure. Then, methanol was added to the residue, and crystals were collected by filtration using a Kiriyama funnel. Then, compound 58 was obtained as a yellow solid (155 mg, 0.418 mmol, 45%). NMR measurement spectra and results of mass spectrometry by HR-ESI-MS are as follows. ¹H NMR (DMSO, 400 MHz): δ 6.93 (2H, d, J = 16.0 Hz), 7.45-7.52 (2H, m), 7.62 (2H, d, J = 16.0 Hz), 7.69 (4H, s), 8.04 (2H, d, J = 6.8 Hz), 8.57-8.60 (2H, m), 8.82 (2H, s); HRMS (ESI-TOF) m/z: [M - H]⁻ calcd for C₂₂H₁₇N₄O₂ 369.1352; Found 369.1364.

The confirmed structure is as follows.

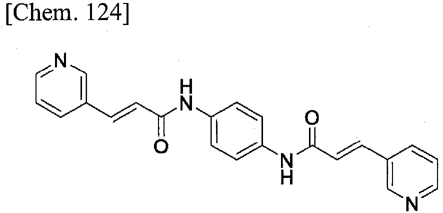

### [Example 59: Production of compound 59]

To a solution of pyridine-2,6-diamine (80.0 mg, 0.733 mmol) in chloroform (3 ml), trans-3-(3-pyridyl)acrylic acid (219 mg, 1.47 mmol), 1-(3-dimethylaminopropyl)-3-ethylcarbodiimide hydrochloride (422 mg, 2.20 mmol), and dimethylaminopyridine (18.0 mg, 0.147 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 59 was obtained as a yellow solid (8.10 mg, 0.0218 mmol, 2.9%). NMR measurement spectra and results of mass spectrometry by HR-ESI-MS are as follows.
¹H NMR (DMSO, 400 MHz): δ 5.51 (2H, d, J = 8.0 Hz), 6.16 (2H, d, J = 14.8 Hz), 6.57-6.72 (5H, m), 6.88 (2H, d, J = 16.0 Hz), 7.30 (2H, d, J = 8.0 Hz), 7.73 (2H, s), 7.96 (2H, s); HRMS (ESI-TOF) m/z: [M + Na]⁺ calcd for C₂₁H₁₇N₅O₂Na 394.1280; Found 394.1279.

The confirmed structure is as follows.

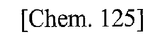

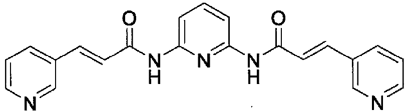

### [Example 60: Production of compound 60]

To a solution of 2-(aminomethyl)aniline (120 mg, 0.982 mmol) in dichloromethane (10 ml), trans-3-(3-pyridyl)acrylic acid (310 mg, 2.08 mmol), 1-(3-dimethylaminopropyl)-3-ethylcarbodiimide hydrochloride (565 mg, 2.95 mmol), and dimethylaminopyridine (23.9 mg, 0.196 mmol) were added at room temperature, and the mixture was stirred at the same temperature as above for 15 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 89:11). Compound 60 was obtained as a yellow solid (29.6 mg, 0.0770 mmol, 7.8%). NMR measurement spectra and results of mass spectrometry by HR-ESI-MS are as follows.
¹H NMR (CDCl₃, 400 MHz): δ 4.46 (2H, d, J = 6.0 Hz), 6.81 (1H, d, J = 16.0 Hz), 7.04 (1H, d, J = 16.0 Hz), 7.18 (1H, t, J = 7.6 Hz), 7.28-7.35 (2H, m), 7.44 (1H, dd, J = 8.0, 4.8 Hz), 7.50 (1H, dd, J = 8.0, 4.8 Hz), 7.56 (1H, d, J = 16.0 Hz), 7.66 (1H, d, J = 16.0 Hz), 7.79 (1H, d, J = 3.6 Hz), 7.99 (1H, d, J = 7.6 Hz), 8.09 (1H, d, J = 7.6 Hz), 8.55 (1H, dd, J = 4.8, 1.6 Hz), 8.60 (1H, dd, J = 4.8, 1.6 Hz), 8.75 (1H, d, J = 2.4 Hz), 8.83-8.90 (2H, m); HRMS (ESI-TOF) m/z: [M + Na]⁺ calcd for C₂₃H₂₀N₄O₂Na 407.1484; Found 407.1480.

The confirmed structure is as follows.

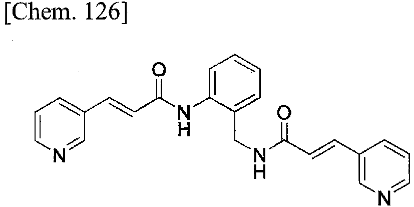

### [Example 61: Production of compound 61]

To a solution of 3-(aminomethyl)aniline (150 mg, 1.23 mmol) in dichloromethane (12 ml), trans-3-(3-pyridyl)acrylic acid (385 mg, 2.58 mmol), 1-(3-dimethylaminopropyl)-3-ethylcarbodiimide hydrochloride (495 mg, 2.58 mmol), and dimethylaminopyridine (30.1 mg, 0.246 mmol) were added at room temperature, and the mixture was stirred at the same temperature as above for 23 hours. The reaction mixture was concentrated under reduced pressure. Chloroform was added to the residue, and crystals were collected by filtration using a Kiriyama funnel. Then, compound 61 was obtained as a yellow solid (24.3 mg, 0.0632 mmol, 5.1%). NMR measurement spectra and results of mass spectrometry by HR-ESI-MS are as follows. ¹H NMR (CDCl₃, 400 MHz): δ 4.41 (2H, d, J = 5.6 Hz), 6.82 (1H, d, J = 16.0 Hz), 6.93 (1H, d, J = 16.0 Hz), 7.02 (1H, d, J = 8.0 Hz), 7.31 (1H, t, J = 8.0 Hz), 7.43-7.69 (7H, m), 7.98-8.04 (2H, m), 8.55-8.59 (2H, m), 8.74 (1H, t, J = 6.0 Hz), 8.78 (1H, d, J = 2.4 Hz), 8.81 (1H, d, J = 2.4 Hz); HRMS (ESI-TOF) m/z: [M + Na]⁺ calcd for C₂₃H₂₀N₄O₂Na 407.1484; Found 407.1482.

The confirmed structure is as follows.

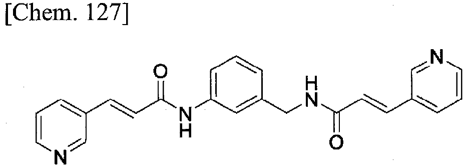

### [Example 62: Production of compound 63]

### (1) Production of intermediate 56

A known compound represented by the following structural formula (intermediate 56) was synthesized in accordance with the method described in EP556738 A1.

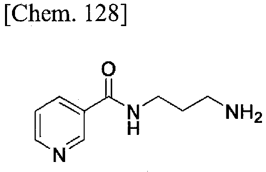

### (2) Production of compound 63

To a solution of intermediate 56 (202 mg, 1.13 mmol) in dichloromethane (3 ml), nosyl chloride (250 mg, 1.13 mmol) and triethylamine (0.189 ml, 1.36 mmol) were added at room temperature, and the mixture was stirred at the same temperature as above for 17 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 92:8). Compound 63 was obtained as a yellow solid (390 mg, 1.07 mmol, 94%). NMR measurement spectra and results of mass spectrometry by HR-ESI-MS are as follows.
¹H NMR (CDCl₃, 400 MHz): δ1.83-1.95 (2H, m), 3.22-3.29 (2H, m), 3.61-3.70 (2H, m), 6.31 (1H, br), 7.60-7.76 (4H, m), 7.84 (1H, d, J = 9.2 Hz), 8.10-8.15 (1H, m), 8.49 (1H, d, J = 6.4 Hz), 8.81 (1H, br), 9.45 (1H, s); HRMS (ESI-TOF) m/z: [M + Na]⁺ calcd for C₁₅H₁₆N₄O₅Na 387.0739; Found 387.0736.

The confirmed structure is as follows.

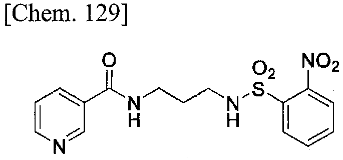

### [Example 63: Production of compound 68]

To a solution of intermediate 55 (35 mg, 0.158 mmol) in dichloromethane (2 ml), 4-(3-butyn-1-yloxy)benzoic acid (30.0 mg, 0.158 mmol), 1-(3-dimethylaminopropyl)-3-ethylcarbodiimide hydrochloride (61.3 mg, 0.320 mmol), and dimethylaminopyridine (19.5 mg, 0.160 mmol) were added at room temperature, and the mixture was stirred at the same temperature as above for 6 hours. Water was added to the reaction mixture, followed by extraction with chloroform. The organic layer was washed with saturated saline, dried over anhydrous magnesium sulfate, then filtered, and concentrated under reduced pressure. Compound 68 was obtained as a yellow solid (11.0 mg, 0.0280 mmol, 18%). NMR measurement spectra and results of mass spectrometry by HR-ESI-MS are as follows.
¹H NMR (CDCl₃, 400 MHz): δ 1.40-1.54 (4H, m), 1.59-1.72 (4H, m), 2.05 (1H, t, J = 2.8 Hz), 2.70 (2H, dt, J =6.8, 2.8 Hz), 3.44-3.53 (4H, m), 4.12 (2H, t, J = 7.2 Hz), 6.35 (1H, br), 6.91 (2H, d, J = 8.4 Hz), 7.64 (1H, dd, J = 8.0, 4.8 Hz), 7.72-7.76 (3H, m), 8.58 (1H, d, J = 7.6 Hz), 8.72 (1H, d, J = 4.0 Hz); HRMS (ESI-TOF) m/z: [M + Na]⁺ calcd for C₂₃H₂₇N₃O₃Na 416.1950; Found 416.1945.

The confirmed structure is as follows.

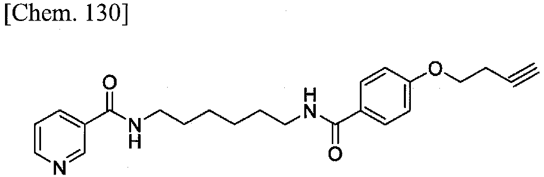

### [Example 64: Production of compound 69]

To a solution of 3,3'-(ethane-1,2-diylbis(oxy))bis(propan-1-amine) (118 mg, 0.669 mmol) in dichloroethane (5 ml), trans-3-(3-pyridyl)acrylic acid (200 mg, 1.33 mmol), 1-(3-dimethylaminopropyl)-3-ethylcarbodiimide hydrochloride (255 mg, 1.33 mmol), and dimethylaminopyridine (163 mg, 1.33 mmol) were added at 60°C, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 7:3). Compound 69 was obtained as a yellow oil (100 mg, 0.228 mmol, 34%). NMR measurement spectra and results of mass spectrometry by HR-ESI-MS are as follows.
¹H NMR (DMSO, 400 MHz): δ 3.20-3.26 (4H, m), 3.42-3.47 (10H, m), 6.71 (2H, d, J = 16.0 Hz), 7.40-7.46 (4H, m), 7.97 (2H, d, J = 6.4 Hz), 8.21 (2H, t, J = 5.6 Hz), 8.53 (2H, d, J = 8.8 Hz), 8.74 (2H, d, J = 2.0 Hz); HRMS (ESI-TOF) m/z: [M + Na]⁺ calcd for C₂₄H₃₀N₄O₄Na 461.2165; Found 461.2164.

The confirmed structure is as follows.

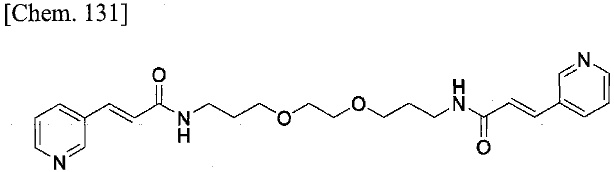

### [Example 65: Production of compound 70]

To a solution of 3,3'-(ethane-1,2-diylbis(oxy))bis(propan-1-amine) (2.47 ml, 13.7 mmol) in chloroform (40 ml), di-t-butyl dicarbonate (1.00 g, 4.58 mmol) was added, and the mixture was stirred at room temperature for 2 days. Water was added to the reaction mixture, followed by extraction with chloroform. The organic layer was washed with saturated saline, dried over anhydrous magnesium sulfate, then filtered, and concentrated under reduced pressure (quantitative). To a solution of the crude compound (150 mg, 0.543 mmol) in dichloromethane (4 ml), nicotinic acid (100 mg, 0.814 mmol), 1-(3-dimethylaminopropyl)-3-ethylcarbodiimide hydrochloride (312 mg, 1.63 mmol), and dimethylaminopyridine (13.0 mg, 0.109 mmol) were added at room temperature, and the mixture was stirred at the same temperature as above for 2 hours. A saturated aqueous solution of sodium bicarbonate was added to the reaction mixture, followed by extraction with dichloromethane. The organic layer was washed with saturated saline, dried over anhydrous magnesium sulfate, then filtered, and concentrated under reduced pressure. The residue was purified by silica gel column chromatography (chloroform:methanol = 19:1). Compound 70 was obtained as a yellow oil (207 mg, 0.543 mmol, 99%). NMR measurement spectra and results of mass spectrometry by HR-ESI-MS are as follows.
¹H NMR (CDCl₃, 400 MHz): δ1.43 (9H, s), 1.68-1.77 (2H, m), 1.89-1.98 (2H, m), 3.15-3.21 (2H, m), 3.52-3.73 (10H, m), 5.69 (1H, s), 7.40 (1H, s), 7.55 (1H, s), 8.23 (1H, d, J = 7.6 Hz), 8.75 (1H, br), 8.99 (1H, br); HRMS (ESI-TOF) m/z: [M + Na]⁺ calcd for C₁₉H₃₁N₃O₅Na 404.2161; Found 404.2156.

The confirmed structure is as follows.

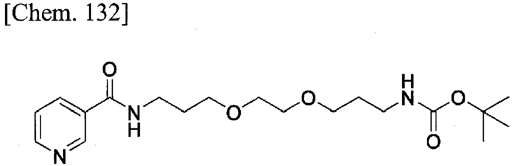

### [Example 66: Production of compound 71]

To a solution of 2,2'-(ethane-1,2-diylbis(oxy))bis(ethan-1-amine) (3.36 ml, 22.9 mmol) in chloroform (40 ml), di-t-butyl dicarbonate (1.00 g, 4.58 mmol) was added, and the mixture was stirred at room temperature for 2 days. Water was added to the reaction mixture, followed by extraction with chloroform. The organic layer was washed with saturated saline, dried over anhydrous magnesium sulfate, then filtered, and concentrated under reduced pressure (quantitative). To a solution of the crude compound (637 mg, 2.57 mmol) in dichloromethane (5 ml), nicotinic acid (316 mg, 2.57 mmol), 1-(3-dimethylaminopropyl)-3-ethylcarbodiimide hydrochloride (985 mg, 5.14 mmol), and dimethylaminopyridine (62.8 mg, 0.514 mmol) were added at room temperature, and the mixture was stirred at the same temperature as above for 14 hours. A saturated aqueous solution of sodium bicarbonate was added to the reaction mixture, followed by extraction with dichloromethane. The organic layer was washed with saturated saline, dried over anhydrous magnesium sulfate, then filtered, and concentrated under reduced pressure. The residue was purified by silica gel column chromatography (chloroform:methanol = 19:1). Compound 71 was obtained as a yellow oil (908 mg, 2.57 mmol, 99%). NMR measurement spectra and results of mass spectrometry by HR-ESI-MS are as follows.
¹H NMR (CDCl₃, 400 MHz): δ 1.62 (9H, s), 3.25-3.35 (2H, m), 3.54-3.71 (10H, m), 6.95 (1H, s), 7.37-7.43 (1H, m), 8.15 (1H, d, J = 8.4 Hz), 8.73 (1H, br), 9.04 (1H, br); HRMS (ESI-TOF) m/z: [M + Na]⁺ calcd for C₁₇H₂₇N₃O₅Na 376.1848; Found 376.1842.

The confirmed structure is as follows.

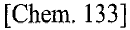

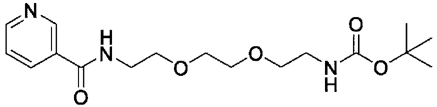

### [Example 67: Production of compound 62]

To a solution of 4-(aminomethyl)aniline (300 mg, 2.46 mmol) in dichloromethane (3 ml), trans-3-(3-pyridyl)-acrylic acid (771 mg, 5.17 mmol), 1-(3-dimethylaminopropyl)-3-ethylcarbodiimide hydrochloride (1.18 g, 6.16 mmol), and dimethylaminopyridine (60.1 mg, 0.492 mmol) were added at room temperature, and the mixture was stirred at the same temperature as above for 15 hours. Water was added to the reaction mixture, and the resulting solid was collected by filtration with a Kiriyama funnel and washed with water and methanol. Then, compound 62 was obtained as a yellow solid (19.5 mg, 0.0507 mmol, 2.1%). NMR measurement spectra and results of mass spectrometry by HR-ESI-MS are as follows.
¹H NMR (DMSO, 400 MHz): δ 4.37 (2H, d, J = 6.0 Hz), 6.80 (1H, d, J = 16.0 Hz), 6.92 (1H, d, J = 15.6 Hz), 7.28 (2H, d, J = 8.8 Hz), 7.42-7.50 (2H, m), 7.51 (1H, d, J = 16.0 Hz), 7.52 (1H, d, J = 15.6 Hz), 7.67 (2H, d, J = 8.8 Hz), 8.01 (2H, ddt, J = 16.8, 8.0, 2.0 Hz), 8.57 (1H, ddd, J = 11.6, 4.8, 1.6 Hz), 8.67 (1H, t-like, J = 6.0 Hz), 8.77 (1H, d, J = 2.4 Hz), 8.82 (1H, d, J = 2.4 Hz), 10.30 (1H, br); HRMS (ESI-TOF) m/z: [M + Na]⁺ calcd for C₂₃H₂₀N₄O₂Na 407.1484; Found 407.1480.

The confirmed structure is as follows.

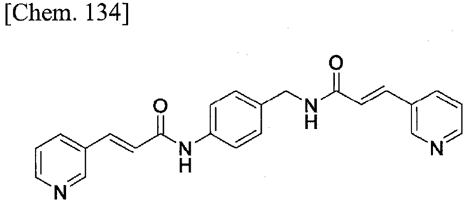

### [Example 68: Production of compound 64]

### (1) Production of intermediate 63

A known compound represented by the following structural formula (intermediate 63) was synthesized in accordance with the method described in Molecules, 2023, 28 (1), 5.

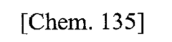

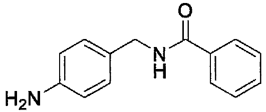

### (2) Production of compound 64

To a solution of intermediate 63 (180 mg, 0.533 mmol) in dichloromethane (5 ml), trans-3-(3-pyridyl)-acrylic acid (131 mg, 0.878 mmol), 1-(3-dimethylaminopropyl)-3-ethylcarbodiimide hydrochloride (305 mg, 1.59 mmol), and dimethylaminopyridine (19.4 mg, 0.159 mmol) were added at room temperature, and the mixture was stirred at the same temperature as above for 3 hours. The reaction mixture was concentrated under reduced pressure. Then, chloroform was added to the residue, and the resulting solid was collected by filtration with a Kiriyama funnel. Compound 64 was obtained as a yellow solid (22.8 mg, 0.0638 mmol, 12%). NMR measurement spectra and results of mass spectrometry by HR-ESI-MS are as follows. ¹H NMR (DMSO, 400 MHz): δ 4.45 (2H, d, J = 6.0 Hz), 6.92 (1H, d, J = 16.0 Hz), 7.29 (2H, d, J = 8.4 Hz), 7.42-7.56 (4H, m), 7.61 (1H, d, J = 16.0 Hz), 7.66 (2H, d, J = 8.4 Hz), 7.90 (2H, d, J = 7.2 Hz), 8.03 (1H, dt, J = 8.4, 2.0 Hz), 8.58 (1H, dd, J = 4.8, 1.6 Hz), 8.81 (1H, d, J = 1.6 Hz), 9.03 (1H, t-like, J = 2.0 Hz), 10.28 (1H, s); HRMS (ESI-TOF) m/z: [M + Na]⁺ calcd for C₂₂H₁₉N₃O₂Na 380.1375; Found 380.1371.

The confirmed structure is as follows.

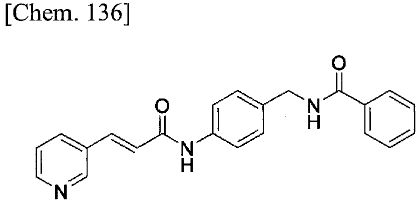

### [Example 69: Production of compound 65]

### (1) Production of intermediate 64

A known compound represented by the following structural formula (intermediate 64) was synthesized in accordance with the method described in European Journal of Medicinal Chemistry, 2020, 185, 111823.

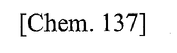

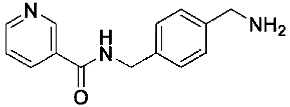

### (2) Production of compound 65

To a solution of intermediate 64 (250 mg, 1.04 mmol) in dichloromethane (5 ml), phenylpropionic acid (312 mg, 2.08 mmol), 1-(3-dimethylaminopropyl)-3-ethylcarbodiimide hydrochloride (598 mg, 3.12 mmol), and dimethylaminopyridine (25.0 mg, 0.205 mmol) were added at room temperature, and the mixture was stirred at the same temperature as above for 14 hours. Water was added to the reaction mixture, followed by extraction with chloroform. The organic layer was washed with an aqueous sodium hydroxide solution, dried over anhydrous magnesium sulfate, then filtered, and concentrated under reduced pressure. The residue was purified by silica gel column chromatography (chloroform:methanol = 97:3). Compound 65 was obtained as a yellow solid (30.5 mg, 0.0817 mmol, 7.9%). NMR measurement spectra and results of mass spectrometry by HR-ESI-MS are as follows.
¹H NMR (CD₃OD, 400 MHz): δ 2.53 (2H, t, J = 7.6 Hz), 2.92 (2H, t, J = 7.6 Hz), 4.30 (2H, s), 4.56 (2H, s), 7.07-7.30 (9H, m), 7.55 (1H, dd, J = 8.0, 4.8 Hz), 8.26 (1H, dt, J = 8.0, 2.0 Hz), 8.69 (1H, dd, J = 4.8, 2.0 Hz), 8.99 (1H, d, J = 2.0 Hz); HRMS (ESI-TOF) m/z: [M + Na]⁺ calcd for C₂₃H₂₃N₃O₂Na 396.1688; Found 396.1682.

The confirmed structure is as follows.

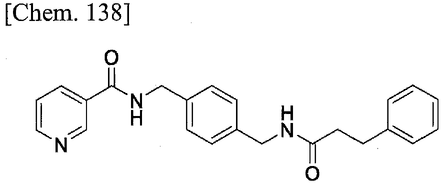

### [Example 70: Production of compound 66]

To a solution of intermediate 64 (270 mg, 1.12 mmol) in dichloromethane (5 ml), cinnamic acid (332 mg, 2.24 mmol), 1-(3-dimethylaminopropyl)-3-ethylcarbodiimide hydrochloride (644 mg, 3.36 mmol), and dimethylaminopyridine (27.4 mg, 0.224 mmol) were added at room temperature, and the mixture was stirred at the same temperature as above for 14 hours. Water was added to the reaction mixture, followed by extraction with chloroform. The organic layer was washed with an aqueous sodium hydroxide solution, dried over anhydrous magnesium sulfate, then filtered, and concentrated under reduced pressure. The residue was purified by silica gel column chromatography (chloroform:methanol = 19:1). Compound 66 was obtained as a yellow solid (9.00 mg, 0.0242 mmol, 2.2%). NMR measurement spectra and results of mass spectrometry by HR-ESI-MS are as follows.
¹H NMR (CD₃OD, 400 MHz): δ4.49 (2H, s), 4.58 (2H, s), 6.65 (1H, d, J = 16.0 Hz), 7.30-7.42 (7H, m), 7.50-7.59 (4H, m), 8.26 (1H, dt, J = 8.4, 2.0 Hz), 8.68 (1H, dd, J = 5.2, 2.0 Hz), 8.99 (1H, d, J = 2.4 Hz); HRMS (ESI-TOF) m/z: [M + Na]⁺ calcd for C₂₃H₂₁N₃O₂Na 394.1531; Found 394.1527.

The confirmed structure is as follows.

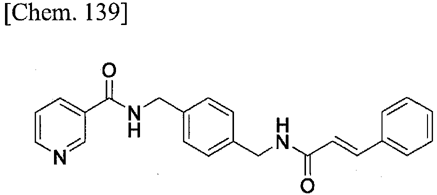

### [Example 71: Production of compound 67]

### (1) Production of intermediate 62

To a solution of 4-(aminomethyl)aniline (300 mg, 2.46 mmol) in dichloromethane (25 ml), triethylamine (0.76 ml, 5.41 mmol) and nicotinoyl chloride hydrochloride (394 mg, 2.21 mmol) were added under an ice bath, and the mixture was stirred at the same temperature as above for 1 hour. The reaction mixture was concentrated under reduced pressure, and insoluble matter was filtered, followed by the concentration of the filtrate under reduced pressure. The residue was purified by silica gel column chromatography (chloroform:methanol = 94:6). Intermediate 62 represented by the following structural formula was obtained as a yellow oil (300 mg, 1.32 mmol, 54%).
HRMS (ESI-TOF) m/z: [M + Na]⁺ calcd for C₁₃H₁₃N₃ONa 250.0951; Found 250.0956.

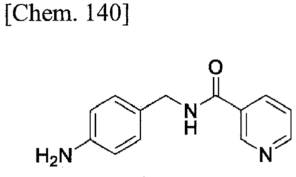

### (2) Production of intermediate 65

To a solution of intermediate 62 (100 mg, 0.440 mmol) in dichloromethane (3 ml), 5-hydroxynicotinic acid (67.3 mg, 0.484 mmol) and N,N'-dicyclohexylcarbodiimide (182 mg, 0.882 mmol) were added at room temperature, and the mixture was stirred at the same temperature as above for 6 hours. The reaction mixture was concentrated under reduced pressure, and the residue was then purified by silica gel column chromatography (chloroform:methanol = 90:10). Intermediate 65 was obtained as a yellow solid (153 mg, 0.439 mmol, 99%).
HRMS (ESI-TOF) m/z: [M - H]⁻ calcd for C₁₉H₁₅N₄O₃ 347.1144; Found 347.1138.

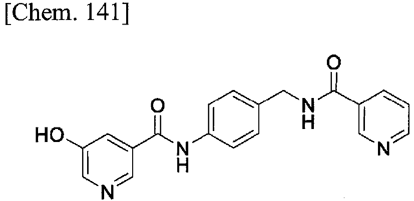

### (3) Production of compound 67

To a solution of intermediate 65 (10.0 mg, 0.0287 mmol) in acetone (2 ml), propargyl bromide (4.10 mg, 0.0345 mmol) and potassium carbonate (4.80 mg, 0.0347 mmol) were added at room temperature, and the mixture was stirred at 50°C for 4 hours. Water was added to the reaction mixture, followed by extraction with ethyl acetate. The organic layer was washed with saturated saline, dried over anhydrous magnesium sulfate, then filtered, and concentrated under reduced pressure. Chloroform was added to the residue, and the resulting solid was collected by filtration with a Kiriyama funnel. Compound 67 was obtained as a yellow solid (9.00 mg, 0.0233 mmol, 81%). NMR measurement spectra and results of mass spectrometry by HR-ESI-MS are as follows. ¹H NMR (DMSO, 400 MHz): δ3.69 (1H, t, J = 2.4 Hz), 4.49 (2H, d, J = 6.0 Hz), 5.00 (2H, d, J = 2.4 Hz), 7.34 (2H, d, J = 8.4 Hz), 7.53 (1H, dd, J = 8.0, 4.8 Hz), 7.72 (2H, d, J = 8.4 Hz), 7.90 (1H, t, J = 2.4 Hz), 8.24 (1H, dt, J = 8.4, 2.0 Hz), 8.52 (1H, d, J = 2.8 Hz), 8.72 (1H, dd, J = 4.8, 1.2 Hz), 8.76 (1H, d, J = 1.6 Hz), 9.06 (1H, d, J = 2.0 Hz), 9.25 (1H, t-like, J = 5.6 Hz), 10.42 (1H, s); HRMS (ESI-TOF) m/z: [M + H]⁺ calcd for C₂₂H₁₉N₄O₃ 387.1457; Found 387.1452.

The confirmed structure is as follows.

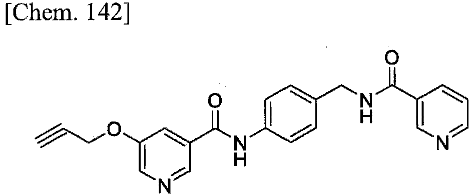

### [Example 72: Production of compound 72]

### (1) Production of intermediate 57

To a solution of 6-aminonicotinonitrile (300 mg, 2.52 mmol) in dichloromethane (3 ml), triethylamine (0.710 ml, 5.04 mmol), dimethylaminopyridine (61.5 mg, 0.505 mmol), and di-t-butyl dicarbonate (1.38 g, 6.30 mmol) were added at room temperature, and the mixture was stirred at the same temperature as above for 14 hours. The reaction mixture was concentrated under reduced pressure, and the residue was then purified by silica gel column chromatography (hexane:ethyl acetate = 3:1). Intermediate 57 represented by the following structural formula was obtained as white crystals (747 mg, 2.34 mmol, 93%).
HRMS (ESI-TOF) m/z: [M + Na]⁺ calcd for C₁₆H₂₁N₃O₄Na 342.1424; Found 342.1426.

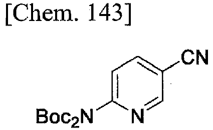

### (2) Production of intermediate 58

To a solution of intermediate 57 (9.43 g, 29.5 mmol) in methanol (100 ml), nickel chloride hexahydrate (7.02 g, 29.5 mmol) and sodium borohydride (3.35 g, 88.6 mmol) were added under an ice bath, and the mixture was stirred at the same temperature as above for 1 hour. Triethylamine (8.30 ml, 59.1 mmol), nicotinoyl chloride hydrochloride (10.5 g, 59.1 mmol), and dichloromethane (100 ml) were added thereto, and the mixture was further stirred at the same temperature as above for 2 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (hexane:ethyl acetate = 2:1). Intermediate 58 represented by the following structural formula was obtained as a yellow oil (4.37 g, 10.2 mmol, 34%). HRMS (ESI-TOF) m/z: [M + Na]⁺ calcd for C₂₂H₂₈N₄O₅Na 451.1952; Found 451.1957.

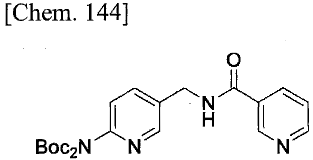

### (3) Production of intermediate 59

To a solution of intermediate 58 (150 mg, 0.340 mmol) in dichloromethane (3 ml), hydrochloric acid (4 M solution in dioxane, 1.70 ml, 6.80 mmol) was added at room temperature, and the mixture was stirred at the same temperature as above for 1 hour. The reaction mixture was concentrated under reduced pressure. Intermediate 59 represented by the following structural formula was obtained as a yellow solid (114 mg, 0.340 mmol, 99%).
HRMS (ESI-TOF) m/z: [M + H]⁺ calcd for C₁₂H₁₃N₄O 229.1084; Found 229.1089.

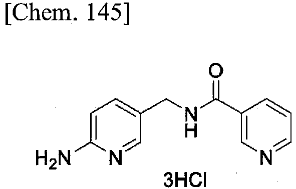

### (4) Production of compound 72

To a solution of intermediate 59 (50.0 mg, 0.148 mmol) in dichloromethane (2 ml), isobutyryl chloride (0.0180 ml, 0.172 mmol) and triethylamine (0.310 ml, 2.22 mmol) were added at room temperature, and the mixture was stirred at the same temperature as above for 2 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 72 was obtained as a yellow solid (18.4 mg, 0.0617 mmol, 42%). NMR measurement spectra and results of mass spectrometry by HR-ESI-MS are as follows. ¹H NMR (CDCl₃, 400 MHz): δ1.23 (6H, d, J = 6.4 Hz), 2.55 (1H, q, J = 6.4 Hz), 4.59 (2H, d, J = 5.2 Hz), 7.14 (1H, s), 7.37 (1H, s), 7.67 (1H, d, J = 8.0 Hz), 8.04-8.24 (4H, m), 8.69 (1H, br), 9.00 (1H, br); HRMS (ESI-TOF) m/z: [M + Na]⁺ calcd for C₁₆H₁₈N₄O₂Na 321.1322; Found 321.1327.

The confirmed structure is as follows.

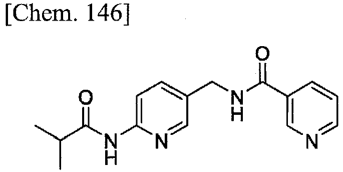

### [Example 73: Production of compound 73]

To a solution of intermediate 59 (50.0 mg, 0.148 mmol) in dichloromethane (2 ml), 4-cyanobenzoic acid (26.2 mg, 0.178 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (3.60 mg, 0.0296 mmol) were added at room temperature, and the mixture was stirred at the same temperature as above for 14 hours. A saturated aqueous solution of sodium bicarbonate was added to the reaction mixture, followed by extraction with dichloromethane. The organic layer was washed with saturated saline, dried over anhydrous magnesium sulfate, then filtered, and concentrated under reduced pressure. The residue was purified by silica gel column chromatography (chloroform:methanol = 19:1). Compound 73 was obtained as a yellow solid (16.0 mg, 0.0448 mmol, 30%). NMR measurement spectra and results of mass spectrometry by HR-ESI-MS are as follows.
¹H NMR (DMSO, 400 MHz): δ 4.51 (2H, d, J = 6.0 Hz), 7.52 (1H, dd, J = 7.6, 5.2 Hz), 7.84 (1H, d, J = 8.4 Hz), 7.99 (2H, d, J = 8.0 Hz), 8.12-8.17 (3H, m), 8.22 (1H, d, J = 8.0 Hz), 8.39 (1H, s), 8.72 (1H, J = 4.8 Hz), 9.04 (1H, s), 9.28 (1H, t-like, J = 5.2 Hz), 11.13 (1H, br); HRMS (ESI-TOF) m/z: [M + Na]⁺ calcd for C₂₀H₁₅N₅O₂Na 380.1123; Found 380.1115.

The confirmed structure is as follows.

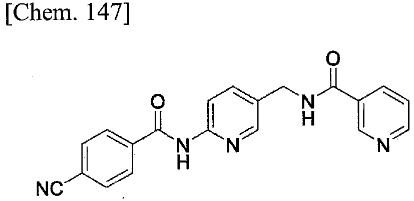

### [Example 74: Production of compound 74]

To a solution of intermediate 59 (50.0 mg, 0.148 mmol) in dichloromethane (2 ml), cinnamic acid (33.8 mg, 0.228 mmol), N,N-diisopropylethylamine (0.330 ml, 1.92 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0377 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 74 was obtained as a yellow solid (25.1 mg, 0.0700 mmol, 37%). NMR measurement spectra and results of mass spectrometry by HR-ESI-MS are as follows. ¹H NMR (DMSO, 400 MHz): δ4.48 (2H, d, J = 5.6 Hz), 7.04 (1H, d, J = 16.0 Hz), 7.39-7.71 (7H, m), 7.79 (1H, d, J = 8.4 Hz), 8.19-8.24 (2H, m), 8.33 (1H, d, J = 8.0 Hz), 8.72 (1H, d, J = 4.8 Hz), 9.04 (1H, s), 9.24 (1H, t-like, J = 6.0 Hz), 10.72 (1H, br); HRMS (ESI-TOF) m/z: [M + Na]⁺ calcd for C₂₁H₁₈N₄O₂Na 381.1327; Found 381.1321.

The confirmed structure is as follows.

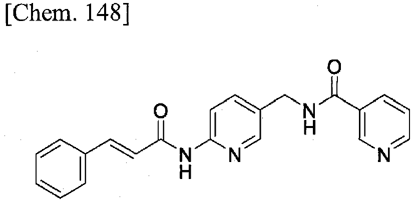

### [Example 75: Production of compound 75]

To a solution of intermediate 59 (50.0 mg, 0.148 mmol) in dichloromethane (2 ml), trans-3-(3-pyridyl)acrylic acid (34.3 mg, 0.230 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (3.60 mg, 0.0296 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 4:1). Compound 75 was obtained as a yellow solid (12.0 mg, 0.0334 mmol, 23%). NMR measurement spectra and results of mass spectrometry by HR-ESI-MS are as follows.
¹H NMR (DMSO, 400 MHz): δ4.48 (2H, d, J = 6.0 Hz), 7.14 (1H, d, J = 15.6 Hz), 7.46-7.54 (2H, m), 7.67 (1H, d, J = 15.6 Hz), 7.80 (1H, dd, J = 8.4, 2.0 Hz), 8.00 (1H, d, J = 7.6 Hz), 8.18-8.25 (2H, m), 8.34 (1H, d, J = 1.6 Hz), 8.59 (1H, d, J = 4.0 Hz), 8.72 (1H, d, J = 4.0 Hz), 8.81 (1H, s), 9.05 (1H, s), 9.29 (1H, t-like, J = 5.6 Hz), 10.79 (1H, s); HRMS (ESI-TOF) m/z: [M + Na]⁺ calcd for C₂₀H₁₇N₅O₂Na 382.1280; Found 382.1274.

The confirmed structure is as follows.

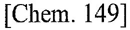

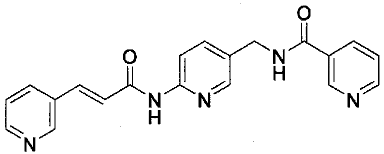

### [Example 76: Production of compound 76]

To a solution of intermediate 59 (50.0 mg, 0.148 mmol) in dichloromethane (2 ml), pivaloyl chloride (0.0300 ml, 0.246 mmol) and triethylamine (0.260 ml, 1.88 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 76 was obtained as a yellow solid (40.9 mg, 0.131 mmol, 89%). NMR measurement spectra and results of mass spectrometry by HR-ESI-MS are as follows. ¹H NMR (DMSO, 400 MHz): δ1.22 (9H, s), 4.46 (2H, d, J = 5.2 Hz), 7.52 (1H, dd, J = 8.0, 5.2 Hz), 7.74 (1H, d, J = 8.8 Hz), 8.00 (1H, d, J = 8.8 Hz), 8.20 (1H, d, J = 8.4 Hz), 8.31 (1H, s), 8.71 (1H, d, J = 4.8 Hz), 9.03 (1H, s), 9.23 (1H, t-like, J = 6.0 Hz), 9.79 (1H, br); HRMS (ESI-TOF) m/z: [M + Na]⁺ calcd for C₁₇H₂₀N₄O₂Na 335.1484; Found 335.1479.

The confirmed structure is as follows.

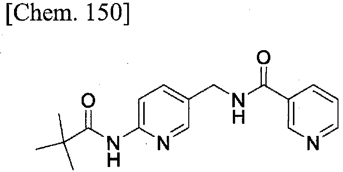

### [Example 77: Production of compound 77]

### (1) Production of intermediate 60

To a solution of intermediate 57 (200 mg, 0.626 mmol) in methanol (2 ml), nickel chloride hexahydrate (149 mg, 0.626 mmol) and sodium borohydride (71.1 mg, 1.88 mmol) were added under an ice bath, and the mixture was stirred at the same temperature as above for 1 hour. A saturated aqueous solution of sodium bicarbonate was added to the reaction mixture, followed by extraction with ethyl acetate. The organic layer was washed with saturated saline, dried over anhydrous magnesium sulfate, then filtered, and concentrated under reduced pressure. To a solution of the residue in dichloromethane (2 ml), triethylamine (0.260 ml, 1.87 mmol) and nicotinoyl chloride hydrochloride (270 mg, 1.52 mmol) were added at room temperature, and the mixture was further stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (hexane: ethyl acetate = 1:2). Intermediate 60 represented by the following structural formula was obtained as a yellow oil (160 mg, 0.352 mmol, 56%).
¹H NMR (DMSO, 400 MHz): δ1.46 (18H, s), 4.35 (2H, d, J = 5.6 Hz), 6.76 (1H, d, J = 16.0 Hz), 7.42-7.53 (2H, m), 7.65 (1H, d, J = 8.4 Hz), 7.75 (1H, d, J = 8.8 Hz), 7.99 (1H, d, J = 6.0 Hz), 8.18 (1H, s), 8.55 (1H, s), 8.67 (1H, s), 8.76 (1H, br).

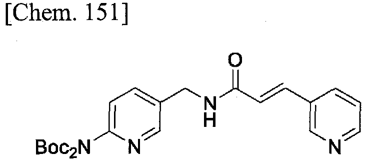

### (2) Production of intermediate 61

To a solution of intermediate 60 (160 mg, 0.352 mmol) in dichloromethane (3 ml), hydrochloric acid (4 M solution in dioxane, 1.80 ml, 7.20 mmol) was added at room temperature, and the mixture was stirred at the same temperature as above for 1 hour. The reaction mixture was concentrated under reduced pressure. Intermediate 61 represented by the following structural formula was obtained as a yellow solid (128 mg, 0.352 mmol, 99%).
¹H NMR (DMSO, 400 MHz): δ4.29 (2H, d, J = 5.2 Hz), 6.85 (1H, d, J = 16.0 Hz), 7.00 (1H, d, J = 9.2 Hz), 7.55 (1H, d, J = 16.0 Hz), 7.69 (1H, br), 7.85-7.91 (2H, m), 8.05 (2H, br), 8.26 (1H, br), 8.68 (1H, br), 8.80-8.93 (2H, m).

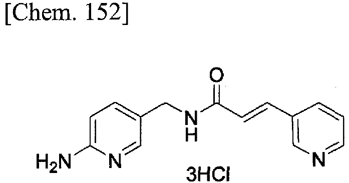

### (3) Production of compound 77

To a solution of intermediate 61 (50.0 mg, 0.148 mmol) in dichloromethane (2 ml), benzoyl chloride (0.0300 ml, 0.258 mmol) and triethylamine (0.120 ml, 0.861 mmol) were added at room temperature, and the mixture was stirred at the same temperature as above for 4 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 77 was obtained as a yellow solid (34.0 mg, 0.0949 mmol, 64%). NMR measurement spectra and results of mass spectrometry by HR-ESI-MS are as follows.
¹H NMR (DMSO, 400 MHz): δ4.40 (2H, d, J = 5.2 Hz), 6.76 (1H, d, J = 16.0 Hz), 7.41-7.61 (6H, m), 7.74-7.83 (4H, m), 7.99 (1H, d, J = 7.6 Hz), 8.24 (1H, s), 8.55 (1H, d, J = 4.4 Hz), 8.72-8.79 (2H, m); HRMS (ESI-TOF) m/z: [M + Na]⁺ calcd for C₂₁H₁₈N₄O₂Na 381.1327; Found 381.1319.

The confirmed structure is as follows.

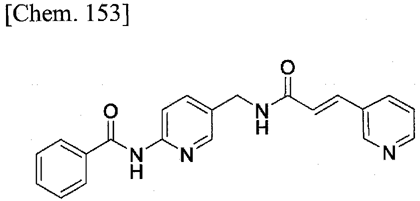

### [Example 78: Production of compound 78]

To a solution of intermediate 61 (50.0 mg, 0.148 mmol) in dichloromethane (2 ml), p-toluic acid (27.8 mg, 0.204 mmol), N,N-diisopropylethylamine(0.150 ml, 0.861 mmol), 1-[bis(dimethylamino)methylene]-1H-1,2,3-triazolo[4,5-b]pyridinium3-oxide hexafluorophosphate (HATU) (129 mg, 0.339 mmol), and dimethylaminopyridine (3.60 mg, 0.0296 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 78 was obtained as a yellow solid (14.3 mg, 0.0384 mmol, 26%). NMR measurement spectra and results of mass spectrometry by HR-ESI-MS are as follows. ¹H NMR (CDCl₃, 400 MHz): δ2.43 (3H, s), 4.59 (2H, d, J = 6.0 Hz), 6.01 (1H, br), 6.49 (1H, d, J = 15.6 Hz), 7.29-7.34 (3H, m), 7.69 (1H, d, J = 15.6 Hz), 7.73-7.84 (4H, m), 8.29 (1H, s), 8.38 (1H, d, J = 8.8 Hz), 8.56 (1H, s), 8.58 (1H, d, J = 4.0 Hz), 8.75 (1H, s); HRMS (ESI-TOF) m/z: [M + Na]⁺ calcd for C₂₂H₂₀N₄O₂Na 395.1484; Found 395.1477.

The confirmed structure is as follows.

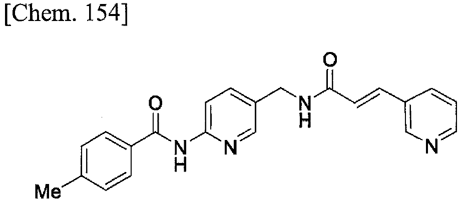

### [Example 79: Production of compound 79]

To a solution of intermediate 59 (50.0 mg, 0.148 mmol) in dichloromethane (2 ml), N,N-diisopropylethylamine (0.170 ml, 0.976 mmol) and benzenesulfonyl chloride(40.6 mg, 0.230 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 4:1). Compound 79 was obtained as a yellow solid (50.0 mg, 0.136 mmol, 92%). NMR measurement spectra and results of mass spectrometry by HR-ESI-MS are as follows.
¹H NMR (DMSO, 400 MHz): δ 4.41 (2H, d, J = 5.6 Hz), 7.40 (1H, d, J = 9.6 Hz), 7.51-7.62 (3H, m), 7.80-7.91 (4H, m), 8.20 (1H, dt, J = 8.0, 2.0 Hz), 8.55 (1H, s), 8.72 (1H, dd, J = 4.8, 1.6 Hz), 9.02 (1H, d, J = 2.0 Hz), 9.16 (1H, t-like, J = 5.6 Hz); HRMS (ESI-TOF) m/z: [M + H]⁺ calcd for C₁₈H₁₇N₄SO₃ 369.1021; Found 369.1014.

The confirmed structure is as follows.

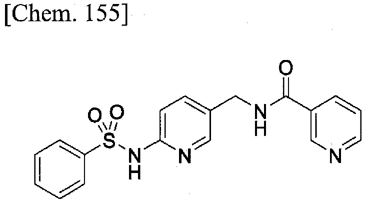

### [Example 80: Production of compound 80]

To a solution of intermediate 59 (50.0 mg, 0.148 mmol) in dichloromethane (2 ml), p-vinylbenzoic acid (32.0 mg, 0.242 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (3.60 mg, 0.0296 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 80 was obtained as a yellow solid (4.50 mg, 0.0126 mmol, 8.5%). NMR measurement spectra and results of mass spectrometry by HR-ESI-MS are as follows. ¹H NMR (CDCl₃, 400 MHz): δ 4.79 (2H, d, J = 4.0 Hz), 5.42 (1H, d, J = 10.4 Hz), 5.89 (1H, d, J = 17.2 Hz), 6.71-6.88 (1H, m), 7.52-7.62 (2H, m), 8.11 (1H, t-like, J = 6.4 Hz), 8.25 (1H, d, J = 8.4 Hz), 8.33 (1H, d, J = 8.0 Hz), 8.45 (1H, t-like, J = 6.8 Hz), 8.82 (1H, d-like, J = 6.0 Hz), 8.93 (1H, d, J = 9.2 Hz), 9.03-9.13 (2H, m), 10.22 (2H, br); HRMS (ESI-TOF) m/z: [M + H]⁺ calcd for C₂₁H₁₉N₄O₂ 359.1508; Found 359.1501.

The confirmed structure is as follows.

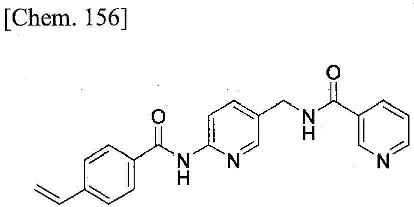

### [Example 81: Production of compound 81]

To a solution of intermediate 59 (50.0 mg, 0.148 mmol) in dichloromethane (2 ml), o-trifluoromethylbenzoic acid (44.0 mg, 0.231 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 81 was obtained as a yellow solid (5.00 mg, 0.0125 mmol, 8.4%). NMR measurement spectra and results of mass spectrometry by HR-ESI-MS are as follows.
¹H NMR (CDCl₃, 400 MHz): δ4.66 (2H, d, J = 5.6 Hz), 6.78 (1H, br), 7.44 (1H, dd, J = 7.6, 3.2 Hz), 7.57-7.88 (6H, m), 8.19 (1H, d, J = 7.6 Hz), 8.26 (1H, s), 8.41 (1H, d, J = 8.8 Hz), 8.75 (1H, d, J = 3.6 Hz), 9.03 (1H, br); HRMS (ESI-TOF) m/z: [M + Na]⁺ calcd for C₂₀H₁₅F₃N₄O₂Na 423.1045; Found 423.1040.

The confirmed structure is as follows.

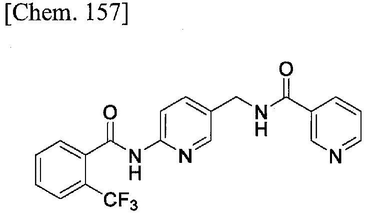

### [Example 82: Production of compound 82]

To a solution of intermediate 59 (50.0 mg, 0.148 mmol) in dichloromethane (2 ml), m-trifluoromethylbenzoic acid (44.0 mg, 0.231 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 82 was obtained as a yellow solid (11.5 mg, 0.0287 mmol, 19%). NMR measurement spectra and results of mass spectrometry by HR-ESI-MS are as follows.
¹H NMR (CDCl₃, 400 MHz): δ4.69 (2H, d, J = 5.6 Hz), 6.50 (1H, br), 7.42 (1H, dd, J = 8.0, 4.8 Hz), 7.67 (1H, t, J = 8.4 Hz), 7.82-7.87 (2H, m), 8.10-8.17 (2H, m), 8.22 (1H, s), 8.35-8.42 (2H, m), 8.73-8.78 (2H, m), 9.00 (1H, s); HRMS (ESI-TOF) m/z: [M + Na]⁺ calcd for C₂₀H₁₅F₃N₄O₂Na 423.1045; Found 423.1039.

The confirmed structure is as follows.

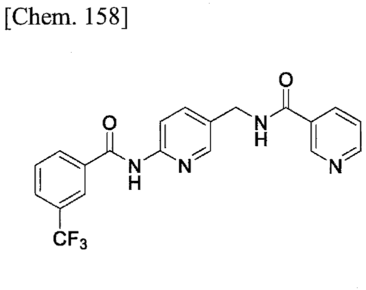

### [Example 83: Production of compound 83]

To a solution of intermediate 59 (50.0 mg, 0.148 mmol) in dichloromethane (2 ml), p-trifluoromethylbenzoic acid (44.0 mg, 0.231 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 83 was obtained as a yellow solid (17.1 mg, 0.0427 mmol, 29%). NMR measurement spectra and results of mass spectrometry by HR-ESI-MS are as follows.
¹H NMR (CDCl₃, 400 MHz): δ4.69 (2H, d, J = 6.0 Hz), 6.57 (1H, br), 7.43 (1H, dd, J = 8.4, 4.8 Hz), 7.74 (1H, d, J = 8.0 Hz), 7.79 (2H, d, J = 8.8 Hz), 8.09 (2H, d, J = 8.8 Hz), 8.17 (1H, d, J = 8.4 Hz), 8.20 (1H, d, J = 8.0 Hz), 8.36 (1H, s), 8.43 (1H, d, J = 8.8 Hz), 8.76 (1H, d, J = 5.6 Hz), 9.01 (1H, s); HRMS (ESI-TOF) m/z: [M + Na]⁺ calcd for C₂₀H₁₅F₃N₄O₂Na 423.1045; Found 423.1039.

The confirmed structure is as follows.

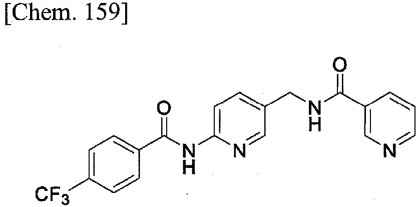

### [Example 84: Production of compound 84]

To a solution of intermediate 59 (50.0 mg, 0.148 mmol) in dichloromethane (2 ml), 3-fluoro-5-trifluoromethylbenzoic acid (47.0 mg, 0.226 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (4.60 mg, 0.0378 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 84 was obtained as a yellow solid (24.4 mg, 0.0583 mmol, 39%). NMR measurement spectra and results of mass spectrometry by HR-ESI-MS are as follows.
¹H NMR (CDCl₃, 400 MHz): δ4.70 (2H, d, J = 6.0 Hz), 6.55 (1H, br), 7.43 (1H, dd, J = 8.0, 3.2 Hz), 7.56 (1H, d, J = 7.2 Hz), 7.85-7.91 (2H, m), 8.04 (1H, s), 8.14 (2H, m), 8.37 (1H, s), 8.41 (1H, d, J = 8.0 Hz), 8.77 (1H, d, J = 3.2 Hz), 9.01 (1H, s); HRMS (ESI-TOF) m/z: [M + Na]⁺ calcd for C₂₀H₁₄F₄N₄O₂Na 441.0951; Found 441.0947.

The confirmed structure is as follows.

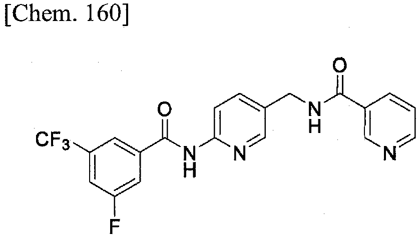

### [Example 85: Production of compound 85]

To a solution of intermediate 59 (50.0 mg, 0.148 mmol) in dichloromethane (2 ml), o-trifluoromethoxybenzoic acid (47.0 mg, 0.228 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 85 was obtained as a yellow solid (16.2 mg, 0.0389 mmol, 26%). NMR measurement spectra and results of mass spectrometry by HR-ESI-MS are as follows.
¹H NMR (CDCl₃, 400 MHz): δ4.69 (2H, d, J = 6.0 Hz), 7.33-7.71 (4H, m), 7.92-7.99 (2H, m), 8.17 (1H, s), 8.42 (1H, d, J = 8.8 Hz), 8.44 (1H, s), 8.57 (1H, d, J = 8.8 Hz), 8.77 (1H, d, J = 3.6 Hz), 9.44 (1H, br), 9.55 (1H, br); HRMS (ESI-TOF) m/z: [M + Na]⁺ calcd for C₂₀H₁₅F₃N₄O₃Na 439.0994; Found 439.0989.

The confirmed structure is as follows.

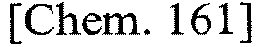

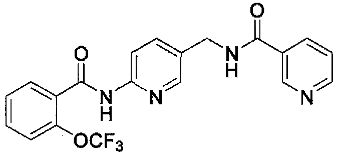

### [Example 86: Production of compound 86]

To a solution of intermediate 59 (50.0 mg, 0.148 mmol) in dichloromethane (2 ml), m-trifluoromethoxybenzoic acid (47.0 mg, 0.228 mmol), N,N-diisopropylethylamine (0.160 ml, 0.918 mmol)1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were| added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 86 was obtained as a yellow solid (15.4 mg, 0.0370 mmol, 25%). NMR measurement spectra and results of mass spectrometry by HR-ESI-MS are as follows.
¹H NMR (CDCl₃, 400 MHz): δ4.78 (2H, d, J = 3.6 Hz), 7.46 (2H, d, J = 8.4 Hz), 7.59 (1H, t, J = 8.0 Hz), 7.95-8.03 (2H, m), 8.24 -8.41 (2H, m), 8.72-8.86 (2H, m), 8.94-9.02 (2H, m), 9.80 (1H, br), 10.07 (1H, br); HRMS (ESI-TOF) m/z: [M + Na]⁺ calcd for C₂₀H₁₅F₃N₄O₃Na 439.0994; Found 439.0990.

The confirmed structure is as follows.

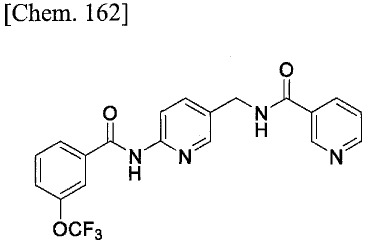

### [Example 87: Production of compound 87]

To a solution of intermediate 59 (50.0 mg, 0.148 mmol) in dichloromethane (2 ml), p-trifluoromethoxybenzoic acid (47.0 mg, 0.228 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 87 was obtained as a yellow solid (13.4 mg, 0.0322 mmol, 22%). NMR measurement spectra and results of mass spectrometry by HR-ESI-MS are as follows.
¹H NMR (CDCl₃, 400 MHz): δ4.80 (2H, d, J = 4.4 Hz), 7.36 (2H, d, J = 8.4 Hz), 8.11-8.16 (1H, m), 8.39 (2H, d, J = 8.4 Hz), 8.47 (1H, d, J = 8.8 Hz), 8.86 (1H, s), 8.94 (1H, d, J = 8.8 Hz), 9.08 (1H, d, J = 4.8 Hz), 9.13 (1H, d, J = 7.2 Hz), 10.25 (2H, s); HRMS (ESI-TOF) m/z: [M + Na]⁺ calcd for C₂₀H₁₅F₃N₄O₃Na 439.0994; Found 439.0992.

The confirmed structure is as follows.

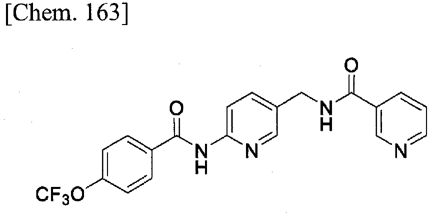

### [Example 88: Production of compound 88]

To a solution of intermediate 59 (50.0 mg, 0.148 mmol) in dichloromethane (2 ml), 2,2-dimethylbutanoic acid (27.9 mg, 0.240 mmol), N,N-diisopropylethylamine (0.170 ml, 0.975 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (3.60 mg, 0.0296 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 88 was obtained as a yellow solid (6.00 mg, 0.0184 mmol, 12%). NMR measurement spectra and results of mass spectrometry by HR-ESI-MS are as follows.
¹H NMR (CDCl₃, 400 MHz): δ0.89 (3H, t, J = 7.2 Hz), 1.65 (2H, q, J = 7.2 Hz), 4.62 (2H, d, J = 6.0 Hz), 6.65 (1H, br), 7.39 (1H, dd, J = 8.0, 4.8 Hz), 7.72 (1H, d, J = 8.8 Hz), 8.05 (1H, s), 8.13 (1H, d, J = 7.6 Hz), 8.24 (1H, d, J = 8.0 Hz), 8.27 (1H, s), 8.72 (1H, d, J = 4.8 Hz), 8.98 (1H, s); HRMS (ESI-TOF) m/z: [M + Na]⁺ calcd for C₁₈H₂₂N₄O₂Na 349.1640; Found 349.1635.

The confirmed structure is as follows.

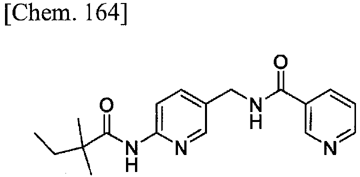

### [Example 89: Production of compound 89]

To a solution of intermediate 59 (50.0 mg, 0.148 mmol) in dichloromethane (2 ml), p-trifluoromethylphenylacetic acid (78.0 mg, 0.382 mmol), N,N-diisopropylethylamine (0.160 ml, 0.918 mmol), 1-[bis(dimethylamino)methylene]-1H-1,2,3-triazolo[4,5-b]pyridinium3-oxide hexafluorophosphate (HATU) (144 mg, 0.378 mmol), and dimethylaminopyridine (5.00 mg, 0.0411 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 89 was obtained as a yellow solid (44.0 mg, 0.106 mmol, 72%). NMR measurement spectra and results of mass spectrometry by HR-ESI-MS are as follows.
¹H NMR (CDCl₃, 400 MHz): δ4.01 (2H, s), 4.74 (2H, s), 6.73 (1H, d, J = 6.4 Hz), 7.55 (2H, d, J = 8.0 Hz), 7.59 (2H, d, J = 8.0 Hz), 8.08 (1H, t, J = 6.4 Hz), 8.20 (1H, t, J = 6.4 Hz), 8.43 (1H, d, J = 8.0 Hz), 8.53 (1H, d, J = 8.0 Hz), 8.73 (1H, s), 8.94 (1H, d, J = 5.2 Hz), 9.11 (1H, d, J = 8.8 Hz), 10.18 (1H, s); HRMS (ESI-TOF) m/z: [M + Na]⁺ calcd for C₂₁H₁₇F₃N₄O₂Na 437.1201; Found 437.1195.

The confirmed structure is as follows.

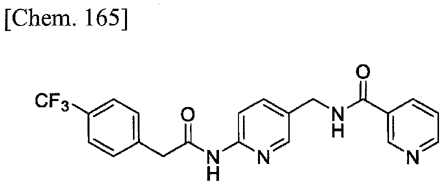

### [Example 90: Production of compound 91]

### (1) Production of intermediate 66

A known compound represented by the following structural formula (intermediate 66) was synthesized in accordance with the method described in Bioorganic & Medicinal Chemistry Letters, 2009, 19 (14), 3716-3720.

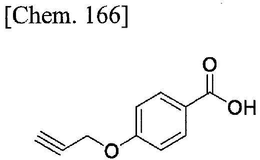

### (2) Production of compound 91

To a solution of intermediate 59 (50.0 mg, 0.148 mmol) in dichloromethane (2 ml), intermediate 66 (27.9 mg, 0.240 mmol), N,N-diisopropylethylamine (0.190 ml, 1.09 mmol), 1-[bis(dimethylamino)methylene]-1H-1,2,3-triazolo[4,5-b]pyridinium3-oxide hexafluorophosphate (HATU) (167 mg, 0.438 mmol), and dimethylaminopyridine (5.30 mg, 0.0436 mmol) were added at room temperature, and the mixture was stirred at the same temperature as above for 38 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 91 was obtained as a yellow solid (2.40 mg, 0.00621 mmol, 4.2%). NMR measurement spectra and results of mass spectrometry by HR-ESI-MS are as follows. ¹H NMR (CDCl₃, 400 MHz): δ2.56 (1H, t, J = 2.0 Hz), 4.66 (2H, d, J = 5.6 Hz), 4.77 (2H, d, J = 2.0 Hz), 6.51 (1H, br), 7.07 (2H, d, J = 8.8 Hz), 7.41 (1H, dd, J = 8.0, 4.8 Hz), 7.78 (1H, dd, J = 8.4, 2.4 Hz), 7.90 (2H, d, J = 8.8 Hz), 8.14 (1H, dt, J = 8.4, 2.0 Hz), 8.32 (1H, d, J = 2.0 Hz), 8.37 (1H, d, J = 8.8 Hz), 8.52 (1H, s), 8.75 (1H, d, J = 5.2 Hz), 9.00 (1H, s); HRMS (ESI-TOF) m/z: [M + Na]⁺ calcd for C₂₂H₁₈N₄O₃Na 409.1277; Found 409.1273.

The confirmed structure is as follows.

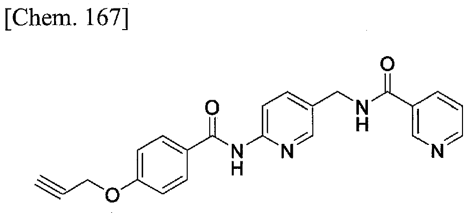

### [Example 91: Production of compound 90]

### (1) Production of intermediate 67

A known compound represented by the following structural formula (intermediate 67) was synthesized in accordance with the method described in Journal of the American Chemical Society, 2008, 130 (38), 12564-12565.

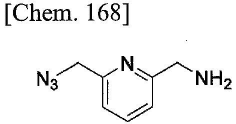

### (2) Production of compound 90

To a solution of intermediate 67 (420 mg, 2.58 mmol) in dichloromethane (3 ml), triethylamine (1.44 ml, 10.3 mmol) and nicotinyl chloride hydrochloride (690 mg, 3.86 mmol) were added at room temperature, and the mixture was stirred at the same temperature as above for 2.5 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (ethyl acetate:methanol = 1:1). Compound 90 was obtained as a yellow solid (545 mg, 2.03 mmol, 79%). NMR measurement spectra and results of mass spectrometry by HR-ESI-MS are as follows. ¹H NMR (CDCl₃, 400 MHz): δ4.45 (2H, s), 4.80 (2H, d, J = 4.4 Hz), 7.23-7.31 (2H, m), 7.41 (1H, dd, J = 8.0, 4.8 Hz), 7.69 (1H, br), 7.74 (1H, t, J = 7.6 Hz), 8.20 (1H, dt, J = 8.0, 2.0 Hz), 8.75 (1H, d, J = 3.6 Hz), 9.11 (1H, s); HRMS (ESI-TOF) m/z: [M + Na]⁺ calcd for C₁₃H₁₂N₆ONa 291.0970; Found 291.0964.

The confirmed structure is as follows.

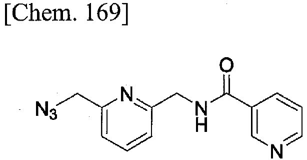

### [Example 92: Production of compound 92]

### (1) Production of intermediate 68

A known compound represented by the following structural formula (intermediate 68) was synthesized in accordance with the method described in Journal of the American Chemical Society, 2008, 130 (38), 12564-12565.

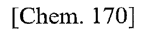

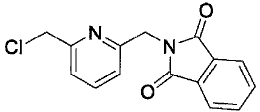

### (2) Production of intermediate 69

To a solution of isoindolinone (440 mg, 3.31 mmol) in dimethylformamide (15 ml), sodium hydride (160 mg, 6.62 mmol) was added under an ice bath, and the mixture was stirred at the same temperature as above for 20 minutes. A solution of intermediate 68 (950 mg, 3.31 mmol) in dimethylformamide (5 ml) was added to the reaction mixture, and the mixture was stirred at room temperature for 12 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 4:1). Intermediate 69 represented by the following structural formula was obtained as a yellow solid (1.01 g, 2.64 mmol, 80%).
HRMS (ESI-TOF) m/z: [M + Na]⁺ calcd for C₂₃H₁₇N₃O₃Na 406.1162; Found 406.1165.

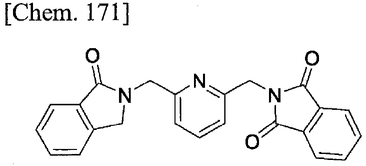

### (3) Production of intermediate 70

To a solution of intermediate 69 (100 mg, 0.261 mmol) in methanol (2 ml), hydrazine monohydrate (160 mg, 6.62 mmol) was added at room temperature, and the mixture was stirred at the same temperature as above for 14 hours. Water was added to the reaction mixture, followed by extraction with chloroform. The organic layer was washed with a saturated aqueous solution of sodium bicarbonate, dried over anhydrous magnesium sulfate, then filtered, and concentrated under reduced pressure. Intermediate 70 represented by the following structural formula was obtained as a yellow solid (60.5 mg, 0.0239 mmol, 92%).

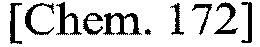

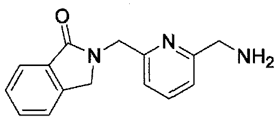

### (4) Production of compound 92

To a solution of intermediate 70 (60.5 mg, 0.239 mmol) in dichloromethane (2 ml), triethylamine (0.100 ml, 0.720 mmol) and nicotinyl chloride hydrochloride (52 mg, 0.290 mmol) were added at room temperature, and the mixture was stirred at the same temperature as above for 3 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 92 was obtained as a yellow solid (19.5 mg, 0.0544 mmol, 23%). NMR measurement spectra and results of mass spectrometry by HR-ESI-MS are as follows. ¹H NMR (CDCl₃, 400 MHz): δ4.63 (2H, s), 4.99 (2H, s), 5.30 (2H, s), 7.42-7.61 (5H, m), 7.79-7.87 (3H, m), 8.21 (1H, br), 8.56 (1H, br), 8.78 (1H, d, J = 4.0 Hz), 9.28 (1H, br); HRMS (ESI-TOF) m/z: [M + Na]⁺ calcd for C₂₁H₁₈N₄O₂Na 381.1327; Found 381.1322.

The confirmed structure is as follows.

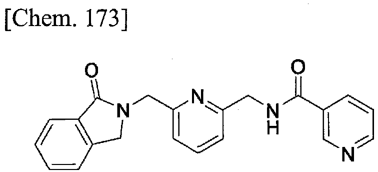

### [Example 93: Production of compound 93]

To a solution of intermediate 62 (100 mg, 0.44 mmol) in dimethylformamide (1.5 ml), oxetane-3-carboxylic acid (49.4 mg, 0.48 mmol), 1-(3-dimethylaminopropyl)-3-ethylcarbodiimide hydrochloride (93.0 mg, 0.48 mmol), and 1-hydroxybenzotriazole (65 mg, 0.48 mmol) were added at room temperature, and the mixture was stirred at the same temperature as above for 3 days. A saturated aqueous solution of sodium bicarbonate was added to the reaction mixture, followed by extraction with chloroform. The organic layer was concentrated under reduced pressure, and the residue was purified by silica gel column chromatography (chloroform:methanol = 100:0 → 93:7). Compound 93 was obtained as a white solid (24 mg, 0.077 mmol, 18%).
¹H NMR (DMSO, 400 MHz): δ3.89-3.99 (1H, m), 4.43 (2H, d, J = 6.0 Hz), 4.64-4.73 (4H, m), 7.27 (2H, d, J = 7.2 Hz), 7.50-7.58 (3H, m), 8.20-8.24 (1H, m), 8.70-8.72 (1H, m), 9.03-9.05 (1H, m), 9.18-9.24 (1H, m), 9.93 (1H, s);
ESI-MS m/z: 312 [M+H]⁺

The confirmed structure is as follows.

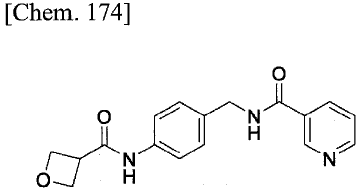

### [Example 94: Production of compound 94]

### (1) Production of intermediate 71

To a solution of t-butyl (5-(aminomethyl)pyridin-2-yl)carbamate (1.12 g, 5.00 mmol) in chloroform (20 ml), diisopropylethylamine (1.92 mL, 11.0 mmol) and nicotinoyl chloride hydrochloride (979 mg, 5.50 mmol) were added at room temperature, and the mixture was stirred at the same temperature as above for 2 hours. A saturated aqueous solution of sodium bicarbonate was added to the reaction mixture, followed by extraction with chloroform. The organic layer was dried over anhydrous sodium sulfate, then filtered, and concentrated under reduced pressure. The residue was purified by silica gel column chromatography (ethyl acetate:methanol = 100:0 → 92:8). Intermediate 71 represented by the following structural formula was obtained as a white solid (1.54 g, 4.67 mmol, 93%).

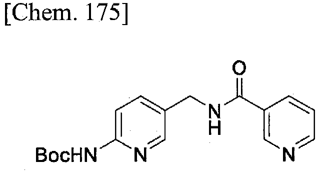

### (2) Production of intermediate 72

To a solution of intermediate 71 (1.54 g, 4.67 mmol) in 1,4-dioxane (23 ml), hydrochloric acid (4 M solution in dioxane, 23 ml) and methanol (5 ml) were added at room temperature, and the mixture was stirred at the same temperature as above for 18 hours. Deposits were filtered, washed with ethyl acetate, and then dried in vacuum. Intermediate 72 represented by the following structural formula was obtained as a white solid (1.25 g, 4.13 mmol, 88%).

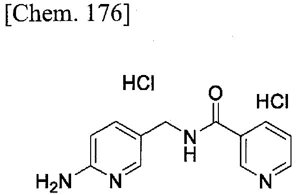

### (3) Production of compound 94

To a solution of 1-(t-butyloxycarbonyl)azetidine-3-carboxylic acid (151 mg, 0.75 mmol) in tetrahydrofuran (10 ml), oxalyl chloride (66 µl, 0.75 mmol) was added at room temperature, and the mixture was stirred at the same temperature as above for 1 hour. Then, intermediate 72 (151 mg, 0.50 mmol) and diisopropylethylamine (437 µl, 2.50 mmol) were added thereto at room temperature, and the mixture was stirred at the same temperature as above for 2 hours. A saturated aqueous solution of ammonium chloride was added thereto, followed by extraction with chloroform. The organic layer was dried over anhydrous sodium sulfate, then filtered, and concentrated under reduced pressure. The residue was purified by silica gel column chromatography (ethyl acetate:methanol = 100:0→92:8). Compound 94 was obtained as a white solid (116 mg, 0.282 mmol, 56%).
¹H NMR (DMSO, 400 MHz): δ1.45 (9H, s), 3.34-3.41 (1H, br), 4.12-4.23 (4H, m), 4.65 (2H, d, J = 6.0 Hz), 6.47 (1H, t, J = 6.0 Hz), 7.40-7.44 (1H, m), 7.77 (1H, dd, J = 8.4, 2.4 Hz), 8.05 (1H, m), 8.13-8.15 (1H, m), 8.23-8.26 (1H, m), 8.29 (1H d, J = 2.0 Hz), 8.76 (1H, dd, 4.8, 1.6 Hz), 8.98 (1H, d, 2.0 Hz);
ESI-MS m/z: 412 [M+H]⁺

The confirmed structure is as follows.

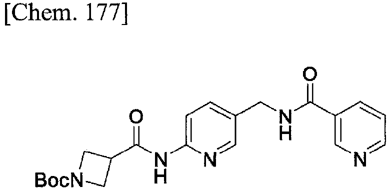

### [Example 95: Production of compound 95]

To a solution of intermediate 10 (441 mg, 1.92 mmol) in dichloromethane (10 ml), nicotinoyl chloride hydrochloride (411 mg, 2.31 mmol) and triethylamine (1.07 ml, 7.69 mmol) were added under an ice bath, and the mixture was stirred at room temperature for 14 hours. The reaction mixture was concentrated under reduced pressure. The residue was purified by silica gel column chromatography (chloroform:methanol = 10:1). Compound 95 was obtained as a yellow solid (266 mg, 0.605 mmol, 32%). NMR measurement spectra and results of mass spectrometry by HR-ESI-MS are as follows.
¹H NMR (DMSO, 400 MHz): δ1.22-1.56 (8H, m), 2.20-2.52 (10H, m), 2.68 (2H, t, J = 5.6 Hz), 3.25 (2H, q, J = 6.4 Hz), 4.40 (2H, t, J = 5.6 Hz), 7.49 (1H, dd, J = 7.2, 5.2 Hz), 7.58 (1H, dd, J = 7.2, 5.2 Hz), 8.16 (1H, d, J = 8.0 Hz), 8.28 (1H, d, J = 8.0 Hz), 8.61 (1H, br), 8.68 (1H, d, J = 3.6 Hz), 8.82 (1H, d, J = 3.6 Hz), 8.98 (1H, s), 9.08 (1H, s); HRMS (ESI-TOF) m/z: [M + H]⁺ calcd for C₂₄H₃₄N₅O₃ 440.2656; Found 440.2654.

The confirmed structure is as follows.

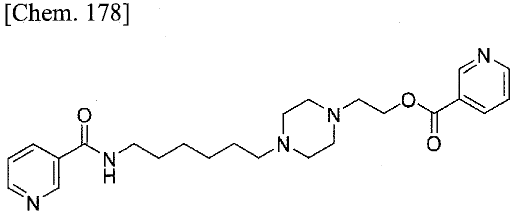

### [Example 96: Production of compound 96]

### (1) Production of intermediate 71

To a solution of compound 4 (677 mg, 2.30 mmol) in dichloromethane (7 ml), mesyl chloride (0.214 ml, 2.76 mmol) and triethylamine (0.962 ml, 6.90 mmol) were added under an ice bath, and the mixture was stirred at room temperature for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 20:1). Intermediate 71 was obtained as a yellow oil (856 mg, 2.30 mmol, 99%).

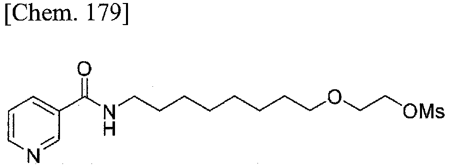

### (2) Production of intermediate 72

To a solution of intermediate 71 (904 mg, 2.43 mmol) in dimethylformamide (8 ml), sodium azide (189 mg, 2.91 mmol) was added at room temperature, and the mixture was stirred at 80°C for 20 hours. The reaction mixture was concentrated under reduced pressure. The residue was purified by silica gel column chromatography (chloroform:methanol = 30:1). Intermediate 72 was obtained as a yellow oil (683 mg, 2.14 mmol, 88%).

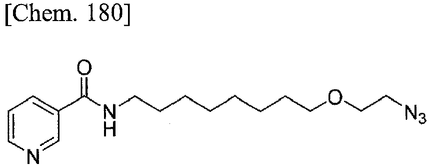

### (3) Production of compound 96

To a solution of intermediate 72 (683 mg, 2.14 mmol) in tetrahydrofuran (10 ml), triphenylphosphine (617 mg, 2.35 mmol) was added at room temperature, and the mixture was stirred at room temperature for 24 hours. Water (0.115 ml, 6.41 mmol) was added thereto, and the mixture was stirred at room temperature for 24 hours. The reaction mixture was concentrated under reduced pressure. The residue was purified by silica gel column chromatography (chloroform:methanol = 7:1). Compound 96 was obtained as yellow crystals (573 mg, 1.95 mmol, 91%). NMR measurement spectra and results of mass spectrometry by HR-ESI-MS are as follows.
¹H NMR (DMSO, 400 MHz): δ1.24-1.34 (8H, m), 1.43-1.58 (4H, m), 2.50 (2H, br), 2.62 (2H, t, J = 5.6 Hz), 3.23-3.37 (6H, m), 7.49 (1H, dd, J = 7.2, 5.2 Hz), 8.16 (1H, d, J = 7.6 Hz), 8.61 (1H, br), 8.68 (1H, d, J = 4.0 Hz), 8.98 (1H, s); HRMS (ESI-TOF) m/z: [M + H]⁺ calcd for C₁₆H₂₈N₃O₂ 294.2176; Found 294.2174.

The confirmed structure is as follows.

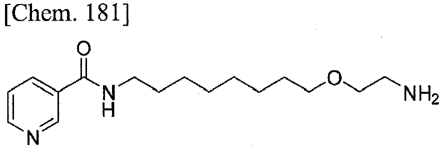

### [Example 97: Production of compound 97]

### (1) Production of intermediate 73

To a solution of compound 6 (580 mg, 1.71 mmol) in dichloromethane (7 ml), mesyl chloride (0.159 ml, 2.06 mmol) and triethylamine (0.717 ml, 5.14 mmol) were added under an ice bath, and the mixture was stirred at room temperature for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 20:1), intermediate 73 was obtained as a yellow oil (651 mg, 1.56 mmol, 91%).

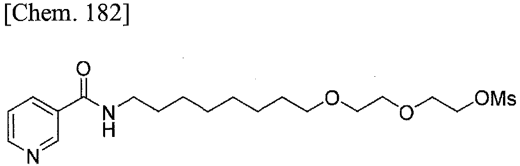

### (2) Production of intermediate 74

To a solution of intermediate 73 (651 mg, 1.56 mmol) in dimethylformamide (5 ml), sodium azide (122 mg, 1.88 mmol) was added at room temperature, and the mixture was stirred at 80°C for 20 hours. The reaction mixture was concentrated under reduced pressure. The residue was purified by silica gel column chromatography (chloroform:methanol = 30:1). Intermediate 74 was obtained as a yellow oil (439 mg, 1.21 mmol, 78%).

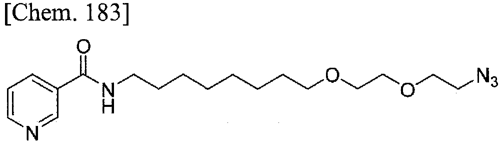

### (3) Production of compound 97

To a solution of intermediate 74 (438 mg, 1.21 mmol) in tetrahydrofuran (6 ml), triphenylphosphine (348 mg, 1.33 mmol) was added at room temperature, and the mixture was stirred at room temperature for 24 hours. Water (0.0651 ml, 3.62 mmol) was added thereto, and the mixture was stirred at room temperature for 24 hours. The reaction mixture was concentrated under reduced pressure. The residue was purified by silica gel column chromatography (chloroform:methanol = 7:1). Compound 97 was obtained as a yellow oil (408 mg, 1.21 mmol, 99%). NMR measurement spectra and results of mass spectrometry by HR-ESI-MS are as follows.
¹H NMR (CDCl₃, 400 MHz): δ1.31-1.67 (12H, m), 2.85 (2H, t, J = 5.6 Hz), 3.43-3.53 (6H, m), 3.55-3.64 (4H, m), 6.36 (1H, br), 7.39 (1H, dd, J = 7.2, 4.8 Hz), 8.12 (1H, d, J = 7.6 Hz), 8.72 (1H, d, J = 3.2 Hz), 8.95 (1H, s); HRMS (ESI-TOF) m/z: [M + Na]⁺ calcd for C₁₈H₃₁N₃O₃Na 360.2263; Found 360.2254.

The confirmed structure is as follows.

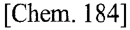

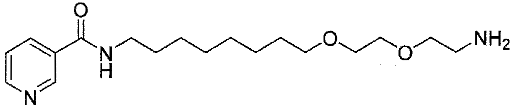

### [Example 98: Production of compound 98]

### (1) Production of intermediate 79

A known compound represented by the following structural formula (intermediate 79) was synthesized in accordance with the method described in WO97/02242.

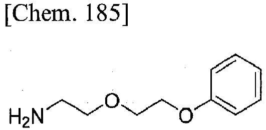

### (2) Production of compound 98

To a solution of intermediate 79 (75 mg, 0.415 mmol) in dichloromethane (3 ml), nicotinic acid (121 mg, 0.983 mmol), 1-(3-dimethylaminopropyl)-3-ethylcarbodiimide hydrochloride (190 mg, 0.991 mmol), and dimethylaminopyridine (12.6 mg, 0.103 mmol) were added at room temperature, and the mixture was stirred at the same temperature as above for 13 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 98 was obtained as a yellow oil (30.8 mg, 0.108 mmol, 22%). NMR measurement spectra and results of mass spectrometry by HR-ESI-MS are as follows.
¹H NMR (DMSO, 400 MHz): δ3.47 (2H, t, J = 6.0 Hz), 3.62 (2H, t, J = 6.0 Hz), 3.76 (2H, t, J = 4.8 Hz), 4.08 (2H, t, J = 4.8 Hz), 6.89-6.94 (3H, m), 7.23-7.29 (2H, m), 7.49 (1H, dd, J = 8.0, 4.8 Hz), 8.17 (1H, dt, J = 8.8, 2.0 Hz), 8.32 (1H, s), 8.70 (1H, dd, J = 4.8, 1.6 Hz), 8.77 (1H, t, J = 4.8 Hz); HRMS (ESI-TOF) m/z: [M + H]⁺ calcd for C₁₆H₁₉N₂O₃ 287.1390; Found 287.1388.

The confirmed structure is as follows.

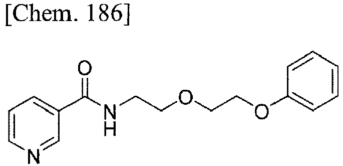

### [Example 99: Production of compound 99]

To a solution of 2-(2-(3-methoxyphenoxy)ethoxy)ethan-1-amine (175 mg, 0.828 mmol) in dichloromethane (8 ml), nicotinic acid (204 mg, 1.66 mmol), 1-(3-dimethylaminopropyl)-3-ethylcarbodiimide hydrochloride (318 mg, 1.66 mmol), and dimethylaminopyridine (20.0 mg, 0.166 mmol) were added at room temperature, and the mixture was stirred at the same temperature as above for 19 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 99 was obtained as a yellow oil (174 mg, 0.550 mmol, 66%). NMR measurement spectra and results of mass spectrometry by HR-ESI-MS are as follows.
¹H NMR (CDCl₃, 400 MHz): δ3.64-3.77 (4H, m), 3.76 (3H, s), 3.85 (2H, t, J = 4.0 Hz), 4.13 (2H, t, J = 4.0 Hz), 6.44-6.53 (3H, m), 6.75 (1H, br), 7.16 (1H, t, J = 8.0 Hz), 7.32 (1H, dd, J = 7.2, 4.8 Hz), 8.03 (1H, d, J = 7.6 Hz), 8.70 (1H, d, J = 3.6 Hz), 8.98 (1H, s); HRMS (ESI-TOF) m/z: [M + H]⁺ calcd for C₁₇H₂₁N₂O₄ 317.1496; Found 317.1493.

The confirmed structure is as follows.

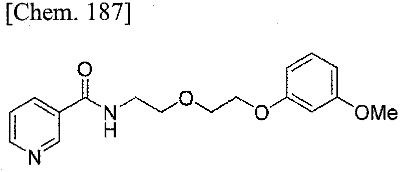

### [Example 100: Production of compound 100]

To a solution of 2,2'-ethylenedianiline (100 mg, 0.471 mmol) in dichloromethane (2 ml), nicotinic acid (145 mg, 1.18 mmol), 1-(3-dimethylaminopropyl)-3-ethylcarbodiimide hydrochloride (271 mg, 1.41 mmol), and dimethylaminopyridine (11.5 mg, 0.0942 mmol) were added at room temperature, and the mixture was stirred at the same temperature as above for 5 hours. Water was added to the reaction mixture, followed by extraction with chloroform. The organic layer was washed with saturated saline, dried over anhydrous magnesium sulfate, then filtered, concentrated under reduced pressure. Compound 100 was obtained as a yellow solid (34.8 mg, 0.0824 mmol, 17%). NMR measurement spectra and results of mass spectrometry by HR-ESI-MS are as follows.
¹H NMR (CDCl₃, 400 MHz): δ3.00 (4H, s), 7.15-7.29 (6H, m), 7.37 (2H, dd, J = 8.0, 4.8 Hz), 7.57-7.62 (4H, m), 7.95 (2H, d, J = 8.0 Hz), 8.74 (2H, d, J = 4.8 Hz), 8.80 (2H, s); HRMS (ESI-TOF) m/z: [M + H]⁺ calcd for C₂₆H₂₃N₄O₂ 423.1816; Found 423.1812.

The confirmed structure is as follows.

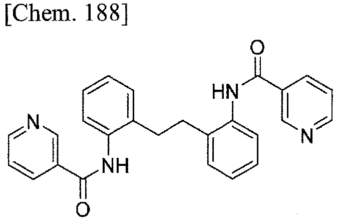

### [Example 101: Production of compound 101]

To a solution of 4,4'-ethylenedianiline (100 mg, 0.471 mmol) in dichloromethane (2 ml), nicotinic acid (145 mg, 1.18 mmol), 1-(3-dimethylaminopropyl)-3-ethylcarbodiimide hydrochloride (271 mg, 1.41 mmol), and dimethylaminopyridine (11.5 mg, 0.0942 mmol) were| added at room temperature, and the mixture was stirred at the same temperature as above for 8 hours. Water was added to the reaction mixture, followed by extraction with chloroform. The organic layer was washed with saturated saline, dried over anhydrous magnesium sulfate, then filtered, concentrated under reduced pressure. Compound 101 was obtained as a yellow solid (16.5 mg, 0.0391 mmol, 8.3%). NMR measurement spectra and results of mass spectrometry by HR-ESI-MS are as follows.
¹H NMR (CDCl₃, 400 MHz): δ2.87 (4H, s), 7.21 (4H, d, J = 3.6 Hz), 7.57 (2H, dd, J = 8.0, 4.8 Hz), 7.66 (4H, d, J = 3.6 Hz), 8.28 (2H, d, J = 8.0 Hz), 8.75 (2H, br), 9.10 (2H, s); HRMS (ESI-TOF) m/z: [M + H]⁺ calcd for C₂₆H₂₃N₄O₂ 423.1816; Found 423.1815.

The confirmed structure is as follows.

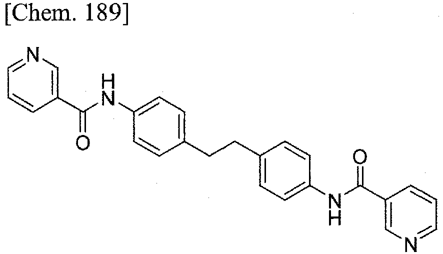

### [Example 102: Production of compound 102]

To a solution of N,N'-dimethyl-1,6-hexanediamine (100 mg, 0.693 mmol) in dichloromethane (3 ml), nicotinoyl chloride hydrochloride (308 mg, 1.73 mmol) and triethylamine (0.280 ml, 2.77 mmol) were added under an ice bath, and the mixture was stirred at the same temperature as above for 1.5 hours. Water was added to the reaction mixture, followed by extraction with chloroform. The organic layer was washed with saturated saline, dried over anhydrous magnesium sulfate, then filtered, and concentrated under reduced pressure. Compound 102 was obtained as a yellow solid (70.9 mg, 0.200 mmol, 29%). NMR measurement spectra and results of mass spectrometry by HR-ESI-MS are as follows.
¹H NMR (CDCl₃, 400 MHz): δ1.16-2.36 (8H, m), 2.92-3.62 (10H, m), 7.39 (2H, s), 7.78 (2H, d, J = 6.4 Hz), 8.60-8.70 (4H, m); HRMS (ESI-TOF) m/z: [M + H]⁺ calcd for C₂₀H₂₇N₄O₂ 355.2129; Found 355.2128.

The confirmed structure is as follows.

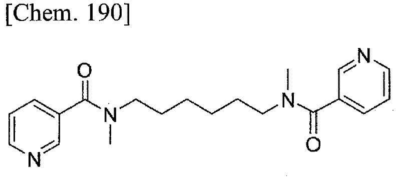

### [Example 103: Production of compound 103]

### (1) Production of intermediate 80

A known compound represented by the following structural formula (intermediate 80) was synthesized in accordance with the method described in CN107459524.

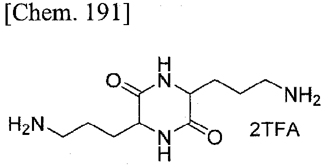

### (2) Production of compound 103

To a solution of intermediate 80 (124 mg, 0.271 mmol) in dimethylformamide (0.9 ml), nicotinic acid (100 mg, 0.813 mmol), 1-(3-dimethylaminopropyl)-3-ethylcarbodiimide hydrochloride (208 mg, 1.08 mmol), N,N-diisopropylethylamine (0.142 ml, 0.813 mmol), and dimethylaminopyridine (13.2 mg, 0.108 mmol) were added at room temperature, and the mixture was stirred at the same temperature as above for 3 hours. Methanol was added to the reaction mixture, and the resulting crystals were collected by filtration using a Kiriyama funnel. Compound 103 was obtained as a yellow solid (33.8 mg, 0.0771 mmol, 44%). NMR measurement spectra and results of mass spectrometry by HR-ESI-MS are as follows.
¹H NMR (DMSO, 400 MHz): δ1.46-1.80 (8H, m), 3.26 (4H, q, J = 6.4 Hz), 3.84-3.91 (2H, m), 7.46-7.52 (2H, m), 8.13-8.21 (4H, m), 8.64-8.70 (4H, m), 8.98 (2H, s); HRMS (ESI-TOF) m/z: [M + H]⁺ calcd for C₂₂H₂₇N₆O₄ 439.2088; Found 439.2089.

The confirmed structure is as follows.

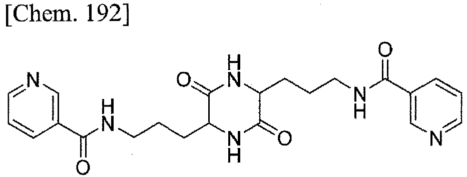

### [Example 104: Production of compound 104]

### (1) Production of intermediate 81

A known compound represented by the following structural formula (intermediate 81) was synthesized in accordance with the method described in WO2017/173202.

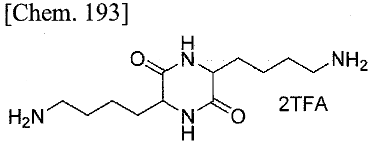

### (2) Production of compound 104

To a solution of intermediate 81 (135 mg, 0.279 mmol) in dimethylformamide (0.9 ml), nicotinic acid (103 mg, 0.837 mmol), 1-(3-dimethylaminopropyl)-3-ethylcarbodiimide hydrochloride (214 mg, 1.12 mmol), N,N-diisopropylethylamine (0.146 ml, 0.837 mmol), and dimethylaminopyridine (13.6 mg, 0.112 mmol) were added at room temperature, and the mixture was stirred at the same temperature as above for 4 hours. Methanol was added to the reaction mixture, and the resulting crystals were collected by filtration using a Kiriyama funnel. Compound 104 was obtained as a yellow solid (8.90 mg, 0.0191 mmol, 6.8%). NMR measurement spectra and results of mass spectrometry by HR-ESI-MS are as follows.
¹H NMR (DMSO, 400 MHz): δ1.24-1.76 (12H, m), 3.25 (4H, q, J = 6.4 Hz), 3.78-3.85 (2H, m), 7.46-7.52 (2H, m), 8.12-8.18 (4H, m), 8.61-8.70 (4H, m), 8.98 (2H, s); HRMS (ESI-TOF) m/z: [M + H]⁺ calcd for C₂₄H₃₁N₆O₄ 467.2401; Found 467.2403.

The confirmed structure is as follows.

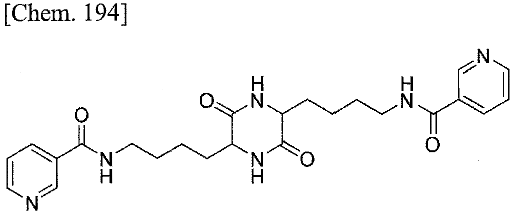

### [Example 105: Production of compound 105]

To a solution of intermediate 59 (100 mg, 0.296 mmol) in dichloromethane (3 ml), benzoyl chloride (0.0500 ml, 0.434 mmol) and triethylamine (0.410 ml, 4.06 mmol) were added at room temperature, and the mixture was stirred at the same temperature as above for 1 hour. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, ethyl acetate 100%). Compound 105 was obtained as a pale yellow solid (76.5 mg, 0.230 mmol, 78%). NMR measurement spectra and results of mass spectrometry by HR-ESI-MS are as follows. ¹H NMR (CDCl₃, 400 MHz): δ4.67 (2H, d, J = 6.0 Hz), 6.57 (1H, br), 7.42 (1H, dd, J = 8.0, 4.8 Hz), 7.49-7.61 (3H, m), 7.82 (1H, dd, J = 8.4, 2.0 Hz), 7.94 (2H, d, J = 7.6 Hz), 8.16 (1H, dt, J = 8.0, 2.0 Hz), 8.34 (1H, d, J = 2.4 Hz), 8.42 (1H, d, J = 8.8 Hz), 8.72-8.78 (2H, m), 9.01 (1H, d, J = 2.0 Hz); HRMS (ESI-TOF) m/z: [M + H]⁺ calcd for C₁₉H₁₇N₄O₂ 333.1352; Found 333.1349.

The confirmed structure is as follows.

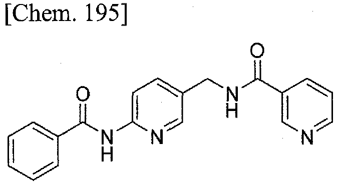

### [Example 106: Production of compound 106]

To a solution of 3-[2-(3-aminopropoxy)ethoxy]propan-1-amine (0.100 ml, 0.560 mmol) in dichloromethane (5 ml), nicotinoyl chloride hydrochloride (100 mg, 0.562 mmol) and triethylamine (0.156 ml, 1.12 mmol) were added at room temperature, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1), then solidified by the addition of 4 M HCl/dioxane (5 ml), and collected by filtration. Compound 106 was obtained as a yellow solid (18.3 mg, 0.0517 mmol, 9.2%). NMR measurement spectra and results of mass spectrometry by HR-ESI-MS are as follows.
¹H NMR (DMSO, 400 MHz): δ1.76 (2H, q, J = 6.4 Hz), 3.29-3.52 (14H, m), 7.49 (1H, dd, J = 8.0, 4.8 Hz), 8.16 (1H, d, J = 7.6 Hz), 8.63-8.70 (2H, m), 8.98 (1H, s); HRMS (ESI-TOF) m/z: [M + H]⁺ calcd for C₁₄H₂₄N₃O₃ 282.1812; Found 282.1812.

The confirmed structure is as follows.

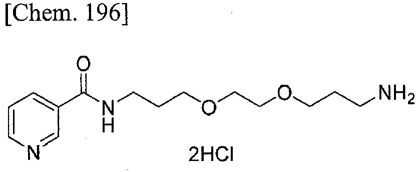

### [Example 107: Production of compound 107]

To a solution of 2-[2-(2-aminoethoxy)ethoxy]ethan-1-amine (83.0 mg, 0.560 mmol) in dichloromethane (5 ml), nicotinoyl chloride hydrochloride (100 mg, 0.562 mmol) and triethylamine (0.156 ml, 1.12 mmol) were added at room temperature, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1), then solidified by the addition of 4 M HCl/dioxane (5 ml), and collected by filtration. Compound 107 was obtained as a yellow solid (23.8 mg, 0.0730 mmol, 13%). NMR measurement spectra and results of mass spectrometry by HR-ESI-MS are as follows.
¹H NMR (DMSO, 400 MHz): δ3.26-3.56 (12H, m), 7.49 (1H, dd, J = 8.0, 4.8 Hz), 8.17 (1H, d, J = 8.0 Hz), 8.67-8.78 (2H, m), 8.99 (1H, s); HRMS (ESI-TOF) m/z: [M + Na]⁺ calcd for C₁₂H₁₉N₃O₃Na 276.1324; Found 276.1317.

The confirmed structure is as follows.

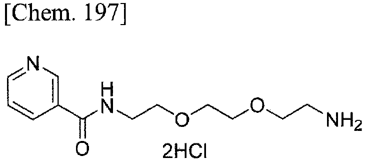

### [Example 108: Production of compound 108]

To a solution of intermediate 62 (100 mg, 0.440 mmol) in dichloroethane (5 ml), oxane-4-carboxylic acid (114 mg, 0.876 mmol), 1-(3-dimethylaminopropyl)-3-ethylcarbodiimide hydrochloride (168 mg, 0.876 mmol), and triethylamine (0.120 ml, 0.861 mmol) were added at 60°C, and the mixture was stirred at the same temperature as above for 3 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 85:15). Compound 108 was obtained as a yellow solid (110 mg, 0.324 mmol, 74%). NMR measurement spectra and results of mass spectrometry by HR-ESI-MS are as follows.
¹H NMR (CD₃OD, 400 MHz): δ1.62-1.89 (4H, m), 2.49-2.57 (1H, m), 3.38-3.76 (4H, m), 4.55 (2H, d, J = 8.4 Hz), 7.32 (2H, d, J = 16.0 Hz), 7.50-7.57 (3H, m), 8.26 (1H, t, J = 8.0 Hz), 8.68 (1H, s), 8.99 (1H, d, J = 6.4 Hz); HRMS (ESI-TOF) m/z: [M + Na]⁺ calcd for C₁₉H₂₁N₃O₃Na 362.1481; Found 362.1478.

The confirmed structure is as follows.

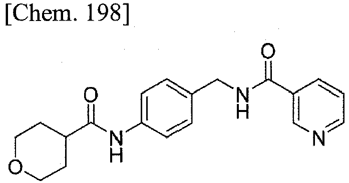

### [Example 109: Production of compound 109]

To a solution of intermediate 62 (100 mg, 0.440 mmol) in dichloroethane (5 ml), cyclobutanecarboxylic acid (88.1 mg, 0.880 mmol), 1-(3-dimethylaminopropyl)-3-ethylcarbodiimide hydrochloride (168 mg, 0.876 mmol), and triethylamine (0.120 ml, 0.861 mmol) were added at 60°C, and the mixture was stirred at the same temperature as above for 3 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 85:15). Compound 109 was obtained as a yellow solid (136 mg, 0.440 mmol, 99%). NMR measurement spectra and results of mass spectrometry by HR-ESI-MS are as follows.
¹H NMR (CD₃OD, 400 MHz): δ1.14-1.20 (2H, m), 1.82-2.08 (4H, m), 2.28-2.39 (1H, m), 4.55 (2H, s), 7.32 (2H, d, J = 8.4 Hz), 7.51-7.58 (3H, m), 8.26 (1H, d, J = 7.6 Hz), 8.68 (1H, s), 9.00 (1H, s); HRMS (ESI-TOF) m/z: [M + Na]⁺ calcd for C₁₈H₁₉N₃O₂Na 332.1375; Found 332.1373.

The confirmed structure is as follows.

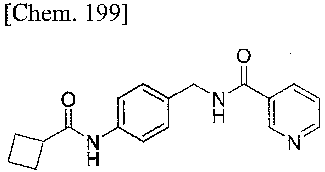

### [Example 110: Production of compound 110]

To a solution of N-[4-(2-aminomethyl)-2-fluorophenyl]benzamide (100 mg, 0.409 mmol) in dichloromethane (5 ml), nicotinoyl chloride hydrochloride (87.6 mg, 0.492 mmol) and triethylamine (0.110 ml, 0.789 mmol) were added at room temperature, and the mixture was stirred at the same temperature as above for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 110 was obtained as a yellow solid (50.0 mg, 0.143 mmol, 35%). NMR measurement spectra and results of mass spectrometry by HR-ESI-MS are as follows. ¹H NMR (CD₃OD, 400 MHz): δ4.61 (2H, s), 7.21-7.27 (2H, m), 7.50-7.77 (6H, m), 7.93-7.98 (2H, m), 8.29 (1H, d, J = 8.4 Hz); HRMS (ESI-TOF) m/z: [M + Na]⁺ calcd for C₂₀H₁₆N₄O₂Na 372.1124; Found 372.1121.

The confirmed structure is as follows.

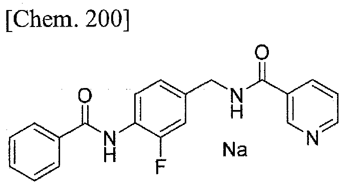

### [Example 111: Production of compound 111]

### (1) Production of intermediate 82

A known compound represented by the following structural formula (intermediate 82) was synthesized in accordance with the method described in WO2008/126731.

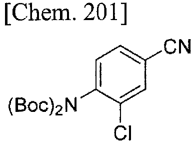

### (2) Production of intermediate 75

To a solution of intermediate 82 (247 mg, 0.700 mmol) in methanol (1.4 ml), nickel chloride hexahydrate (166 mg, 0.698 mmol) and sodium borohydride (79.4 mg, 2.10 mmol) were added under an ice bath, and the mixture was stirred at the same temperature as above for 15 minutes. Insoluble matter was filtered through Celite, and the filtrate was concentrated under reduced pressure. To a solution of the residue in dichloromethane (2.3 ml), nicotinoyl chloride hydrochloride (250 mg, 1.40 mmol) and triethylamine (0.210 ml, 1.51 mmol) were added at room temperature, and the mixture was stirred at the same temperature as above for 14 hours. The reaction mixture was filtered through Celite, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Intermediate 75 was obtained as a yellow solid (226 mg, 0.489 mmol, 70%).

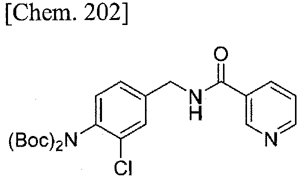

### (3) Production of intermediate 76

To a solution of intermediate 75 (226 mg, 0.489 mmol) in dichloromethane (10 ml), trifluoroacetic acid (0.19 ml) was added under an ice bath, and the mixture was stirred at room temperature for 16 hours. Insoluble matter was filtered through Celite, and the filtrate was concentrated under reduced pressure. To a solution of the residue in dichloromethane (2.3 ml), nicotinoyl chloride hydrochloride (250 mg, 1.40 mmol) and triethylamine (0.210 ml, 1.51 mmol) were added at room temperature, and the mixture was stirred at the same temperature as above for 14 hours. A saturated aqueous solution of sodium bicarbonate was added to the reaction mixture, followed by extraction with dichloromethane. The organic layer was washed with saturated saline, dried over anhydrous magnesium sulfate, then filtered, and concentrated under reduced pressure. Intermediate 76 was obtained as a yellow solid (127 mg, 0.485 mmol, 99%).

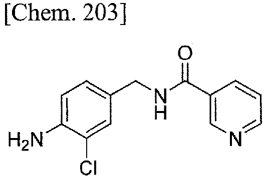

### (4) Production of compound 111

To a solution of intermediate 76 (28.1 mg, 0.107 mmol) in dichloromethane (0.2 ml), benzoyl chloride (0.0200 ml, 0.172 mmol) and triethylamine (0.0300 ml, 0.215 mmol) were added under an ice bath, and the mixture was stirred at room temperature for 2.5 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 10:1). Compound 111 was obtained as a yellow solid (32.0 mg, 0.0875 mmol, 82%). NMR measurement spectra and results of mass spectrometry by HR-ESI-MS are as follows.
¹H NMR (DMSO, 400 MHz): δ4.50 (2H, d, J = 6.0 Hz), 7.27 (1H, d, J = 8.4 Hz), 7.40-7.75 (8H, m), 7.96 (1H, dd, J = 6.8, 6.8 Hz), 8.22 (1H, dt, J = 7.6, 2.0 Hz), 8.72 (1H, d, J = 3.2 Hz), 9.04 (1H, s), 9.30 (1H, t, J = 6.0 Hz); HRMS (ESI-TOF) m/z: [M + Na]⁺ calcd for C₂₀H₁₆ClN₃O₂Na 388.0829; Found 388.0824.

The confirmed structure is as follows.

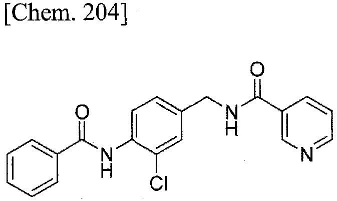

### [Example 112: Production of compound 112]

To a solution of intermediate 76 (27.5 mg, 0.105 mmol) in dichloromethane (0.2 ml), nicotinoyl chloride (29.4 mg, 0.165 mmol) and triethylamine (0.0300 ml, 0.215 mmol) were added under an ice bath, and the mixture was stirred at room temperature for 48 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 10:1). Compound 112 was obtained as a yellow solid (22.0 mg, 0.0600 mmol, 57%). NMR measurement spectra and results of mass spectrometry by HR-ESI-MS are as follows.
¹H NMR (DMSO, 400 MHz): δ4.52 (2H, d, J = 6.0 Hz), 7.36 (1H, d, J = 6.0 Hz), 7.51-7.60 (4H, m), 8.22-8.33 (3H, m), 8.73 (1H, d, J = 3.2 Hz), 8.78 (1H, d, J = 3.2 Hz), 9.06 (1H, s), 9.13 (1H, s), 9.33 (1H, t, J = 5.6 Hz); HRMS (ESI-TOF) m/z: [M + H]⁺ calcd for C₁₉H₁₆ClN₄O₂ 367.0962; Found 367.0958.

The confirmed structure is as follows.

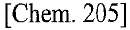

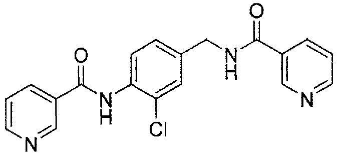

### [Example 113: Production of compound 113]

To a solution of intermediate 59 (150 mg, 0.444 mmol) in dichloromethane (5 ml), cyclohexanecarboxylic acid (0.083 ml, 0.666 mmol), 1-(3-dimethylaminopropyl)-3-ethylcarbodiimide hydrochloride (170 mg, 0.888 mmol), diisopropylethylamine (0.390 ml, 2.24 mmol), and dimethylaminopyridine (5.40 mg, 0.0442 mmol) were added at room temperature, and the mixture was stirred at the same temperature as above for 15 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1), SYK-221 was obtained as a yellow solid (23.5 mg, 0.0694 mmol, 16%). NMR measurement spectra and results of mass spectrometry by HR-ESI-MS are as follows.
¹H NMR (CDCl₃, 400 MHz): δ1.18-2.31 (11H, m), 4.63 (2H, d, J = 5.6 Hz), 6.50 (1H, br), 7.41 (1H, dd, J = 7.2, 4.4 Hz), 7.72 (1H, dd, J = 8.4, 2.0 Hz), 7.91 (1H, br), 8.13 (1H, dt, J = 8.0, 2.0 Hz), 8.23 (1H, d, J = 8.4 Hz), 8.28 (1H, d, J = 2.0 Hz); HRMS (ESI-TOF) m/z: [M + Na]⁺ calcd for C₁₉H₂₂N₄O₂Na 361.1640; Found 361.1637.

The confirmed structure is as follows.

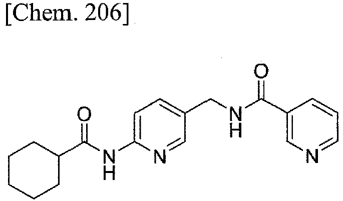

### [Example 114: Production of compound 114]

To a solution of intermediate 59 (100 mg, 0.296 mmol) in dichloromethane (5 ml), oxane-4-carboxylic acid (54.6 mg, 0.420 mmol), 1-(3-dimethylaminopropyl)-3-ethylcarbodiimide hydrochloride (72.8 mg, 0.380 mmol), triethylamine (0.520 ml, 3.73 mmol), and dimethylaminopyridine (9.30 mg, 0.0761 mmol) were added at room temperature, and the mixture was stirred at the same temperature as above for 6 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 94:6). Compound 114 was obtained as a yellow solid (15.0 mg, 0.0441 mmol, 15%). NMR measurement spectra and results of mass spectrometry by HR-ESI-MS are as follows.
¹H NMR (CDCl₃, 400 MHz): δ1.85-1.91 (4H, m), 2.50-2.59 (1H, m), 3.46 (2H, dt, J = 12.0, 3.2 Hz), 4.06 (2H, dt, J = 10.8, 3.2 Hz), 4.65 (2H, d, J = 6.0 Hz), 6.52 (1H, br), 7.42 (1H, dd, J = 8.0, 4.8 Hz), 7.77 (1H, dd, J = 8.8, 2.4 Hz), 8.09 (1H, br), 8.14 (1H, dt, J = 8.0, 2.0 Hz), 8.24 (1H, d, J = 8.4 Hz), 8.29 (1H, d, J = 0.8 Hz), 8.75 (1H, dd, J = 4.8, 1.6 Hz), 8.99 (1H, d, J = 1.6 Hz); HRMS (ESI-TOF) m/z: [M + Na]⁺ calcd for C₁₈H₂₀N₄O₃Na 363.1433; Found 363.1429.

The confirmed structure is as follows.

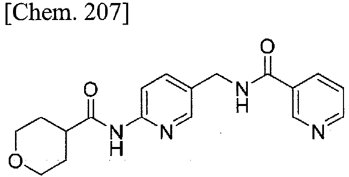

### [Example 115: Production of compound 115]

To a solution of intermediate 59 (51.7 mg, 0.196 mmol) in dichloroethane (5 ml), 3-phenylpropionic acid (32.3 mg, 0.215 mmol), 1-(3-dimethylaminopropyl)-3-ethylcarbodiimide hydrochloride (37.5 mg, 0.196 mmol), triethylamine (0.270 ml, 1.94 mmol), and dimethylaminopyridine (4.80 mg, 0.0393 mmol) were added at room temperature, and the mixture was stirred at 60°C for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 94:6). Compound 115 was obtained as a yellow solid (7.20 mg, 0.0200 mmol, 10.2%). NMR measurement spectra and results of mass spectrometry by HR-ESI-MS are as follows.
¹H NMR (CDCl₃, 400 MHz): δ2.70 (2H, t, J = 7.6 Hz), 3.05 (2H, t, J = 7.6 Hz), 4.62 (2H, d, J = 5.6 Hz), 6.67 (1H, br), 7.18-7.32 (5H, m), 7.39 (1H, dd, J = 8.0, 4.8 Hz), 7.72 (1H, dd, J = 8.8, 2.0 Hz), 7.97 (1H, br), 8.13 (1H, dt, J = 8.0, 2.0 Hz), 8.20 (1H, d, J = 8.4 Hz), 8.25 (1H, J = 2.0 Hz), 8.73 (1H, dd, J = 4.8, 1.6 Hz); HRMS (ESI-TOF) m/z: [M + Na]⁺ calcd for C₂₁H₂₀N₄O₂Na 383.1484; Found 383.1477.

The confirmed structure is as follows.

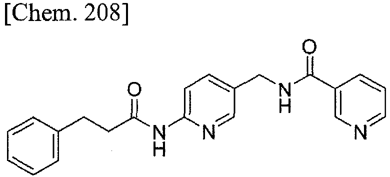

### [Example 116: Production of compound 116]

To a solution of intermediate 59 (50.0 mg, 0.148 mmol) in dichloromethane (2 ml), 3-fluorobenzoic acid (31.8 mg, 0.227 mmol), 1-(3-dimethylaminopropyl)-3-ethylcarbodiimide hydrochloride (72.6 mg, 0.379 mmol), triethylamine (0.260 ml, 1.87 mmol), and dimethylaminopyridine (4.60 mg, 0.0376 mmol) were added at room temperature, and the mixture was stirred at 60°C for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 94:6). Compound 116 was obtained as a yellow solid (10.8 mg, 0.0308 mmol, 21%). NMR measurement spectra and results of mass spectrometry by HR-ESI-MS are as follows.
¹H NMR (CDCl₃, 400 MHz): δ4.62 (2H, d, J = 6.0 Hz), 7.08 (1H, br), 7.22-7.77 (6H, m), 8.14 (1H, d, J = 7.6 Hz), 8.24-8.31 (2H, m), 8.70 (1H, br), 8.86 (1H, s), 9.01 (1H, br); HRMS (ESI-TOF) m/z: [M + Na]⁺ calcd for C₁₉H₁₅FN₄O₂Na 373.1077; Found 373.1072.

The confirmed structure is as follows.

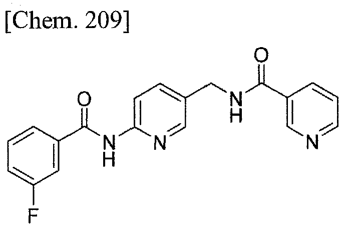

### [Example 117: Production of compound 117]

To a solution of 4-fluorobenzoic acid (32.0 mg, 0.228 mmol) in dichloroethane (5 ml), oxalyl chloride (0.0210 ml, 0.245 mmol) and dimethylformamide (1 drop) were added, and the mixture was stirred at 60°C for 1 hour. After concentration under reduced pressure, intermediate 59 (50.0 mg, 0.148 mmol) and triethylamine (0.320 ml, 2.28 mmol) were added to a solution of the residue in dichloroethane (2 ml), and the mixture was stirred at 60°C for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 117 was obtained as a white solid (7.10 mg, 0.0203 mmol, 8.9%). NMR measurement spectra and results of mass spectrometry by HR-ESI-MS are as follows. ¹H NMR (CDCl₃, 400 MHz): δ4.64 (2H, d, J = 5.6 Hz), 6.86 (1H, br), 7.03 (2H, d, J = 8.8 Hz), 7.23 (1H, d, J = 8.0 Hz), 7.40 (1H, dd, J = 8.0, 4.8 Hz), 7.73-7.79 (4H, m), 8.12 (1H, d, J = 8.0 Hz), 8.34 (1H, d, J = 2.4 Hz), 8.73 (1H, d, J = 3.6 Hz), 8.99 (1H, s); HRMS (ESI-TOF) m/z: [M + Na]⁺ calcd for C₁₉H₁₅FN₄O₂Na 373.1077; Found 373.1068.

The confirmed structure is as follows.

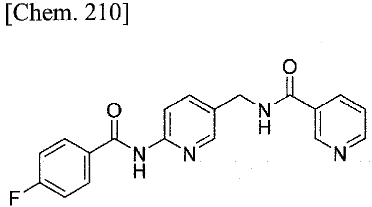

### [Example 118: Production of compound 118]

To a solution of intermediate 59 (50.0 mg, 0.148 mmol) in dichloromethane (2 ml), 4-chlorobenzoic acid (36.0 mg, 0.230 mmol), 1-(3-dimethylaminopropyl)-3-ethylcarbodiimide hydrochloride (73.0 mg, 0.381 mmol), and triethylamine (0.260 ml, 1.87 mmol) were added at room temperature, and the mixture was stirred at room temperature for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 95:5). Compound 118 was obtained as a yellow solid (6.20 mg, 0.0169 mmol, 11%). NMR measurement spectra and results of mass spectrometry by HR-ESI-MS are as follows.
¹H NMR (CD₃OD, 400 MHz): δ4.74 (2H, s), 7.63 (2H, d, J = 8.4 Hz), 7.87 (1H, s), 8.05-8.11 (3H, m), 8.47-8.51 (2H, m), 8.90 (1H, d, J = 8.0 Hz), 8.97 (1H, s), 9.29 (1H, s); HRMS (ESI-TOF) m/z: [M + Na]⁺ calcd for C₁₉H₁₅ClN₄O₂Na 389.0781; Found 389.0777.

The confirmed structure is as follows.

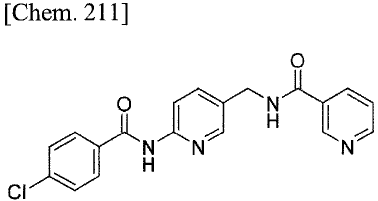

### [Example 119: Production of compound 119]

To a solution of intermediate 59 (50.0 mg, 0.148 mmol) in dichloromethane (2 ml), 4-methylbenzoic acid (31.0 mg, 0.228 mmol), 1-(3-dimethylaminopropyl)-3-ethylcarbodiimide hydrochloride (73.0 mg, 0.381 mmol), and triethylamine (0.260 ml, 1.87 mmol) were added at room temperature, and the mixture was stirred at room temperature for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 95:5). Compound 119 was obtained as a yellow solid (13.6 mg, 0.0393 mmol, 27%). NMR measurement spectra and results of mass spectrometry by HR-ESI-MS are as follows.
¹H NMR (CD₃OD, 400 MHz): δ2.41 (3H, s), 4.59 (2H, s), 7.26 (1H, d, J = 7.6 Hz), 7.33 (2H, d, J =7.6 Hz), 7.54 (1H, dd, J = 8.0, 4.8 Hz), 7.83-7.91 (3H, m), 8.20 (1H, d, J = 8.4 Hz), 8.26 (1H, d, J = 7,6 Hz), 8.68 (1H, s), 9.00 (1H, s); HRMS (ESI-TOF) m/z: [M + Na]⁺ calcd for C₂₀H₁₈N₄O₂Na 369.1327; Found 369.1321.

The confirmed structure is as follows.

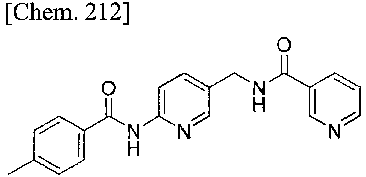

### [Example 120: Production of compound 120]

To a solution of intermediate 59 (50.0 mg, 0.148 mmol) in dichloroethane (2 ml), nicotinoyl chloride hydrochloride (37.0 mg, 0.208 mmol) and triethylamine (0.184 ml, 1.32 mmol) were added under an ice bath, and the mixture was stirred at room temperature for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 120 was obtained as a yellow solid (14.3 mg, 0.0429 mmol, 29%). NMR measurement spectra and results of mass spectrometry by HR-ESI-MS are as follows.
¹H NMR (CDCl₃, 400 MHz): δ4.64 (2H, d, J = 6.0 Hz), 7.06 (1H, br), 7.29-7.46 (3H, m), 7.75-7.82 (1H, m), 8.05 (1H, dt, J = 8.0, 2.0 Hz), 8.11-8.18 (1H, m), 8.28-8.34 (2H, m), 8.67-8.73 (2H, m), 8.90 (1H, d, J = 1.6 Hz), 9.00 (1H, t, J = 2.4 Hz); HRMS (ESI-TOF) m/z: [M + Na]⁺ calcd for C₁₈H₁₅N₅O₂Na 356.1123; Found 356.1118.

The confirmed structure is as follows.

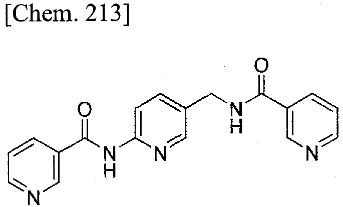

### [Example 121: Production of compound 121]

To a solution of intermediate 59 (50.0 mg, 0.148 mmol) in dichloromethane (2 ml), 1-naphthalenecarboxylic acid (39.3 mg, 0.228 mmol), 1-(3-dimethylaminopropyl)-3-ethylcarbodiimide hydrochloride (72.8 mg, 0.380 mmol), triethylamine (0.260 ml, 1.87 mmol), and dimethylaminopyridine (7.30 mg, 0.0598 mmol) were added at room temperature, and the mixture was stirred at the same temperature as above for 72 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 121 was obtained as a yellow solid (46.9 mg, 0.123 mmol, 83%). NMR measurement spectra and results of mass spectrometry by HR-ESI-MS are as follows.
¹H NMR (CDCl₃, 400 MHz): δ4.47 (2H, d, J = 5.6 Hz), 6.97 (1H, br), 7.31 (1H, dd, J = 8.0, 5.6 Hz), 7.44-7.97 (8H, m), 8.07 (1H, d, J = 8.0 Hz), 8.31 (1H, d, J = 9.2 Hz), 8.37 (1H, d, J = 8.4 Hz), 8.65 (1H, d, J = 8.4 Hz), 8.95 (1H, s), 9.31 (1H, s); HRMS (ESI-TOF) m/z: [M + H]⁺ calcd for C₂₃H₁₉N₄O₂ 383.1508; Found 383.1501.

The confirmed structure is as follows.

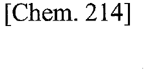

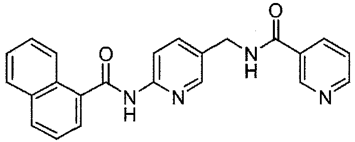

### [Example 122: Production of compound 122]

To a solution of intermediate 59 (50.0 mg, 0.148 mmol) in dichloromethane (2 ml), 6-quinolinecarboxylic acid (39.5 mg, 0.228 mmol), 1-(3-dimethylaminopropyl)-3-ethylcarbodiimide hydrochloride (72.8 mg, 0.380 mmol), triethylamine (0.260 ml, 1.87 mmol), and dimethylaminopyridine (7.30 mg, 0.0598 mmol) were added at room temperature, and the mixture was stirred at the same temperature as above for 96 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 5:1). Compound 122 was obtained as a yellow solid (36.9 mg, 0.0962 mmol, 65%). NMR measurement spectra and results of mass spectrometry by HR-ESI-MS are as follows.
¹H NMR (CDCl₃, 400 MHz): δ4.67 (2H, d, J = 5.6 Hz), 6.86 (1H, br), 7.39-8.50 (11H, m), 8,74 (1H, dd, J = 4.8, 2.0 Hz), 9.02 (1H, dd, J = 4.8, 2.0 Hz), 9.04 (1H, d, J = 2.0 Hz); HRMS (ESI-TOF) m/z: [M + H]⁺ calcd for C₂₂H₁₈N₅O₂ 384.1460; Found 384.1458.

The confirmed structure is as follows.

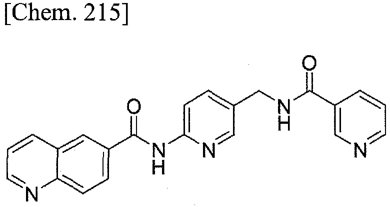

### [Example 123: Production of compound 123]

To a solution of intermediate 59 (50.0 mg, 0.148 mmol) in dichloromethane (2 ml), 2-naphthaleneacetic acid (42.8 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 123 was obtained as a yellow solid (54.9 mg, 0.138 mmol, 93%). NMR measurement spectra and results of mass spectrometry by HR-ESI-MS are as follows.
¹H NMR (DMSO, 400 MHz): δ3.89 (2H, s), 4.45 (2H, d, J = 6.4 Hz), 7.45-7.53 (3H, m), 7.73 (1H, d, J = 8.0 Hz), 7.82-7.90 (4H, m), 8.02 (1H, d, J = 8.0 Hz), 8.20 (1H, d, J = 8.0 Hz), 8.30-8.32 (2H, m), 8.70 (1H, d, J = 5.6 Hz), 9.02 (1H, s), 9.22 (1H, t, J = 5.6 Hz), 10.80 (1H, s); HRMS (ESI-TOF) m/z: [M + Na]⁺ calcd for C₂₄H₂₀N₄O₂Na 419.1484; Found 419.1478.

The confirmed structure is as follows.

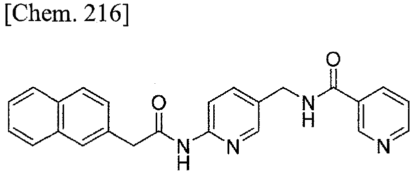

### [Example 124: Production of compound 124]

To a solution of intermediate 59 (50.0 mg, 0.148 mmol) in dichloromethane (2 ml), 4-pyridinecarboxylic acid (28.3 mg, 0.230 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 124 was obtained as a yellow solid (25.0 mg, 0.0750 mmol, 51%). NMR measurement spectra and results of mass spectrometry by HR-ESI-MS are as follows.
¹H NMR (DMSO, 400 MHz): δ4.52 (2H, d, J = 6.0 Hz), 7.52 (1H, dd, J = 8.0, 4.8 Hz), 7.85 (1H, d, J = 8.8 Hz), 7.91 (2H, d, J = 6.0 Hz), 8.16 (1H, d, J = 8.8 Hz), 8.22 (1H, d, J = 7.6 Hz), 8.40 (1H, s), 8.72 (1H, d, J = 4.8 Hz), 8.76 (2H, d, J = 6.0 Hz), 9.05 (1H, s), 9.27 (1H, t, J = 5.6 Hz), 11.14 (1H, s); HRMS (ESI-TOF) m/z: [M + Na]⁺ calcd for C₁₈H₁₅N₅O₂Na 356.1123; Found 356.1118.

The confirmed structure is as follows.

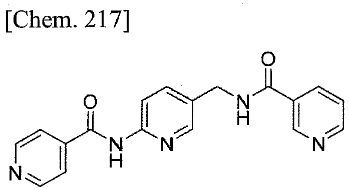

### [Example 125: Production of compound 125]

To a solution of intermediate 59 (50.0 mg, 0.148 mmol) in dichloromethane (2 ml), 2-pyridinecarboxylic acid (36.7 mg, 0.298 mmol), N,N-diisopropylethylamine(0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 125 was obtained as a yellow solid (48.3 mg, 0.145 mmol, 98%). NMR measurement spectra and results of mass spectrometry by HR-ESI-MS are as follows.
¹H NMR (DMSO, 400 MHz): δ4.52 (2H, d, J = 5.6 Hz), 7.52 (1H, t, J = 6.0 Hz), 7.73 (1H, d, J = 5.2 Hz), 7.88 (1H, d, J = 8.4 Hz), 8.12 (1H, t, J = 8.0 Hz), 8.19-8.27 (3H, m), 8.39 (1H, s), 8.72 (1H, d, J = 4.4 Hz), 8.76 (1H, d, J = 4.4 Hz), 9.05 (1H, s), 9.28 (1H, t, J = 4.8 Hz), 10.44 (1H, s); HRMS (ESI-TOF) m/z: [M + Na]⁺ calcd for C₁₈H₁₅N₅O₂Na 356.1123; Found 356.1116.

The confirmed structure is as follows.

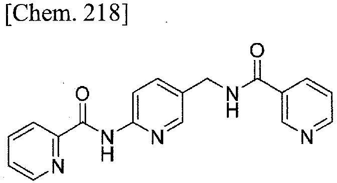

### [Example 126: Production of compound 126]

To a solution of intermediate 59 (50.0 mg, 0.148 mmol) in dichloromethane (2 ml), 3-quinolinecarboxylic acid (39.8 mg, 0.230 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, chloroform:methanol = 19:1). Compound 126 was obtained as a yellow solid (20.0 mg, 0.0522 mmol, 35%). NMR measurement spectra and results of mass spectrometry by HR-ESI-MS are as follows.
¹H NMR (DMSO, 400 MHz): δ4.52 (2H, s), 7.53 (1H, br), 7.72 (1H, t, J = 6.8 Hz), 7.83-7.94 (2H, m), 8.08-8.27 (4H, m), 8.42 (1H, s), 8.72 (1H, s), 9.06 (2H, s), 9.30 (1H, br), 9.37 (1H, s), 11.24 (1H, s); HRMS (ESI-TOF) m/z: [M + Na]⁺ calcd for C₂₂H₁₇N₅O₂Na 406.1280; Found 406.1270.

The confirmed structure is as follows.

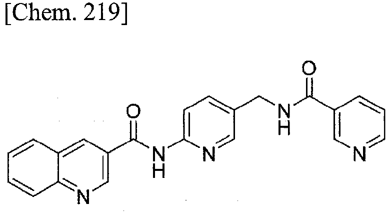

### [Example 127: Production of compound 127]

To a solution of intermediate 59 (50.0 mg, 0.148 mmol) in dichloromethane (2 ml), 2-naphthalenecarboxylic acid (39.6 mg, 0.230 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 127 was obtained as a yellow solid (5.00 mg, 0.0131 mmol, 8.9%). NMR measurement spectra and results of mass spectrometry by HR-ESI-MS are as follows.
¹H NMR (CDCl₃, 400 MHz): δ4.71 (2H, d, J = 5.6 Hz), 7.51-7.63 (3H, m), 7.85-8.14 (6H, m), 8.44 (1H, d, J = 7.6 Hz), 8.50 (1H, s), 8.61-8.69 (3H, m), 8.77 (1H, d, J = 4.4 Hz), 9.39 (1H, s); HRMS (ESI-TOF) m/z: [M + Na]⁺ calcd for C₂₃H₁₈N₄O₂Na 405.1327; Found 405.1320.

The confirmed structure is as follows.

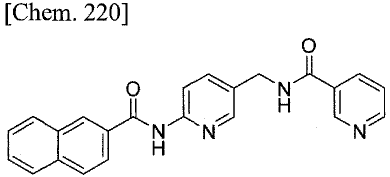

### [Example 128: Production of compound 128]

To a solution of intermediate 59 (50.0 mg, 0.148 mmol) in dichloromethane (2 ml), [1,1'-biphenyl]-4-carboxylic acid (45.6 mg, 0.230 mmol), triethylamine (0.260 ml, 1.87 mmol), 1-(3-dimethylaminopropyl)-3-ethylcarbodiimide hydrochloride (72.6 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 128 was obtained as a yellow solid (52.2 mg, 0.128 mmol, 86%). NMR measurement spectra and results of mass spectrometry by HR-ESI-MS are as follows.
¹H NMR (DMSO, 400 MHz): δ4.52 (2H, d, J = 5.6 Hz), 7.40-7.55 (4H, m), 7.74-7.85 (5H, m), 8.11-8.25 (4H, m), 8.39 (1H, s), 8.72 (1H, d, J = 5.2 Hz), 9.05 (1H, s), 9.27 (1H, t, J = 6.0 Hz), 10.86 (1H, s); HRMS (ESI-TOF) m/z: [M + H]⁺ calcd for C₂₅H₂₁N₄O₂ 409.1665; Found 409.1658.

The confirmed structure is as follows.

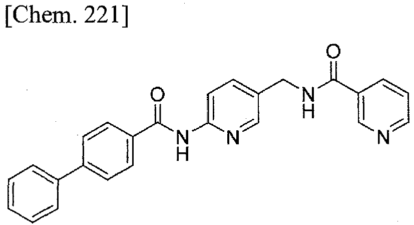

### [Example 129: Production of compound 129]

To a solution of intermediate 59 (50.0 mg, 0.148 mmol) in dichloroethane (2 ml), 4-methoxybenzoyl chloride (39.2 mg, 0.230 mmol) and triethylamine (0.130 ml, 0.953 mmol) were added at room temperature, and the mixture was stirred at room temperature for 14 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 129 was obtained as a yellow solid (8.70 mg, 0.0240 mmol, 16%). NMR measurement spectra and results of mass spectrometry by HR-ESI-MS are as follows.
¹H NMR (CDCl₃, 400 MHz): δ3.87 (3H, s), 4.67 (2H, d, J = 5.2 Hz), 6.98 (2H, d, J = 8.4 Hz), 7.40-7.47 (2H, m), 7.96 (1H, d, J = 8.8 Hz), 8.00 (2H, d, J = 8.8 Hz), 8.25 (1H, d, J = 8.0 Hz), 8.36 (1H, s), 8.49 (1H, d, J = 8.4 Hz), 8.74 (1H, br), 9.14 (1H, br), 9.60 (1H, br); HRMS (ESI-TOF) m/z: [M + Na]⁺ calcd for C₂₀H₁₈N₄O₃Na 385.1277; Found 385.1270.

The confirmed structure is as follows.

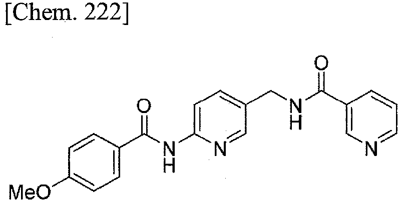

### [Example 130: Production of compound 130]

To a solution of intermediate 59 (50.0 mg, 0.148 mmol) in dichloromethane (2 ml), 2-methylbenzoic acid (31.3 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 130 was obtained as a yellow solid (12.9 mg, 0.0372 mmol, 25%). NMR measurement spectra and results of mass spectrometry by HR-ESI-MS are as follows.
¹H NMR (DMSO, 400 MHz): δ2.09 (3H, s), 4.49 (2H, d, J = 6.4 Hz), 7.24-7.55 (5H, m), 7.81 (1H, d, J = 8.0 Hz), 8.15 (1H, d, J = 8.8 Hz), 8.22 (1H, d, J = 8.0 Hz), 8.33 (1H, d, J = 8.4 Hz), 8.72 (1H, d, J = 5.2 Hz), 9.04 (1H, s), 9.26 (1H, s), 10.75 (1H, s); HRMS (ESI-TOF) m/z: [M + Na]⁺ calcd for C₂₀H₁₈N₄O₃Na 369.1327; Found 369.1320.

The confirmed structure is as follows.

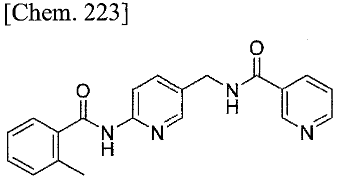

### [Example 131: Production of compound 131]

### (1) Production of intermediate 83

A known compound represented by the following structural formula (intermediate 83) was synthesized in accordance with the method described in Catalysis Science Technology (2023), 13 (8), 2508-2516.

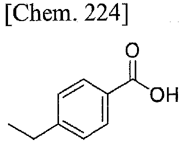

### (2) Production of compound 131

To a solution of intermediate 59 (50.0 mg, 0.148 mmol) in dichloromethane (2 ml), 4-ethylbenzoic acid (intermediate 83) (28.1 mg, 0.187 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 131 was obtained as a yellow solid (2.00 mg, 0.00555 mmol, 3.8%). NMR measurement spectra and results of mass spectrometry by HR-ESI-MS are as follows.
¹H NMR (CDCl₃, 400 MHz): δ1.35 (3H, t, J = 7.6 Hz), 2.71 (2H, q, J = 7.6 Hz), 4.78 (2H, s), 7.36 (2H, d, J = 7.6 Hz), 8.12 (1H, s), 8.20 (2H, d, J = 7.6 Hz), 8.43 (1H, d, J = 8.0 Hz), 8.80 (1H, s), 8.93 (1H, d, J = 8.4 Hz), 9.05-9.14 (2H, m), 10.20-10.27 (2H, m), 12.13 (1H, s); HRMS (ESI-TOF) m/z: [M + H]⁺ calcd for C₂₁H₂₁N₄O₂ 361.1665; Found 361.1656.

The confirmed structure is as follows.

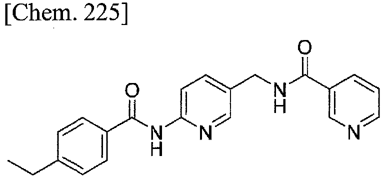

### [Example 132: Production of compound 132]

To a solution of intermediate 59 (50.0 mg, 0.148 mmol) in dichloromethane (2 ml), 3-chlorobenzoic acid (36.0 mg, 0.230 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 132 was obtained as a yellow solid (16.9 mg, 0.0461 mmol, 31%). NMR measurement spectra and results of mass spectrometry by HR-ESI-MS are as follows.:
¹H NMR (DMSO, 400 MHz): δ4.46 (2H, d, J = 5.6 Hz), 7.45-7.52 (2H, m), 7.61 (1H, d, J = 8.4 Hz), 7.74 (1H, d, J = 8.8 Hz), 7.92 (1H, d, J = 7.6 Hz), 8.02 (1H, s), 8.09 (1H, d, J = 8.8 Hz), 8.17 (1H, d, J = 8.0 Hz), 8.34 (1H, s), 8.67 (1H, d, J = 3.6 Hz), 9.00 (1H, s), 9.22 (1H, t, J = 6.0 Hz), 10.91 (1H, s); HRMS (ESI-TOF) m/z: [M + Na]⁺ calcd for C₁₉H₁₅ClN₄O₂Na 389.0781; Found 389.0774.

The confirmed structure is as follows.

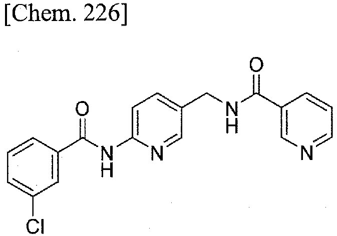

### [Example 133: Production of compound 133]

To a solution of intermediate 59 (50.0 mg, 0.148 mmol) in dichloromethane (2 ml), 2-fluorobenzoic acid (32.2 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 133 was obtained as a yellow solid (18.8 mg, 0.0537 mmol, 36%). NMR measurement spectra and results of mass spectrometry by HR-ESI-MS are as follows.
¹H NMR (CDCl₃, 400 MHz): δ4.70 (2H, d, J = 6.0 Hz), 7.20 (1H, dd, J = 8.0, 4.8 Hz), 7.31 (1H, t, J = 7.6 Hz), 7.51-7.58 (1H, m), 7.71 (1H, t, J = 6.4 Hz), 7.95 (1H, d, J = 8.8 Hz), 8.12 (1H, t, J = 8.0 Hz), 8.25 (1H, br), 8.42-8.50 (2H, m), 8.60 (1H, d, J = 7.6 Hz), 8.79 (1H, d, J = 4.4 Hz), 9.46 (1H, d, J = 12.0 Hz), 9.60 (1H, s); HRMS (ESI-TOF) m/z: [M + Na]⁺ calcd for C₁₉H₁₅FN₄O₂Na 373.1077; Found 373.1069.

The confirmed structure is as follows.

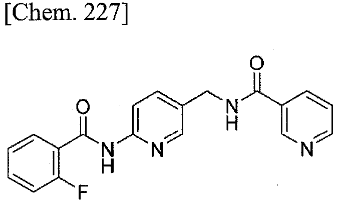

### [Example 134: Production of compound 134]

To a solution of intermediate 59 (50.0 mg, 0.148 mmol) in dichloromethane (2 ml), 3,4-dimethylbenzoic acid (34.5 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 134 was obtained as a yellow solid (15.0 mg, 0.0416 mmol, 28%). NMR measurement spectra and results of mass spectrometry by HR-ESI-MS are as follows.
¹H NMR (DMSO, 400 MHz): δ2.29 (6H, s), 4.50 (2H, d, J = 5.6 Hz), 7.26 (1H, d, J = 8.0 Hz), 7.52 (1H, dd, J = 8.0, 4.8 Hz), 7.74-7.86 (3H, m), 8.15 (1H, d, J = 8.4 Hz), 8.22 (1H, d, J = 8.4 Hz), 8.37 (1H, s), 8.72 (1H, d, J = 4.4 Hz), 9.05 (1H, s), 9.26 (1H, t, J = 5.6 Hz), 10.62 (1H, s); HRMS (ESI-TOF) m/z: [M + Na]⁺ calcd for C₂₁H₂₀N₄O₂Na 383.1484; Found 383.1478.

The confirmed structure is as follows.

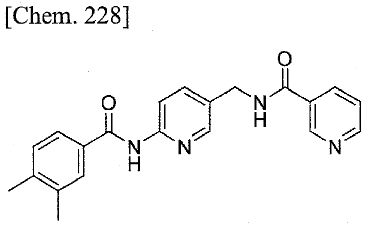

### [Example 135: Production of compound 135]

To a solution of intermediate 59 (50.0 mg, 0.148 mmol) in dichloromethane (2 ml), 2,4-dimethylbenzoic acid (34.5 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 135 was obtained as a yellow solid (6.80 mg, 0.0189 mmol, 13%). NMR measurement spectra and results of mass spectrometry by HR-ESI-MS are as follows.
¹H NMR (CDCl₃, 400 MHz): δ2.35 (3H, s), 2.48 (3H, s), 4.63 (2H, d, J = 6.0 Hz), 7.04-7.15 (3H, m), 7.41-7.49 (2H, m), 7.84 (1H, d, J = 8.8 Hz), 8.20-8.26 (2H, m), 8.38 (1H, d, J = 8.4 Hz), 8.73 (1H, d, J = 4.4 Hz), 8.81 (1H, br), 9.10 (1H, s); HRMS (ESI-TOF) m/z: [M + Na]⁺ calcd for C₂₁H₂₀N₄O₂Na 383.1484; Found 383.1475.

The confirmed structure is as follows.

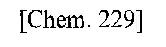

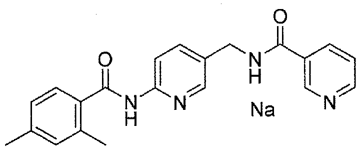

### [Example 136: Production of compound 136]

To a solution of intermediate 59 (50.0 mg, 0.148 mmol) in dichloromethane (2 ml), 3,5-dimethylbenzoic acid (34.5 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 136 was obtained as a yellow solid (11.8 mg, 0.0327 mmol, 22%). NMR measurement spectra and results of mass spectrometry by HR-ESI-MS are as follows.
¹H NMR (CDCl₃, 400 MHz): δ2.38 (6H, s), 4.65 (2H, d, J = 6.0 Hz), 7.14 (1H, br), 7.20 (1H, s), 7.42 (1H, dd, J = 8.0, 4.8 Hz), 7.57 (1H, s), 7.71 (1H, s), 7.83 (1H, d, J = 8.8 Hz), 8.21 (1H, d, J = 8.0 Hz), 8.32 (1H, s), 8.41 (1H, d, J = 8.8 Hz), 8.73 (1H, d, J = 4.8 Hz), 9.09 (1H, s), 9.25 (1H, br); HRMS (ESI-TOF) m/z: [M + H]⁺ calcd for C₂₁H₂₁N₄O₂ 361.1665; Found 361.1658.

The confirmed structure is as follows.

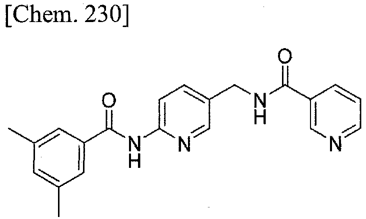

### [Example 137: Production of compound 137]

To a solution of intermediate 59 (50.0 mg, 0.148 mmol) in dichloromethane (2 ml), 3,4-difluorobenzoic acid (36.4 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 137 was obtained as a yellow solid (16.8 mg, 0.0456 mmol, 31%). NMR measurement spectra and results of mass spectrometry by HR-ESI-MS are as follows.
¹H NMR (CDCl₃, 400 MHz): δ4.74 (2H, d, J = 5.6 Hz), 7.28-7.36 (1H, m), 7.78 (1H, t, J = 8.0 Hz), 7.85-8.05 (4H, m), 8.19 (1H, d, J = 8.8 Hz), 8.54 (1H, s), 8.64-8.71 (2H, m), 8.85 (1H, d, J = 5.2 Hz), 9.69 (1H, s); HRMS (ESI-TOF) m/z: [M + Na]⁺ calcd for C₁₉H₁₄F₂N₄O₂Na 391.0983; Found 391.0976.

The confirmed structure is as follows.

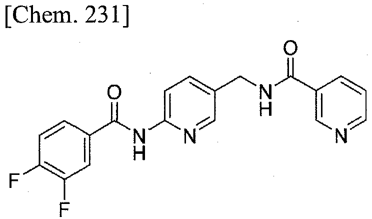

### [Example 138: Production of compound 138]

To a solution of intermediate 59 (50.0 mg, 0.148 mmol) in dichloromethane (2 ml), 3,5-difluorobenzoic acid (36.4 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 138 was obtained as a yellow solid (25.5 mg, 0.0692 mmol, 47%). NMR measurement spectra and results of mass spectrometry by HR-ESI-MS are as follows.
¹H NMR (CDCl₃, 400 MHz): δ4.83 (2H, d, J = 6.0 Hz), 6.97-7.05 (2H, m), 7.41-7.61 (4H, m), 7.85 (1H, dd, J = 8.8, 1.6 Hz), 8.24-8.30 (2H, m), 8.39 (1H, d, J = 8.8 Hz), 8.73 (1H, d, J = 3.6 Hz), 9.14 (1H, s); HRMS (ESI-TOF) m/z: [M + Na]⁺ calcd for C₁₉H₁₄F₂N₄O₂Na 391.0983; Found 391.0975.

The confirmed structure is as follows.

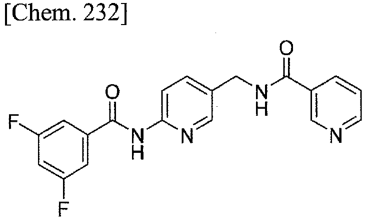

### [Example 139: Production of compound 139]

To a solution of intermediate 59 (50.0 mg, 0.148 mmol) in dichloromethane (2 ml), 2,4-difluorobenzoic acid (36.4 mg, 0.230 mmol), N,N-diisopropylethylamine (0.330 ml, 1.89 mmol), 1-[bis(dimethylamino)methylene]-1H-1,2,3-triazolo[4,5-b]pyridinium3-oxide hexafluorophosphate (HATU) (145 mg, 0.381 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 139 was obtained as a yellow solid (22.2 mg, 0.0603 mmol, 41%). NMR measurement spectra and results of mass spectrometry by HR-ESI-MS are as follows.
¹H NMR (CDCl₃, 400 MHz): δ4.67 (2H, d, J = 6.0 Hz), 6.91-6.98 (1H, m), 7.02-7.09 (1H, m), 7.20 (1H, br), 7.49 (1H, dd, J = 8.0, 4.8 Hz), 7.82 (1H, d, J = 8.4 Hz), 8.12-8.20 (1H, m), 8.26-8.38 (3H, m), 8.74 (1H, s), 9.09 (1H, d, J = 12.4 Hz), 9.18 (1H, s); HRMS (ESI-TOF) m/z: [M + Na]⁺ calcd for C₁₉H₁₄F₂N₄O₂Na 391.0983; Found 391.0975.

The confirmed structure is as follows.

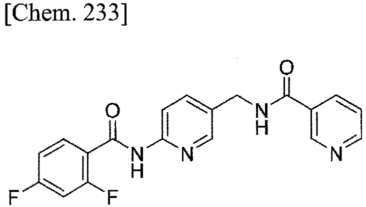

### [Example 140: Production of compound 140]

### (1) Production of intermediate 95

To a solution of intermediate 57 (100 mg, 0.313 mmol) in methanol (2 ml), nickel chloride hexahydrate (73.7 mg, 0.310 mmol) and sodium borohydride (35.2 mg, 0.930 mmol) were added under an ice bath, and the mixture was stirred at the same temperature as above for 0.5 hours. Triethylamine (0.0900 ml, 0.646 mmol) and 3-quinolinecarbonyl chloride (119 mg, 0.621 mmol) were added thereto at the same temperature as above, and the mixture was further stirred at the same temperature as above for 1 hour. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 98:2). Intermediate 95 was obtained as a yellow oil (100 mg, 0.352 mmol, 21%).

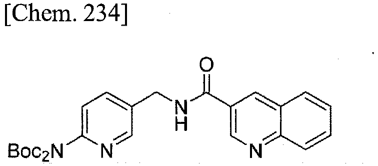

### (2) Production of intermediate 96

To a solution of intermediate 95 (100 mg, 0.352 mmol) in dichloromethane (1.5 ml), 4 M HCl/dioxane (1 ml) was added, and the mixture was stirred at room temperature for 4 hours and concentrated under reduced pressure to obtain intermediate 96 (64.8 mg, 0.167 mmol, 47%).

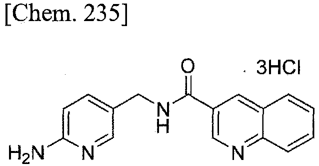

### (3) Production of compound 140

To a solution of intermediate 96 (64.8 mg, 0.167 mmol) in dichloromethane (2 ml), benzoyl chloride (0.0300 ml, 0.280 mmol) and N,N-diisopropylethylamine (0.200 ml, 1.15 mmol) were added at room temperature, and the mixture was stirred at the same temperature as above for 2 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 140 was obtained as a yellow solid (40.6 mg, 0.106 mmol, 63%). NMR measurement spectra and results of mass spectrometry by HR-ESI-MS are as follows. ¹H NMR (CDCl₃, 400 MHz): δ4.65 (2H, d, J = 6.0 Hz), 7.20-7.93 (12H, m), 8.13 (1H, d, J = 8.4 Hz), 8.33 (1H, s), 8.62 (1H, s), 9.30 (1H, d, J = 2.4 Hz); HRMS (ESI-TOF) m/z: [M + Na]⁺ calcd for C₂₃H₁₈N₄O₂Na 405.1327; Found 405.1320.

The confirmed structure is as follows.

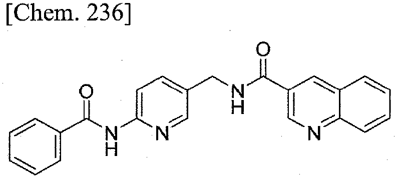

### [Example 141: Production of compound 141]

### (1) Production of intermediate 97

To a solution of intermediate 57 (100 mg, 0.313 mmol) in methanol (2 ml), nickel chloride hexahydrate (73.7 mg, 0.310 mmol) and sodium borohydride (35.2 mg, 0.930 mmol) were added under an ice bath, and the mixture was stirred at the same temperature as above for 0.5 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. To a solution of the residue in dichloromethane (2 ml), 3-pyridineacetic acid (51.0 mg, 0.372 mmol), N,N-diisopropylethylamine (0.270 ml, 1.55 mmol), 1-[bis(dimethylamino)methylene]-1H-1,2,3-triazolo[4,5-b]pyridinium3-oxide hexafluorophosphate (HATU) (236 mg, 0.621 mmol), and dimethylaminopyridine (7.60 mg, 0.0622 mmol) were added at room temperature, and the mixture was stirred at the same temperature as above for 3 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Intermediate 97 was obtained as a yellow oil (59.5 mg, 0.134 mmol, 43%).

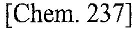

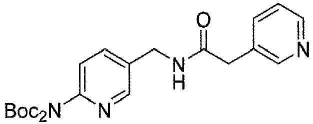

### (2) Production of intermediate 98

To a solution of intermediate 97 (59.5 mg, 0.134 mmol) in dichloromethane (1 ml), 4 M HCl/dioxane (0.65 ml) was added, and the mixture was stirred at room temperature for 1 hour. The reaction mixture was concentrated under reduced pressure to obtain intermediate 98 (0.134 mmol).

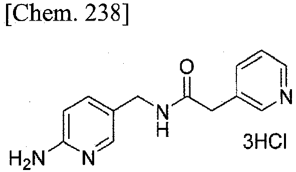

### (3) Production of compound 141

To a solution of intermediate 98 (32.5 mg, 0.134 mmol) in dichloromethane (2 ml), benzoyl chloride (0.0200 ml, 0.187 mmol) and N,N-diisopropylethylamine (0.100 ml, 0.574 mmol) were added at room temperature, and the mixture was stirred at the same temperature as above for 2 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 141 was obtained as a yellow solid (17.0 mg, 0.0491 mmol, 37%). NMR measurement spectra and results of mass spectrometry by HR-ESI-MS are as follows. ¹H NMR (DMSO, 400 MHz): δ3.52 (2H, s), 4.26 (2H, d, J = 6.0 Hz), 7.30-7.77 (12H, m), 8.17 (1H, d, J = 2.4 Hz), 8.39 (1H, s), 8.42-8.46 (2H, m); HRMS (ESI-TOF) m/z: [M + Na]⁺ calcd for C₂₀H₁₈N₄O₂Na 369.1327; Found 369.1323.

The confirmed structure is as follows.

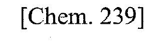

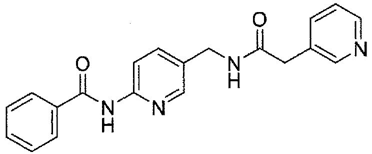

### [Example 142: Production of compound 142]

To a solution of intermediate 59 (50.0 mg, 0.148 mmol) in dichloromethane (2 ml), 1-adamantanecarboxylic acid (41.5 mg, 0.230 mmol), N,N-diisopropylethylamine (0.170 ml, 0.976 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (4.60 mg, 0.0376 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 142 was obtained as a yellow solid (11.8 mg, 0.0302 mmol, 20%). NMR measurement spectra and results of mass spectrometry by HR-ESI-MS are as follows.
¹H NMR (CDCl₃, 400 MHz): δ1.22-2.14 (15H, m), 4.63 (2H, d, J = 5.6 Hz), 6.51 (1H, br), 7.41 (1H, dd, J = 8.0, 4.8 Hz), 7.72 (1H, dd, J = 8.8, 2.4 Hz), 8.11-8.16 (2H, m), 8.27 (2H, s), 8.74 (1H, d, J = 3.6 Hz), 8.98 (1H, s); HRMS (ESI-TOF) m/z: [M + Na]⁺ calcd for C₂₃H₂₆N₄O₂Na 413.1953; Found 413.1948.

The confirmed structure is as follows.

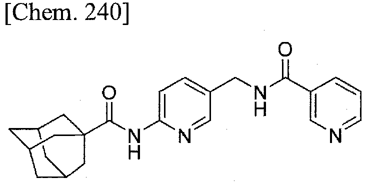

### [Example 143: Production of compound 143]

To a solution of intermediate 59 (50.0 mg, 0.148 mmol) in dichloromethane (3 ml), p-tolylacetic acid (57.0 mg, 0.380 mmol), N,N-diisopropylethylamine (0.160 ml, 0.919 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (5.00 mg, 0.0409 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 143 was obtained as a yellow solid (30.6 mg, 0.0849 mmol, 57%). NMR measurement spectra and results of mass spectrometry by HR-ESI-MS are as follows. ¹H NMR (CDCl₃, 400 MHz): δ2.33 (3H, s), 3.75 (2H, s), 4.61 (2H, d, J = 5.6 Hz), 7.15 (2H, d, J = 7.6 Hz), 7.21 (2H, d, J = 7.6 Hz), 7.49 (1H, dd, J = 8.0, 4.8 Hz), 7.76 (1H, br), 7.87 (1H, d, J = 7.2 Hz), 8.20 (1H, d, J = 8.4 Hz), 8.28 (1H, s), 8.34 (1H, d, J = 8.4 Hz), 8.72 (1H, dd, J = 4.8, 1.6 Hz), 9.14 (1H, br), 9.24 (1H, s); HRMS (ESI-TOF) m/z: [M + Na]⁺ calcd for C₂₁H₂₀N₄O₂Na 383.1484; Found 383.1479.

The confirmed structure is as follows.

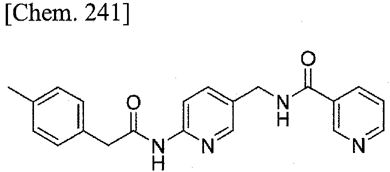

### [Example 144: Production of compound 144]

To a solution of intermediate 59 (50.0 mg, 0.148 mmol) in dichloromethane (3 ml), (4-fluorophenyl)acetic acid (59.0 mg, 0.383 mmol), N,N-diisopropylethylamine (0.160 ml, 0.919 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (5.00 mg, 0.0409 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 97:3). Compound 144 was obtained as a yellow solid (31.7 mg, 0.0870 mmol, 59%). NMR measurement spectra and results of mass spectrometry by HR-ESI-MS are as follows.
¹H NMR (CDCl₃, 400 MHz): δ3.75 (2H, s), 4.62 (2H, d, J = 5.6 Hz), 7.05 (2H, t, J = 8.4 Hz), 7.27-7.33 (3H, m), 7.47 (1H, dd, J = 8.0, 4.8 Hz), 7.83 (1H, dd, J = 8.4, 2.0 Hz), 8.19-8.29 (3H, m), 8.70-8.75 (2H, m), 9.15 (1H, s); HRMS (ESI-TOF) m/z: [M + Na]⁺ calcd for C₂₀H₁₇FN₄O₂Na 387.1233; Found 387.1225.

The confirmed structure is as follows.

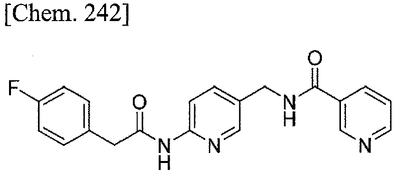

### [Example 145: Production of compound 145]

To a solution of intermediate 59 (50.0 mg, 0.148 mmol) in dichloromethane (3 ml), (4-chlorophenyl)acetic acid (65.0 mg, 0.381 mmol), N,N-diisopropylethylamine (0.160 ml, 0.919 mmol), 1-[bis(dimethylamino)methylene]-1H-1,2,3-triazolo[4,5-b]pyridinium3-oxide hexafluorophosphate (HATU) (144 mg, 0.379 mmol), and dimethylaminopyridine (5.00 mg, 0.0409 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 96:4). Compound 145 was obtained as a yellow solid (36.8 mg, 0.0966 mmol, 65%). NMR measurement spectra and results of mass spectrometry by HR-ESI-MS are as follows.
¹H NMR (CDCl₃, 400 MHz): δ3.87 (2H, s), 4.69 (2H, d, J = 6.0 Hz), 7.29 (2H, d, J = 8.4 Hz), 7.33 (2H, d, J = 8.4 Hz), 7.86-7.93 (1H, m), 8.21-8.28 (1H, m), 8.42-8.55 (2H, m), 8.80-8.90 (2H, m), 9.90 (1H, s); HRMS (ESI-TOF) m/z: [M + Na]⁺ calcd for C₂₀H₁₇ClN₄O₂Na 403.0938; Found 403.0929.

The confirmed structure is as follows.

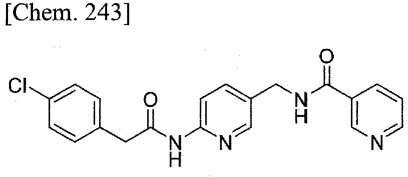

### [Example 146: Production of compound 146]

### (1) Production of intermediate 84

A known compound represented by the following structural formula (intermediate 84) was synthesized in accordance with the method described in Journal of Organic Chemistry (2015), 80 (5), 2676-2699.

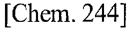

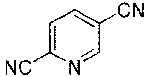

### (2) Production of intermediate 77

To a solution of intermediate 84 (50 mg, 0.387 mmol) in methanol (2 ml), nickel chloride hexahydrate (93.0 mg, 0.391 mmol) and sodium borohydride (22.0 mg, 0.581 mmol) were added under an ice bath, and the mixture was stirred at the same temperature as above for 0.5 hours. Triethylamine (0.0500 ml, 0.359 mmol) and nicotinoyl chloride (70.0 mg, 0.393 mmol) were added thereto at the same temperature as above, and the mixture was further stirred at the same temperature as above for 1 hour. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Intermediate 77 was obtained as a yellow solid (19.0 mg, 0.0797 mmol, 21%).

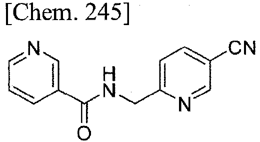

### (3) Production of compound 146

To a solution of intermediate 77 (19.0 mg, 0.0797 mmol) in methanol (1 ml), nickel chloride hexahydrate (19.0 mg, 0.0799 mmol) and sodium borohydride (9.00 mg, 0.238 mmol) were added under an ice bath, and the mixture was stirred at the same temperature as above for 0.5 hours. Triethylamine (0.0200 ml, 0.143 mmol) and nicotinoyl chloride (28.0 mg, 0.157 mmol) were added thereto at the same temperature as above, and the mixture was further stirred at the same temperature as above for 1 hour. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 146 was obtained as a yellow solid (1.50 mg, 0.00432 mmol, 5.4%). NMR measurement spectra and results of mass spectrometry by HR-ESI-MS are as follows.
¹H NMR (CDCl₃, 400 MHz): δ4.71 (2H, d, J = 5.6 Hz), 4.80 (2H, d, J = 4.0 Hz), 7.38-7.50 (3H, m), 7.88 (1H, s), 8.17-8.25 (2H, m), 8.65-8.75 (3H, m), 9.06 (1H, s), 9.12 (1H, s); HRMS (ESI-TOF) m/z: [M + Na]⁺ calcd for C₁₉H₁₇N₅O₂Na 370.1280; Found 370.1274.

The confirmed structure is as follows.

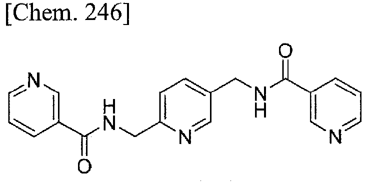

### [Example 147: Production of compound 147]

### (1) Production of intermediate 85

A known compound represented by the following structural formula (intermediate 85) was synthesized in accordance with the method described in Bioorganic Medicinal Chemistry (2015), 23 (15), 4428-4433.

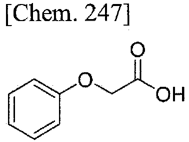

### (2) Production of compound 147

To a solution of intermediate 59 (50.0 mg, 0.148 mmol) in dichloromethane (2 ml), phenoxyacetic acid (intermediate 85) (39.9 mg, 0.262 mmol), N,N-diisopropylethylamine (0.190 ml, 1.09 mmol), 1-[bis(dimethylamino)methylene]-1H-1,2,3-triazolo[4,5-b]pyridinium3-oxide hexafluorophosphate (HATU) (167 mg, 0.439 mmol), and dimethylaminopyridine (5.30 mg, 0.0433 mmol) were added at room temperature, and the mixture was stirred at the same temperature as above for 30 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 20:1). Compound 147 was obtained as a yellow solid (8.00 mg, 0.0221 mmol, 20%). NMR measurement spectra and results of mass spectrometry by HR-ESI-MS are as follows.
¹H NMR (CDCl₃, 400 MHz): δ4.63 (2H, s), 4.66 (2H, d, J = 5.6 Hz), 6.46 (1H, br), 6.92-7.08 (3H, m), 7.32-7.43 (3H, m), 7.78 (1H, dd, J = 8.0, 2.4 Hz), 8.14 (1H, dt, J = 7.6, 2.0 Hz), 8.29 (1H, d, J = 8.8 Hz), 8.34 (1H, d, J = 1.6 Hz), 8.75 (1H, d, J = 3.2 Hz), 8.94 (1H, s), 8.99 (1H, s); HRMS (ESI-TOF) m/z: [M + Na]⁺ calcd for C₂₀H₁₈N₄O₃Na 385.1277; Found 385.1274.

The confirmed structure is as follows.

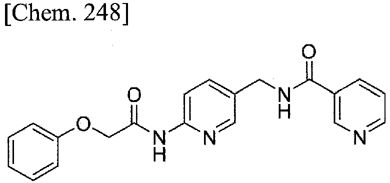

### [Example 148: Production of compound 148]

To a solution of compound 90 (100 mg, 0.353 mmol) in methanol (20 ml), palladium carbon (10%, 20.0 mg, 0.0188 mmol) was added at room temperature in a hydrogen atmosphere (1 atm), and the mixture was stirred at the same temperature as above for 14 hours. Insoluble matter was filtered off, and the filtrate was concentrated under reduced pressure. Compound 148 was obtained as a green oil (85.0 mg, 0.351 mmol, 99%). NMR measurement spectra and results of mass spectrometry by HR-ESI-MS are as follows.
¹H NMR (CD₃OD, 400 MHz): δ4.28 (2H, s), 4.75 (2H, s), 7.35 (1H, d, J = 8.0 Hz), 7.42 (1H, d, J = 8.0 Hz), 7.58 (1H, dd, J = 8.0, 4.8 Hz), 7.85 (1H, t, J = 8.0 Hz), 8.32 (1H, d, J = 8.0 Hz), 8.72 (1H, dd, J = 4.8, 1.6 Hz), 9.05 (1H, d, J = 1.6 Hz); HRMS (ESI-TOF) m/z: [M + Na]⁺ calcd for C₁₃H₁₄N₄ONa 265.1065; Found 265.1061.

The confirmed structure is as follows.

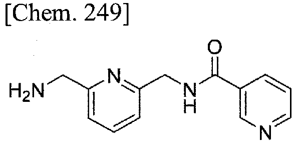

### [Example 149: Production of compound 149]

To a solution of compound 148 (57.0 mg, 0.235 mmol) in dichloromethane (2.4 ml), benzoyl chloride (0.0360 ml, 0.310 mmol) and triethylamine (0.100 ml, 0.733 mmol) were added at room temperature, and the mixture was stirred at the same temperature as above for 17 hours. A saturated aqueous solution of ammonium chloride was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 4:1). Compound 149 was obtained as a yellow solid (32.8 mg, 0.0948 mmol, 40%). NMR measurement spectra and results of mass spectrometry by HR-ESI-MS are as follows. ¹H NMR (CDCl₃, 400 MHz): δ4.79 (4H, d, J = 4.4 Hz), 7.18-7.53 (8H, m), 7.70 (1H, t, J = 8.0 Hz), 7.82 (2H, d, J = 7.2 Hz), 8.12 (1H, d, J = 7.6 Hz), 8.73 (1H, d, J = 3.6 Hz), 9.08 (1H, s); HRMS (ESI-TOF) m/z: [M + Na]⁺ calcd for C₂₀H₁₈N₄O₂Na 369.1327; Found 369.1322.

The confirmed structure is as follows.

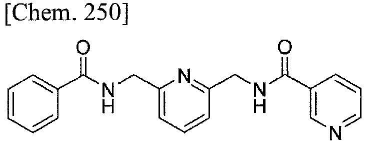

### [Example 150: Production of compound 150]

To a solution of compound 148 (50.0 mg, 0.206 mmol) in dichloromethane (1 ml), nicotinoyl chloride hydrochloride (21.0 mg, 0.118 mmol) and triethylamine (0.0370 ml, 0.265 mmol) were added at room temperature, and the mixture was stirred at room temperature for 14 hours. A saturated aqueous solution of sodium bicarbonate was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 9:1). Compound 150 was obtained as a yellow solid (24.4 mg, 0.0702 mmol, 34%). NMR measurement spectra and results of mass spectrometry by HR-ESI-MS are as follows. ¹H NMR (CDCl₃, 400 MHz): δ4.79 (4H, d, J = 5.2 Hz), 7.28 (2H, d, J = 7.6 Hz), 7.38 (4H, m), 7.72 (1H, t, J = 8.0 Hz), 8.15 (2H, dt, J = 7.6, 2.0 Hz), 8.73 (2H, dd, J = 4.8, 1.6 Hz), 9.06 (2H, d, J = 2,0 Hz); HRMS (ESI-TOF) m/z: [M + Na]⁺ calcd for C₁₉H₁₇N₅O₂Na 370.1280; Found 370.1276.

The confirmed structure is as follows.

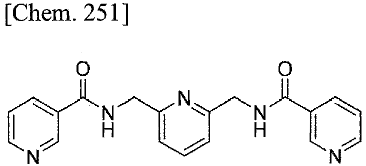

### [Example 151: Production of compound 151]

To a solution of compound 148 (50.0 mg, 0.206 mmol) in dichloromethane (2 ml), 4-pyridinecarboxylic acid (31.0 mg, 0.252 mmol), N,N-diisopropylethylamine (0.180 ml, 1.03 mmol), 1-[bis(dimethylamino)methylene]-1H-1,2,3-triazolo[4,5-b]pyridinium3-oxide hexafluorophosphate (HATU) (160 mg, 0.421 mmol), and dimethylaminopyridine (5.00 mg, 0.0409 mmol) were added at room temperature, and the mixture was stirred at the same temperature as above for 15 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 151 was obtained as a yellow solid (19.9 mg, 0.0573 mmol, 28%). NMR measurement spectra and results of mass spectrometry by HR-ESI-MS are as follows.
¹H NMR (CDCl₃, 400 MHz): δ4.76 (2H, d, J = 5.2 Hz), 4.78 (2H, d, J = 5.2 Hz), 7.23-7.41 (5H, m), 7.60-7.63 (2H, m), 7.70 (1H, t, J = 8.0 Hz), 8.12 (1H, dt, J = 8.4, 2.0 Hz), 8.68-8.73 (3H, m), 9.03 (1H, d, J = 1.6 Hz); HRMS (ESI-TOF) m/z: [M + Na]⁺ calcd for C₁₉H₁₇N₅O₂Na 370.1280; Found 370.1276.

The confirmed structure is as follows.

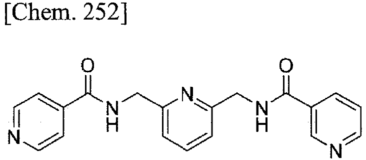

### [Example 152: Production of compound 152]

To a solution of compound 152 (50.0 mg, 0.206 mmol) in dichloromethane (2 ml), 3,5-difluorobenzoic acid (40.0 mg, 0.253 mmol), N,N-diisopropylethylamine (0.180 ml, 1.03 mmol), 1-[bis(dimethylamino)methylene]-1H-1,2,3-triazolo[4,5-b]pyridinium3-oxide hexafluorophosphate (HATU) (160 mg, 0.421 mmol), and dimethylaminopyridine (5.00 mg, 0.0409 mmol) were added at room temperature, and the mixture was stirred at the same temperature as above for 15 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 9:1). Compound 152 was obtained as a yellow solid (67.7 mg, 0.177 mmol, 86%). NMR measurement spectra and results of mass spectrometry by HR-ESI-MS are as follows.
¹H NMR (CDCl₃, 400 MHz): δ4.76 (2H, d, J = 4.8 Hz), 4.80 (2H, d, J = 4.8 Hz), 6.92-6.98 (1H, m), 7.18-7.40 (7H, m), 7.72 (1H, t, J = 8.0 Hz), 8.15 (1H, d, J = 7.6 Hz), 8.74 (1H, d, J = 5.2 Hz), 9.05 (1H, s); HRMS (ESI-TOF) m/z: [M + Na]⁺ calcd for C₂₀H₁₆N₄O₂Na 405.1139; Found 405.1135.

The confirmed structure is as follows.

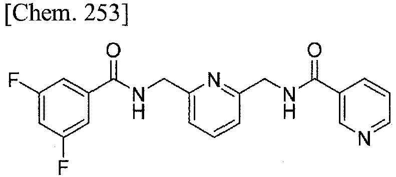

### [Example 153: Production of compound 153]

### (1) Production of intermediate 78

To a solution of intermediate 84 (100 mg, 0.774 mmol) in methanol (8 ml), nickel chloride hexahydrate (184 mg, 0.774 mmol) and sodium borohydride (44.0 mg, 1.16 mmol) were added under an ice bath, and the mixture was stirred at the same temperature as above for 2.5 hours. The reaction solution was concentrated under reduced pressure, and dichloromethane (8 ml) was added to the residue. Triethylamine (0.430 ml, 3.09 mmol) and benzoyl chloride (0.180 ml, 1.55 mmol) were added thereto at room temperature, and the mixture was stirred at the same temperature as above for 3 hours. A saturated aqueous solution of ammonium chloride was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (ethyl acetate 100%). Intermediate 78 was obtained as a yellow solid (50.0 mg, 0.211 mmol, 27%).

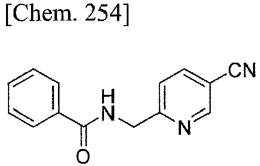

### (2) Production of compound 153

To a solution of intermediate 78 (25.0 mg, 0.105 mmol) in methanol (1 ml), nickel chloride hexahydrate (25.0 mg, 0.105 mmol) and sodium borohydride (6.00 mg, 0.159 mmol) were added under an ice bath, and the mixture was stirred at the same temperature as above for 2 hours. The reaction solution was concentrated under reduced pressure, and dichloromethane (1 ml) was added to the residue. Triethylamine (0.0600 ml, 0.430 mmol) and nicotinoyl chloride (38.0 mg, 0.213 mmol) were added thereto at room temperature, and the mixture was stirred at the same temperature as above for 3 hours. A saturated aqueous solution of ammonium chloride was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 4:1). Compound 153 was obtained as a yellow solid (3.00 mg, 0.00866 mmol, 8.2%). NMR measurement spectra and results of mass spectrometry by HR-ESI-MS are as follows.
¹H NMR (CDCl₃, 400 MHz): δ4.69 (2H, d, J = 5.6 Hz), 4.75 (2H, d, J = 4.4 Hz), 6.67 (1H, br), 7.33-7.54 (6H, m), 7.73 (1H, dd, J = 7.6, 2.0 Hz), 7.86 (2H, d, J = 7.6 Hz), 8.15 (1H, d, 8.0 Hz), 8.58 (1H, s), 8.74 (1H, d, J = 4.8 Hz), 9.01 (1H, s); HRMS (ESI-TOF) m/z: [M + Na]⁺ calcd for C₂₀H₁₈N₄O₂Na 369.1327; Found 369.1327.

The confirmed structure is as follows.

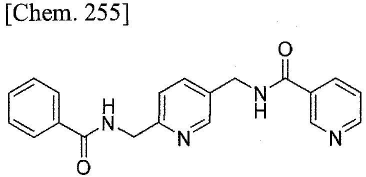

### [Example 154: Production of compound 154]

To a solution of intermediate 59 (50.0 mg, 0.148 mmol) in dichloromethane (3 ml), (4-cyanophenyl)acetic acid (71.0 mg, 0.441 mmol), N,N-diisopropylethylamine (0.160 ml, 0.919 mmol), 1-[bis(dimethylamino)methylene]-1H-1,2,3-triazolo[4,5-b]pyridinium3-oxide hexafluorophosphate (HATU) (167 mg, 0.439 mmol), and dimethylaminopyridine (5.00 mg, 0.0409 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 154 was obtained as a yellow solid (33.4 mg, 0.0899 mmol, 61%). NMR measurement spectra and results of mass spectrometry by HR-ESI-MS are as follows.
¹H NMR (CDCl₃, 400 MHz): δ3.87 (2H, s), 4.65 (2H, d, J = 4.4 Hz), 7.49 (2H, d, J = 7.6 Hz), 7.55 (1H, s), 7.65 (2H, d, J = 7.6 Hz), 7.91 (1H, d, J = 9.2 Hz), 8.22-8.39 (3H, m), 8.74 (1H, s), 9.29 (1H, s); HRMS (ESI-TOF) m/z: [M + Na]⁺ calcd for C₂₁H₁₇N₅O₂Na 394.1280; Found 394.1271.

The confirmed structure is as follows.

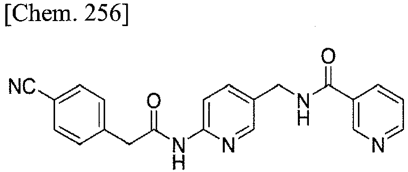

### [Example 155: Production of compound 155]

To a solution of intermediate 59 (50.0 mg, 0.148 mmol) in dichloromethane (3 ml), [2-(trifluoromethyl)phenyl]acetic acid (90.0 mg, 0.441 mmol), N,N-diisopropylethylamine (0.150 ml, 0.861 mmol), 1-[bis(dimethylamino)methylene]-1H-1,2,3-triazolo[4,5-b]pyridinium3-oxide hexafluorophosphate (HATU) (167 mg, 0.439 mmol), and dimethylaminopyridine (5.00 mg, 0.0409 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 155 was obtained as a yellow solid (48.0 mg, 0.116 mmol, 78%). NMR measurement spectra and results of mass spectrometry by HR-ESI-MS are as follows.
¹H NMR (CDCl₃, 400 MHz): δ4.06 (2H, s), 4.68 (2H, s), 7.39-7.83 (5H, m), 8.09-8.15 (1H, m), 8.37 (1H, d, J = 8.0 Hz), 8.49 (1H, s), 8.75 (1H, s), 8.80 (1H, s), 9.75 (1H, s); HRMS (ESI-TOF) m/z: [M + Na]⁺ calcd for C₂₁H₁₇F₃N₄O₂Na 437.1201; Found 437.1197.

The confirmed structure is as follows.

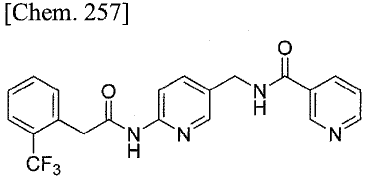

### [Example 156: Production of compound 156]

To a solution of intermediate 59 (50.0 mg, 0.148 mmol) in dichloromethane (3 ml), [3-(trifluoromethyl)phenyl]acetic acid (90.0 mg, 0.441 mmol), N,N-diisopropylethylamine (0.150 ml, 0.861 mmol), 1-[bis(dimethylamino)methylene]-1H-1,2,3-triazolo[4,5-b]pyridinium3-oxide hexafluorophosphate (HATU) (167 mg, 0.439 mmol), and dimethylaminopyridine (5.00 mg, 0.0409 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 19:1). Compound 156 was obtained as a yellow solid (32.3 mg, 0.0779 mmol, 53%). NMR measurement spectra and results of mass spectrometry by HR-ESI-MS are as follows.
¹H NMR (CDCl₃, 400 MHz): δ3.94 (2H, s), 4.67 (2H, s), 7.40-7.79 (5H, m), 8.13 (1H, s), 8.33 (1H, s), 8.46 (1H, s), 8.70 (1H, s), 8.77 (1H, s), 9.01 (1H, br), 9.71 (1H, s), 10.97 (1H, br); HRMS (ESI-TOF) m/z: [M + Na]⁺ calcd for C₂₁H₁₇F₃N₄O₂Na 437.1201; Found 437.1194.

The confirmed structure is as follows.

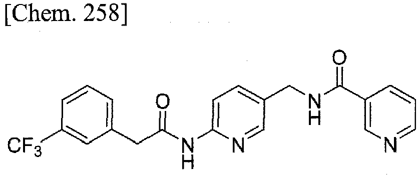

### [Example 157: Production of compound 157]

To a solution of intermediate 59 (50.0 mg, 0.148 mmol) in dichloromethane (1.9 ml), propionic acid (0.0170 ml, 0.227 mmol), 1-(3-dimethylaminopropyl)-3-ethylcarbodiimide hydrochloride (87.0 mg, 0.454 mmol), triethylamine (0.260 ml, 1.87 mmol), and dimethylaminopyridine (4.60 mg, 0.0376 mmol) were added at room temperature, and the mixture was stirred at room temperature for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 10:1). Compound 157 was obtained as a yellow solid (12.5 mg, 0.0440 mmol, 30%). NMR measurement spectra and results of mass spectrometry by HR-ESI-MS are as follows.
¹H NMR (CDCl₃, 400 MHz): δ1.24 (3H, t, J = 7.6 Hz), 2.43 (2H, q, J = 7.6 Hz), 4.62 (2H, d, J = 6.0 Hz), 6.64 (1H, br), 7.40 (1H, dd, J = 7.6, 4.8 Hz), 7.71 (1H, dd, J = 8.8, 2.4 Hz), 8.07-8.28 (4H, m), 8.74 (1H, s), 8.99 (1H, s); HRMS (ESI-TOF) m/z: [M + Na]⁺ calcd for C₁₅H₁₆N₄O₂Na 307.1171; Found 307.1165.

The confirmed structure is as follows.

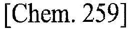

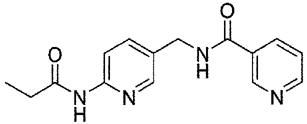

### [Example 158: Production of compound 158]

To a solution of intermediate 59 (100 mg, 0.296 mmol) in dichloromethane (3.8 ml), butyric acid (0.0417 ml, 0.454 mmol), 1-(3-dimethylaminopropyl)-3-ethylcarbodiimide hydrochloride (174 mg, 0.909 mmol), triethylamine (0.524 ml, 3.79 mmol), and dimethylaminopyridine (9.30 mg, 0.0757 mmol) were added at room temperature, and the mixture was stirred at room temperature for 15 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 20:1). Compound 158 was obtained as a yellow solid (15.3 mg, 0.0513 mmol, 17%). NMR measurement spectra and results of mass spectrometry by HR-ESI-MS are as follows.
¹H NMR (CDCl₃, 400 MHz): δ0.98 (3H, t, J = 7.6 Hz), 1.62-1.78 (2H, m), 2.37 (2H, t, J = 7.2 Hz), 4.59 (2H, d, J = 6.4 Hz), 7.10 (1H, br), 7.37 (1H, dd, J = 8.0, 4.8 Hz), 7.69 (1H, dd, J = 8.4, 2.0 Hz), 8.11-8.21 (3H, m), 8.69 (1H, d, J =3.6 Hz), 8.94 (1H, s), 9.00 (1H, s); HRMS (ESI-TOF) m/z: [M + Na]⁺ calcd for C₁₆H₁₈N₄O₂Na 321.1327; Found 321.1327.

The confirmed structure is as follows.

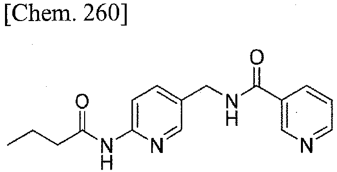

### [Example 159: Production of compound 159]

To a solution of intermediate 59 (50.0 mg, 0.148 mmol) in dichloromethane (1 ml), valeric acid (0.0290 ml, 0.264 mmol), N,N-diisopropylethylamine (0.190 ml, 1.09 mmol), 1-[bis(dimethylamino)methylene]-1H-1,2,3-triazolo[4,5-b]pyridinium3-oxide hexafluorophosphate (HATU) (167 mg, 0.379 mmol), and dimethylaminopyridine (5.40 mg, 0.0442 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 15:1). Compound 159 was obtained as a pale yellow solid (37.8 mg, 0.121 mmol, 82%). NMR measurement spectra and results of mass spectrometry by HR-ESI-MS are as follows.
¹H NMR (CDCl₃, 400 MHz): δ0.95 (3H, t, J = 7.6 Hz), 1.35-1.45 (2H, m), 1.67-1.76 (2H, m), 2.40 (1H, t, J = 7.6 Hz), 4.64 (2H, d, J = 5.6 Hz), 7.00 (1H, br), 7.41 (1H, dd, J = 8.0, 4.8 Hz), 7.73 (1H, d, J = 8.4 Hz), 7.91 (1H, s), 8.13 (1H, d, J = 7.6 Hz), 8.22 (1H, d, J = 8.8 Hz), 8.28 (1H, s), 8.75 (1H, d, J = 4.8 Hz), 8.98 (1H, s); HRMS (ESI-TOF) m/z: [M + Na]⁺ calcd for C₁₇H₂₀N₄O₂Na 335.1484; Found 335.1480.

The confirmed structure is as follows.

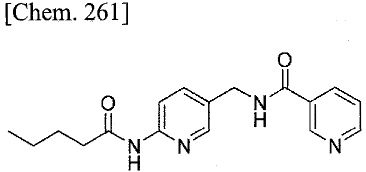

### [Example 160: Production of compound 160]

To a solution of intermediate 59 (54.0 mg, 0.160 mmol) in dichloromethane (2 ml), hexanoic acid (0.0360 ml, 0.284 mmol), N,N-diisopropylethylamine (0.210 ml, 1.21 mmol), 1-[bis(dimethylamino)methylene]-1H-1,2,3-triazolo[4,5-b]pyridinium3-oxide hexafluorophosphate (HATU) (180 mg, 0.473 mmol), and dimethylaminopyridine (5.70 mg, 0.0466 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 50:1). Compound 160 was obtained as a yellow solid (21.9 mg, 0.0701 mmol, 44%). NMR measurement spectra and results of mass spectrometry by HR-ESI-MS are as follows. ¹H NMR (CDCl₃, 400 MHz): δ0.91 (3H, t, J = 7.6 Hz), 1.31-1.77 (6H, m), 2.40 (1H, t, J = 7.6 Hz), 4.63 (2H, d, J = 5.6 Hz), 6.48 (1H, br), 7.41 (1H, dd, J = 7.6, 5.2 Hz), 7.74 (1H, d, J = 9.2 Hz), 8.14 (1H, d, J = 7.6 Hz), 8.25 (1H, s), 8.26 (1H, d, J = 7.6 Hz), 8.53 (1H, s), 8.75 (1H, d, J = 4.8 Hz), 8.98 (1H, s); HRMS (ESI-TOF) m/z: [M + Na]⁺ calcd for C₁₈H₂₃N₄O₂ 327.1821; Found 327.1818.

The confirmed structure is as follows.

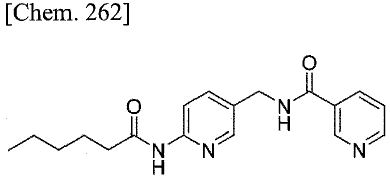

### [Example 161: Production of compound 161]

To a solution of intermediate 59 (100 mg, 0.296 mmol) in dichloromethane (3.8 ml), 2-methylbutyric acid (0.0417 ml, 0.454 mmol), 1-(3-dimethylaminopropyl)-3-ethylcarbodiimide hydrochloride (174 mg, 0.909 mmol), triethylamine (0.524 ml, 3.79 mmol), and dimethylaminopyridine (9.30 mg, 0.0757 mmol) were| added at room temperature, and the mixture was stirred at room temperature for 23 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 10:1). Compound 161 was obtained as a yellow solid (35.8 mg, 0.0513 mmol, 11%). NMR measurement spectra and results of mass spectrometry by HR-ESI-MS are as follows.
¹H NMR (CDCl₃, 400 MHz): δ0.92-1.80 (7H, m), 2.27-2.36 (lH, m), 4.63 (2H, d, J = 6.0 Hz), 6.58 (1H, br), 7.40 (1H, dd, J = 8.0, 4.8 Hz), 7.73 (1H, dd, J = 8.4, 2.0 Hz), 8.10-8.16 (2H, m), 8.23-8.28 (2H, m), 8.73 (1H, s), 8.98 (1H, s); HRMS (ESI-TOF) m/z: [M + Na]⁺ calcd for C₁₇H₂₀N₄O₂Na 335.1484; Found 335.1482.

The confirmed structure is as follows.

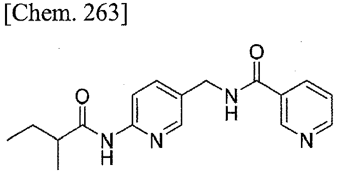

### [Example 162: Production of compound 162]

To solution of intermediate 59 (100 mg, 0.296 mmol) in dichloromethane (3 ml), isovaleric acid (0.0650 ml, 0.592 mmol), N,N-diisopropylethylamine (0.516 ml, 2.96 mmol), 1-[bis(dimethylamino)methylene]-1H-1,2,3-triazolo[4,5-b]pyridinium3-oxide hexafluorophosphate (HATU) (225 mg, 0.592 mmol), and dimethylaminopyridine (7.20 mg, 0.0589 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 20:1). Compound 162 was obtained as a yellow solid (36.2 mg, 0.116 mmol, 39%). NMR measurement spectra and results of mass spectrometry by HR-ESI-MS are as follows.
¹H NMR (CDCl₃, 400 MHz): δ1.01 (6H, d, J = 6.0 Hz), 1.60-1.70 (1H, m), 2.27 (1H, d, J = 6.0 Hz), 4.63 (2H, d, J = 6.0 Hz), 6.51 (1H, br), 7.41 (1H, dd, J = 7.2, 4.8 Hz), 7.74 (1H, d, J = 9.2 Hz), 8.14 (1H, d, J = 8.4 Hz), 8.21-8.28 (2H, m), 8.36 (1H, s), 8.74 (1H, s), 8.98 (1H, s); HRMS (ESI-TOF) m/z: [M + H]⁺ calcd for C₁₇H₂₁N₄O₂ 313.1665; Found 313.1661.

The confirmed structure is as follows.

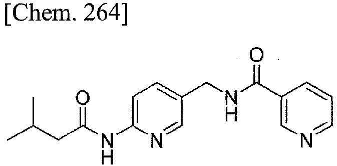

### [Example 163: Production of compound 163]

To a solution of intermediate 59 (50.0 mg, 0.148 mmol) in dichloromethane (1.5 ml), 2-ethylbutyryl chloride (0.0120 ml, 0.0890 mmol) and triethylamine (0.210 ml, 1.48 mmol) were added at room temperature, and the mixture was stirred at room temperature for 1 hour. A saturated aqueous solution of sodium bicarbonate was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 20:1). Compound 163 was obtained as a yellow solid (2.00 mg, 0.00613 mmol, 4.1%). NMR measurement spectra and results of mass spectrometry by HR-ESI-MS are as follows.
¹H NMR (CDCl₃, 400 MHz): δ0.95 (6H, t, J = 7.6 Hz), 1.52-1.64 (4H, m), 2.05-2.14 (1H, m), 4.63 (2H, d, J = 5.6 Hz), 6.53 (1H, br), 7.40 (1H, dd, J = 7.2, 5.2 Hz), 7.74 (1H, d, J = 8.4 Hz), 8.03 (1H, s), 8.13 (1H, d, J = 7.6 Hz), 8.28 (1H, d, J = 7.6 Hz), 8.29 (1H, s), 8.73 (1H, s), 8.98 (1H, s); HRMS (ESI-TOF) m/z: [M + H]⁺ calcd for C₁₈H₂₃N₄O₂ 327.1821; Found 327.1815.

The confirmed structure is as follows.

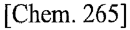

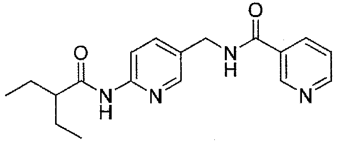

### [Example 164: Production of compound 164]

To a solution of intermediate 59 (50.0 mg, 0.148 mmol) in dichloromethane (1 ml), 4-methylvaleric acid (0.0350 ml, 0.242 mmol), N,N-diisopropylethylamine (0.200 ml, 1.15 mmol), 1-[bis(dimethylamino)methylene]-1H-1,2,3-triazolo[4,5-b]pyridinium3-oxide hexafluorophosphate (HATU) (167 mg, 0.439 mmol), and dimethylaminopyridine (5.30 mg, 0.0433 mmol) were added at room temperature, and the mixture was stirred at the same temperature as above for 15 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 5:1). Compound 164 was obtained as a pale yellow solid (25.0 mg, 0.0766 mmol, 52%). NMR measurement spectra and results of mass spectrometry by HR-ESI-MS are as follows.
¹H NMR (CD₃OD, 400 MHz): δ0.94 (6H, d, J = 6.0 Hz), 1.54-1.66 (3H, m), 2.44 (1H, t, J = 7.6 Hz), 4.56 (2H, s), 7.54 (1H, dd, J = 8.0, 4.8 Hz), 7.80 (1H, dd, J = 8.8, 2.4 Hz), 8.05 (1H, d, J = 8.8 Hz), 8.26 (1H, dt, J = 8.4, 2.0 Hz), 8.31 (1H, s), 8.69 (1H, dd, J = 5.2, 1.2 Hz), 9.00 (1H, d, J = 2.0 Hz); HRMS (ESI-TOF) m/z: [M + H]⁺ calcd for C₁₈H₂₃N₄O₂ 327.1821; Found 327.1819.

The confirmed structure is as follows.

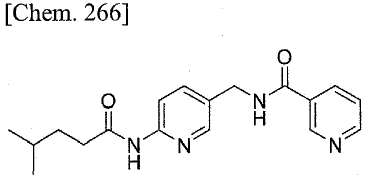

### [Example 165: Production of compound 165]

To a solution of intermediate 59 (50.0 mg, 0.148 mmol) in dichloromethane (2 ml), 3-methylvaleric acid (0.0330 ml, 0.264 mmol), N,N-diisopropylethylamine (0.190 ml, 1.09 mmol), 1-[bis(dimethylamino)methylene]-1H-1,2,3-triazolo[4,5-b]pyridinium3-oxide hexafluorophosphate (HATU) (167 mg, 0.439 mmol), and dimethylaminopyridine (5.40 mg, 0.0442 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 25:1). Compound 165 was obtained as a yellow oil (27.7 mg, 0.0849 mmol, 57%). NMR measurement spectra and results of mass spectrometry by HR-ESI-MS are as follows.
¹H NMR (CDCl₃, 400 MHz): δ0.86 (3H, t, J = 7.6 Hz), 0.93 (3H, d, J = 6.4 Hz), 1.15-1.28 (2H, m), 1.87-1.99 (1H, m), 2.08-2.16 (1H, m), 2.32-2.40 (1H, m), 4.57 (2H, d, J = 5.6 Hz), 7.35 (1H, t, J = 6.0 Hz), 7.67 (1H, d, J = 8.4 Hz), 8.12 (1H, t, J = 8.0 Hz), 8.23 (1H, s), 8.47 (1H, s), 8.65 (1H, s), 8.99 (1H, s); HRMS (ESI-TOF) m/z: [M + H]⁺ calcd for C₁₈H₂₃N₄O₂ 327.1821; Found 327.1818.

The confirmed structure is as follows.

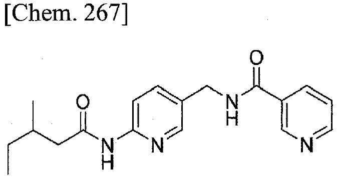

### [Example 166: Production of compound 166]

### (1) Production of intermediate 87

A known compound represented by the following structural formula (intermediate 87) was synthesized in accordance with the method described in European Journal of Medicinal Chemistry (2015), 97, 32-41.

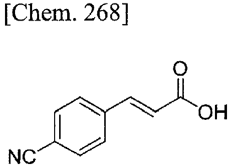

### (2) Production of compound 166

To a solution of intermediate 59 (200 mg, 0.592 mmol) in dichloromethane (8 ml), intermediate 87 (205 mg, 1.18 mmol), triethylamine (0.826 ml, 5.92 mmol), 1-[bis(dimethylamino)methylene]-1H-1,2,3-triazolo[4,5-b]pyridinium3-oxide hexafluorophosphate (HATU) (450 mg, 1.18 mmol), and dimethylaminopyridine (14.5 mg, 0.118 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:1). Compound 166 was obtained as a yellow solid (6.80 mg, 0.0177 mmol, 3.0%). NMR measurement spectra and results of mass spectrometry by HR-ESI-MS are as follows. ¹H NMR (CDCl₃, 400 MHz): δ4.66 (2H, d, J = 6.0 Hz), 6.58 (1H, br), 6.69 (1H, d, J = 15.2 Hz), 7.35-7.83 (8H, m), 8.10-8.50 (3H, m), 8.75 (1H, s), 9.00 (1H, d, J = 10.0 Hz); HRMS (ESI-TOF) m/z: [M + H]⁺ calcd for C₂₂H₁₈N₅O₂ 384.1460; Found 384.1453.

The confirmed structure is as follows.

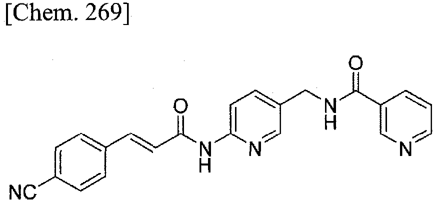

### [Example 167: Production of compound 167]

### (1) Production of intermediate 88

A known compound represented by the following structural formula (intermediate 88) was synthesized in accordance with the method described in Bioorganic Medicinal Chemistry Letters (2013), 23 (13), 3857-3863.

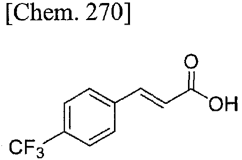

### (2) Production of compound 167

To a solution of intermediate 59 (180 mg, 0.533 mmol) in dichloromethane (6 ml), intermediate 88 (230 mg, 1.07 mmol), triethylamine (0.740 ml, 5.33 mmol), 1-[bis(dimethylamino)methylene]-1H-1,2,3-triazolo[4,5-b]pyridinium3-oxide hexafluorophosphate (HATU) (405 mg, 1.07 mmol), and dimethylaminopyridine (13.0 mg, 0.107 mmol) were added at room temperature, and the mixture was stirred at the same temperature as above for 12 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:1). Compound 167 was obtained as a yellow solid (72.1 mg, 0.169 mmol, 32%). NMR measurement spectra and results of mass spectrometry by HR-ESI-MS are as follows. ¹H NMR (CDCl₃, 400 MHz): δ4.66 (2H, d, J = 6.0 Hz), 6.57 (1H, br), 6.64 (1H, d, J = 15.2 Hz), 7.37-7.83 (8H, m), 8.10-8.43 (3H, m), 8.74 (1H, s), 8.99 (1H, s); HRMS (ESI-TOF) m/z: [M + H]⁺ calcd for C₂₂H₁₈F₃N₄O₂ 427.1382; Found 427.1375.

The confirmed structure is as follows.

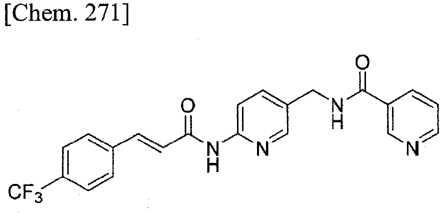

### [Example 168: Production of compound 168]

### (1) Production of intermediate 89

A known compound represented by the following structural formula (intermediate 89) was synthesized in accordance with the method described in WO2008/078291.

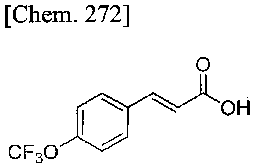

### (2) Production of compound 168

To a solution of intermediate 59 (200 mg, 0.592 mmol) in dichloromethane (5 ml), intermediate 89 (165 mg, 0.711 mmol), triethylamine (0.820 ml, 5.88 mmol), 1-[bis(dimethylamino)methylene]-1H-1,2,3-triazolo[4,5-b]pyridinium3-oxide hexafluorophosphate (HATU) (450 mg, 1.18 mmol), and dimethylaminopyridine (14.4 mg, 0.118 mmol) were added at room temperature, and the mixture was stirred at the same temperature as above for 12 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:4). Compound 168 was obtained as a yellow solid (21.3 mg, 0.0504 mmol, 8.5%). NMR measurement spectra and results of mass spectrometry by HR-ESI-MS are as follows. ¹H NMR (CDCl₃, 400 MHz): δ4.67 (2H, d, J = 6.0 Hz), 6.45 (1H, br), 6.52 (1H, d, J = 16.0 Hz), 7.37-7.80 (7H, m), 8.08 (1H, s), 8.15 (1H, d, J = 7.2 Hz), 8.31-8.37 (2H, m), 8.75 (1H, s), 8.99 (1H, s); HRMS (ESI-TOF) m/z: [M + H]⁺ calcd for C₂₂H₁₈F₃N₄O₃ 443.1331; Found 443.1327.

The confirmed structure is as follows.

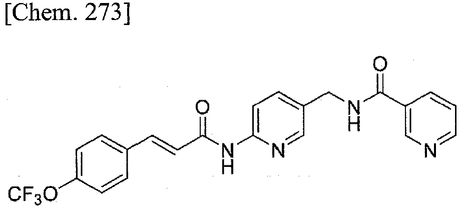

### [Example 169: Production of compound 169]

To a solution of compound 167 (53.4 mg, 0.125 mmol) in ethanol (10 ml), palladium carbon (10%, 26.7 mg, 0.0250 mmol) was added at room temperature in a hydrogen atmosphere (1 atm), and the mixture was stirred at the same temperature as above for 15 hours. Insoluble matter was filtered off, and the filtrate was concentrated under reduced pressure. Compound 169 was obtained as a yellow oil (28.7 mg, 0.0670 mmol, 54%). NMR measurement spectra and results of mass spectrometry by HR-ESI-MS are as follows.
¹H NMR (CDCl₃, 400 MHz): δ2.73 (2H, t, J = 7.2 Hz), 3.10 (2H, t, J = 7.2 Hz), 4.63 (2H, s), 6.64 (1H, br), 7.31-7.79 (8H, m), 8.09-8.21 (2H, m), 8.73 (1H, s), 8.98 (1H, s); HRMS (ESI-TOF) m/z: [M + H]⁺ calcd for C₂₂H₂₀F₃N₄O₂ 429.1538; Found 429.1530.

The confirmed structure is as follows.

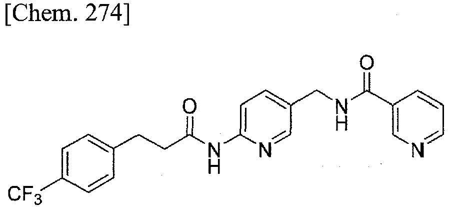

### [Example 170: Production of compound 170]

To a solution of compound 168 (24.5 mg, 0.0580 mmol) in a mixture of ethanol and tetrahydrofuran (2 ml + 2 ml), palladium carbon (10%, 12.3 mg, 0.0116 mmol) was added at room temperature in a hydrogen atmosphere (1 atm), and the mixture was stirred at the same temperature as above for 14 hours. Insoluble matter was filtered off, and the filtrate was concentrated under reduced pressure. Compound 170 was obtained as a yellow oil (22.9 mg, 0.0515 mmol, 89%). NMR measurement spectra and results of mass spectrometry by HR-ESI-MS are as follows.
¹H NMR (CDCl₃, 400 MHz): δ2.70 (2H, t, J = 7.6 Hz), 3.06 (2H, t, J = 7.6 Hz), 4.64 (2H, d, J = 5.2 Hz), 6.46 (1H, br), 7.14 (2H, d, J = 8.0 Hz), 7.38-8.35 (8H, m), 8.75 (1H, d, J = 3.6 Hz), 8.98 (1H, s); HRMS (ESI-TOF) m/z: [M + H]⁺ calcd for C₂₂H₂₀F₃N₄O₃ 445.1488; Found 445.1482.

The confirmed structure is as follows.

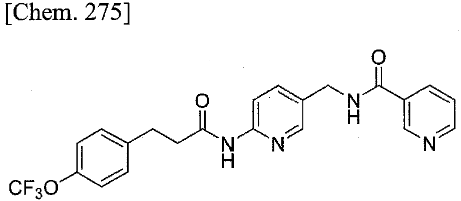

### [Example 171: Production of compound 171]

To a solution of intermediate 59 (50.0 mg, 0.148 mmol) in dichloromethane (2 ml), valeryl chloride (0.0320 ml, 0.270 mmol) and triethylamine (0.300 ml, 2.15 mmol) were| added at room temperature, and the mixture was stirred at room temperature for 2 hours. A saturated aqueous solution of sodium bicarbonate was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 50:1). Compound 171 was obtained as a white solid (31.8 mg, 0.102 mmol, 69%). NMR measurement spectra and results of mass spectrometry by HR-ESI-MS are as follows.
¹H NMR (CDCl₃, 400 MHz): δ0.88 (6H, t, J = 7.2 Hz), 1.24-1.35 (4H, m), 1.56-1.65 (2H, m), 2.54 (4H, t, J = 7.6 Hz); HRMS (ESI-TOF) m/z: [M - H]⁺ calcd for C₂₂H₂₇N₄O₃ 395.2083; Found 395.2084.

The confirmed structure is as follows.

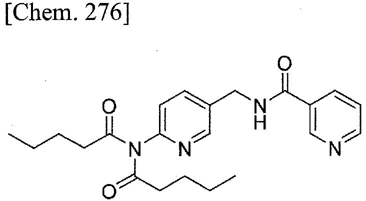

### [Example 172: Production of compound 172]

To a solution of intermediate 59 (50.0 mg, 0.148 mmol) in dichloromethane (1.9 ml), isobutyryl chloride (0.0240 ml, 0.227 mmol) and triethylamine (0.264 ml, 1.89 mmol) were added at room temperature, and the mixture was stirred at room temperature for 1 hour. A saturated aqueous solution of sodium bicarbonate was added to the reaction mixture, followed by extraction with ethyl acetate. The organic layer was washed with saturated saline, dried over anhydrous magnesium sulfate, then filtered, and concentrated under reduced pressure. The residue was purified by silica gel column chromatography (chloroform:methanol = 20:1). Compound 172 was obtained as a yellow solid (52.0 mg, 0.174 mmol, 92%). NMR measurement spectra and results of mass spectrometry by HR-ESI-MS are as follows.
¹H NMR (CDCl₃, 400 MHz): δ1.17 (12H, d, J = 6.4 Hz), 2.89-2.99 (2H, m), 4.73 (2H, d, J = 6.0 Hz), 6.73 (1H, br), 7.20 (1H, d, J = 8.0 Hz), 7.44 (1H, dd, J = 7.6, 5.2 Hz), 7.85 (1H, d, J = 8.4 Hz), 8.17 (1H, d, J = 8.4 Hz), 8.56 (1H, d, J = 8.8 Hz), 8.76 (1H, d, J = 4.8 Hz), 9.02 (1H, s); HRMS (ESI-TOF) m/z: [M + Na]⁺ calcd for C₂₀H₂₄N₄O₃Na 391.1746; Found 391.1741.

The confirmed structure is as follows.

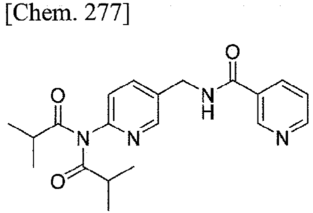

### [Example 173: Production of compound 173]

To a solution of compound 166 (30.0 mg, 0.0782 mmol) in ethanol (5 ml), palladium carbon (10%, 7.5 mg, 0.00702 mmol) was added at room temperature in a hydrogen atmosphere (1 atm), and the mixture was stirred at the same temperature as above for 15 hours. Insoluble matter was filtered off, and the filtrate was concentrated under reduced pressure. Compound 173 was obtained as a yellow oil (9.30 mg, 0.0241 mmol, 31%). NMR measurement spectra and results of mass spectrometry by HR-ESI-MS are as follows.
¹H NMR (CDCl₃, 400 MHz): δ2.66 (2H, t, J = 6.8 Hz), 3.04 (2H, t, J = 6.8 Hz), 4.55 (2H, d, J = 4.0 Hz), 6.72 (1H, br), 7.27 (2H, d, J = 7.6 Hz), 7.33 (1H, dd, J = 7.6, 4.4 Hz), 7.50 (2H, d, J = 7.6 Hz), 7.66 (1H, d, J = 7.2 Hz), 8.08 (1H, t, J = 7.2 Hz), 8.18 (1H, s), 8.65 (1H, s), 8.93 (1H, s); HRMS (ESI-TOF) m/z: [M + H]⁺ calcd for C₂₂H₂₀N₅O₂ 386.1617; Found 386.1609.

The confirmed structure is as follows.

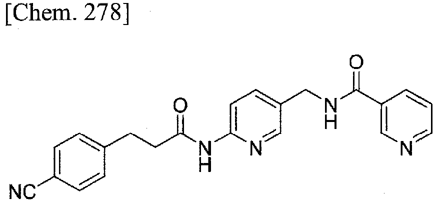

### [Example 174: Production of compound 174]

### (1) Production of intermediate 90

A known compound represented by the following structural formula (intermediate 90) was synthesized in accordance with the method described in Journal of Organic Chemistry (2014), 79 (3), 852-866.

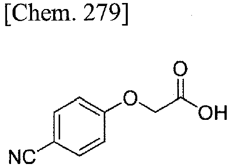

### (2) Production of compound 174

To a solution of intermediate 59 (50.0 mg, 0.148 mmol) in dichloromethane (1.5 ml), intermediate 90 (52.5 mg, 0.296 mmol), triethylamine (0.206 ml, 1.48 mmol), 1-[bis(dimethylamino)methylene]-1H-1,2,3-triazolo[4,5-b]pyridinium3-oxide hexafluorophosphate (HATU) (113 mg, 0.297 mmol), and dimethylaminopyridine (3.60 mg, 0.0294 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:5). Compound 174 was obtained as a yellow solid (24.2 mg, 0.0625 mmol, 42%). NMR measurement spectra and results of mass spectrometry by HR-ESI-MS are as follows. ¹H NMR (CDCl₃, 400 MHz): δ4.67 (2H, d, J = 6.0 Hz), 4.68 (2H, s), 6.51 (1H, br), 7.08 (2H, d, J = 8.8 Hz), 7.42 (1H, dd, J = 8.0, 4.8 Hz), 7.67 (2H, d, J = 8.8 Hz), 7.80 (1H, d, J = 8.4 Hz), 8.14 (1H, d, J = 7.6 Hz), 8.27 (1H, d, J = 8.8 Hz), 8.35 (1H, s), 8.75 (1H, d, J = 4.4 Hz), 8.82 (1H, s), 8.98 (1H, s); HRMS (ESI-TOF) m/z: [M + H]⁺ calcd for C₂₁H₁₈N₅O₃ 388.1410; Found 388.1404.

The confirmed structure is as follows.

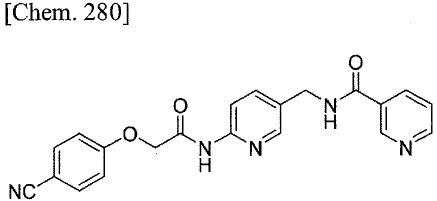

### [Example 175: Production of compound 175]

### (1) Production of intermediate 91

A known compound represented by the following structural formula (intermediate 91) was synthesized in accordance with the method described in WO2008/078291.

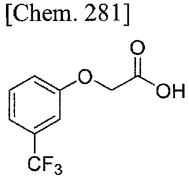

### (2) Production of compound 175

To a solution of intermediate 59 (50.0 mg, 0.148 mmol) in dichloromethane (1.5 ml), intermediate 91 (65.2 mg, 0.296 mmol), triethylamine (0.210 ml, 1.48 mmol), 1-[bis(dimethylamino)methylene]-1H-1,2,3-triazolo[4,5-b]pyridinium3-oxide hexafluorophosphate (HATU) (113 mg, 0.297 mmol), and dimethylaminopyridine (3.60 mg, 0.0294 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:5). Compound 175 was obtained as a yellow solid (3.60 mg, 0.00836 mmol, 5.7%). NMR measurement spectra and results of mass spectrometry by HR-ESI-MS are as follows. ¹H NMR (CDCl₃, 400 MHz): δ4.66 (2H, d, J = 6.0 Hz), 4.67 (2H, s), 6.58 (1H, br), 7.17 (1H, d, J = 8.0 Hz), 7.33 (1H, d, J = 8.0 Hz), 7.41 (1H, dd, J = 7.6, 4.8 Hz), 7.47 (1H, t, J = 8.0 Hz), 7.79 (1H, d, J = 8.4 Hz), 8.14 (1H, d, J = 7.6 Hz), 8.28 (1H, d, J = 8.4 Hz), 8.34 (1H, s), 8.74 (1H, d, J = 4.8 Hz), 8.90 (1H, s), 8.99 (1H, s); HRMS (ESI-TOF) m/z: [M + H]⁺ calcd for C₂₁H₁₈F₃N₄O₃ 431.1331; Found 431.1323.

The confirmed structure is as follows.

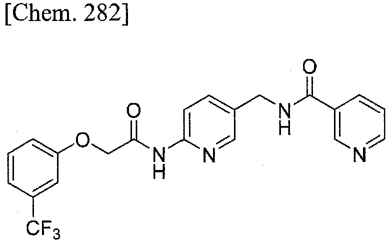

### [Example 176: Production of compound 176]

### (1) Production of intermediate 92

A known compound represented by the following structural formula (intermediate 92) was synthesized in accordance with the method described in WO2020/216766.

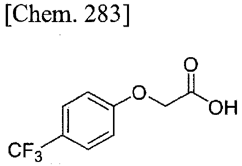

### (2) Production of compound 176

To a solution of intermediate 59 (50.0 mg, 0.148 mmol) in dichloromethane (1.5 ml), intermediate 92 (65.2 mg, 0.296 mmol), triethylamine (0.210 ml, 1.48 mmol), 1-[bis(dimethylamino)methylene]-1H-1,2,3-triazolo[4,5-b]pyridinium3-oxide hexafluorophosphate (HATU) (113 mg, 0.297 mmol), and dimethylaminopyridine (3.60 mg, 0.0294 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:9). Compound 176 was obtained as a yellow solid (10.5 mg, 0.0244 mmol, 16%). NMR measurement spectra and results of mass spectrometry by HR-ESI-MS are as follows. ¹H NMR (CDCl₃, 400 MHz): δ4.65 (2H, d, J = 6.0 Hz), 4.66 (2H, s), 6.72 (1H, br), 7.07 (2H, d, J = 8.8 Hz), 7.40 (1H, dd, J = 7.2, 5.2 Hz), 7.61 (2H, d, J = 8.8 Hz), 7.77 (1H, d, J = 8.4 Hz), 8.13 (1H, d, J = 8.0 Hz), 8.25 (1H, d, J = 8.4 Hz), 8.33 (1H, s), 8.73 (1H, d, J = 3.6 Hz), 8.88 (1H, s), 8.98 (1H, s); HRMS (ESI-TOF) m/z: [M + H]⁺ calcd for C₂₁H₁₈F₃N₄O₃ 431.1331; Found 431.1325.

The confirmed structure is as follows.

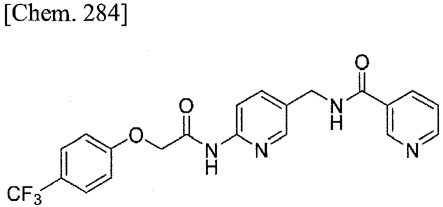

### [Example 177: Production of compound 177]

### (1) Production of intermediate 93

A known compound represented by the following structural formula (intermediate 93) was synthesized in accordance with the method described in Synthesis (2010), (10), 1697-1701.

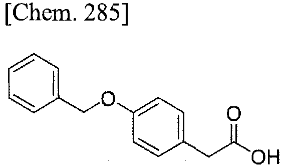

### (2) Production of compound 177

To a solution of intermediate 59 (100 mg, 0.296 mmol) in dichloromethane (3 ml), intermediate 93 (144 mg, 0.594 mmol), triethylamine (0.410 ml, 2.94 mmol), 1-[bis(dimethylamino)methylene]-1H-1,2,3-triazolo[4,5-b]pyridinium3-oxide hexafluorophosphate (HATU) (225 mg, 0.592 mmol), and dimethylaminopyridine (7.20 mg, 0.0294 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:5). Compound 177 was obtained as a yellow solid (61.7 mg, 0.136 mmol, 92%). NMR measurement spectra and results of mass spectrometry by HR-ESI-MS are as follows. ¹H NMR (CDCl₃, 400 MHz): δ3.70 (2H, s), 4.61 (2H, d, J = 5.6 Hz), 5.07 (2H, s), 6.42 (1H, br), 6.99 (2H, d, J = 8.8 Hz), 7.22-7.46 (8H, m), 7.71 (1H, d, J = 8.8 Hz), 7.82 (1H, s), 8.11 (1H, d, J = 8.4 Hz), 8.20-8.24 (2H, m), 8.74 (1H, d, J = 4.4 Hz), 8.96 (1H, s); HRMS (ESI-TOF) m/z: [M + H]⁺ calcd for C₂₇H₂₅N₄O₃ 453.1927; Found 453.1921.

The confirmed structure is as follows.

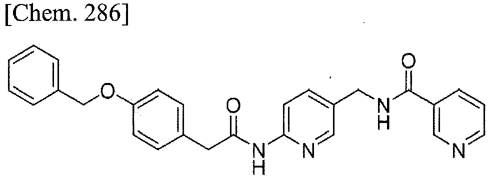

### [Example 178: Production of compound 178]

### (1) Production of intermediate 94

A known compound represented by the following structural formula (intermediate 94) was synthesized in accordance with the method described in European Journal of Organic Chemistry (2008), (2), 337-342.

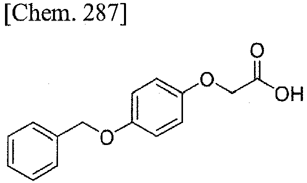

### (2) Production of compound 178

To a solution of intermediate 59 (100 mg, 0.296 mmol) in dichloromethane (3 ml), intermediate 94 (153 mg, 0.592 mmol), triethylamine (0.410 ml, 2.94 mmol), 1-[bis(dimethylamino)methylene]-1H-1,2,3-triazolo[4,5-b]pyridinium3-oxide hexafluorophosphate (HATU) (225 mg, 0.592 mmol), and dimethylaminopyridine (7.20 mg, 0.0294 mmol) were added at room temperature, and the mixture was stirred at the same temperature as above for 16 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, hexane:ethyl acetate = 1:5). Compound 178 was obtained as a yellow solid (138 mg, 0.295 mmol, 99%). NMR measurement spectra and results of mass spectrometry by HR-ESI-MS are as follows. ¹H NMR (CDCl₃, 400 MHz): δ4.58 (2H, s), 4.66 (2H, d, J = 6.0 Hz), 5.03 (2H, s), 6.47 (1H, br), 6.92 (2H, d, J = 10.0 Hz), 6.94 (2H, d, J = 10.0 Hz), 7.28-7.46 (6H, m), 7.78 (1H, d, J = 8.0 Hz), 8.14 (1H, d, J = 8.0 Hz), 8.28 (1H, d, J = 8.4 Hz), 8.34 (1H, s), 8.75 (1H, d, J = 4.0 Hz), 8.95 (1H, s), 8.98 (1H, s); HRMS (ESI-TOF) m/z: [M + H]⁺ calcd for C₂₇H₂₅N₄O₄ 469.1876; Found 469.1870.

The confirmed structure is as follows.

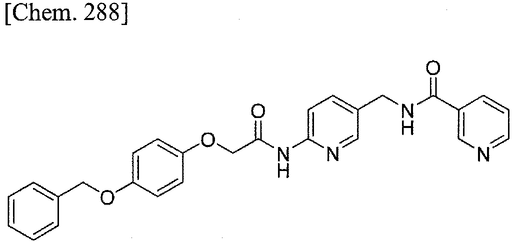

### [Example 179: Production of compound 179]

### (1) Production of intermediate 99

A known compound represented by the following structural formula (intermediate 99) was synthesized in accordance with the method described in Bioorganic Medicinal Chemistry (2018), 26(4), 869-874.

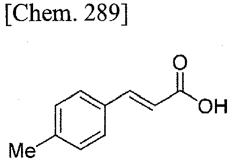

### (2) Production of compound 179

To a solution of intermediate 59 (50.0 mg, 0.148 mmol) in dichloromethane (2 ml), intermediate 99 (46.1 mg, 0.284 mmol), triethylamine (0.329 ml, 2.36 mmol), 1-[bis(dimethylamino)methylene]-1H-1,2,3-triazolo[4,5-b]pyridinium3-oxide hexafluorophosphate (HATU) (180 mg, 0.473 mmol), and dimethylaminopyridine (5.80 mg, 0.0474 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, ethyl acetate 100%). Compound 179 was obtained as a white solid (7.80 mg, 0.0209 mmol, 14%). NMR measurement spectra and results of mass spectrometry by HR-ESI-MS are as follows.
¹H NMR (CD₃OD, 400 MHz): δ2.38 (3H, s), 4.59 (2H, s), 6.84 (1H, d, J = 15.6 Hz), 7.24 (2H, d, J = 8.4 Hz), 7.52 (2H, d, J = 8.4 Hz), 7.56 (1H, dd, J = 8.0, 5.2 Hz), 7.68 (1H, d, J = 15.6 Hz), 7.83 (1H, dd, J = 8.4, 2.4 Hz), 8.22 (1H, d, J = 8.8 Hz), 8.25-8.29 (1H, m), 8.36 (1H, s), 8.69 (1H, dd, J = 4.8, 1.6 Hz), 9.01 (1H, d, J = 1.6 Hz); HRMS (ESI-TOF) m/z: [M + H]⁺ calcd for C₂₂H₂₁N₄O₂ 373.1665; Found 373.1659.

The confirmed structure is as follows.

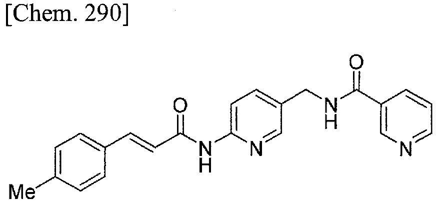

### [Example 180: Production of compound 180]

### (1) Production of intermediate 100

A known compound represented by the following structural formula (intermediate 100) was synthesized in accordance with the method described in JP2018-123127.

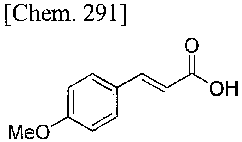

### (2) Production of compound 180

To a solution of intermediate 59 (50.0 mg, 0.148 mmol) in dichloromethane (2 ml), intermediate 100 (50.6 mg, 0.284 mmol), triethylamine (0.330 ml, 2.37 mmol), 1-[bis(dimethylamino)methylene]-1H-1,2,3-triazolo[4,5-b]pyridinium3-oxide hexafluorophosphate (HATU) (180 mg, 0.473 mmol), and dimethylaminopyridine (5.80 mg, 0.0474 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, ethyl acetate 100%). Compound 180 was obtained as a white solid (3.50 mg, 0.00901 mmol, 6.1%). NMR measurement spectra and results of mass spectrometry by HR-ESI-MS are as follows. ¹H NMR (CD₃OD, 400 MHz): δ3.84 (3H, s), 4.59 (2H, s), 6.75 (1H, d, J = 15.6 Hz), 6.98 (2H, d, J = 8.8 Hz), 7.53-7.61 (3H, m), 7.67 (1H, d, J = 15.6 Hz), 7.82 (1H, d, J = 8.8 Hz), 8.22 (1H, d, J = 8.0 Hz), 8.27 (1H, d, J = 8.0 Hz), 8.35 (1H, s), 8.69 (1H, s), 9.01 (1H, s); HRMS (ESI-TOF) m/z: [M + H]⁺ calcd for C₂₂H₂₁N₄O₃ 389.1614; Found 389.1607.

The confirmed structure is as follows.

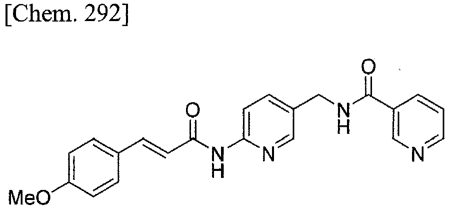

### [Example 181: Production of compound 181]

### (1) Production of intermediate 101

A known compound represented by the following structural formula (intermediate 101) was synthesized in accordance with the method described in Acta Farmaceutica Bonaerense (2001), 20 (1), 5-8.

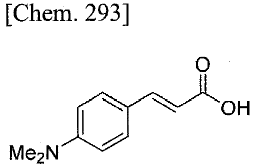

### (2) Production of compound 181

To a solution of intermediate 59 (250 mg, 0.740 mmol) in dichloromethane (2 ml), intermediate 101 (271 mg, 1.42 mmol), triethylamine (1.64 ml, 11.8 mmol), 1-[bis(dimethylamino)methylene]-1H-1,2,3-triazolo[4,5-b]pyridinium3-oxide hexafluorophosphate (HATU) (901 mg, 2.37 mmol), and dimethylaminopyridine (28.9 mg, 0.236 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, ethyl acetate 100%). Compound 181 was obtained as a yellow solid (15.2 mg, 0.0379 mmol, 5.1%). NMR measurement spectra and results of mass spectrometry by HR-ESI-MS are as follows. ¹H NMR (CDCl₃, 400 MHz): δ3.03 (6H, s), 4.64 (2H, d, J = 5.6 Hz), 6.31 (1H, d, J = 15.2 Hz), 6.50 (1H, br), 6.68 (2H, d, J = 8.8 Hz), 7.40 (1H, dd, J = 7.2, 5.6 Hz), 7.45 (2H, d, J = 8.4 Hz), 7.67-7.76 (2H, m), 7.98 (1H, s), 8.14 (1H, d, J = 8.4 Hz), 8.29 (1H, s), 8.35 (1H, d, J = 8.4 Hz), 8.73 (1H, d, J = 3.6 Hz), 9.00 (1H, s); HRMS (ESI-TOF) m/z: [M + H]⁺ calcd for C₂₃H₂₄N₅O₂ 402.1930; Found 402.1923.

The confirmed structure is as follows.

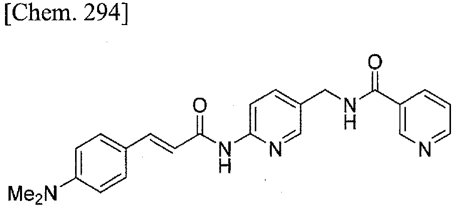

### [Example 182: Production of compound 182]

To a solution of intermediate 59 (50.0 mg, 0.148 mmol) in dichloromethane (1.2 ml), 3,3-dimethylbutanoic acid (0.0360 ml, 0.284 mmol), triethylamine (0.330 ml, 2.37 mmol), 1-[bis(dimethylamino)methylene]-1H-1,2,3-triazolo[4,5-b]pyridinium3-oxide hexafluorophosphate (HATU) (188 mg, 0.474 mmol), and dimethylaminopyridine (5.80 mg, 0.0474 mmol) were added at room temperature, and the mixture was stirred at the same temperature as above for 14 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (basic, ethyl acetate 100%). Compound 182 was obtained as a yellow solid (11.9 mg, 0.0364 mmol, 25%). NMR measurement spectra and results of mass spectrometry by HR-ESI-MS are as follows.
¹H NMR (CDCl₃, 400 MHz): δ1.09 (9H, s), 2.25 (2H, s), 4.62 (2H, d, J = 5.2 Hz), 6.67 (1H, br), 7.39 (1H, dd, J = 7.6, 4.8 Hz), 7.71 (1H, d, J = 8.4 Hz), 8.03 (1H, s), 8.13 (1H, d, J = 8.0 Hz), 8.21 (1H, d, J = 8.4 Hz), 8.26 (1H, s), 8.73 (1H, d, J = 4.0 Hz), 8.98 (1H, s); HRMS (ESI-TOF) m/z: [M + H]⁺ calcd for C₁₈H₂₃N₄O₂ 327.1821; Found 327.1815.

The confirmed structure is as follows.

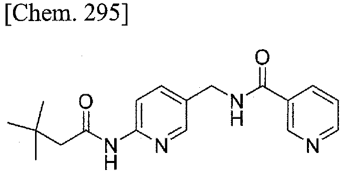

### [Example 183: Production of compound 183]

To a solution of intermediate 59 (50.0 mg, 0.148 mmol) in dichloromethane (1 ml), 2-butynoic acid (14.9 mg, 0.178 mmol), triethylamine (0.200 ml, 1.43 mmol), 1-[bis(dimethylamino)methylene]-1H-1,2,3-triazolo[4,5-b]pyridinium3-oxide hexafluorophosphate (HATU) (113 mg, 0.297 mmol), and dimethylaminopyridine (3.60 mg, 0.0294 mmol) were added at room temperature, and the mixture was stirred at the same temperature as above for 15 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 25:1). Compound 183 was obtained as a pale yellow solid (4.30 mg, 0.0146 mmol, 9.9%). NMR measurement spectra and results of mass spectrometry by HR-ESI-MS are as follows.
¹H NMR (DMSO, 400 MHz): δ2.64 (3H, s), 4.42 (2H, d, J = 5.2 Hz), 7.50 (1H, dd, J = 8.0, 5.2 Hz), 7.71 (1H, d, J = 8.0 Hz), 8.05 (1H, d, J = 8.8 Hz), 8.17-8.21 (2H, m), 8.70 (1H, dd, J = 4.4, 2.0 Hz), 8.78 (1H, dd, J = 8.8, 1.6 Hz), 8.87 (1H, dd, J = 5.6, 1.2 Hz), 9.01 (1H, d, J = 2.8 Hz); HRMS (ESI-TOF) m/z: [M + H]⁺ calcd for C₁₆H₁₅N₄O₂ 295.1195; Found 295.1187.

The confirmed structure is as follows.

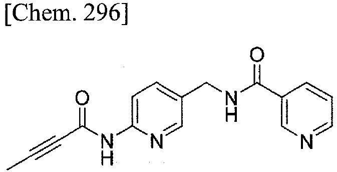

### [Example 184: Production of compound 184]

To a solution of intermediate 59 (50.0 mg, 0.148 mmol) in dichloromethane (1 ml), phenylpropiolic acid (14.9 mg, 0.178 mmol), triethylamine (0.200 ml, 1.43 mmol), 1-[bis(dimethylamino)methylene]-1H-1,2,3-triazolo[4,5-b]pyridinium3-oxide hexafluorophosphate (HATU) (113 mg, 0.297 mmol), and dimethylaminopyridine (3.60 mg, 0.0294 mmol) were added at room temperature, and the mixture was stirred at the same temperature as above for 4 hours. A saturated aqueous solution of sodium bicarbonate was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 25:1). Compound 184 was obtained as a red solid (1.90 mg, 0.00533 mmol, 3.6%). NMR measurement spectra and results of mass spectrometry by HR-ESI-MS are as follows. ¹H NMR (DMSO, 400 MHz): δ4.31 (2H, d, J = 6.4 Hz), 7.06 (1H, d, J = 10.5 Hz), 7.23 (1H, s), 7.36-7.56 (5H, m), 7.72 (1H, s), 8.22 (1H, d, J = 7.2 Hz), 8.30 (2H, d, J = 8.0 Hz), 8.72 (1H, d, J = 5.6 Hz), 9.04 (1H, s), 9.17 (1H, s); HRMS (ESI-TOF) m/z: [M - H]⁺ calcd for C₂₁H₁₅N₄O₂ 355.1195; Found 355.1196.

The confirmed structure is as follows.

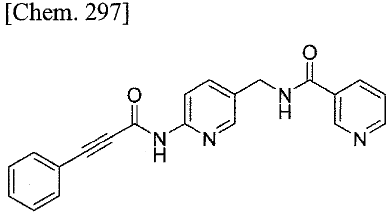

### [Example 185: Production of compound 185]

### (1) Production of intermediate 102

A known compound represented by the following structural formula (intermediate 102) was synthesized in accordance with the method described in Med. Chem. Commum. (2019), 10 (6), 1037-1041.

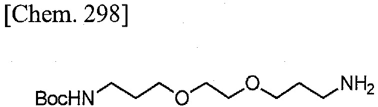

### (2) Production of intermediate 103

To a solution of intermediate 102 (590 mg, 2.13 mmol) in chloroform (20 ml), nicotinoyl chloride hydrochloride (570 mg, 3.20 mmol) and triethylamine (0.890 ml, 6.39 mmol) were added at room temperature, and the mixture was stirred at the same temperature as above for 1 hour. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (ethyl acetate:methanol = 4:1). Intermediate 103 was obtained as a yellow oil (798 mg, 2.09 mmol, 98%).

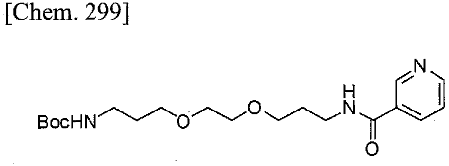

### (3) Production of intermediate 104

To a solution of intermediate 103 (798 mg, 2.09 mmol) in tetrahydrofuran (10 ml), 6 M hydrochloric acid (10.5 ml) was added under an ice bath, and the mixture was stirred at room temperature for 15 hours. The reaction mixture was concentrated under reduced pressure. Intermediate 104 was obtained as a yellow oil (662 mg, 2.08 mmol, 99%).

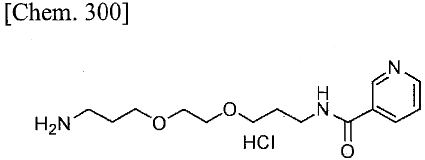

### (4) Production of compound 185

To a solution of intermediate 104 (81.6 mg, 0.257 mmol) in dichloromethane (1.45 ml), 3-quinolinecarboxylic acid (50.2 mg, 0.290 mmol), 1-(3-dimethylaminopropyl)-3-ethylcarbodiimide hydrochloride (83.4 mg, 0.435 mmol), and triethylamine (0.0800 ml, 0.574 mmol) were added at room temperature, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (ethyl acetate:methanol = 9:1). Compound 185 was obtained as a yellow oil (27.0 mg, 0.0619 mmol, 24%). NMR measurement spectra and results of mass spectrometry by HR-ESI-MS are as follows.
¹H NMR (CDCl₃, 400 MHz): δ1.86 (2H, q, J = 6.0 Hz), 1.95 (2H, q, J = 5.2 Hz), 3.55-3.75 (12H, m), 7.32 (1H, t, J = 6.0 Hz), 7.59 (1H, t, J = 7.6 Hz), 7.67 (1H, br), 7.76 (1H, t, J = 7.2 Hz), 7.88 (1H, d, J = 7.6 Hz), 8.05 (1H, d, J = 8.4 Hz), 8.13 (1H, br), 8.22 (1H, d, J = 7.6 Hz), 8.54 (1H, d, J = 5.2 Hz), 8.71 (1H, s), 8.99 (1H, s), 9.32 (1H, s); HRMS (ESI-TOF) m/z: [M + H]⁺ calcd for C₂₄H₂₉N₄O₄ 437.2189; Found 437.2186.

The confirmed structure is as follows.

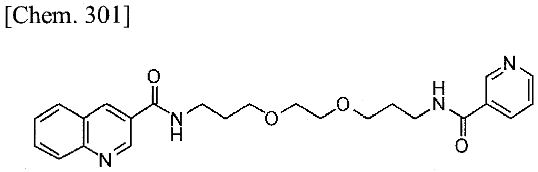

### [Example 186: Production of compound 186]

To a solution of intermediate 104 (50.0 mg, 0.158 mmol) in dichloromethane (1.8 ml), benzoyl chloride (25.3 mg, 0.180 mmol) and triethylamine (0.0500 ml, 0.359 mmol) were added at room temperature, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 98:2). Compound 186 was obtained as a yellow oil (35.3 mg, 0.0916 mmol, 58%). NMR measurement spectra and results of mass spectrometry by HR-ESI-MS are as follows.
¹H NMR (CDCl₃, 400 MHz): δ1.83-1.92 (4H, m), 3.51-3.70 (12H, m), 7.33-7.54 (6H, m), 7.79 (2H, d, J = 7.2 Hz), 8.22 (1H, d, J = 8.4 Hz), 8.54 (1H, d, J = 5.2 Hz), 8.97 (1H, s); HRMS (ESI-TOF) m/z: [M + H]⁺ calcd for C₂₁H₂₈N₃O₄ 386.2074; Found 386.2081.

The confirmed structure is as follows.

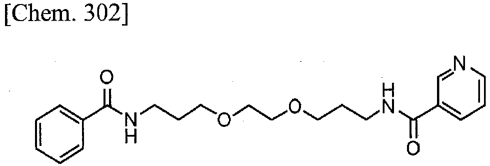

### [Example 187: Production of compound 187]

To a solution of intermediate 104 (81.6 mg, 0.257 mmol) in dichloromethane (1.45 ml), naphthalenecarboxylic acid (50.0 mg, 0.290 mmol), 1-(3-dimethylaminopropyl)-3-ethylcarbodiimide hydrochloride (83.4 mg, 0.435 mmol), and triethylamine (0.0800 ml, 0.574 mmol) were added at room temperature, and the mixture was stirred at the same temperature as above for 2 hours. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (ethyl acetate:methanol = 9:1). Compound 187 was obtained as a yellow oil (12.5 mg, 0.0287 mmol, 11%). NMR measurement spectra and results of mass spectrometry by HR-ESI-MS are as follows.
¹H NMR (CDCl₃, 400 MHz): δ1.79-1.86 (2H, m), 1.89-1.97 (2H, m), 3.52-3.74 (12H, m), 7.29-7.35 (2H, m), 7.49-7.58 (2H, m), 7.75 (1H, br), 7.84 (4H, m), 8.20 (1H, d, J = 7.2 Hz), 8.33 (1H, s), 8.53 (1H, d, J = 5.2 Hz), 8.98 (1H, s); HRMS (ESI-TOF) m/z: [M + H]⁺ calcd for C₂₅H₃₀N₃O₄ 436.2236.; Found 436.2235.

The confirmed structure is as follows.

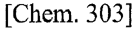

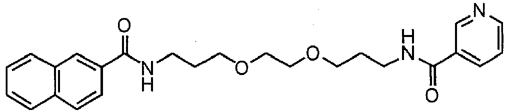

### [Example 188: Production of compound 188]

To a solution of intermediate 104 (100 mg, 0.315 mmol) in dichloromethane (1.5 ml), 6-quinolinecarboxylic acid (62.3 mg, 0.543 mmol), 1-(3-dimethylaminopropyl)-3-ethylcarbodiimide hydrochloride (104 mg, 0.435 mmol), and triethylamine (0.100 ml, 0.717 mmol) were added at room temperature, and the mixture was stirred at the same temperature as above for 1 hour. Water was added to the reaction mixture, followed by extraction with chloroform. The organic layer was washed with saturated saline, dried over anhydrous magnesium sulfate, then filtered, and concentrated under reduced pressure. The residue was purified by silica gel column chromatography (chloroform:methanol = 95:5). Compound 188 was obtained as a yellow oil (11.1 mg, 0.0254 mmol, 8.1%). NMR measurement spectra and results of mass spectrometry by HR-ESI-MS are as follows.
¹H NMR (CDCl₃, 400 MHz): δ1.81-1.88 (2H, m), 1.89-1.97 (2H, m), 3.54-3.73 (12H, m), 7.32-7.43 (2H, m), 7.46 (1H, dd, J = 8.4, 4.0 Hz), 7.93 (1H, br), 8.08-8.15 (2H, m), 8.18-8.26 (2H, m), 8.37 (1H, s), 8.52 (1H, dd, J = 4.4, 1.6 Hz), 8.96-9.00 (2H, m); HRMS (ESI-TOF) m/z: [M + Na]⁺ calcd for C₂₄H₂₈N₄O₄Na 459.2003; Found 459.2006.

The confirmed structure is as follows.

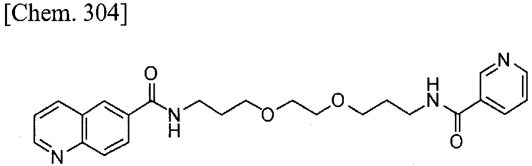

### [Example 189: Production of active metabolite of compound 35]

### (1) Production of intermediate 105

To a solution of (2S,3R,4R,5R)-2-acetoxy-5-((benzyloxy)methyl)tetrahydrofuran-3,4-diyl dibenzoate (1.11 g, 2.20 mmol) in dichloromethane (88 ml), compound 35 (935 mg, 2.42 mmol) and trimethylsilyl triflate (1.59 ml, 8.80 mmol) were added at room temperature, and the mixture was stirred at 45°C for 3 hours. The reaction mixture was concentrated under reduced pressure, and the residue was purified by reverse-phase column chromatography (water:acetonitrile = 95:5 → 5:95). Intermediate 105 represented by the following structural formula was obtained as a colorless liquid (1.06 g, 1.08 mmol, 49%).

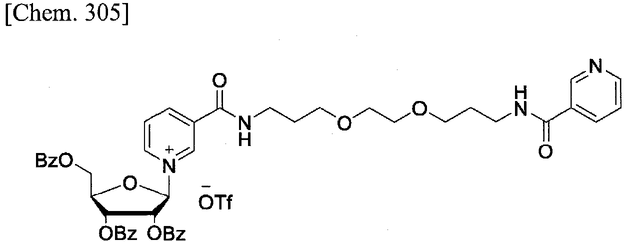

### (2) Production of intermediate 106 (compound 35-ribo)

Intermediate 105 (1.06 g, 1.08 mmol) was dissolved in a 2 M solution of ammonia in methanol (70 ml), and the solution was stirred at -10°C for 3 days. The reaction mixture was concentrated under reduced pressure. The residue was purified by reverse-phase column chromatography (water:acetonitrile = 100:0 → 60:40). Intermediate 106 represented by the following structural formula was obtained as colorless liquid (274 mg, 0.41 mmol, 38%).
ESI-MS m/z: 519 [M+H]⁺

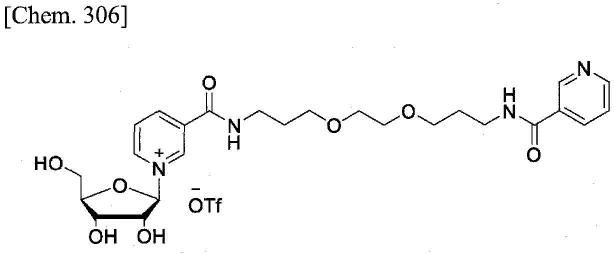

### (3) Production of compound 35-NMN-like metabolite

To a solution of intermediate 106 (264 mg, 0.40 mmol) in trimethyl phosphate (2 ml), phosphorus oxychloride (184 µl, 1.97 mmol) was added at 0°C, and the mixture was stirred at the same temperature as above for 2 hours. The reaction solution was neutralized by the addition of saturated sodium bicarbonate water, and this mixed solution was purified by reverse-phase column chromatography (water:acetonitrile = 100:0 → 60:40). Compound 35-NMN-like metabolite represented by the following structural formula was obtained as a white solid (203 mg, 0.34 mmol, 86%).
ESI-MS m/z: 599 [M+H]⁺

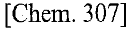

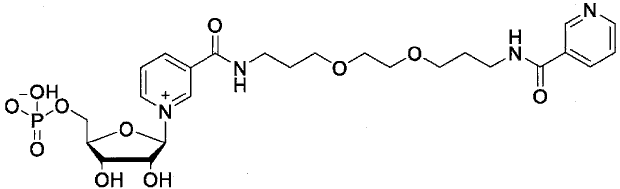

### (4) Production of compound 35-NAD-like metabolite

To a solution of compound 35-NMN-like metabolite (166 mg, 0.28 mmol) in formamide (4 ml), sodium ((2R,3S,4R,5R)-5-(6-amino-9H-purin-9-yl)-3,4-dihydrooxytetrahydrofuran-2-yl)methylmorpholinophosphonate (365 mg, 0.83 mmol), magnesium sulfate (67 mg, 0.56 mmol), and manganese(II) chloride (70 mg, 0.56 mmol) were added at room temperature, and the mixture was stirred at the same temperature as above for 2 days. This reaction mixture was purified by reverse-phase column chromatography (water:acetonitrile = 100:0 → 60:40). Compound 35-NAD-like metabolite represented by the following structural formula was obtained as a white solid (88 mg, 0.095 mmol, 34%).
ESI-MS m/z: 928 [M+H]⁺

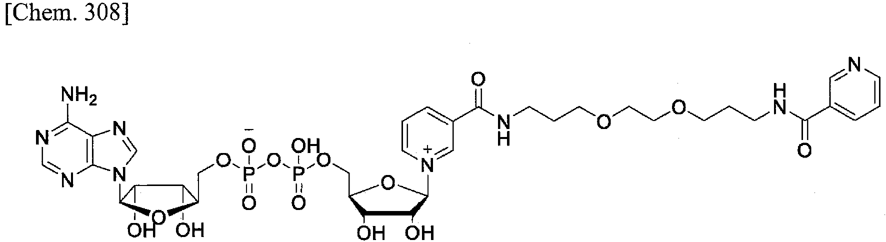

### [Example 190: Production of active metabolite of compound 76]

### (1) Production of intermediate 107

To a solution of (2S,3R,4R,5R)-2-acetoxy-5-((benzyloxy)methyl)tetrahydrofuran-3,4-diyl dibenzoate (565 mg, 1.12 mmol) in dichloromethane (40 ml), compound 76 (318 mg, 1.02 mmol) and trimethylsilyl triflate (736 µl, 4.07 mmol) were added at room temperature, and the mixture was stirred at 45°C for 3 hours. The reaction mixture was concentrated under reduced pressure, and the residue was diluted with diethyl ether. Deposits were filtered, washed with diethyl ether, and then dried in vacuum. Intermediate 107 represented by the following structural formula was obtained as a white solid (1.12 g, 1.23 mmol, 121%).

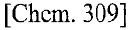

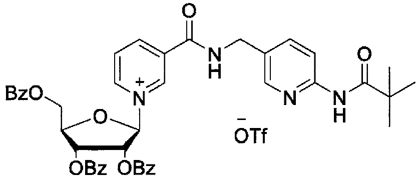

### (2) Production of intermediate 108 (compound 76-ribo)

Intermediate 107 (1.02 g, 1.12 mmol) was dissolved in a 2 M solution of ammonia in methanol (75 ml), and the solution was stirred at -10°C for 3 days. The reaction mixture was concentrated under reduced pressure, and the residue was purified by reverse-phase column chromatography (water:acetonitrile = 100:0 → 60:40). Intermediate 108 represented by the following structural formula was obtained as a white solid (302 mg, 0.51 mmol, 45%).
ESI-MS m/z: 445 [M+H]⁺

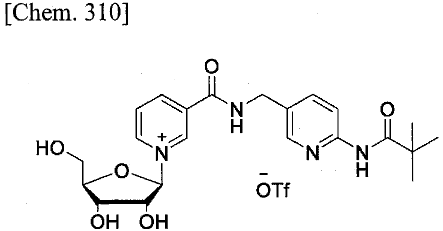

### (3) Production of compound 76-NMN-like metabolite

To a solution of intermediate 108 (282 mg, 0.47 mmol) in trimethyl phosphate (2.4 ml), phosphorus oxychloride (221 µl, 2.37 mmol) was added at 0°C, and the mixture was stirred at the same temperature as above for 2 hours. The reaction solution was neutralized by the addition of saturated sodium bicarbonate water, followed by extraction with ethyl acetate. The aqueous phase was purified by reverse-phase column chromatography (water:acetonitrile = 100:0 → 60:40). Compound 76-NMN-like metabolite represented by the following structural formula was obtained as a white solid (160 mg, 0.47 mmol, 31%).
ESI-MS m/z: 525 [M+H]⁺

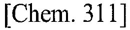

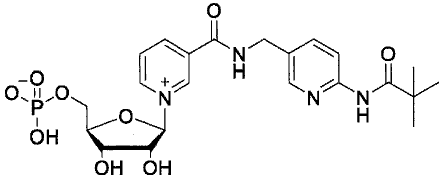

### (4) Production of compound 76-NAD-like metabolite

To a solution of compound 76-NMN-like metabolite (79 mg, 0.15 mmol) in formamide (2.3 ml), sodium ((2R,3S,4R,5R)-5-(6-amino-9H-purin-9-yl)-3,4-dihydrooxytetrahydrofuran-2-yl)methylmorpholinophosphonate (197 mg, 0.45 mmol), magnesium sulfate (36 mg, 0.30 mmol), and manganese(II) chloride (28 mg, 0.30 mmol) were added at room temperature, and the mixture was stirred at the same temperature as above for 3 days. This reaction mixture was purified by reverse-phase column chromatography (water: acetonitrile = 100:0 → 60:40). Compound 76-NAD-like metabolite represented by the following structural formula was obtained as a white solid (36 mg, 0.042 mmol, 28%).
ESI-MS m/z: 854 [M+H]⁺

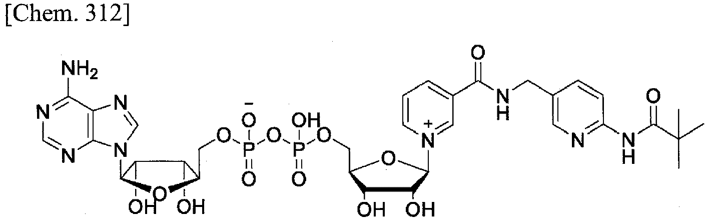

### [Example 191: Production of active metabolite of compound 105]

### (1) Production of intermediate 109

To a solution of (2S,3R,4R,5R)-2-acetoxy-5-((benzyloxy)methyl)tetrahydrofuran-3,4-diyl dibenzoate (471 mg, 0.933 mmol) in dichloromethane (34 ml), compound 105 (282 mg, 0.848 mmol) and trimethylsilyl triflate (0.614 ml, 3.39 mmol) were added at room temperature, and the mixture was stirred at 45°C for 3 hours. The reaction mixture was concentrated under reduced pressure, and the residue was diluted with diethyl ether. Deposits were filtered, washed with diethyl ether, and then dried in vacuum. Intermediate 109 represented by the following structural formula was obtained as a white solid (874 mg, 0.943 mmol, quantitative).

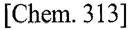

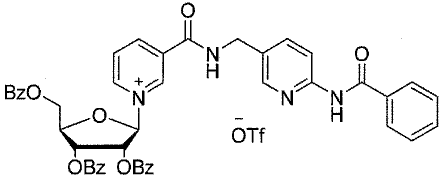

### (2) Production of intermediate 110 (compound 105-ribo)

Intermediate 109 (786 mg, 0.848 mmol) was dissolved in a 7 M solution of ammonia in methanol (32 ml), and the solution was stirred at -10°C for 3 days. The reaction mixture was concentrated under reduced pressure, and the residue was purified by reverse-phase column chromatography (water:acetonitrile = 100:0 → 60:40). Intermediate 110 represented by the following structural formula was obtained as a white solid (235 mg, 0.382 mmol, 45%).
ESI-MS m/z: 465 [M]⁺

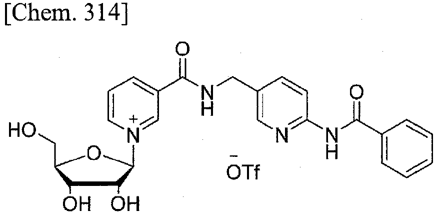

### (3) Production of compound 105-NMN-like metabolite

To a solution of intermediate 110 (235 mg, 0.382 mmol) in trimethyl phosphate (2 ml), phosphorus oxychloride (178 µl, 1.91 mmol) was added at 0°C, and the mixture was stirred at the same temperature as above for 2 hours. The reaction solution was neutralized by the addition of saturated sodium bicarbonate water, and this mixed solution was purified by reverse-phase column chromatography (water:acetonitrile = 100:0 → 60:40). Compound 105-NMN-like metabolite represented by the following structural formula was obtained as a white solid (150 mg, 0.276 mmol, 72%).
ESI-MS m/z: 545 [M+H]⁺

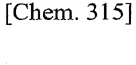

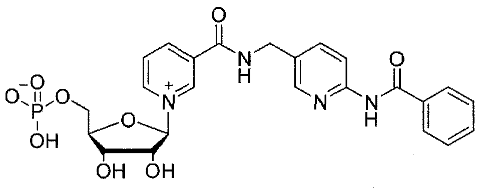

### (4) Production of compound 105-NAD-like metabolite

To a solution of compound 105-NMN-like metabolite (53 mg, 0.097 mmol) in formamide (1.5 ml), sodium ((2R,3S,4R,5R)-5-(6-amino-9H-purin-9-yl)-3,4-dihydrooxytetrahydrofuran-2-yl)methylmorpholinophosphonate (128 mg, 0.292 mmol), magnesium sulfate (23 mg, 0.20 mmol), and manganese(II) chloride (18 mg, 0.15 mmol) were added at room temperature, and the mixture was stirred at the same temperature as above for 3 days. This reaction mixture was purified by reverse-phase column chromatography (water:acetonitrile = 100:0 → 60:40). Compound 105-NAD-like metabolite represented by the following structural formula was obtained as a white solid (32 mg, 0.037 mmol, 38%).
ESI-MS m/z: 874 [M+H]⁺

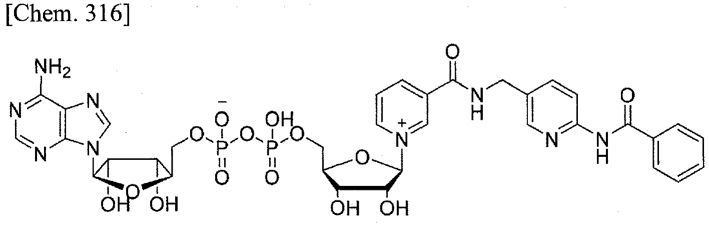

### [Evaluation Example 1: Human SARM1 activity measurement]

### Transient transfection of HEK293 cell with hSARM1

HEK293 cells cultured in a prepared medium of nicotinamide-free DMEM (Dulbecco's modified Eagle medium) supplemented with 10% FBS (BioSera), a 1% penicillin-streptomycin solution (FUJIFILM Wako Pure Chemical Corp.), 5 µM nicotinamide were inoculated at approximately 50,000 cells/well to a 96-well microplate, and the cells were cultured at 37°C under 5% CO₂, transiently transfected on the next day with a pcDNA3 expression vector containing a DNA sequence (SEQ ID NO: 1) deficient in a mitochondrial targeting sequence encoded by an N-terminal 78-base sequence in cDNA encoding human sterile α- and armadillo-motif-containing protein (hSARM1) using PEI MAX(R) reagent (PolySciences Inc.), and forced to express ΔN-hSARM1 (SEQ ID NO: 2). Approximately 8 hours later, PC6, which reacts as a substrate of SARM1-specific base exchange reaction and functions as a fluorescent probe, was added thereto at a final concentration of 50 µM, and intracellular hSARM1 activity was measured (Li et al, eLife 2021 10: e67381). A compound for inhibitory activity evaluation was added at a final concentration of 30 µM at the same time with PC6, while dimethyl sulfoxide (Nacalai Tesque Inc.) was added to samples of a control group at the same time with PC6. The cells were incubated at 37°C under 5% CO₂ for approximately 48 hours from transfection, followed by fluorescent signal measurement using SpectraMax M2 (Molecular Devices) (excitation: 405 nm, fluorescence: 525 nm, fluorescence cutoff: 515 nm). Results of this screening are reported as an inhibition rate (%) relative to a negative control defined as follows by using fluorescence intensity measured from a control group without transfection with ΔN-hSARM1 as a background whereas using fluorescence intensity measured from a control group transfected with ΔN-hSARM1 as the negative control.$Inhibitory rate \left(%\right) = 100 - \frac{\left(Signal of the evaluated compound-Background\right)}{\left(Signal of the negative control-Background\right)} \times 100$

The nicotinamide-free medium was prepared so as to have the same composition as that of DMEM having known composition which is generally used in the art except for nicotinamide.

The hSARM1 inhibitory activity was evaluated as A when the inhibition rate was 75% or more, as B when the inhibition rate was 50% or more, as C when the inhibition rate was 25% or more, and as D when the inhibition rate was 10% or more. The results are shown in Table 2.

**[Table 2]**

| Compound No. | Evaluation on hSARM1 inhibitory | Compound No. | Evaluation on hSARM1 inhibitory | Compound No. | Evaluation on hSARM1 inhibitory | Compound No. | Evaluation on hSARM1 inhibitory |
|---|---|---|---|---|---|---|---|
| 1 | A | 56 | B | 99 | B | 146 | B |
| 3 | A | 57 | A | 101 | A | 147 | A |
| 4 | A | 58 | A | 103 | D | 149 | C |
| 6 | A | 59 | C | 104 | D | 150 | C |
| 8 | B | 60 | D | 105 | B | 151 | C |
| 9 | A | 61 | A | 106 | C | 152 | C |
| 11 | D | 62 | A | 108 | A | 153 | A |
| 12 | A | 63 | D | 109 | A | 154 | A |
| 13 | A | 64 | A | 110 | C | 155 | A |
| 14 | A | 65 | A | 111 | D | 156 | A |
| 15 | A | 66 | A | 112 | D | 157 | D |
| 16 | A | 67 | A | 113 | A | 158 | C |
| 17 | A | 68 | A | 114 | A | 159 | B |
| 18 | A | 69 | A | 115 | A | 160 | A |
| 19 | A | 70 | B | 116 | A | 161 | D |
| 20 | A | 71 | B | 117 | A | 162 | C |
| 21 | B | 72 | B | 118 | A | 163 | C |
| 22 | A | 73 | B | 119 | A | 164 | A |
| 23 | A | 74 | A | 120 | C | 165 | A |
| 24 | A | 75 | A | 121 | A | 166 | A |
| 25 | A | 76 | B | 122 | A | 167 | A |
| 26 | A | 77 | A | 123 | A | 168 | B |
| 27 | A | 78 | A | 124 | A | 169 | A |
| 28 | A | 79 | D | 125 | A | 170 | A |
| 29 | A | 80 | A | 126 | A | 171 | B |
| 30 | B | 81 | B | 127 | A | 172 | D |
| 31 | A | 82 | A | 128 | A | 173 | A |
| 32 | B | 83 | B | 129 | A | 174 | A |
| 33 | A | 84 | A | 130 | A | 175 | A |
| 34 | A | 85 | A | 131 | A | 176 | A |
| 35 | A | 86 | A | 132 | A | 177 | A |
| 36 | D | 87 | B | 133 | A | 178 | A |
| 37 | A | 88 | B | 134 | A | 179 | A |
| 38 | A | 89 | A | 135 | A | 180 | A |
| 39 | A | 90 | D | 136 | A | 182 | A |
| 40 | D | 91 | B | 137 | A | 183 | B |
| 41 | A | 92 | D | 138 | A | 184 | B |
| 42 | A | 93 | B | 139 | C | 185 | C |
| 43 | A | 94 | A | 140 | D | 186 | C |
| 52 | B | 95 | B | 142 | A | 187 | C |
| 53 | A | 96 | A | 143 | A | 188 | C |
| 54 | A | 97 | A | 144 | A | | |
| 55 | A | 98 | D | 145 | A | | |

### [Evaluation Example 2: Evaluation on suppression of degeneration]

### Method for evaluating compound by DRG explant culture

### (Cell culture)

The compound of the present invention was studied for its protective effect against axonal injury using primary cultured cells of mouse fetus-derived dorsal root ganglia (DRGs).

DRGs were collected from a fetus derived from an ICR mouse (13.5 days of pregnancy). DRGs were cultured using a plate coated by incubation with 0.1 mg/mL poly-L-lysine at 4°C for 24 hours or longer, washing with PBS, and then incubation with 2.5 µg/mL Cultrex Mouse Laminin I at 37°C for 4 to 6 hours.

One of DRGs collected was plated to each well of the coated 24-well plate. The DRG was cultured in a nicotinamide-free medium for neuron (supplemented with 10% FBS, 1 mM glutamine, 50 ng/mL NGF2.5S, and penicillin-streptomycin) for 24 hours at maximum from the start of culture and then cultured in a nicotinamide-free medium for neuron (supplemented with 2% MACS NeuroBrew-21, 50 ng/mL NGF2.5S, 1% Glutamax, 20 µM uridine, 20 µM 5-fluoro-2'-deoxyuridine, and penicillin-streptomycin) for 7 to 8 days.

### (Nicotinamide-free medium for neuron)

The nicotinamide-free medium for neuron was prepared so as to have the same constituents as those of the composition published in a previously reported paper (Brewer et al., J. Neuroscience Research 35: 567-576) except for nicotinamide with reference to this composition.

### (Test compound)

The test compound is dissolved in DMSO and added to cells such that the final concentration of DMSO is 0.1% (1000-fold dilution). 10 mM, 1 mM, 100 µM, 10 µM, 1 µM, and 100 nM samples of the test compound are prepared and added at a 1000-fold dilution to cells.

### (Axonal injury ascribable to axotomy and addition of compound)

Axotomy was carried out by scooping out a cell body portion of the cultured DRG with a chip for a micropipette. The test compound was added at the following concentrations (final concentration: 10 µM, 1 µM, 100 nM, 10 nM, 1 nM, and 100 pM) within 30 minutes after axotomy. The compound was evaluated for its protective effect against axonal degeneration by imaging (Leica DMIL microscope, 20× objective lens) the state of neurites 24 hours after axotomy and evaluating the degree of degeneration. The degree of degeneration was quantified using a program uniquely developed using the R language. A degeneration index (DI) was used as an indicator for the degree of degeneration, and the ratio of the area of a fragmented axon to the total area of axons was calculated as defined in the equation of Expression 2. Suppressed axonal degeneration was evaluated when the value of DI was 0.3 or less. The minimum concentration at which axonal degeneration was suppressed was determined for each compound.$Degeneratioin index \left(DI\right) = \frac{Degenerated axons}{All axons}$

The axonal protective activity was evaluated as A ≤ 0.01 µM, 0.01 µM < B ≤ 0.1 µM, and 0.1 µM < C ≤ 1 µM depending on the minimum concentration at which axonal degeneration was suppressed. The results are shown in Table 3.

**[Table 3]**

| Compound No. | Evaluation on protection against degeneration | Compound No. | Evaluation on protection against degeneration | Compound No. | Evaluation on protection against degeneration | Compound No. | Evaluation on protection against degeneration |
|---|---|---|---|---|---|---|---|
| 1 | A | 52 | A | 100 | B | 144 | A |
| 2 | A | 53 | A | 101 | A | 145 | A |
| 3 | A | 54 | A | 102 | A | 146 | B |
| 4 | A | 55 | A | 103 | B | 147 | A |
| 5 | A | 56 | A | 104 | C | 148 | C |
| 6 | A | 57 | B | 105 | A | 149 | C |
| 7 | A | 58 | A | 106 | A | 150 | C |
| 8 | A | 59 | A | 107 | A | 151 | C |
| 9 | A | 61 | A | 108 | A | 153 | B |
| 10 | B | 62 | A | 109 | A | 154 | A |
| 11 | B | 63 | C | 110 | A | 155 | A |
| 12 | B | 64 | A | 111 | A | 156 | A |
| 13 | B | 65 | A | 112 | B | 157 | A |
| 14 | A | 66 | A | 113 | A | 158 | A |
| 15 | A | 67 | A | 114 | A | 159 | A |
| 16 | A | 68 | A | 115 | A | 160 | A |
| 17 | A | 69 | A | 116 | A | 161 | A |
| 18 | A | 70 | A | 117 | A | 162 | A |
| 19 | A | 71 | A | 118 | A | 163 | A |
| 20 | B | 72 | B | 119 | A | 164 | A |
| 21 | A | 73 | A | 120 | A | 165 | A |
| 22 | A | 74 | B | 121 | B | 166 | A |
| 23 | A | 75 | A | 122 | A | 167 | A |
| 24 | A | 76 | A | 123 | A | 168 | A |
| 25 | B | 77 | A | 124 | A | 169 | A |
| 26 | A | 78 | A | 125 | A | 170 | A |
| 27 | B | 80 | A | 126 | A | 171 | A |
| 28 | A | 81 | B | 127 | A | 172 | A |
| 29 | A | 82 | A | 128 | A | 173 | B |
| 30 | B | 83 | A | 129 | A | 174 | B |
| 31 | A | 84 | B | 130 | A | 175 | B |
| 32 | A | 85 | B | 131 | A | 176 | B |
| 33 | B | 86 | B | 132 | A | 177 | B |
| 34 | A | 87 | A | 133 | A | 178 | B |
| 35 | A | 88 | B | 134 | A | 179 | A |
| 36 | - | 89 | A | 135 | A | 180 | A |
| 37 | B | 91 | A | 136 | A | 181 | A |
| 38 | A | 93 | B | 137 | A | 182 | A |
| 39 | A | 94 | A | 138 | A | 183 | B |
| 40 | C | 95 | A | 139 | A | 184 | B |
| 41 | A | 96 | A | 140 | C | 185 | C |
| 42 | A | 97 | A | 141 | C | 186 | B |
| 43 | A | 98 | B | 142 | B | 187 | C |
| 48 | C | 99 | B | 143 | A | 188 | A |

### [Evaluation Example 3: NAMPT activity measurement]

The compound of the present invention was evaluated for the ability to influence the activity of nicotinamide phosphoribosyl transferase (NAMPT) which is an enzyme that metabolizes nicotinamide (Nam), not the target SARM1.

The enzymatic reaction of NAMPT was carried out by adding NAMPT recombinant protein (manufactured by Medical & Biological Laboratories Co., Ltd. (MBL)) to a reaction solution containing the substrate Nam, phosphoribosylpyrophosphate (PRPP), ATP, and the like (composition: 20 µM Tris (pH = 7.4), 2 µM ATP, 0.5 µM PRPP, 0.02% BSA, 2 µM DTT, 1.2 µM MgCl₂, and 10 µM Nam). The amount of the reaction product NMN was detected by detecting the fluorescent signal of a fluorescent substance obtained through the reaction of 2-acetylbenzofuran with NMN using SpectraMax M2 (Molecular Devices) (excitation: 421 nm, fluorescence: 480 nm) (Moriya et al, Chemical Communications 2013, 49 11500). The test compound was added at final concentrations of 10 µM, 3 µM, 1 µM, 300 nM, 100 nM, 30 nM, 10 nM, and 3 nM to the NMN synthesis reaction system described above. The NMN-derived fluorescent signal was normalized against a control group and plotted as the function of the test compound concentrations, and the compound was evaluated for its NAMPT inhibitory effect by calculating IC₅₀.

The IC₅₀ value was evaluated as ∘ for ≥1 µM and as Δ for ≥0.1 µM. The results are shown in Table 4.

**[Table 4]**

| Compound No. | Evaluation on NAMPT inhibitory | Compound No. | Evaluation on NAMPT inhibitory | Compound No. | Evaluation on NAMPT inhibitory | Compound No. | Evaluation on NAMPT inhibitory |
|---|---|---|---|---|---|---|---|
| 2 | Δ | 59 | ○ | 106 | ○ | 146 | ○ |
| 4 | ○ | 60 | ○ | 107 | ○ | 148 | ○ |
| 5 | Δ | 61 | Δ | 108 | Δ | 149 | ○ |
| 6 | Δ | 63 | ○ | 109 | ○ | 150 | ○ |
| 8 | Δ | 64 | Δ | 110 | ○ | 151 | ○ |
| 11 | ○ | 65 | ○ | 111 | ○ | 152 | ○ |
| 13 | ○ | 67 | Δ | 112 | ○ | 153 | ○ |
| 15 | Δ | 70 | ○ | 113 | ○ | 154 | Δ |
| 16 | Δ | 71 | ○ | 114 | ○ | 158 | ○ |
| 17 | Δ | 72 | ○ | 115 | ○ | 159 | ○ |
| 18 | Δ | 73 | ○ | 116 | ○ | 160 | Δ |
| 19 | Δ | 74 | ○ | 117 | ○ | 161 | ○ |
| 20 | ○ | 75 | Δ | 118 | ○ | 162 | ○ |
| 21 | ○ | 76 | ○ | 119 | ○ | 163 | ○ |
| 22 | Δ | 78 | ○ | 120 | ○ | 164 | Δ |
| 25 | ○ | 79 | ○ | 121 | Δ | 165 | ○ |
| 27 | Δ | 80 | Δ | 122 | ○ | 166 | ○ |
| 28 | Δ | 81 | ○ | 123 | Δ | 167 | ○ |
| 30 | ○ | 82 | ○ | 124 | ○ | 168 | ○ |
| 31 | Δ | 83 | ○ | 125 | Δ | 169 | ○ |
| 32 | ○ | 84 | Δ | 126 | Δ | 171 | ○ |
| 34 | ○ | 85 | ○ | 127 | Δ | 172 | ○ |
| 35 | ○ | 86 | Δ | 128 | ○ | 173 | ○ |
| 36 | ○ | 87 | ○ | 129 | ○ | 174 | Δ |
| 37 | ○ | 88 | ○ | 130 | ○ | 175 | Δ |
| 39 | ○ | 90 | ○ | 131 | ○ | 176 | Δ |
| 40 | ○ | 91 | ○ | 132 | ○ | 177 | Δ |
| 41 | ○ | 92 | ○ | 133 | ○ | 178 | Δ |
| 43 | Δ | 95 | ○ | 134 | ○ | 179 | ○ |
| 44 | ○ | 96 | Δ | 135 | ○ | 180 | Δ |
| 45 | ○ | 97 | Δ | 136 | ○ | 181 | Δ |
| 46 | ○ | 98 | ○ | 137 | ○ | 182 | Δ |
| 47 | ○ | 99 | ○ | 138 | ○ | 183 | Δ |
| 48 | ○ | 100 | ○ | 139 | ○ | 184 | ○ |
| 49 | ○ | 102 | ○ | 140 | ○ | 185 | ○ |
| 50 | ○ | 103 | ○ | 141 | ○ | 186 | ○ |
| 51 | ○ | 104 | ○ | 143 | Δ | 187 | ○ |
| 57 | Δ | 105 | ○ | 144 | Δ | 188 | ○ |

### [Evaluation Example 4: SARM1 inhibitory activity evaluation and pharmacokinetic evaluation of compound 35 and its metabolite]

### (In vitro evaluation)

The SARM1 inhibitory activity was evaluated by the following method using recombinant SARM1 from BPS Bioscience.

NMN, an endogenous activator for SARM1, was added at a final concentration of 10 µM to a reaction solution (composition: 40 mM HEPES-KOH (pH 7.4), 0.1% polyvinyl alcohol, and 110 mM potassium acetate) using a 96-well black plate. Subsequently, serial dilutions of the test compound and recombinant human SARM1 (final concentration: 2 ng/µL) were added thereto and preincubated at room temperature for 5 to 10 minutes. NAD and PC6 were added as substrates at final concentrations of 1 mM and 50 µM, respectively, to each well and incubated at room temperature for 1 hour, followed by fluorescent signal measurement using SpectraMax M2 (Molecular Devices) (excitation: 390 nm, fluorescence: 520 nm, fluorescence cutoff: 515 nm) (Li et al, eLife 2021 10: e67381). The fluorescent signal was normalized by using fluorescence intensity measured from a control group without addition of recombinant human SARM1 as a background whereas using fluorescence intensity measured from a control group supplemented with recombinant human SARM1 as the negative control, and plotted as the function of the test compound concentrations, and the compound was evaluated for its SARM1 inhibitory activity by calculating IC₅₀.

As a result of conducting analysis with a UV (254 nm) chromatogram, the production of metabolites compound 35-NMN-like metabolite (see Example 189(3)) and compound 35-NAD-like metabolite (see Example 189(4)) was confirmed in addition to compound 35.

As a result of evaluating SARM1 inhibitory activity, compound 35-NMN-like metabolite and compound 35-NAD-like metabolite exhibited concentration-dependent activity and had stronger activity than that of compound 35.

### (In vivo evaluation)

Compound 35 was intravenously (iv: 1 mg/5 ml/kg) administered at a single dose to C57BL/6J male mice (about 10 weeks old (non-fasted), n = 3), and blood was collected 5, 15, and 30 minutes, and 1, 2, 4, 8, and 24 hours later. Likewise, compound 35 was orally (po: 10 mg/10 ml/kg) administered at a single dose to C57BL/6J male mice (about 10 weeks old (non-fasted), n = 3), and blood was collected 15 and 30 minutes, and 1, 2, 4, 8, and 24 hours later. Urine was collected from each mouse up to 24 hours after administration (pooled urine).

The concentrations of compound 35 and its metabolites in plasma were measured, and oral absorbability (bioavailability) was evaluated. Systemic clearance was calculated for the iv administration, and pharmacokinetic features were determined. The results are shown in Table 5.

**[Table 5]**

| | C₀ (ng/mL) | t_{1/2} (hr) | AUC₀₋ₗₐₛₜ (ng·hr/mL) | AUC_{0-∞} (ng·hr/mL) | Vss (L/kg) | CLtot (mL/hr/kg) |
|---|---|---|---|---|---|---|
| iv | 1598 | 1.90 | 227.8 | 233.5 | 1.57 | 4283 |
| | | | | | | |

| | Cmax (ng/mL) | tmax (hr) | t_{1/2} (hr) | AUC₀₋ₗₐₛₜ (ng·hr/mL) | AUC_{0-∞} (ng·hr/mL) | BA (%) |
|---|---|---|---|---|---|---|
| po | 501.1 | 0.25 | 1.89 | 293.1 | 296.9 | 12.7 |

While the retrieval of metabolites was performed using plasma obtained 4 hours after iv administration and 1 and 8 hours after po administration and urine up to 24 hours, compound 35 and a metabolite with [M+H]⁺ of 195.0764 were detected in the plasma of the mice intravenously or orally given compound 35. The volume of distribution was large as compared with the amount of intercellular fluid, suggesting that the compound was widely distributed in tissues.

BA was approximately 13%. Absorption was faster for the oral administration. The volume of distribution was also relatively large, and the compound was presumed to reach the inside of cells.

The results described above suggested that compound 35 absorbed from the digestive tract widely migrates in tissues and is metabolized into, for example, an NMN-like metabolite, an NAD-like metabolite, and a riboside form in the tissues so that the metabolites exert *in vivo* efficacy.

### [Evaluation Example 5: SARM1 inhibitory activity evaluation and pharmacokinetic evaluation of compound 76 and its metabolite]

### (In vitro evaluation)

As a result of evaluating SARM1 inhibitory activity by the same method as in Test Example 4, compound 76-NMN-like metabolite (see Example 190(3)) and compound 76-NAD-like metabolite (see Example 190(4)) exhibited concentration-dependent activity and had stronger activity than that of compound 76.

### (In vivo evaluation)

Compound 76 was intravenously (iv: 1 mg/5 ml/kg) administered at a single dose to C57BL/6J male mice (about 10 weeks old (non-fasted), n = 3), and blood was collected 5 and 15 minutes, and 1, 2, 4, and 8 hours later. Likewise, compound 76 was orally (po: 10 mg/10 ml/kg) administered at a single dose to C57BL/6J male mice (about 10 weeks old (non-fasted), n = 3), and blood was collected 15 and 30 minutes, and 2, 4, 8, and 24 hours later.

The concentrations of compound 76 and its metabolites in plasma were measured, and oral absorbability (bioavailability) was evaluated. Systemic clearance was calculated for the iv administration, and pharmacokinetic features were determined. The results are shown in Table 6.

**[Table 6]**

| | C₀ (ng/mL) | t_{1/2} (hr) | AUC₀₋ₗₐₛₜ (ng·hr/mL) | AUC_{0-∞} (ng·hr/mL) | Vss (L/kg) | CLtot (mL/hr/kg) |
|---|---|---|---|---|---|---|
| iv | 1940 | 1.14 | 1160 | 1160 | 0.89 | 878 |
| | | | | | | |

| | Cmax (ng/mL) | tmax (hr) | t_{1/2} (hr) | AUC₀₋ₗₐₛₜ (ng·hr/mL) | AUC_{0-∞} (ng·hr/mL) | BA (%) |
|---|---|---|---|---|---|---|
| po | 9850 | 0.25 | 3.21 | 14100 | 14200 | 124 |

While the retrieval of metabolites was performed using plasma obtained after iv and po administration, compound 76-ribo (see Example 190(2)) metabolite and compound 76-NMN-like metabolite (see Example 190(3)) were detected, albeit at a much lower concentration than that of compound 76, in the plasma of the mice intravenously or orally given compound 76. The volume of distribution of compound 76 was also large, as was the case for compound 35, suggesting that the compound was widely distributed in tissues.

The results described above suggested that compound 35 and compound 76 absorbed from the digestive tract widely migrate in tissues and are metabolized into, for example, an NMN-like metabolite, an NAD-like metabolite, and a riboside form in the tissues so that the metabolites exert *in vivo* efficacy.

The compounds of the other Examples, which are nicotinamide derivatives, might produce an NMN-like metabolite, an NAD-like metabolite, and the like through a metabolism mechanism based on the salvage pathway and were considered to exert *in vivo* efficacy through such a mechanism.

### [Evaluation Example 6: SARM1 inhibitory activity evaluation and pharmacokinetic evaluation of compound 105 and its metabolite]

### (In vitro evaluation)

As a result of conducting analysis with a UV (254 nm) chromatogram, the production of metabolites compound 105-NMN-like metabolite (see Example 191(3)), compound 105-NAD-like metabolite (see Example 191(4)), and compound 105-riboside form was confirmed in addition to compound 105.

As a result of evaluating SARM1 inhibitory activity by the same method as in Test Example 4, compound 105-NMN-like metabolite, compound 105-NAD-like metabolite, and compound 105-riboside metabolite exhibited stronger activity in this order, and all the metabolites of the compound had equivalent SARM1 inhibitory activity.

### (In vivo evaluation)

Compound 105 was intravenously (iv: 1 mg/5 ml/kg) administered at a single dose to C57BL/6J male mice (about 10 weeks old (non-fasted), n = 3), and blood was collected 5, 15, and 30 minutes, and 1, 2, 4, 8, and 24 hours later. Likewise, compound 105 was orally (po: 10 mg/10 ml/kg) administered at a single dose to C57BL/6J male mice (about 10 weeks old (non-fasted), n = 3), and blood was collected 15 and 30 minutes, and 1, 2, 4, 8, and 24 hours later. Also, compound 105 was orally (po: 10 mg/10 ml/kg) administered at a single dose to C57BL/6J male mice (about 10 weeks old (non-fasted), n = 3), and the liver, the brain, and muscle were collected 30 minutes and 1, 4, and 8 hours later.

The concentration of compound 105 in plasma was measured, and oral absorbability (bioavailability: BA) was evaluated. A half-life, the volume of distribution in a stationary state, and systemic clearance, and the like were calculated for the iv administration, and pharmacokinetic features were determined. The results are shown in Table 7.

**[Table 7]**

| | C₀ (ng/mL) | t_{1/2} (hr) | AUC₀₋ₗₐₛₜ (ng·hr/mL) | AUC_{0-∞} (ng·hr/mL) | Vss (L/kg) | CLtot (mL/hr/kg) |
|---|---|---|---|---|---|---|
| iv | 1052 | 2.81 | 391 | 408 | 3.14 | 2451 |
| | | | | | | |

| | Cmax (ng/mL) | tmax (hr) | t_{1/2} (hr) | AUC₀₋ₗₐₛₜ (ng·hr/mL) | AUC_{0-∞} (ng·hr/mL) | BA (%) |
|---|---|---|---|---|---|---|
| po | 2524 | 0.25 | 4.13 | 2414 | 2422 | 59.4 |

As a result of analyzing metabolites in the liver, the brain, and muscle by LC-MS/MS, the production of compound 105-NMN-like metabolite (see Example 191(3)) and compound 105-NAD-like metabolite (see Example 191(4)) was confirmed in addition to compound 105.

The volume of distribution after iv administration was large as compared with the amount of intercellular fluid, suggesting that the compound was widely distributed in tissues.

BA was approximately 59%. Absorption was faster for the oral administration.

The results described above suggested that compound 105 absorbed from the digestive tract widely migrates in tissues and is metabolized into, for example, an NMN-like metabolite and an NAD-like metabolite in the tissues so that the metabolites exert *in vivo* efficacy.

### Industrial Applicability

The compound represented by the general formula (I) of the present invention or the pharmaceutically acceptable salt thereof has SARM1 inhibitory activity and/or has weak NAMPT inhibitory activity, and has excellent pharmacokinetic characteristics, and is therefore useful for treatment of a disease subject to prevention, alleviation, and/or therapy through the suppression of axonal degeneration, typically, a neurodegenerative disease, via these properties.

## Claims

1. A compound represented by the following general formula (I): 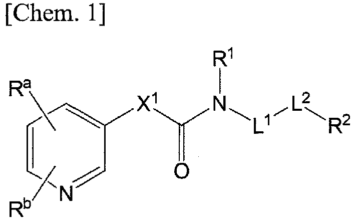
or a pharmaceutically acceptable salt thereof, wherein
R^{a} and R^{b} are each independently a hydrogen atom, halogen, nitro, cyano, amino, hydroxy, C₁₋₆ alkyl, halo-C₁₋₆ alkyl, amino-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, or C₂₋₆ alkynyloxy, or R^{a} and R^{b}, which present on adjacent carbon atoms, optionally form a substituted or unsubstituted carbocyclic ring or heterocyclic ring condensed with a pyridine ring, together with the carbon atoms to which they are bonded,
X¹ is a direct bond, -CR¹¹=CR¹¹-, or -(CONR¹¹)ₘCH₂-,
R¹ is a hydrogen atom or C₁₋₆ alkyl,
L¹ is C₁₋₁₀ alkylene, arylene, C₁₋₁₀ alkylene-arylene, arylene-C₁₋₁₀ alkylene, C₁₋₁₀ alkylene-arylene-C₁₋₁₀ alkylene, arylene-C₁₋₁₀ alkylene-arylene, heterocyclylene, C₁₋₁₀ alkylene-heterocyclylene, heterocyclylene-C₁₋₁₀ alkylene, or C₁₋₁₀ alkylene-heterocyclylene-C₁₋₁₀ alkylene, wherein the alkylene, arylene, or heterocyclylene moiety is optionally substituted with halogen, -COOR¹², -CONHR¹², or oxo,
L² is a direct bond, -O-, -NR¹³-, -NR¹³SO₂-, or -(OCH₂CH₂)ₙO-, and
R² is a hydrogen atom, azide, substituted or unsubstituted C₁₋₆ alkyl, substituted or unsubstituted C₂₋₆ alkenyl, substituted or unsubstituted C₂₋₆ alkynyl, -COR¹⁴, substituted or unsubstituted aryl, or substituted or unsubstituted heterocyclyl,
wherein
m is 0 or 1,
n is 1 to 6,
each R¹¹ is independently a hydrogen atom or C₁₋₆ alkyl,
R¹² is a hydrogen atom or C₁₋₆ alkyl,
R¹³ is a hydrogen atom, C₁₋₆ alkyl, or -COR¹⁴, and
R¹⁴ is substituted or unsubstituted C₁₋₆ alkyl, substituted or unsubstituted C₂₋₆ alkenyl, substituted or unsubstituted C₂₋₆ alkynyl, substituted or unsubstituted C₃₋₁₀ cycloalkyl,
substituted or unsubstituted C₃₋₁₀ cycloalkenyl, substituted or unsubstituted aryl, or substituted or unsubstituted heterocyclyl,
provided that the compound is not a compound in which L¹ is arylene, C₁₋₃ alkylene-arylene, arylene-C₁₋₃ alkylene, or C₁₋₃ alkylene-arylene-C₁₋₃ alkylene, and L² is a direct bond, a compound in which L¹ is C₁₋₃ alkylene-arylene, and L² is -O-, or a compound in which L¹ is C₁₋₃ alkylene, L² is -NH-, and R² is -CO-5-membered heterocyclyl.

2. The compound or the pharmaceutically acceptable salt thereof according to claim 1, wherein the compound is a compound represented by the following general formula (Ia): 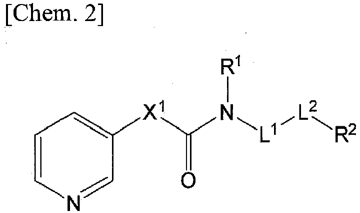 wherein
X¹ is a direct bond, -CR¹¹=CR¹¹-, or -CONR¹¹CH₂-,
R¹ is a hydrogen atom or C₁₋₆ alkyl,
L¹ is C₁₋₁₀ alkylene, arylene, C₁₋₃ alkylene-arylene, arylene-C₁₋₃ alkylene, C₁₋₃ alkylene-arylene-C₁₋₃ alkylene, heterocyclylene, C₁₋₃ alkylene-heterocyclylene, heterocyclylene-C₁₋₃ alkylene, or C₁₋₃ alkylene-heterocyclylene-C₁₋₃ alkylene, wherein the alkylene moiety is optionally substituted with -COOR¹² or -CONHR¹²,
L² is a direct bond, -O-, -NR¹³-, -NR¹³SO₂-, or -(OCH₂CH₂)ₙO-, and
R² is a hydrogen atom, azide, substituted or unsubstituted C₁₋₆ alkyl, substituted or unsubstituted C₂₋₆ alkenyl, substituted or unsubstituted C₂₋₆ alkynyl, -COR¹⁴, substituted or unsubstituted aryl, or substituted or unsubstituted heterocyclyl,
wherein
n is 1 to 6,
each R¹¹ is independently a hydrogen atom or C₁₋₆ alkyl,
R¹² is a hydrogen atom or C₁₋₆ alkyl,
R¹³ is a hydrogen atom, C₁₋₆ alkyl, or -COR¹⁴,
R¹⁴ is substituted or unsubstituted C₁₋₆ alkyl, substituted or unsubstituted C₂₋₆ alkenyl, substituted or unsubstituted aryl, or substituted or unsubstituted heterocyclyl, provided that the compound is not a compound in which L¹ is arylene, C₁₋₃ alkylene-arylene, arylene-C₁₋₃ alkylene, or C₁₋₃ alkylene-arylene-C₁₋₃ alkylene, and L² is a direct bond, a compound in which L¹ is C₁₋₃ alkylene-arylene, and L² is -O-, or a compound in which L¹ is C₁₋₃ alkylene, L² is -NH-, and R² is -CO-5-membered heterocyclyl.

3. The compound or the pharmaceutically acceptable salt thereof according to claim 1 or 2, wherein L² is -O- or -NH-.

4. The compound or the pharmaceutically acceptable salt thereof according to claim 3, wherein the compound is represented by the following general formula (II): 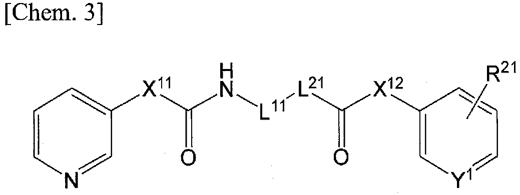 wherein
X¹¹ is a direct bond, -CR¹¹=CR¹¹-, or -CONR¹¹CH₂-,
X¹² is a direct bond, C₁₋₃ alkylene, -CR¹¹=CR¹¹-, or -CH₂NR¹¹CO-,
L¹¹ is C₁₋₁₀ alkylene, arylene, C₁₋₃ alkylene-arylene, arylene-C₁₋₃ alkylene, C₁₋₃ alkylene-arylene-C₁₋₃ alkylene, heterocyclylene, C₁₋₃ alkylene-heterocyclylene, heterocyclylene-C₁₋₃ alkylene, or C₁₋₃ alkylene-heterocyclylene-C₁₋₃ alkylene,
L²¹ is -O- or -NH-,
Y¹ is N or CH,
each R¹¹ is independently a hydrogen atom or C₁₋₆ alkyl,
R¹² is a hydrogen atom or C₁₋₆ alkyl, and
each R²¹ is independently selected from the group consisting of halogen, nitro, cyano, amino, hydroxy, C₁₋₆ alkyl, halo-C₁₋₆ alkyl, hydroxy-C₁₋₆ alkyl, C₁₋₆ alkoxy, halo-C₁₋₆ alkoxy, C₁₋₆ alkoxy-C₁₋₆ alkoxy, C₁₋₆ alkoxycarbonyl, C₂₋₆ alkenyl, C₂₋₆ alkenyloxy, C₂₋₆ alkynyl, and C₂₋₆ alkynyloxy.

5. The compound or the pharmaceutically acceptable salt thereof according to claim 1 or 2, wherein L² is a direct bond or -(OCH₂CH₂)ₙO-.

6. The compound or the pharmaceutically acceptable salt thereof according to claim 5, wherein R² is a hydrogen atom, substituted or unsubstituted C₁₋₆ alkyl, substituted or unsubstituted C₂₋₆ alkenyl, substituted or unsubstituted C₂₋₆ alkynyl, substituted or unsubstituted aryl, or substituted or unsubstituted heterocyclyl.

7. The compound or the pharmaceutically acceptable salt thereof according to claim 1, wherein the compound is the following compound:

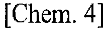

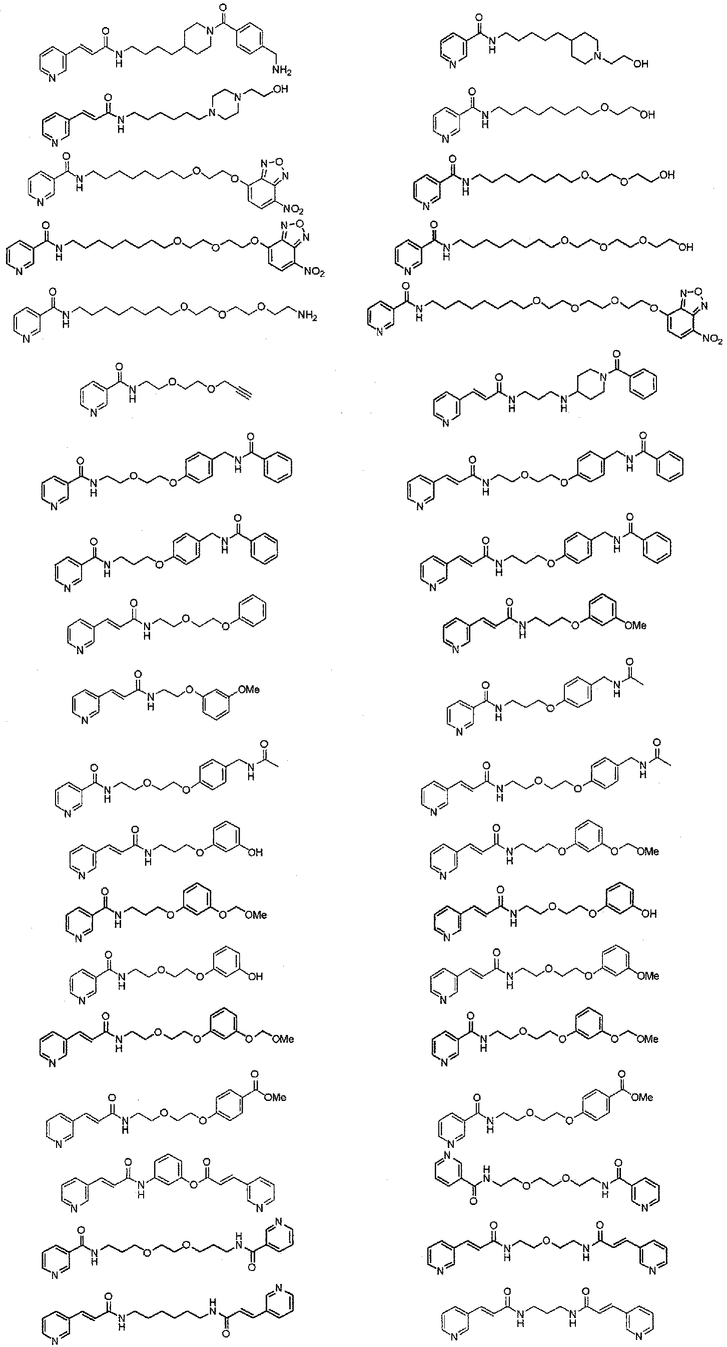

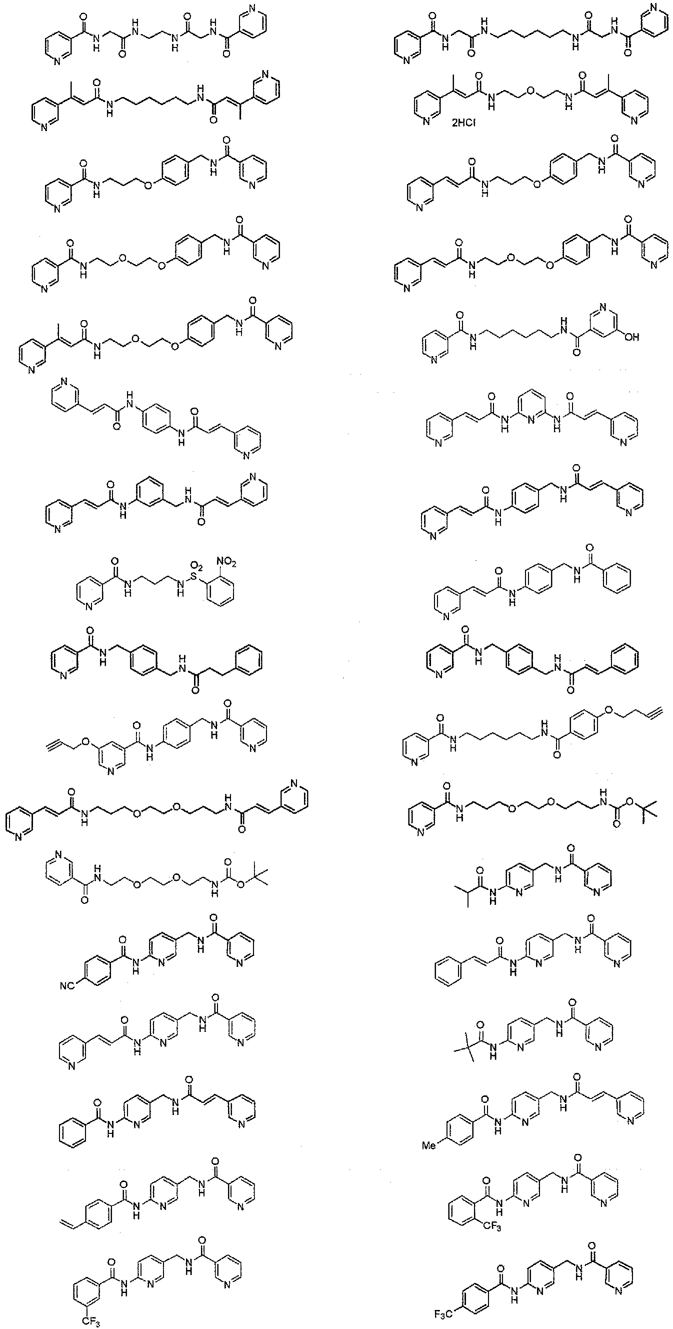

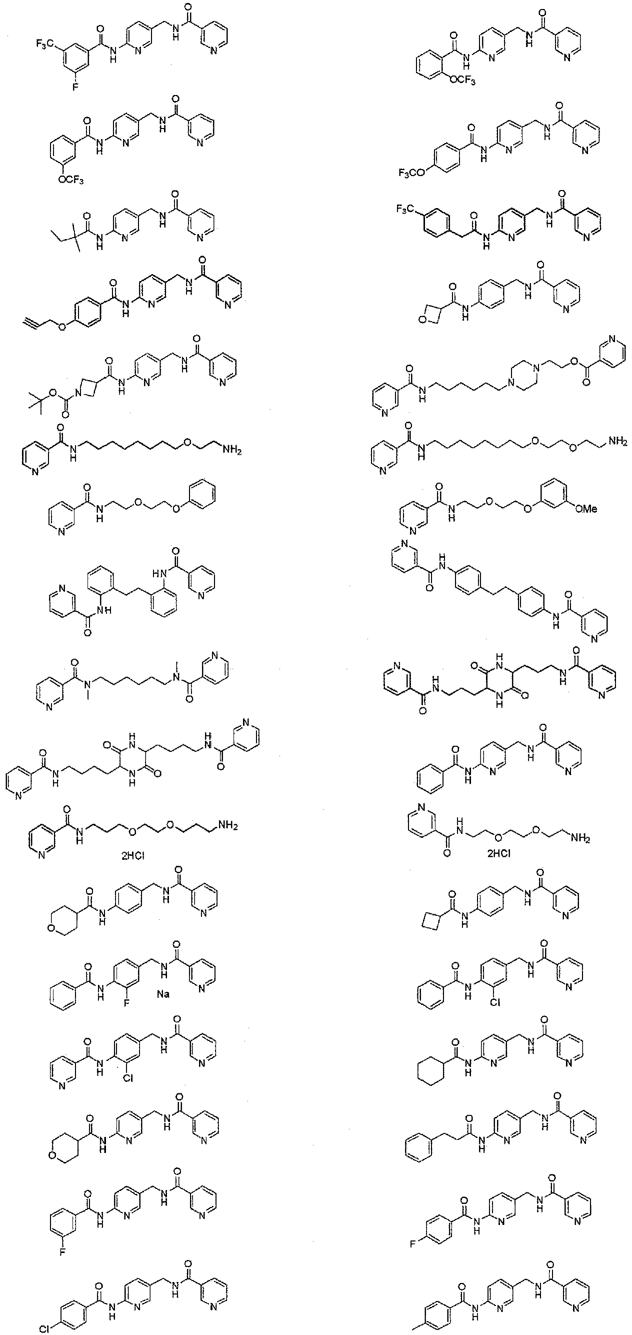

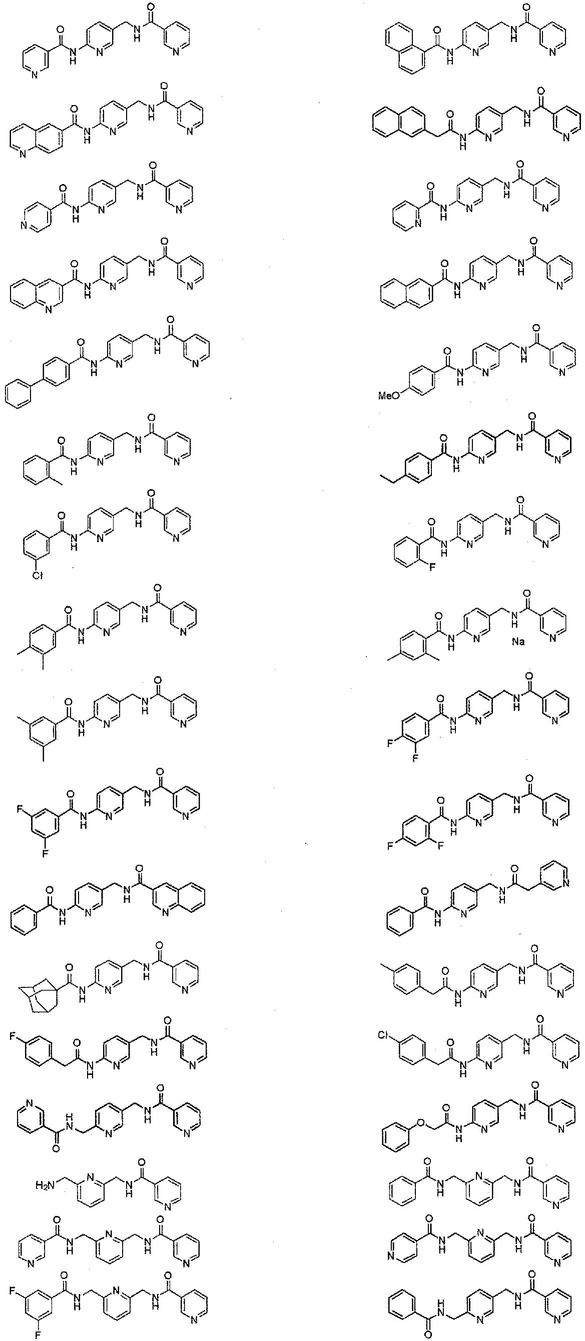

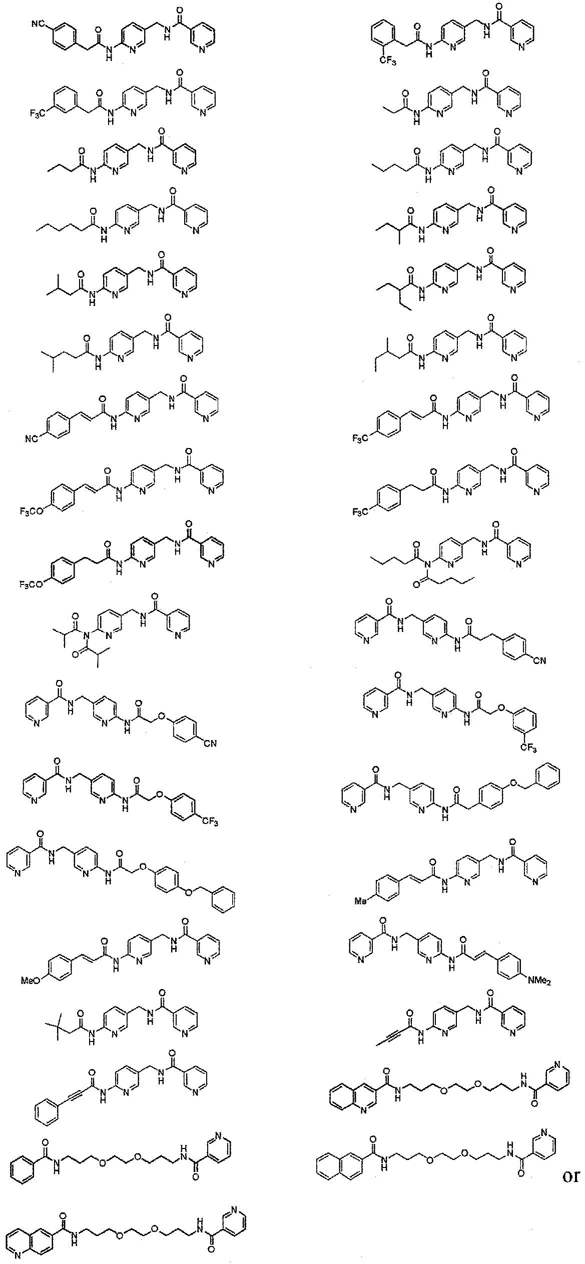

8. A pharmaceutical composition comprising the compound or a pharmaceutically acceptable salt thereof according to claim 1 and at least one pharmaceutically acceptable additive.

9. The pharmaceutical composition according to claim 8 for treatment of a disease subject to prevention, alleviation, and/or therapy through the suppression of axonal degeneration.

10. The pharmaceutical composition according to claim 9, wherein the disease is a neurodegenerative disease.

11. Use of the compound or a pharmaceutically acceptable salt thereof according to claim 1 for producing a medicament for treatment of a disease subject to prevention, alleviation, and/or therapy through the suppression of axonal degeneration.

12. A method for treatment of a disease subject to prevention, alleviation, and/or therapy through the suppression of axonal degeneration, comprising administering a therapeutically effective amount of the compound or a pharmaceutically acceptable salt thereof according to claim 1 to a patient in need thereof.

13. The pharmaceutical composition according to claim 8 for treatment of a disease subject to prevention, alleviation, and/or therapy through the inhibition of SARM1.

14. The pharmaceutical composition according to claim 13, wherein the disease is a neurodegenerative disease.

15. Use of the compound or a pharmaceutically acceptable salt thereof according to claim 1 for producing a medicament for treatment of a disease subject to prevention, alleviation, and/or therapy through the inhibition of SARM1.

16. A method for treatment of a disease subject to prevention, alleviation, and/or therapy through the inhibition of SARM1, comprising administering a therapeutically effective amount of the compound or a pharmaceutically acceptable salt thereof according to claim 1 to a patient in need thereof.
